# EUROPEAN PATENT APPLICATION

(11) **EP 4 219 504 A1**
(43) Date of publication of application: **02.08.2023**
(21) Application number: 21871647.0
(22) Date of filing: 26.09.2021
(51) Int. Cl.: C07D 487/16, A61K 31/52, A61P 31/12, A61P 35/00, A61P 31/16, A61P 31/18, A61P 31/20

(54) **MACROCYCLIC TLR7 AGONIST, PREPARATION METHOD THEREFOR, PHARMACEUTICAL COMPOSITION AND USE THEREOF**

(30) Priority: 27.09.2020 CN 202011035500
(71) Applicant: Shanghai Visonpharma Co., Ltd., Shanghai 201321 (CN)
(72) Inventor: TANG, Guozhi, Shanghai 201321 (CN); MA, Dawei, Shanghai 200030 (CN); HUANG, Mengwei, Shanghai 201321 (CN); LIU, Yongfu, Shanghai 201321 (CN); WANG, Yingyi, Shanghai 201321 (CN)
(74) Representative: Cabinet Laurent & Charras
(86) International application number: PCT/CN2021/120751
(87) International publication number: WO 2022/063278

(57) **Abstract**

A macrocyclic TLR7 agonist, a preparation method therefor, a pharmaceutical composition and use thereof. The macrocyclic TLR7 agonist disclosed is as represented by formula I, has good TLR7 agonistic activity, and can be used for treating or preventing tumors or infections caused by viruses.

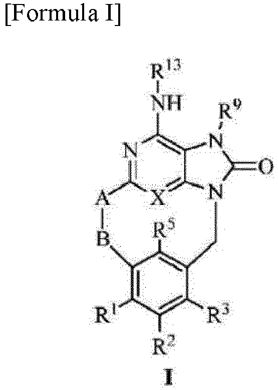

## Description

### TECHNICAL FIELD

The invention relates to a large ring TLR7 agonist, its preparation method, a pharmaceutical composition and its use.

### BACKGROUND TECHNOLOGY

Toll-like receptors (TLRs) are a class of structure-conserved proteins that form the first barrier in innate immune response. By recognizing a variety of conserved pathogen-associated molecular patterns (PAMPs), TLRs can recognize invasive microorganisms and endogenous molecules released after tissue injury or nonphysiological cell death, and activate signaling cascades, which leads to the production of proinflammatory cytokines. Inflammatory processes are essential for the initiation and progression of a variety of diseases, such as type I diabetes, sepsis, cancer, and viral infections. Therefore, strategies to manipulate inflammatory responses through small molecule TLR modulators to treat related diseases are promising.

Human TLR family consists of 10 known members, which are type I transmembrane proteins characterized by a leucine-rich extracellular domain and a cytosolic tail containing a conserved Toll/ interleukin-1 receptor (TIR) domain. In this family, TLR3, TLR7, TLR8 and TLR9 are located in the intracellular endosomes (Vijay K., Int Immunopharmacol., 2018, 59, 391-412).

TLR7 recognizes single-stranded RNA (ssRNA) fragments. TLR7 is mainly expressed in plasmacytoid dendritic cells and B cells. Stimulation of TLR7 in those cells mainly induces the production of type I interferons, including interferon-α (IFN-α), and induces the transcription of interferon-stimulated genes (ISGs) (Gorden KB., J Immunol., 2005, 174, 1259-1268; Shah M., Expert Opin Investig Drugs, 2016, 25, 437-453). IFN-α is one of the major drugs for the treatment of chronic hepatitis B or C. Therefore, the development of TLR7 agonists for the treatment of viral infectious diseases is of great clinical significance.

Studies of treatment of cancer with TLR7 agonists were reported. Treatment of HER2-positive cancers with TLR7 agonist-anti-HER2 conjugates was reported in WO201772662. Yosuke Ota et al. reported that intravenous administration of the TLR7 agonist DSP-0509 and anti-PD-1 antibodies shown synergistical effects in antitumor immune responses (AACR 2018 Proceedings: Abstract 4726).

Currently there are several TLR7 agonist patent applications, but it is still necessary to continuously develop highly active, safer and highly therapeutic TLR7 agonists.

### SUMMARY OF THE INVENTION

In view of the shortcomings of the existing TLR7 agonists which have a relatively homogeneous structure, the technical problem to be solved by the invention is to provide a large ring TLR7 agonist, its preparation method, pharmaceutical composition and its use, the compound structure of the present invention is novel, the activity and selectivity are better.

The present invention provides a compound as shown in Formula I, a solvate thereof, a prodrug thereof, a metabolite thereof, or a pharmaceutically acceptable salt thereof (referring to the compound as shown in Formula I, a solvate thereof, a prodrug thereof, a metabolite thereof): 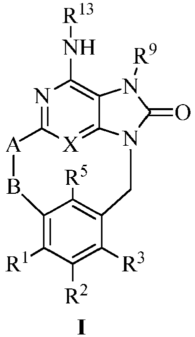
wherein X is N or CH;
A is O, S, S(=O)₂, S(=O)(=NH), NR⁴ or CR⁶R⁷; R⁴, R⁶and R⁷ are independently H or C₁-C₆alkyl;
B is C₂-C₁₀alkylene, C₂-C₁₀unsaturated hydrocarbylene, R¹⁻¹ substituted C₂-C₁₀alkylene, R¹⁻¹ substituted C₂-C₁₀ unsaturated hydrocarbylene, -Z¹-NH-C(=O)-Z²-, -Z³-NH-C(=O)-Z⁴-L¹-, -Z⁵-L²-, -Z⁶-O-Z⁷-, -Z⁸-O-Z⁹-L³-or -(CH₂)ₙ-L⁵-(CH₂)ᵣ-L⁴-;
L¹, L², L³ and L⁴ are independently O, S, S(=O)₂, NR⁸, R⁸ is H or C₁-C₆alkyl;
L⁵ is C₃-C₆ cycloalkylene, halogenated C₃-C₆cycloalkylene, "3-6 membered heterocycloalkylene having 1-3 hetero atoms selected from one or more of N, O and S" or halogenated "3-6 membered heterocycloalkylene having 1-3 hetero atoms selected from one or more of N, O and S"; n and r are independently 1, 2 or 3;
Z¹, Z², Z³, Z⁴, Z⁶, Z⁷, Z⁸ and Z⁹are independently C₁-C₆alkylene, C₁-C₆unsaturated hydrocarbylene, R¹⁻² substituted C₁-C₆alkylene or R¹⁻²substituted C₁-C₆unsaturated hydrocarbylene;
Z⁵ is C₂-C₁₀ alkylene, C₂-C₁₀ unsaturated hydrocarbylene, R¹⁻³ is substituted C₂-C₁₀ alkylene or R¹⁻³ substituted C₂-C₁₀ unsaturated hydrocarbylene;
R¹⁻¹, R¹⁻² and R¹⁻³ are independently OH, CN, NH₂, halogen, C₁-C₆ alkyl, C₁-C₆ alkoxy or COOR¹⁻¹⁻¹; and R¹⁻¹⁻¹ is H or C₁-C₃ alkyl;
R⁵ is H, CN, halogen, C₃-C₅ cycloalkyl, C₁-C₆ alkyl or C₁-C₆ alkoxy;
R⁹ is H, CONR¹⁰R¹¹ or C(=O)R¹², R¹⁰and R¹² are independently C₁-C₆alkyl or C₁-C₆alkoxy substituted C₁-C₆alkyl; R¹¹ is C₁-C₆alkyl or R¹⁻³⁰substituted C₁-C₆alkyl; or R¹⁰ and R¹¹ together with the nitrogen atom to which they are attached form a "5-7-membered heterocycloalkyl having 1-2 hetero atoms, and the heteroatom is N";
R¹³ is H, CONR¹⁴R¹⁵, C(=O)R¹⁶ or COOR¹⁷, and R¹⁴, R¹⁵, R¹⁶ and R¹⁷ are independently C₁-C₆alkyl or R¹³⁻¹ substituted C₁-C₆alkyl; R¹³⁻¹ is CN, halogen, C₁-C₆alkoxy or -(CH₂CH₂O)_{q}-R¹³⁻² , R¹³⁻² is C₁-C₆alkyl, and q is an integer of 0-460;
R¹⁻³⁰ is C₁-C₆alkoxy, -C(=O)-O-R¹⁻³⁰⁻¹, phenyl, NR¹⁻³⁰⁻²R¹⁻³⁰⁻³, or -O-C(=O)-R¹⁻³⁰⁻⁵;
R¹⁻³⁰⁻¹ and R¹⁻³⁰⁻² are each independently C₁-C₆alkyl;
R¹⁻³⁰⁻³ is C₁-C₆alkyl or C(=O)R¹⁻³⁰⁻⁴ , and R¹⁻³⁰⁻⁴ is C₁-C₆alkyl or C₁-C₆alkoxy;
R¹⁻³⁰⁻⁵ is C₁-C₆alkoxy or NR¹⁻³⁰⁻⁶R¹⁻³⁰⁻⁷, R¹⁻³⁰⁻⁶ and R¹⁻³⁰⁻⁷ are independently C₁-C₆alkyl, or R¹⁻³⁰⁻⁶ and R¹⁻³⁰⁻⁷ together with the nitrogen atom to which they are attached form a "5-7 membered heterocycloalkyl having 1-2 heteroatoms, and the heteroatom is N";
the definitions of R¹, R² and R³ are selected from the following Scheme A or Scheme B:
   scheme A: R¹, R² together with the carbon atoms to which they attached form a "4-7 membered heterocycloalkylene having 1-3 heteroatoms selected from one or more of N, O and S", R¹⁻⁴ substituted "4-7 membered heterocycloalkylene having 1-3 heteroatoms selected from one or more of N, O and S", C₄-C₇cycloalkylene or R¹⁻⁵ substituted C₄-C₇cycloalkylene; or, 1 or 2 or more arbitrary methylenes in the "4-7 membered heterocycloalkylene having 1-3 heteroatoms selected from one or more of N, O and S" of the "4-7 membered heterocycloalkylene having 1-3 heteroatoms selected from one or more of N, O and S" or the R¹⁻⁴ substituted "4-7 membered heterocycloalkylene having 1-3 heteroatoms selected from one or more of N, O and S" formed by R¹, R² together with the carbon atoms to which they are attached are independently replaced by carbonyl or S(=O)₂; and R³ is H, halogen, C₁-C₆alkyl or halogenated C₁-C₆alkyl;
   scheme B: R¹ is H, halogen, C₁-C₆alkyl or halogenated C₁-C₆alkyl; R², R³ together with the carbon atoms to which they are attached form a "4-7 membered heterocycloalkylene having 1-3 heteroatoms selected from one or more of N, O and S", R¹⁻⁴ substituted "4-7 membered heterocycloalkylene having 1-3 heteroatoms selected from one or more of N, O and S", C₄-C₇cycloalkylene or R¹⁻⁵-substituted C₄-C₇cycloalkylene; or, 1 or 2 or more arbitrary methylenes in the "4-7 membered heterocycloalkylene having 1-3 heteroatoms selected from one or more of N, O and S" of the "4-7 membered heterocycloalkylene having 1-3 heteroatoms selected from one or more of N, O and S" or the R¹⁻⁴ substituted "4-7 membered heterocycloalkylene having 1-3 heteroatoms selected from one or more of N, O and S" formed by R², R³ together with the carbon atoms to which they are attached are independently replaced by carbonyl or S(=O)₂;

In scheme A or scheme B, R¹⁻⁴ and R¹⁻⁵ are independently OH, halogen, CN, C₁-C₆alkyl, R¹⁻⁶substituted C₁-C₆alkyl, C₁-C₆alkoxy, R¹⁻⁹ substituted C₁-C₆alkoxy, -S(=O)₂R¹⁻⁷, -C(=O)R¹⁻⁸, NR¹⁻¹⁰R¹⁻¹¹, COOR¹⁻¹², SR¹⁻¹³, C₃-C₇cycloalkyl, R¹⁻¹⁹substituted C₃-C₇cycloalkyl, "4-7 membered heterocycloalkyl having 1-3 heteroatoms selected from one or more of N, O and S", R¹⁻²⁰substituted "4-7 membered heterocycloalkyl having 1-3 heteroatoms selected from one or more of N, O and S", "C₁-C₁₀heteroaryl having 1-4 heteroatoms selected from one or more of N, O and S", R¹⁻²¹substituted "C₁-C₁₀ heteroaryl having 1-4 heteroatoms selected from one or more of N, O and S" or -Y(CR¹⁻¹⁴R¹⁻¹⁵)ᵤ-COOR¹⁻¹⁶; Y is O, S, S(=O)₂ or NH; and u is 1, 2 or 3;
R¹⁻⁶ and R¹⁻⁹ are independently halogen, amino, CN, OH, COOR¹⁻¹⁷, S(=O)₂R¹⁻³¹, - C(=O)NH₂, -S(=O)₂NH₂, C₁-C₃alkoxy, C₃-C₇cycloalkyl, COOR¹⁻¹⁸substituted C₃-C₇cycloalkyl, "4-7 membered heterocycloalkyl having 1-3 heteroatoms selected from one or more of N, O and S", R¹⁻²² substituted "4-7 membered heterocycloalkyl having 1-3 heteroatoms selected from one or more of N, O and S", "C₁-C₁₀heteroaryl having 1-4 heteroatoms selected from one or more of N, O and S" or R¹⁻²³ substituted "C₁-C₁₀heteroaryl having 1-4 heteroatoms selected from one or more of N, O and S";
R¹⁻⁷and R¹⁻⁸are independently C₁-C₃alkyl, C₃-C₇cycloalkyl, R¹⁻²⁴substituted C₃-C₇cycloalkyl, "4-7 membered heterocycloalkyl having 1-3 heteroatoms selected from one or more ofN, O and S", R¹⁻²⁵-substituted" 4-7 membered heterocycloalkyl having 1-3 heteroatoms selected from one or more of N, O and S", "C₁-C₁₀heteroaryl having 1-4 heteroatoms selected from one or more of N, O and S" or R¹⁻²⁶ substituted "C₁-C₁₀heteroaryl having 1-4 heteroatoms selected from one or more of N, O and S", C₆-C₁₀aryl, R¹⁻²⁷substituted C₆-C₁₀aryl or NR¹⁻²⁸R¹⁻²⁹;
R¹⁻¹⁰, R¹⁻¹¹, R¹⁻¹², R¹⁻¹⁶, R¹⁻¹⁷, R¹⁻¹⁸, R¹⁻²⁸and R¹⁻²⁹are independently H or C₁-C₃alkyl; R¹⁻³¹is C₁-C₃alkyl;
R¹⁻¹³is H, C₁-C₆alkyl or halogenated C₁-C₆alkyl;
R¹⁻¹⁴ and R¹⁻¹⁵are independently H, C₁-C₆alkyl or halogenated C₁-C₆alkyl; and
R¹⁻¹⁹, R¹⁻²⁰, R¹⁻²¹ , R¹⁻²², R¹⁻²³and R¹⁻²⁴are independently OH, halogen, amino, CN or C₁-C₆alkyl.

In some schemes, in the compound shown in formula I, its solvate, its prodrug, its metabolite, or their pharmaceutically acceptable salt, the definition of some groups may be as follows, and the definition of the remaining groups is as described in any of the above schemes (the content of this paragraph is hereinafter referred to as "in some embodiments"):
when R⁴, R⁶ and R⁷are independently C₁-C₆alkyl, the C₁-C₆alkyl is C₁-C₃alkyl, such as methyl;
In some embodiments, when R⁸is C₁-C₆alkyl, the C₁-C₆alkyl is C₁-C₃alkyl, such as methyl.

In some embodiments, when B is C₂-C₁₀alkylene, the C₂-C₁₀alkylene is C₄-C₆alkylene, preferably-(CH₂)ᵥ-, and v is 4, 5 or 6.

In some embodiments, when B is C₂-C₁₀unsaturated alkylene, the C₂-C₁₀unsaturated alkylene is C₄-C₆unsaturated alkylene, preferably 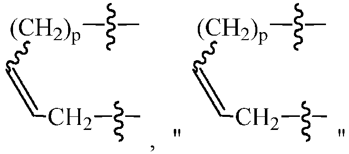 is Z configuration olefin 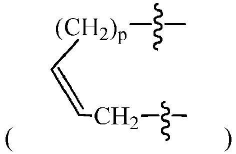 and/or E configuration olefin 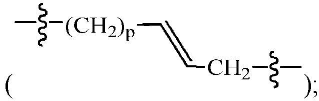 p is 1, 2 or 3.

In some embodiments, when B is C₂-C₁₀alkylene substituted by R¹⁻¹, the C₂-C₁₀alkylene is C₄-C₆alkylene, preferably-(CH₂)ᵥ-, and v is 4, 5 or 6.

In some aspects, when B is R¹⁻¹ substituted C₂-C₁₀alkylene, the number of the R¹⁻¹is one or more, and when the number of the R¹⁻¹ is multiple, the R¹⁻¹ are the same or different; the multiple is 2 or 3.

In some embodiments, when B is R¹⁻¹ substituted C₂-C₁₀unsaturated alkylene, the C₂-C₁₀unsaturated alkylene is C₄-C₆unsaturated alkylene, preferably 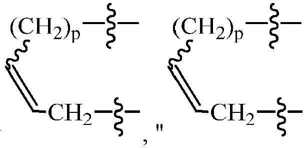 " is Z configuration olefin 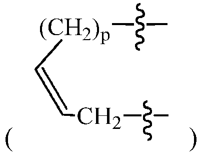 and/or E configuration olefin 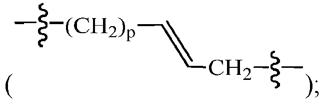 p is 1, 2 or 3.

In some embodiments, when B is R¹⁻¹ substituted C₂-C₁₀unsaturated hydrocarbylene, the number of R¹⁻¹ is one or more, when the number of R¹⁻¹is multiple, each R¹⁻¹ are the same or different, the multiple is 2 or 3.

In some embodiments, when L⁵ is C₃-C₆cycloalkylene, the C₃-C₆cycloalkylene is C₃-C₅cyclopropylene, cyclobutylene, or cyclopentylene (e. g., 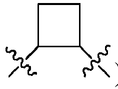 ), or cyclopentylene (e. g., 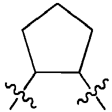 )•

In some embodiments, when L⁵ is a halogenated C₃to C₆cycloalkylene, the C₃to C₆cycloalkylene is a C₃to C₅cycloalkylene, such as cyclopropylene, cyclobutylene (e. g., 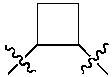 ), or cyclopentylene (e. g., 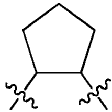 )•

In some embodiments, when L⁵ is a halogenated C₃-C₆cycloalkylene, the number of the halogen is one or multiple, and the multiple can be 2 or 3.

In some embodiments, when L⁵ is "3-6 membered heterocycloalkylene having 1-3 hetero atoms selected from one or more of N, O and S", the heteroatom in the "3-6 membered heterocycloalkylene having 1-3 hetero atoms selected from one or more of N, O and S" is O.

In some embodiments, when L⁵ is "3-6 membered heterocycloalkylene having 1-3 hetero atoms selected from one or more of N, O and S", the number of the heteroatoms in the "3-6 membered heterocycloalkylene having 1-3 hetero atoms selected from one or more of N, O and S" is 1 or 2.

In some embodiments, when L⁵ is halogenated "3-6 membered heterocycloalkylene having 1-3 hetero atoms selected from one or more of N, O and S", the heteroatom in the "3-6 membered heterocycloalkylene having 1-3 hetero atoms selected from one or more of N, O and S" is O.

In some embodiments, when L⁵ is halogenated "3-6 membered heterocycloalkylene having 1-3 hetero atoms selected from one or more of N, O and S", the number of the heteroatom in the "3-6 membered heterocycloalkylene having 1-3 hetero atoms selected from one or more of N, O and S" is 1 or 2.

In some embodiments, when L⁵ is a halogenated "3-6 membered heterocycloalkylene having 1-3 heteroatoms selected from one or more of N, O and S", the number of the halogen is one or multiple, and the multiple can be 2 or 3.
in some embodiments, when Z¹, Z², Z³, Z⁴, Z⁶, Z⁷, Z⁸ and Z⁹ are independently C₁-C₆ alkylene, the C₁-C₆ alkylene is C₁-C₄ alkylene.
in some embodiments, when Z¹, Z², Z³, Z⁴, Z⁶, Z⁷, Z⁸, and Z⁹are independently C₁-C₆unsaturated hydrocarbylene, the C₁-C₆unsaturated hydrocarbylene is C₁-C₄unsaturated hydrocarbylene;
in some embodiments, when Z¹, Z², Z³, Z⁴, Z⁶, Z⁷, Z⁸ and Z⁹are independently R¹⁻²substituted C₁-C₆ alkylene, the C₁-C₆alkylene is C₁-C₄alkylene.

In some embodiments, when Z¹, Z², Z³, Z⁴, Z⁶, Z⁷, Z⁸and Z⁹are independently R¹⁻²substituted C₁-C₆alkylene, the number of R¹⁻²is one or more, and when the number of R¹⁻² is multiple, the R¹⁻² are the same or different, and the multiple can be 2 or 3.

In some embodiments, when Z¹, Z², Z³, Z⁴, Z⁶, Z⁷, Z⁸, and Z⁹are independentlyR¹⁻²substituted C₁-C₆unsaturated hydrocarbylene, the C₁-C₆ unsaturated hydrocarbylene is C₁-C₄unsaturated hydrocarbylene.

In some embodiments, when Z¹, Z², Z³, Z⁴, Z⁶, Z⁷, Z⁸and Z⁹are independentlyR¹⁻²substituted C₁-C₆unsaturated hydrocarbylene, the number of R¹⁻²is one or more, when the number of R¹⁻²is multiple, the R¹⁻²are the same or different; the multiple can be 2 or 3.

In some embodiments, when Z⁵ is C₂-C₁₀ alkylene, the C₂-C₁₀ alkylene is C₃-C₅alkylene, preferably-(CH₂)_{w}-; w is 3, 4 or 5.

In some embodiments, when Z⁵ is C₂-C₁₀unsaturated alkylene, the C₂-C₁₀unsaturated alkylene is C₃-C₅unsaturated alkylene, preferably 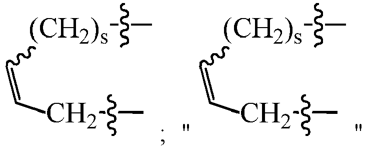 is Z configuration olefin 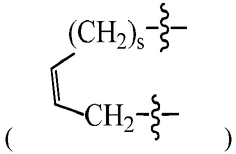 and/or E configuration olefin 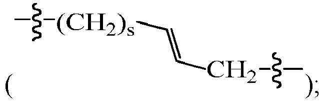 s is 1 or 2.

In some embodiments, when Z⁵ is R¹⁻³substituted C₂-C₁₀alkylene, the C₂-C₁₀alkylene is C₃-C₅alkylene, preferably-(CH₂)_{w}-; w is 3, 4 or 5.

In some embodiments, when Z⁵ is R¹⁻³substituted C₂-C₁₀alkylene, the number of the R¹⁻³ is one or more, when the number of the R¹⁻³ is multiple, the R¹⁻³are the same or different; the multiple can be 2 or 3.

In some embodiments, when Z⁵ is R¹⁻³substituted C₂-C₁₀unsaturated alkylene, the C₂-C₁₀unsaturated alkylene is C₃-C₅unsaturated alkylene, preferably 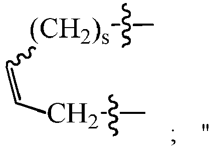 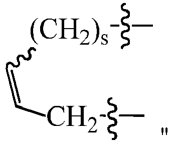 is Z configuration olefin 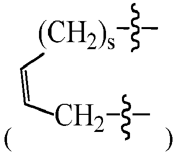 and/or E configuration olefin 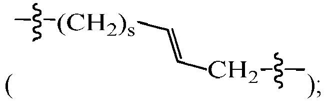 s is 1 or 2.

In some embodiments, when Z⁵ is R¹⁻³substituted C₂-C₁₀unsaturated hydrocarbylene, the number of R¹⁻³ is one or more, when the number of R¹⁻³is multiple, each R¹⁻³ are the same or different, the multiple can be 2 or 3.

In some embodiments, when R¹⁻¹, R¹⁻², and R¹⁻³are independently halogen, the halogen is fluorine, chlorine, bromine or iodine.

In some embodiments, when R¹⁻¹, R¹⁻², and R¹⁻³ are independently C₁-C₆ alkyl, the C₁-C₆ alkyl is C₁-C₃alkyl, such as methyl, ethyl, n-propyl or isopropyl.

In some embodiments, when R¹⁻¹, R¹⁻², and R¹⁻³are independently C₁-C₆alkoxy, the C₁-C₆alkoxy is C₁-C₃ alkoxy, such as methoxy, ethoxy, n-propoxy or isopropoxy.

In some embodiments, when R¹⁻¹⁻¹ is C₁-C₃alkyl, the C₁-C₃ alkyl is methyl, ethyl, n-propyl or isopropyl.

In some embodiments, when R⁵ is halogen, the halogen is fluorine, chlorine, bromine or iodine.

In some embodiments, when R⁵ is C₁-C₆alkyl, the C₁-C₆ alkyl is C₁-C₃ alkyl, such as methyl, ethyl, n-propyl or isopropyl.

In some embodiments, when R¹⁰and R¹²are independently C₁-C₆alkyl, the C₁-C₆alkyl is C₁-C₃alkyl, such as methyl, ethyl, n-propyl or isopropyl.

In some embodiments, when R¹⁰and R¹² are independently C₁-C₆ alkoxy-substituted C₁-C₆alkyl, the C₁-C₆alkyl is C₁-C₃alkyl, such as methyl, ethyl, n-propyl or isopropyl.

In some embodiments, when R¹⁰and R¹² are independently C₁-C₆alkoxy substituted C₁-C₆alkyl, the C₁-C₆alkoxy are C₁-C₃alkoxy, such as methoxy, ethoxy, n-propoxy or isopropoxy groups.

In some embodiments, when R¹¹ is C₁-C₆alkyl, the C₁-C₆ alkyl is C₁-C₃ alkyl, such as methyl, ethyl, n-propyl or isopropyl.

In some embodiments, when R¹¹is R¹⁻³⁰substituted C₁-C₆alkyl, the C₁-C₆alkyl is C₁-C₃alkyl, such as methyl, ethyl, n-propyl or isopropyl.

In some embodiments, when R¹¹ is R¹⁻³⁰substituted C₁-C₆ alkyl, the number of the R¹⁻³⁰ is one or more, when the number of the R¹⁻³⁰ is multiple, the R¹⁻³⁰are the same or different; the multiple can be 2 or 3.

In some embodiments, when R¹⁻³⁰ is C₁-C₆alkoxy, the C₁-C₆alkoxy is C₁-C₃alkoxy, such as methoxy, ethoxy, n-propoxy or isopropoxy.

In some embodiments, when R¹⁰ and R¹¹ together with the nitrogen atom to which they are attached form a "5-7 membered heterocycloalkyl having 1-2 heteroatoms, and the heteroatom is N", the " 5-7 membered heterocycloalkyl having 1-2 heteroatoms, and the heteroatom is N" is pyrrolidyl or piperidinyl;

In some embodiments, when R¹⁴, R¹⁵, R¹⁶, and R¹⁷are independently C₁-C₆alkyl, the C₁-C₆ alkyl are C₁-C₄ alkyl, such as methyl, ethyl, n-propyl, isopropyl, n-butyl, isobutyl, sec-butyl or t-butyl.

In some embodiments, when R¹⁴, R¹⁵, R¹⁶, and R¹⁷are independently R¹³⁻¹ substituted C₁-C₆alkyl, the C₁-C₆alkyl is C₁-C₃alkyl, such as methyl, ethyl, n-propyl or isopropyl.

In some embodiments, when R¹⁴, R¹⁵, R¹⁶, and R¹⁷are independently R¹³⁻¹substituted C₁-C₆alkyl, the number of R¹³⁻¹is one or more, and when the number of R¹³⁻¹is multiple, the R¹³⁻¹ are the same or different; the multiple can be 2 or 3.

In some embodiments, when R¹³⁻¹ is C₁-C₆alkoxy, the C₁-C₆alkoxy is C₁-C₃alkoxy, such as methoxy, ethoxy, n-propoxy or isopropoxy.

In some embodiments, R¹³⁻² is C₁-C₃ alkyl, such as methyl, ethyl, n-propyl or isopropyl.

In some embodiments, when R^{1-30 -1}and R^{1 -30-2} are independently C₁-C₆alkyl, the C₁-C₆alkyl is C₁-C₃alkyl, such as methyl, ethyl, n-propyl or isopropyl.

In some embodiments, when R¹⁻³⁰⁻³ is C₁-C₆alkyl, the C₁-C₆ alkyl is C₁-C₃ alkyl, such as methyl, ethyl, n-propyl or isopropyl.

In some embodiments, when R¹⁻³⁰⁻⁴ is C₁-C₆alkyl, the C₁-C₆ alkyl is C₁-C₃ alkyl, such as methyl, ethyl, n-propyl or isopropyl.

In some embodiments, when R¹⁻³⁰⁻⁴ is C₁-C₆ alkoxy, the C₁-C₆alkoxy is C₁-C₃alkoxy, such as methoxy, ethoxy, n-propoxy or isopropoxy.

In some embodiments, when R¹⁻³⁰⁻⁵ is C₁-C₆ alkoxy, the C₁-C₆alkoxy is C₁-C₃alkoxy, such as methoxy, ethoxy, n-propoxy or isopropoxy.

In some embodiments, when R^{1-30 -6}and R^{1 -30-7} are independently C₁-C₆alkyl, the C₁-C₆alkyl is C₁-C₃alkyl, such as methyl, ethyl, n-propyl or isopropyl.

In some embodiments, when R^{1-30 -6} and R^{1 -30-7} together with the nitrogen atom to which they are attached form a "5-7 membered heterocycloalkyl having 1-2 heteroatoms, and the heteroatom is N", the "5-7 membered heterocycloalkyl having 1-2 heteroatoms, and the heteroatom is N" is pyrrolidyl or piperidinyl.

In some embodiments, when R⁵ is C₁-C₆alkoxy, the C₁-C₆alkoxy is C₁-C₃alkoxy, such as methoxy, ethoxy, n-propoxy or isopropoxy.

In some embodiments, when R¹, R² together with the carbon atoms to which they are attached form a "4-7 membered heterocycloalkylene having 1-3 heteroatoms selected from one or more of N, O and S", the heteroatoms in the "4-7 membered heterocycloalkylene having 1-3 heteroatoms selected from one or more of N, O and S" are N and/or O.

In some embodiments, when R¹, R² together with the carbon atoms to which they are attached form a "4-7 membered heterocycloalkylene having 1-3 heteroatoms selected from one or more of N, O and S", the number of heteroatoms in the "4-7 membered heterocycloalkylene having 1-3 heteroatoms selected from one or more of N, O and S" is 1 or 2.

In some embodiments, when R¹, R² together with the carbon atoms to which they are attached form a "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is 5-6 membered heterocycloalkylene.

In some embodiments, when R¹, R² together with the carbon atoms to which they are attached form a "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene group is replaced by a carbonyl group.

In some embodiments, when the R¹, R² together with the carbon atoms to which they are attached form a "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² is

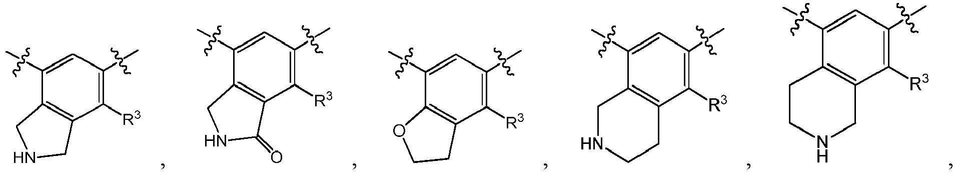

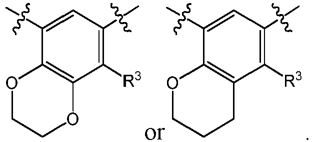

In some embodiments, when R¹, R² together with the carbon atoms to which they are attached form a R¹⁻⁴substituted "4-7 membered heterocycloalkylene having 1-3 heteroatoms selected from one or more of N, O and S", the heteroatoms in the "4-7 membered heterocycloalkylene having 1-3 heteroatoms selected from one or more of N, O and S" are N and/or O.

In some embodiments, when R¹, R² together with the carbon atoms to which they are attached form R¹⁻⁴substituted "heteroatoms are selected from one or more of N, O and S, and the number of heteroatoms is 1-3 4-7 membered heterocycloalkyls", the "heteroatoms are selected from one or more of N, O and S, 4-7 membered heterocycloalkyl with 1-3 heteroatoms", the number of heteroatoms is 1 or 2.

In some embodiments, when R¹, R² together with the carbon atoms to which they are attached form R¹⁻⁴substituted "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is 5-6 membered heterocycloalkylene.

In some embodiments, when R¹, R² together with the carbon atoms to which they are attached form a R¹⁻⁴substituted "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene is replaced by a carbonyl group.

In some embodiments, when R¹, R² together with the carbon atoms to which they are attached form a R¹⁻⁴substituted "4-7 membered heterocycloalkylene having 1-3 heteroatoms selected from one or more of N, O and S", the number of R¹⁻⁴is one or more, when the number of R¹⁻⁴is multiple, the R¹⁻⁴ are the same or different; the multiple can be 2 or 3.

In some embodiments, when R¹, R² together with the carbon atoms to which they are attached form a R¹⁻⁴substituted "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² is

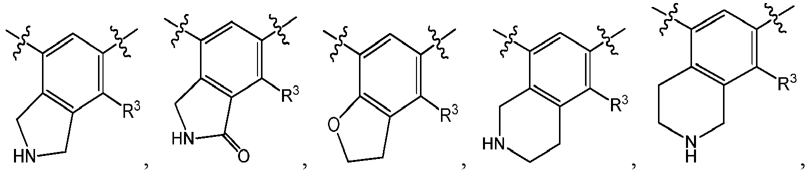

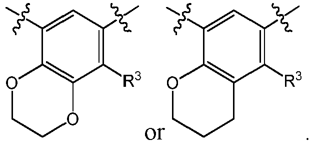

In some embodiments, when R¹, R² together with the carbon atoms to which they are attached form a R¹⁻⁴ substituted "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² form 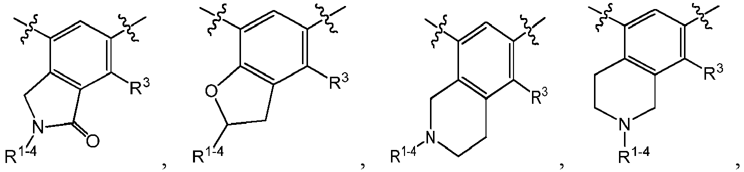 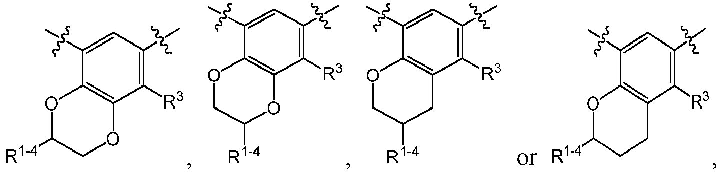 and the number of R¹⁻⁴ is one or more, when the number of R¹⁻⁴ is multiple, each R¹⁻⁴ are the same or different.

In some embodiments, when R¹, R²and the carbon atom to which they are attached together form a C₄-C₇, the C₄-C₇cycloalkylene is C₅-C₆ cycloalkylene , such as a cyclopentylene (e. g., 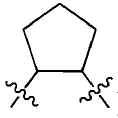 ) or a cyclohexylene (e. g., 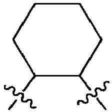 )•

In some embodiments, when R¹, R² and the carbon atom to which they are attached together form a R¹⁻⁵substituted C₄-C₇cycloalkylene, the C₄-C₇cycloalkylene is C₅-C₆ cycloalkylene, such as cyclopentylene (e. g., 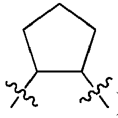 ) or cyclohexylene (e. g., 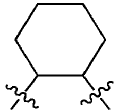 ).

In some embodiments, when R¹, R² together with the carbon atoms to which they are attached form a R¹⁻⁵-substituted C₄-C₇cycloalkylene, the number of R¹⁻⁵is one or more, and when the number of R¹⁻⁵is multiple, the R¹⁻⁵ are the same or different; the multiple can be 2 or 3.

In some embodiments, when R¹, R² and their attached carbon atoms together form R¹⁻⁵ substituted C₄-C₇ cycloalkylene, the R¹⁻⁵ substituted C₄-C₇ cycloalkylene together with the benzene ring bonded to R¹ and R² form 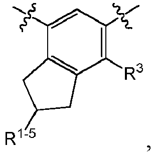 and the number of R¹⁻⁵ is 1 or 2, when the number of R¹⁻⁵ is 2, the R¹⁻⁵ are the same or different.

In some embodiments, in scheme A, when R³ is halogen, the halogen is fluorine, chlorine, bromine or iodine.

In some embodiments, in scheme A, when R³ is C₁-C₆ alkyl, the C₁-C₆ alkyl is C₁-C₃ alkyl, suchas methyl, ethyl, n-propyl or isopropyl.

In some embodiments, in scheme A, when R³ is a halogenated C₁-C₆alkyl, the C₁-C₆ alkyl is a C₁-C₃ alkyl, such as methyl, ethyl, n-propyl or isopropyl.

In some embodiments, in scheme A, when R³is a halogenated C₁-C₆alkyl, the halogen is fluorine, chlorine, bromine or iodine.

In some embodiments, in scheme A, when R³ is a halogenated C₁-C₆alkyl, the number of the halogens is one or multiple, and the multiple can be 2 or 3.

In some embodiments, when R², R³ together with the carbon atoms to which they are attached form a "4-7 membered heterocycloalkylene having 1-3 heteroatoms selected from one or more of N, O and S", the heteroatoms in the "4-7 membered heterocycloalkylene having 1-3 heteroatoms selected from one or more of N, O and S" are N and/or O.

In some embodiments, when R², R³ together with the carbon atoms to which they are attached form a "4-7 membered heterocycloalkylene having 1-3 heteroatoms selected from one or more of N, O and S", the number of heteroatoms in the "4-7 membered heterocycloalkylene having 1-3 heteroatoms selected from one or more of N, O and S" is 1 or 2.

In some embodiments, when R², R³ together with the carbon atoms to which they are attached form a "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is 5-6 membered heterocycloalkylene.

In some embodiments, when R², R³ together with the carbon atoms to which they are attached form a "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene group is replaced by a carbonyl group.

In some embodiments, when the R², R³ together with the carbon atoms to which they are attached form a "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R² and R³ from

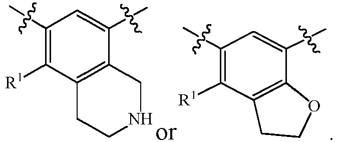

In some embodiments, when R², R³ together with the carbon atoms to which they are attached form a R¹⁻⁴ substituted "4-7 membered heterocycloalkylene having 1-3 heteroatoms selected from one or more of N, O and S", the heteroatoms in the "4-7 membered heterocycloalkylene having 1-3 heteroatoms selected from one or more of N, O and S" are N and/or O.

In some embodiments, when R², R³ together with the carbon atoms to which they are attached form R¹⁻⁴substituted "4-7 membered heterocycloalkylene having 1-3 heteroatoms selected from one or more of N, O and S", the number of heteroatom in "4-7 membered heterocycloalkylene having 1-3 heteroatoms selected from one or more of N, O and S" is 1 or 2.

In some embodiments, when R², R³ together with the carbon atoms to which they are attached form R¹⁻⁴substituted "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is 5-6 membered heterocycloalkylene.

In some embodiments, when R², R³ together with the carbon atoms to which they are attached form a R¹⁻⁴ substituted "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene is replaced by a carbonyl group.

In some embodiments, when R², R³ together with the carbon atoms to which they are attached form a R¹⁻⁴ substituted "4-7 membered heterocycloalkylene having 1-3 heteroatoms selected from one or more of N, O and S", the number of R¹⁻⁴ is one or more, when the number of R¹⁻⁴ is multiple, the R¹⁻⁴ are the same or different; the multiple can be 2 or 3.

In some embodiments, when R², R³ together with the carbon atoms to which they are attached form a R¹⁻⁴substituted "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² from 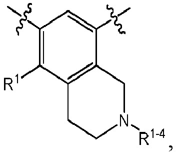 and the number of R¹⁻⁴ is one or more, when the number of R¹⁻⁴is multiple, the R¹⁻⁴ are the same or different.

In some embodiments, when R², R³and the carbon atom to which they are attached together form a C₄-C₇ cycloalkylene, the C₄-C₇ cycloalkylene is C₅-C₆ cycloalkylene , such as a cyclopentylene or a cyclohexylene.

In some embodiments, when R², R³ and the carbon atom to which they are attached together form a R¹⁻⁵substituted C₄-C₇cycloalkylene, the C₄-C₇cycloalkylene is C₅-C₆ cycloalkylene, such as cyclopentylene (e. g., 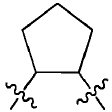 ) or cyclohexylene (e. g., 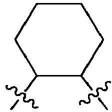 ).

In some embodiments, when R², R³ together with the carbon atoms to which they are attached form R¹⁻⁵ substituted C₄-C₇cycloalkylene, the number of R¹⁻⁵ is one or more, and when the number of R¹⁻⁵ is multiple, the R¹⁻⁵ are the same or different; the multiple can be 2 or 3.

In some embodiments, in scheme B, when R¹ is halogen, the halogen is fluorine, chlorine, bromine or iodine.

In some embodiments, in scheme B, when R¹ is C₁-C₆alkyl, the C₁-C₆ alkyl is C₁-C₃ alkyl, such as methyl, ethyl, n-propyl or isopropyl.

In some embodiments, in scheme B, when R¹ is a halogenated C₁-C₆alkyl, the C₁-C₆ alkyl is a C₁-C₃ alkyl, such as methyl, ethyl, n-propyl or isopropyl.

In some embodiments, in scheme B, when R¹ is a halogenated C₁-C₆alkyl, the halogen is fluorine, chlorine, bromine or iodine.

In some embodiments, in scheme B, when R¹ is a halogenated C₁-C₆alkyl, the number of the halogens is one or multiple, and the multiple can be 2 or 3.

In some embodiments, when R¹⁻⁴ and R¹⁻⁵ are independently halogen, the halogen is fluorine, chlorine, bromine or iodine.

In some embodiments, when R¹⁻⁴and R¹⁻⁵ are independently C₁-C₆alkyl, the C₁-C₆alkyl is C₁-C₄alkyl, such as methyl, ethyl, isopropyl or tertiary butyl.

In some embodiments, when R¹⁻⁴and R¹⁻⁵are independently R¹⁻⁶ substituted C₁-C₆alkyl, the C₁-C₆alkyl is a C₁-C₄alkyl, such as methyl, ethyl, isopropyl or isobutyl.

In some embodiments, when R¹⁻⁴ and R¹⁻⁵ are independently R¹⁻⁶ substituted C₁-C₆alkyl, the number of R¹⁻⁶ is one or more, and when the number of R¹⁻⁶ is multiple, the R¹⁻⁶ are the same or different; the multiple can be 2 or 3.

In some embodiments, when R¹⁻⁴ and R¹⁻⁵ are independently C₁-C₆alkoxy, the C₁-C₆alkoxy is C₁-C₃ alkoxy, such as methoxy, ethoxy, n-propoxy or isopropoxy.

In some embodiments, when R¹⁻⁴and R¹⁻⁵are independently R¹⁻⁹ substituted C₁-C₆alkoxy, the C₁-C₆ alkoxy is C₁-C₃ alkoxy, such as methoxy, ethoxy, n-propoxy or isopropoxy.

In some embodiments, when R¹⁻⁴ and R¹⁻⁵ are independently R¹⁻⁹ substituted C₁-C₆alkoxy, the number of R¹⁻⁹ is one or more, and when the number of R¹⁻⁹ is multiple, the R¹⁻⁹ are the same or different; the multiple can be 2 or 3.

In some embodiments, when R¹⁻⁴and R¹⁻⁵are independently C₃-C₇cycloalkyl, the C₃-C₇cycloalkyl is C₃-C₅cycloalkyl, such as cyclopropyl or cyclobutyl.

In some embodiments, when R¹⁻⁴and R¹⁻⁵are independently R¹⁻¹⁹ substituted C₃-C₇cycloalkyl, the C₃-C₇cycloalkyl is a C₃-C₅cycloalkyl, such as cyclopropyl or cyclobutyl.

In some embodiments, when R¹⁻⁴and R¹⁻⁵are independently R¹⁻¹⁹ substituted C₃-C₇cycloalkyl, the number of R¹⁻¹⁹ is one or more, when the number of R¹⁻¹⁹ is multiple, each R¹⁻⁶ are the same or different; the multiple can be 2 or 3.

In some embodiments, when R¹⁻⁴ and R¹⁻⁵ are independently "4-7 membered heterocycloalkyl having 1-3 heteroatoms selected from one or more of N, O and S", the heteroatom in the "4-7 membered heterocycloalkyl having 1-3 heteroatoms selected from one or more of N, O and S" is N or O.

In some embodiments, when R¹⁻⁴ and R¹⁻⁵ are independently "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1.

In some embodiments, when R¹⁻⁴ and R¹⁻⁵ are independently "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl.

In some embodiments, when R¹⁻⁴ and R¹⁻⁵ are independently "4-7 membered heterocycloalkyl having 1-3 heteroatoms selected from one or more of N, O and S", the "4-7 membered heterocycloalkyl having 1-3 heteroatoms selected from one or more of N, O and S" is piperidinyl (e. g., 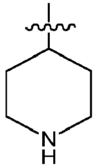 ) or tetrahydropyranyl (e. g., 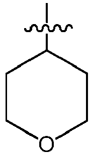 ).

In some embodiments, when R¹⁻⁴ and R¹⁻⁵ are independently R¹⁻²⁰ substituted "4-7 membered heterocycloalkyl having 1-3 heteroatoms selected from one or more of N, O and S", the heteroatoms in the "4-7 membered heterocycloalkyl having 1-3 heteroatoms selected from one or more of N, O and S" is N or O.

In some embodiments, when R¹⁻⁴ and R¹⁻⁵are independently R¹⁻²⁰ substituted "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1.

In some embodiments, when R¹⁻⁴and R¹⁻⁵are independently R¹⁻²⁰ substituted "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl.

In some embodiments, when R¹⁻⁴and R¹⁻⁵are independently R¹⁻²⁰ substituted "4-7 membered heterocycloalkyl having 1-3 heteroatoms selected from one or more of N, O and S", the "4-7 membered heterocycloalkyl having 1-3 heteroatoms selected from one or more of N, O and S" is piperidinyl (e. g., 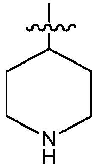 ) or tetrahydropyran (e. g., 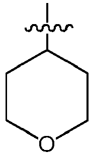 ).

In some embodiments, when R¹⁻⁴ and R¹⁻⁵ are independently R¹⁻²⁰ substituted "4-7 membered heterocycloalkyl having 1-3 heteroatoms selected from one or more of N, O and S", the number of R¹⁻²⁰ is one or more, when the number of R¹⁻²⁰ is multiple, the R¹⁻²⁰ are the same or different; the multiple can be 2 or 3.

In some embodiments, when R¹⁻⁴and R¹⁻⁵are independently "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" is N and/or O.

In some embodiments, when R¹⁻⁴and R¹⁻⁵are independently "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1 or 2.

In some embodiments, when R¹⁻⁴and R¹⁻⁵are independently "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl.

In some embodiments, when R¹⁻⁴ and R¹⁻⁵ are independently "C₁-C₁₀heteroaryl having 1-4 heteroatoms selected from one or more of N, O and S", the "C₁-C₁₀heteroaryl having 1-4 heteroatoms selected from one or more of N, O and S" is pyrimidinyl (for example, 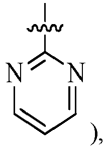 pyridyl (for example, 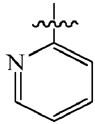 ) or oxazolyl (for example, 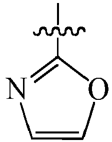 ).

In some embodiments, when R¹⁻⁴and R¹⁻⁵are independently R¹⁻²¹ substituted "C₁-C₁₀heteroaryl having 1-4 heteroatoms selected from one or more of N, O and S", the heteroatom in "C₁-C₁₀heteroaryl having 1-4 heteroatoms selected from one or more of N, O and S" is N and/or O.

In some embodiments, when R¹⁻⁴and R¹⁻⁵are independently R¹⁻²¹ substituted "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1 or 2.

In some embodiments, when R¹⁻⁴and R¹⁻⁵are independently R¹⁻²¹ substituted "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl.

In some embodiments, when R¹⁻⁴ and R¹⁻⁵ are independently R¹⁻²¹ substituted "C₁-C₁₀heteroaryl having 1-4 heteroatoms selected from one or more of N, O and S", the "C₁-C₁₀heteroaryl having 1-4 heteroatoms selected from one or more of N, O and S" is pyrimidinyl (for example, 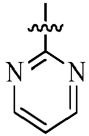 ), pyridyl (for example, 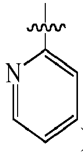 ) or oxazolyl (for example, 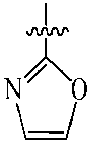 ).

In some embodiments, when R¹⁻⁴and R¹⁻⁵are independently R¹⁻²¹ substituted "C₁-C₁₀heteroaryl having 1-4 heteroatoms selected from one or more of N, O and S", the number of R¹⁻²¹ is one or more, when the number of R¹⁻²¹ is multiple, each R¹⁻²¹ are the same or different; the multiple can be 2 or 3.

In some embodiments, when R¹⁻⁶ and R¹⁻⁹ are independently halogen, the halogen is fluorine, chlorine, bromine or iodine.

In some embodiments, when R¹⁻⁶and R¹⁻⁹are independently C₁-C₃alkoxy, the C₁-C₃alkoxy is methoxy, ethoxy, n-propoxy or isopropoxy, such as methoxy.

In some embodiments, when R¹⁻⁶and R¹⁻⁹are independently C₃-C₇cycloalkyl, the C₃-C₇cycloalkyl is C₃-C₅cycloalkyl, such as cyclopropyl or cyclobutyl.

In some embodiments, when R¹⁻⁶and R¹⁻⁹are independently COOR¹⁻¹⁸ substituted C₃-C₇cycloalkyl, the C₃-C₇cycloalkyl is a C₃-C₅cycloalkyl, such as cyclopropyl or cyclobutyl.

In some embodiments, when R¹⁻⁶and R¹⁻⁹are independently a COOR¹⁻¹⁸ substituted C₃₋C₇cycloalkyl, the number of the COOR¹⁻¹⁸ is one or more, and when the number of the COOR¹⁻¹⁸ is multiple, the COOR¹⁻¹⁸ are the same or different; the multiple can be 2 or 3.

In some embodiments, when R¹⁻⁶and R¹⁻⁹are independently "4-7 membered heterocycloalkylene having 1-3 heteroatoms selected from one or more of N, O and S", the heteroatom is N.

In some embodiments, when R¹⁻⁶ and R¹⁻⁹ are independently "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1.

In some embodiments, when R¹⁻⁶ and R¹⁻⁹ are independently "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl.

In some embodiments, when R¹⁻⁶ and R¹⁻⁹ are independently "4-7 membered heterocycloalkyl having 1-3 heteroatoms selected from one or more of N, O and S", the "4-7 membered heterocycloalkyl having 1-3 heteroatoms selected from one or more of N, O and S" is tetrahydropyrrolyl (e. g., 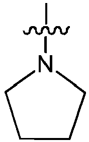 ).

In some embodiments, when R¹⁻⁶ and R¹⁻⁹are independently R¹⁻²² substituted "4-7 membered heterocycloalkyl having 1-3 heteroatoms selected from one or more of N, O and S", the heteroatom in the "4-7 membered heterocycloalkyl having 1-3 heteroatoms selected from one or more of N, O and S" is N.

In some embodiments, when R¹⁻⁶ and R¹⁻⁹are independently R¹⁻²² substituted "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1.

In some embodiments, when R¹⁻⁶and R¹⁻⁹are independently R¹⁻²² substituted "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl.

In some embodiments, when R¹⁻⁶ and R¹⁻⁹ are independently R¹⁻²² substituted "4-7 membered heterocycloalkyl having 1-3 heteroatoms selected from one or more of N, O and S", the "4-7 membered heterocycloalkyl having 1-3 heteroatoms selected from one or more of N, O and S" is tetrahydropyrrolyl (e. g., 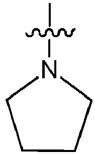 ).

In some embodiments, when R¹⁻⁶ and R¹⁻⁹ are independently R¹⁻²² substituted "4-7 membered heterocycloalkyl having 1-3 heteroatoms selected from one or more of N, O and S", the number of R¹⁻²² is one or more, when the number of R¹⁻²² is multiple, the R¹⁻²² are the same or different; the multiple can be 2 or 3.

In some embodiments, when R¹⁻⁶and R¹⁻⁹are independently "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" is N.

In some embodiments, when R¹⁻⁶and R¹⁻⁹are independently "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1.

In some embodiments, when R¹⁻⁶and R¹⁻⁹are independently "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl.

In some embodiments, when R¹⁻⁶ and R¹⁻⁹ are independently "C₁-C₁₀heteroaryl having 1-4 heteroatoms selected from one or more of N, O and S", the "C₁-C₁₀heteroaryl having 1-4 heteroatoms selected from one or more of N, O and S" is pyridinyl (such as 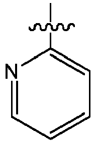 or 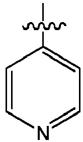 ).

In some embodiments, when R¹⁻⁶and R¹⁻⁹are independently R¹⁻²³ substituted "C₁-C₁₀heteroaryl having 1-4 heteroatoms selected from one or more of N, O and S", the heteroatom in "C₁-C₁₀heteroaryl having 1-4 heteroatoms selected from one or more of N, O and S" is N.

In some embodiments, when R¹⁻⁶and R¹⁻⁹are independently R¹⁻²³ substituted "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1.

In some embodiments, when R¹⁻⁶and R¹⁻⁹are independently R¹⁻²³ substituted "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl.

In some embodiments, when R¹⁻⁶ and R¹⁻⁹ are independently R¹⁻²³ substituted "C₁-C₁₀heteroaryl having 1-4 heteroatoms selected from one or more of N, O and S", the "C₁-C₁₀heteroaryl having 1-4 heteroatoms selected from one or more of N, O and S" is pyridinyl (such as 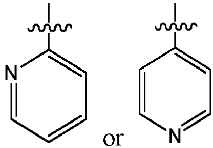 )•

In some embodiments, when R¹⁻⁶and R¹⁻⁹are independently R¹⁻²³ substituted "C₁-C₁₀heteroaryl having 1-4 heteroatoms selected from one or more of N, O and S", the number of R¹⁻²³ is one or more, when the number of R¹⁻²³ is multiple, each R¹⁻²³ are the same or different; the multiple can be 2 or 3.

In some embodiments, when R¹⁻⁷and R¹⁻⁸are independently C₁-C₃alkyl, the C₁-C₃ alkyl is methyl, ethyl, n-propyl or isopropyl, for example methyl.

In some embodiments, when R¹⁻⁷and R¹⁻⁸are independently C₃-C₇cycloalkyl, the C₃-C₇cycloalkyl is C₃-C₅cycloalkyl, such as cyclopropyl or cyclobutyl.

In some embodiments, when R¹⁻⁷and R¹⁻⁸are independently R¹⁻²⁴ substituted C₃-C₇cycloalkyl, the C₃-C₇cycloalkyl is a C₃-C₅cycloalkyl, such as cyclopropyl or cyclobutyl.

In some embodiments, when R¹⁻⁷and R¹⁻⁸are independently R¹⁻²⁴ substituted C₃-C₇ cycloalkyl, the number of R¹⁻²⁴ is one or more, when the number of R¹⁻²⁴is multiple, each R¹⁻²⁴ are the same or different; the multiple can be 2 or 3.

In some embodiments, when R¹⁻⁷ and R¹⁻⁸ are independently "4-7 membered heterocycloalkyl having 1-3 heteroatoms selected from one or more of N, O and S", the heteroatom in the "4-7 membered heterocycloalkyl having 1-3 heteroatoms selected from one or more of N, O and S" is N or O.

In some embodiments, when R¹⁻⁷ and R¹⁻⁸ are independently "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1 or 2.

In some embodiments, when R¹⁻⁷ and R¹⁻⁸ are independently "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl.

In some embodiments, when R¹⁻⁷ and R¹⁻⁸are independently R¹⁻¹⁵ substituted "4-7 membered heterocycloalkyl having 1-3 heteroatoms selected from one or more of N, O and S", the heteroatom in the "4-7 membered heterocycloalkyl having 1-3 heteroatoms selected from one or more of N, O and S" is N and/or O.

In some embodiments, when R¹⁻⁷ and R¹⁻⁸are independently R¹⁻¹⁵ substituted "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1 or 2.

In some embodiments, when R¹⁻⁷and R¹⁻⁸are independently R¹⁻²⁵ substituted "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl.

In some embodiments, when R¹⁻⁷ and R¹⁻⁸ are independently R¹⁻²⁵ substituted "4-7 membered heterocycloalkyl having 1-3 heteroatoms selected from one or more of N, O and S", the number of R¹⁻¹⁵ is one or more, when the number of R¹⁻²⁵ is multiple, the R¹⁻²⁵ are the same or different; the multiple can be 2 or 3.

In some embodiments, when R¹⁻⁷and R¹⁻⁸are independently "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" is N and/or O.

In some embodiments, when R¹⁻⁷and R¹⁻⁸are independently "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1 or 2.

In some embodiments, when R¹⁻⁷and R¹⁻⁸are independently "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl.

In some embodiments, when R¹⁻⁷and R¹⁻⁸are independently R¹⁻²⁶ substituted "C₁-C₁₀heteroaryl having 1-4 heteroatoms selected from one or more of N, O and S", the heteroatom in "C₁-C₁₀heteroaryl having 1-4 heteroatoms selected from one or more of N, O and S" is N and/or O.

In some embodiments, when R¹⁻⁷and R¹⁻⁸are independently R¹⁻²⁶ substituted "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1 or 2.

In some embodiments, when R¹⁻⁷and R¹⁻⁸are independently R¹⁻²⁶ substituted "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl.

In some embodiments, when R¹⁻⁷and R¹⁻⁸are independently C₆-C₁₀aryl, the C₆-C₁₀aryl is phenyl.

In some embodiments, when R¹⁻⁷and R¹⁻⁸are independently R¹⁻²⁷ substituted C₆-C₁₀aryl , the C₆-C₁₀aryl is phenyl.

In some embodiments, when R¹⁻⁷and R¹⁻⁸ are independently R¹⁻²⁷ substituted C₆-C₁₀aryl, the number of R¹⁻²⁷ is one or more, when the number of R¹⁻²⁷is multiple, each R¹⁻²⁷ are the same or different; the multiple can be 2 or 3.

In some embodiments, when R¹⁻¹⁰, R¹⁻¹¹, R¹⁻¹², R¹⁻¹⁶, R¹⁻¹⁷, R¹⁻¹⁸, R¹⁻²⁸and R¹⁻²⁹are independently C₁-C₃alkyl, the C₁-C₃alkyl is methyl, ethyl, n-propyl or isopropyl.

In some embodiments, when R¹⁻¹³ is C₁-C₆alkyl, the C₁-C₆ alkyl is C₁-C₃ alkyl, such as methyl, ethyl, n-propyl or isopropyl.

In some embodiments, when R¹⁻¹³ is halogenated C₁-C₆alkyl, the C₁-C₆ alkyl is a C₁-C₃ alkyl, such as methyl, ethyl, n-propyl or isopropyl.

In some embodiments, when R¹⁻¹³ is halogenated C₁-C₆alkyl, the halogen is fluorine, chlorine, bromine or iodine.

In some embodiments, when R¹⁻¹³ is halogenated C₁-C₆alkyl, the number of the halogens is one or multiple, and the multiple can be 2 or 3.

In some embodiments, when R¹⁻¹⁴ and R¹⁻¹⁵ are independently C₁-C₆alkyl, the C₁-C₆alkyl is C₁-C₃alkyl, such as methyl, ethyl, n-propyl or isopropyl.

In some embodiments, when R¹⁻¹⁴ and R¹⁻¹⁵ are independently halogenated C₁-C₆alkyl, the C₁-C₆alkyl is C₁-C₃alkyl, such as methyl, ethyl, n-propyl or isopropyl.

In some embodiments, when R¹⁻¹⁴and R¹⁻¹⁵are independently halogenated C₁-C₆alkyl, the halogen is fluorine, chlorine, bromine or iodine.

In some embodiments, when R¹⁻¹⁴ and R¹⁻¹⁵ are independently halogenated C₁-C₆alkyl, wherein the number of the halogen is one or multiple, and the multiple can be 2 or 3.

In some embodiments, when R¹⁻¹⁹, R¹⁻²⁰, R¹⁻²¹, R¹⁻²², R¹⁻²³, and R¹⁻²⁴are independently halogen, the halogen is fluorine, chlorine, bromine or iodine.

In some embodiments, when R¹⁻¹⁹, R¹⁻²⁰, R¹⁻²¹, R¹⁻²², R¹⁻²³and R¹⁻²⁴ are independently C₁-C₆alkyl, the C₁-C₆alkyl is C₁-C₃alkyl, for example, methyl, ethyl, n-propyl or isopropyl.

In some embodiments, X is N.

In some embodiments, A is O, S, S(=O)₂, NH or NCH₃.

In some embodiments, A is O, S or S(=O)₂.

In some embodiments, A is O or S(=O)₂.

In some embodiments, n and r are 1.

In some embodiments, L⁵ is C₃-C₆ cycloalkylene.

In some embodiments, L²and L⁴are independently O.

In some embodiments, Z⁵ is C₂-C₁₀alkylene or C₂-C₁₀ unsaturated alkylene.

In some embodiments, B is C₂-C₁₀alkylene, C₂-C₁₀unsaturated alkylene, -Z⁵-L²-or-(CH₂)ₙ-L⁵-(CH₂)ᵣ-L⁴-.

In some embodiments, B is C₄-C₆alkylene, C₄-C₆unsaturated alkylene, -Z⁵-L²-or-(CH₂)ₙ-L⁵-(CH₂)ᵣ-L⁴-, Z⁵is C₃-C₅alkylene or C₃-C₅unsaturated alkylene.

In some embodiments, B is 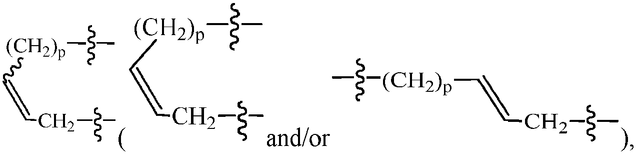 -(CH₂)ᵥ-, 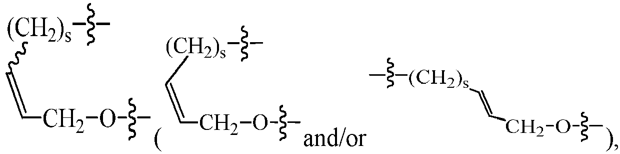 -(CH₂)_{w}-O- or -CH₂-L⁵-CH₂-O-; p is 1, 2 or 3; v is 4, 5 or 6; s is 1 or 2; w is 3, 4 or 5; L⁵ is C₃-C₅cycloalkylene.

In some embodiments, p is 2; v is 5; s is 1; w is 4; L⁵ is cyclopropylene or cyclobutylene.

In some embodiments, R¹⁻⁴ is C₁-C₆alkyl, R¹⁻⁶substituted C₁-C₆alkyl, -S(=O)₂R¹⁻⁷, - C(=O)R¹⁻⁸, COOR¹⁻¹², C₃-C₇cycloalkyl, "4-7 membered heterocycloalkyl having 1-3 heteroatoms selected from one or more of N, O and S", "C₁-C₁₀ heteroaryl having 1-4 heteroatoms selected from one or more of N, O and S" or R¹⁻²¹substituted "C₁-C₁₀ heteroaryl having 1-4 heteroatoms selected from one or more of N, O and S".

In some embodiments, R¹⁻⁶ is halogen, OH, COOR¹⁻¹⁷, S(=O)₂R¹⁻³¹, C₁-C₃alkoxy, "4-7 membered heterocycloalkyl having 1-3 heteroatoms selected from one or more of N, O and S" (e. g. 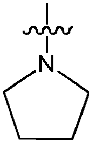 ) or "C₁-C₁₀ heteroaryl having 1-4 heteroatoms selected from one or more of N, O and S" (e. g 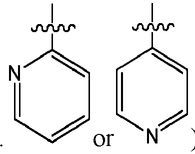 ).

In some embodiments, R¹⁻⁷and R¹⁻⁸ are independently C₁-C₃alkyl (e. g. methyl) or C₆-C₁₀aryl (e. g. phenyl).

In some embodiments, R¹⁻⁷ and R¹⁻⁸are independently C₁-C₃alkyl (e. g. methyl).

In some embodiments, R¹⁻²¹ is C₁-C₃alkyl.

In some embodiments, R¹⁻⁵is C₁-C₄alkyl or COOH.

In some embodiments, where R¹⁻⁴ is COOH, C₁-C₄alkyl, R¹⁻⁶substituted C₁-C₄alkyl, - C(=O)R¹⁻⁸, -S(=O)₂R¹⁻⁷, 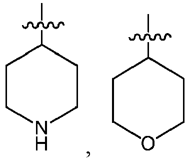 or cyclobutyl; R¹⁻⁶ is COOR¹⁻¹⁷, C₁-C₃alkoxy, 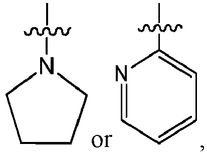 R¹⁻⁷is methyl; and R¹⁻⁸is methyl.

In some embodiments, in scheme A, R³ is H.

In some embodiments, in scheme A, R¹, R² together with the carbon atoms to which they are attached form a "5-6 membered heterocycloalkylene having 1-2 heteroatoms selected from one or more of N and/or O", R¹⁻⁴substituted "5-6 membered heterocycloalkylene having 1-2 heteroatoms selected from one or more of N and/or O", C₅-C₆cycloalkylene or R¹⁻⁵substituted C₅-C₆cycloalkylene; wherein the methylene in the "5-6 membered heterocycloalkylene having 1-2 heteroatoms selected from one or more of N and/or O" and R¹⁻⁴substituted "5-6 membered heterocycloalkylene having 1-2 heteroatoms selected from one or more of N and/or O" is not replaced, or one methylene isreplaced by a carbonyl group.

In some embodiments, in scheme B, R¹ is H.

In some embodiments, in scheme B, R², R³ together with the carbon atoms to which they are attached form a "5-6 membered heterocycloalkylene having 1-2 heteroatoms selected from one or more of N and/or O", R¹⁻⁴substituted "5-6 membered heterocycloalkylene having 1-2 heteroatoms selected from one or more of N and/or O"; wherein the methylene in the "5-6 membered heterocycloalkylene having 1-2 heteroatoms selected from one or more of N and/or O" and R¹⁻⁴substituted "5-6 membered heterocycloalkylene having 1-2 heteroatoms selected from one or more of N and/or O" is not replaced.

In some embodiments, R⁵ is H.

In some embodiments, R¹⁰ is C₁-C₆alkyl, and R¹¹is C₁-C₆alkyl.

In some embodiments, R¹² is C₁-C₆alkyl.

In some embodiments, R⁹ is H.

In some embodiments, R¹³is H or COOR¹⁷.

In some embodiments, R¹⁷ is C₁-C₆alkyl.

In some embodiments, R¹³ is H.

In some embodiments, -A-B- is 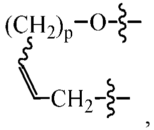 -O-(CH₂)ᵥ-, 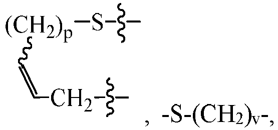 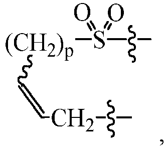 -S(=O)₂-(CH₂)ᵥ-, 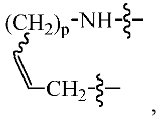 -NH-(CH₂)ᵥ-, 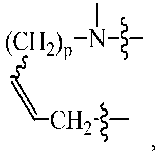 -N(CH₃)-(CH₂)ᵥ-, 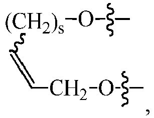 -O-(CH₂)_{w}-O- or -O-CH₂-L⁵-CH₂-O-(e. g. 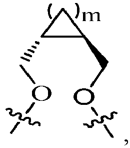 m is 1, 2 or 3); p is 1, 2 or 3; v is 4, 5 or 6; s is 1 or 2; w is 3, 4 or 5; L⁵ is C₃-C₅ cycloalkylene.

In some embodiments, -A-B- is 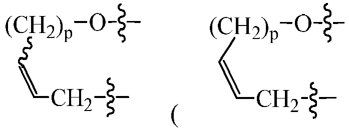 and/or 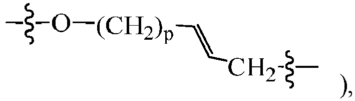 -O-(CH₂)ᵥ-, 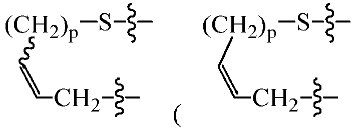 and/or 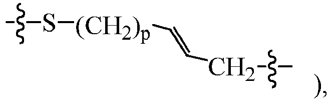 -S-(CH₂)ᵥ-, 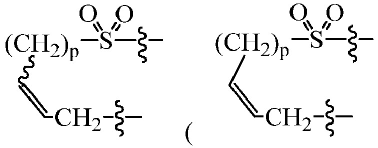 and/or 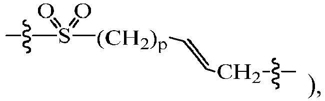 -S(=O)₂-(CH₂)ᵥ-, 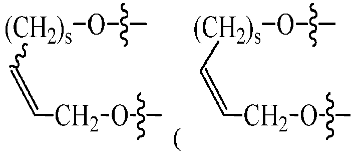 and/or 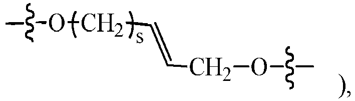 -O-(CH₂)_{w}-O-, 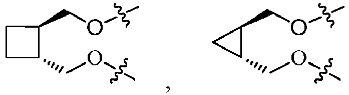 or 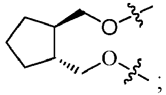 p is 1, 2 or 3; v is 4, 5 or 6; s is 1 or 2; w is 3 or 4.

In some embodiments, 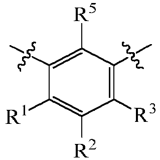 is 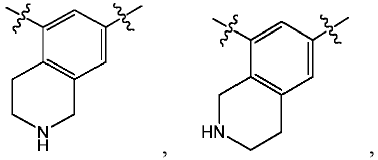 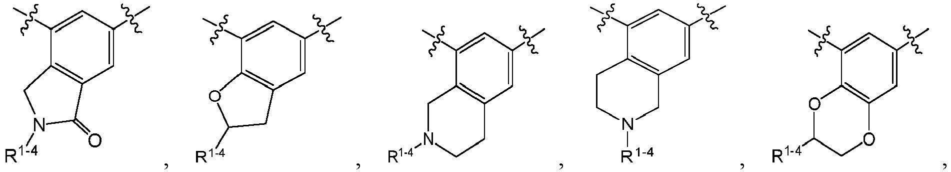 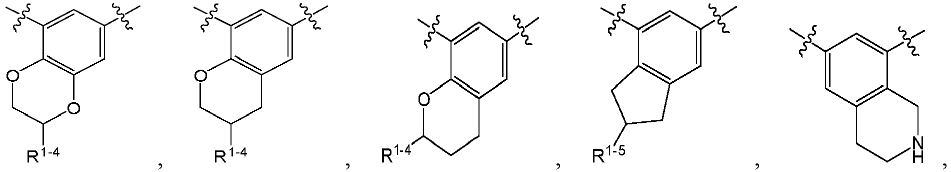 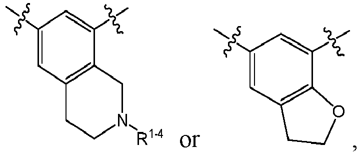 the number of R¹⁻⁴ is 1 or more, when number of of R¹⁻⁴ is multiple, the R¹⁻⁴ are the same or different; the number of R¹⁻⁵ is 1 or 2, and when the number of R¹⁻⁵ is 2, the R¹⁻⁵ are the same or different.

In some embodiments, the compound as shown in formula I is any one of the following compounds:

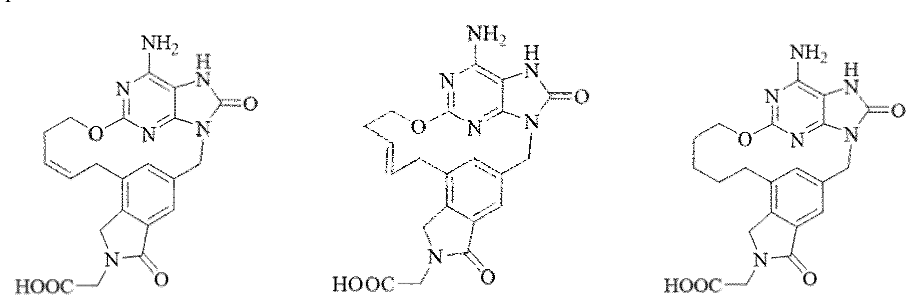

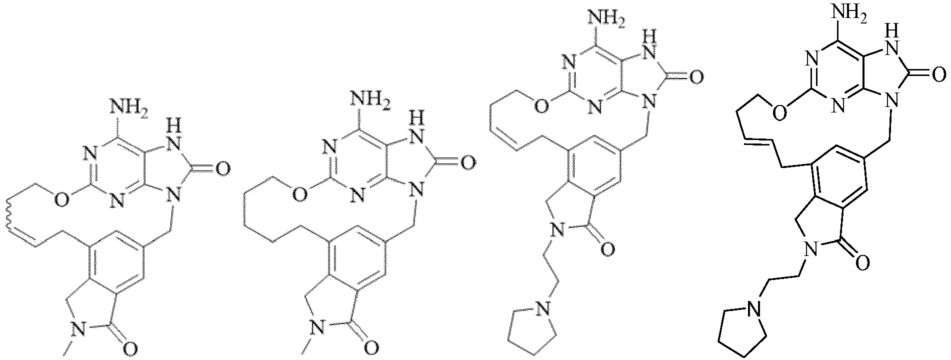

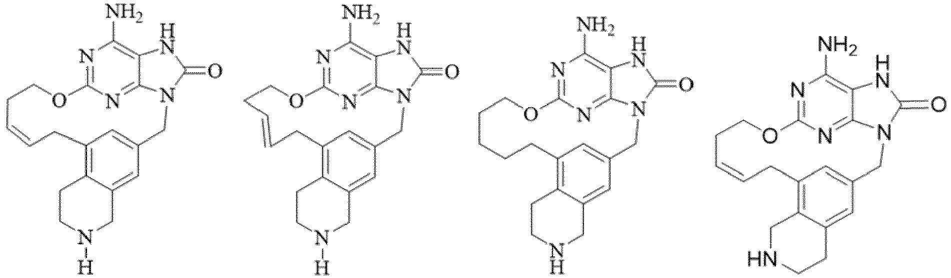

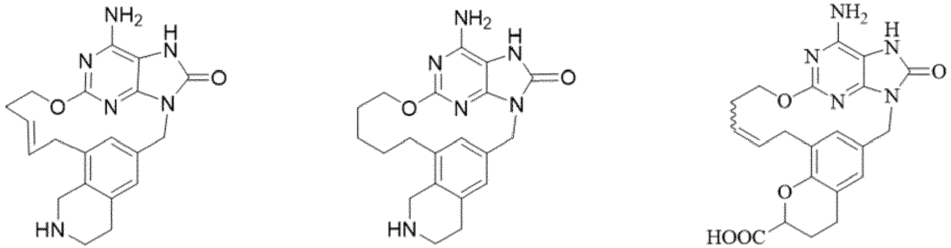

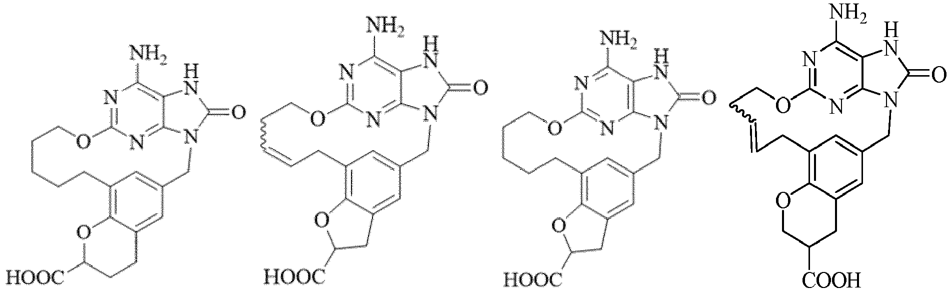

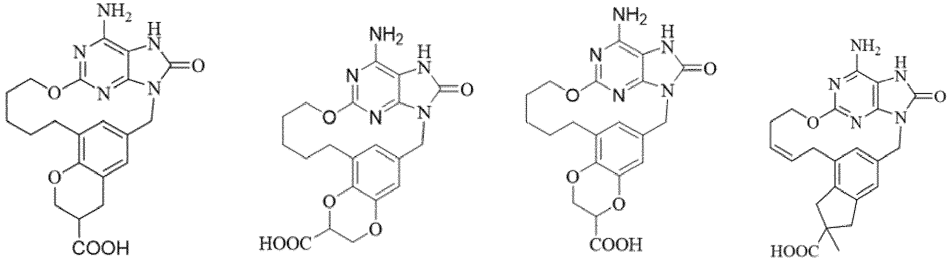

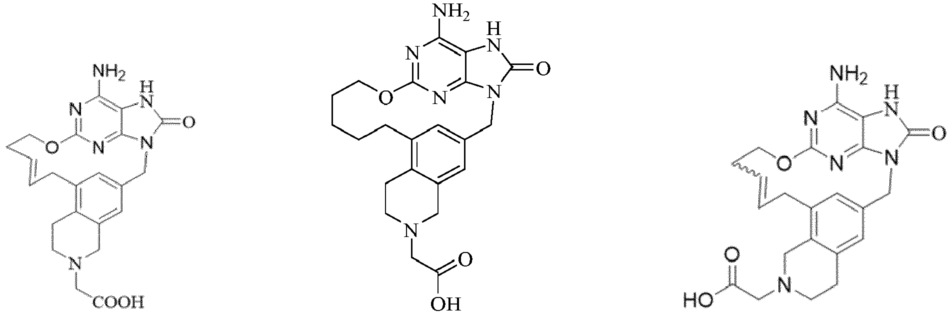

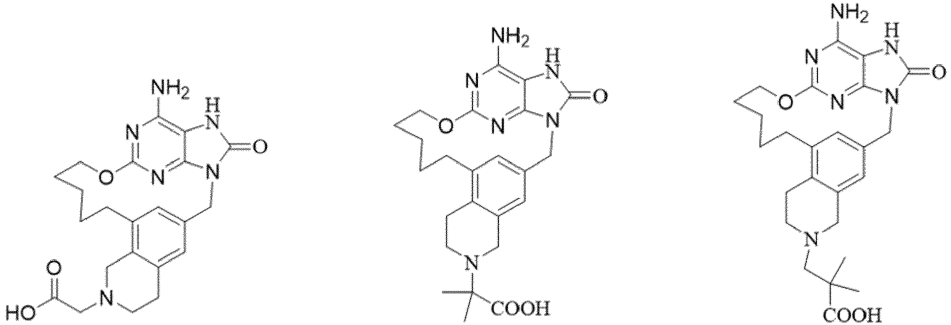

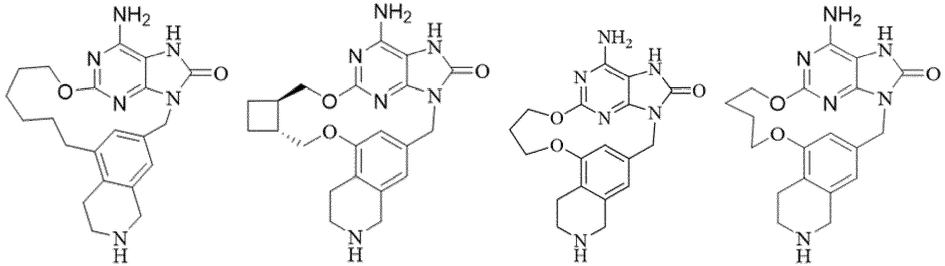

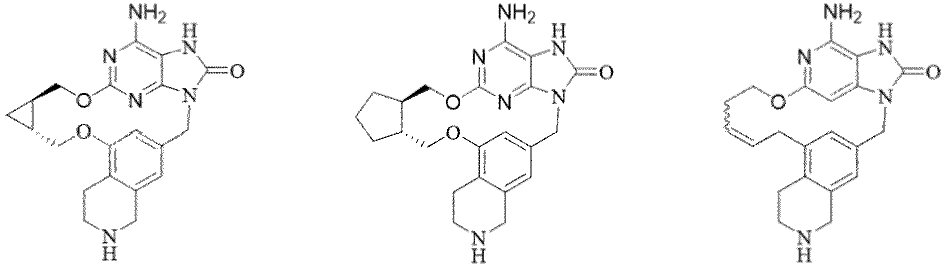

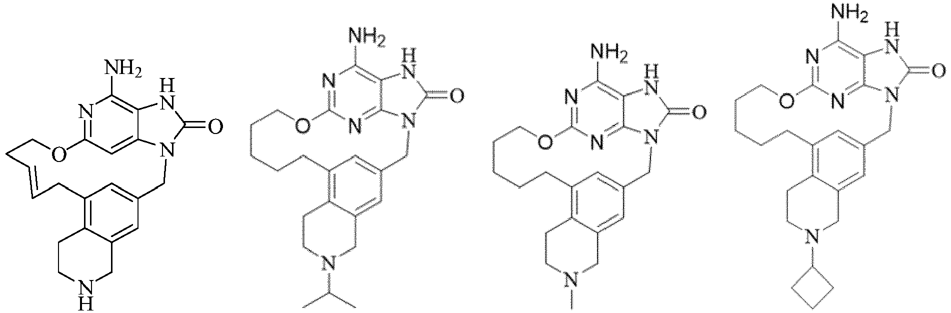

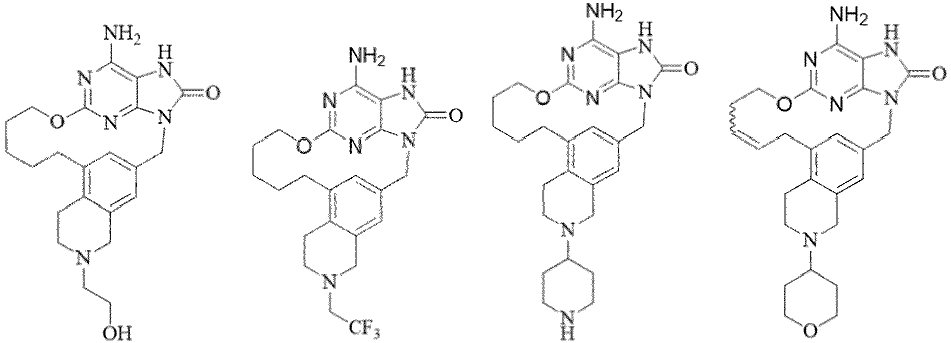

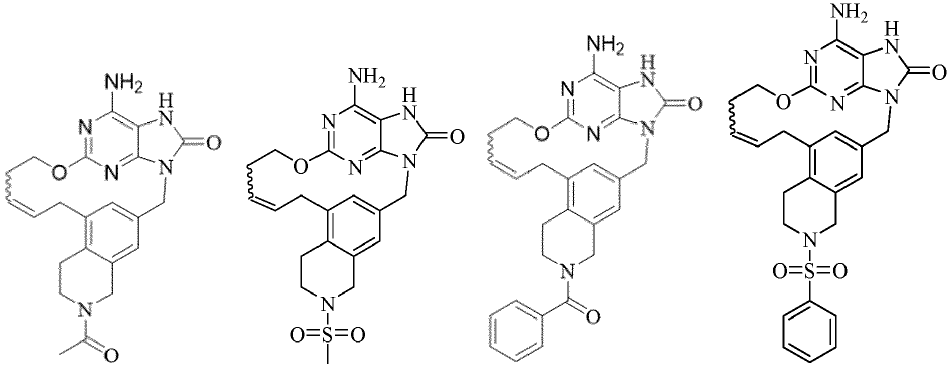

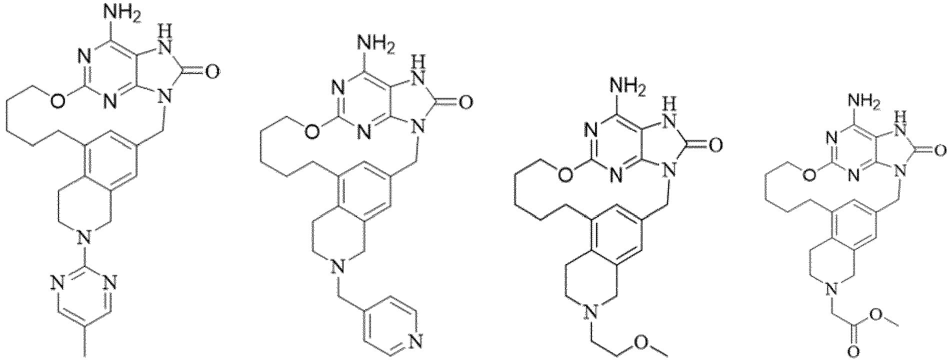

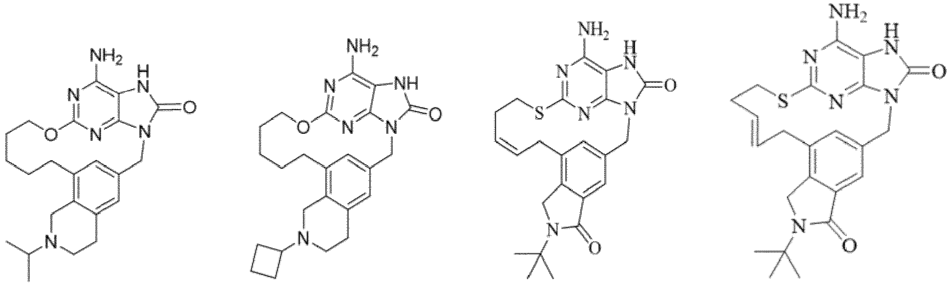

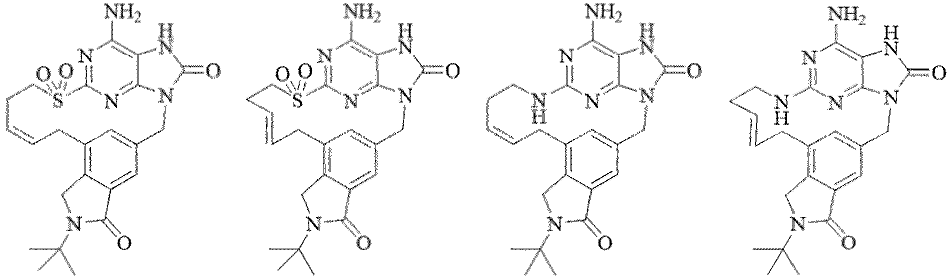

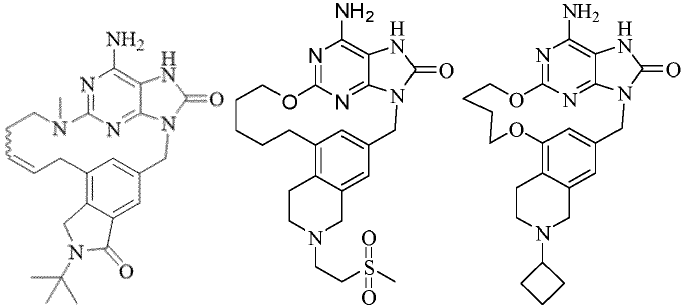

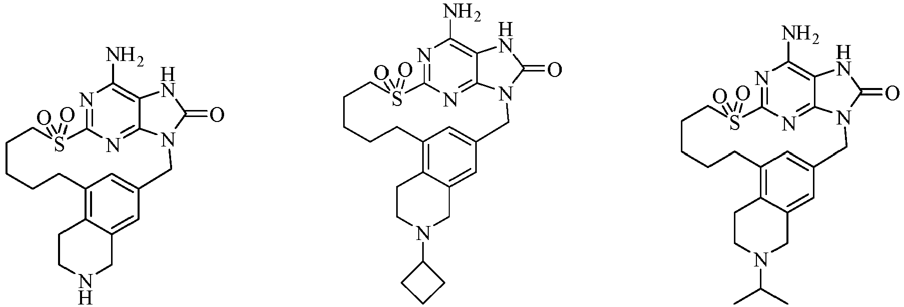

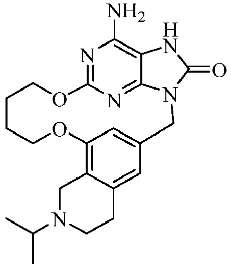

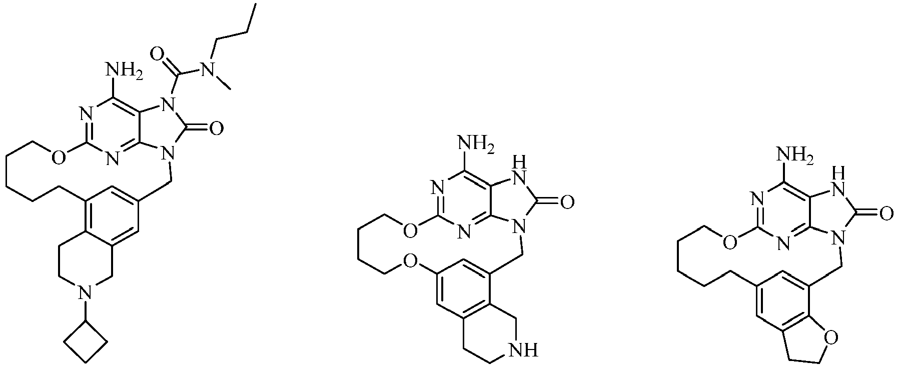

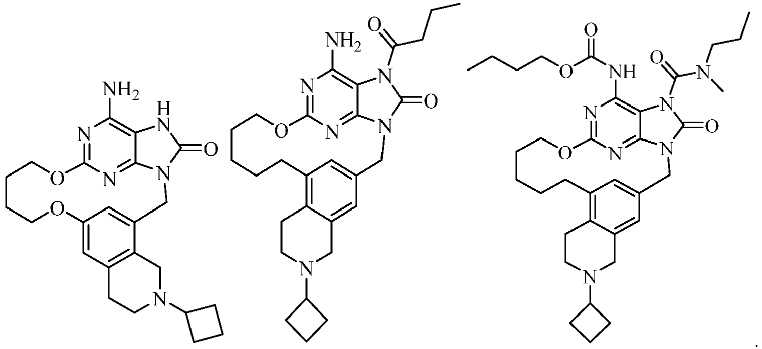

The invention also provides a compound as shown in formula **II**: 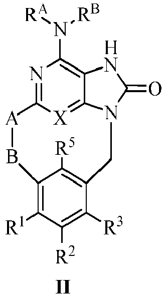 wherein, R^{A} and R^{B}are independently H or amino protecting groups, and R^{A}and R^{B} cannot simultaneously be H; X, A, B, R¹, R², R³ and R⁵are defined as described above.

In formula II, the amino protecting group may be a common amino protecting group in the art, such as p-methoxybenzyl (PMB).

The compound shown in Formula **II** is any of the following compounds:

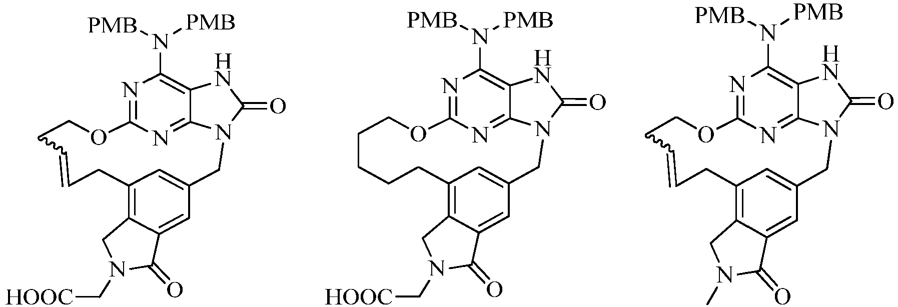

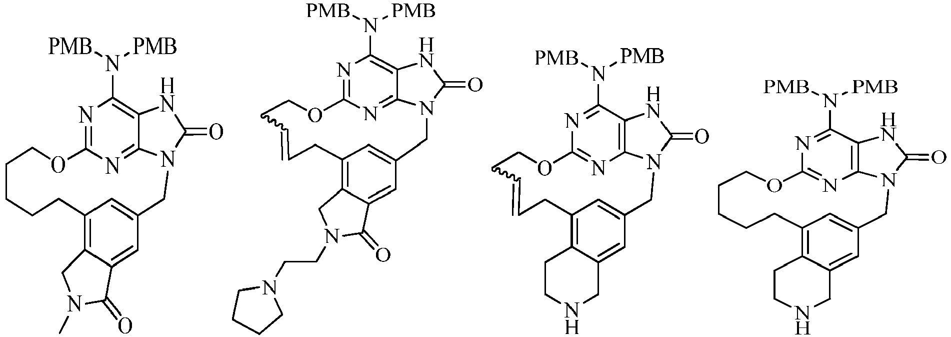

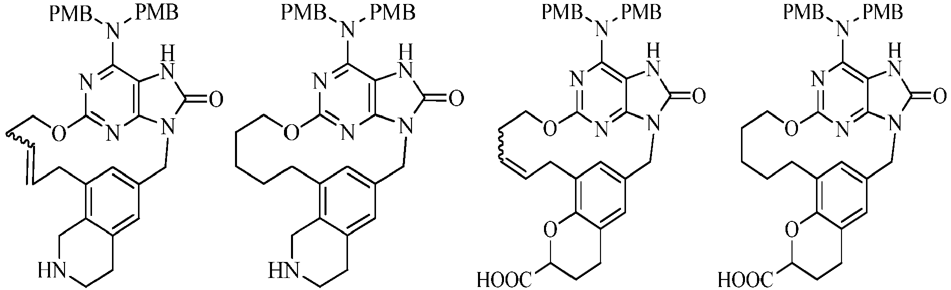

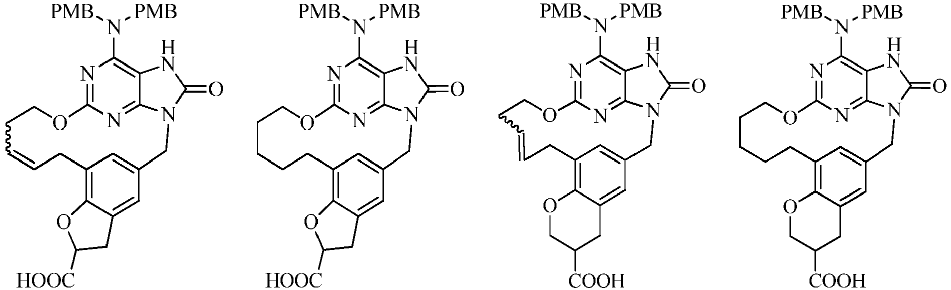

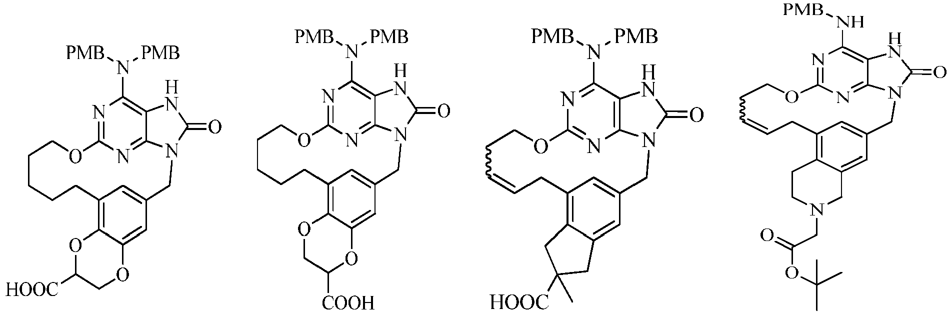

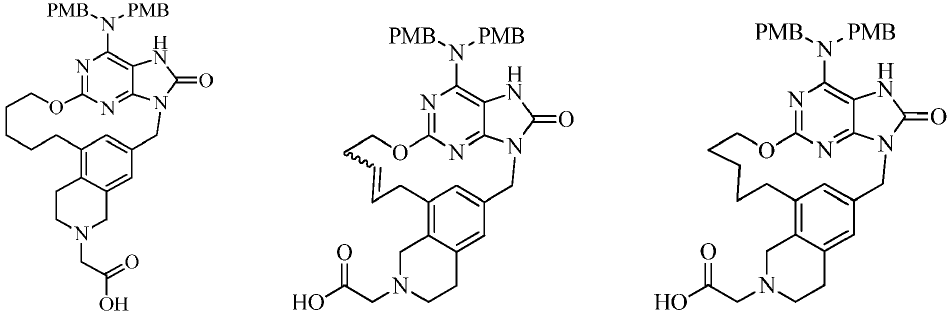

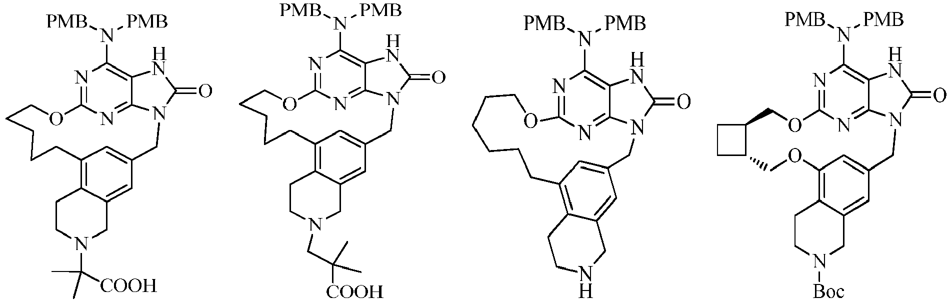

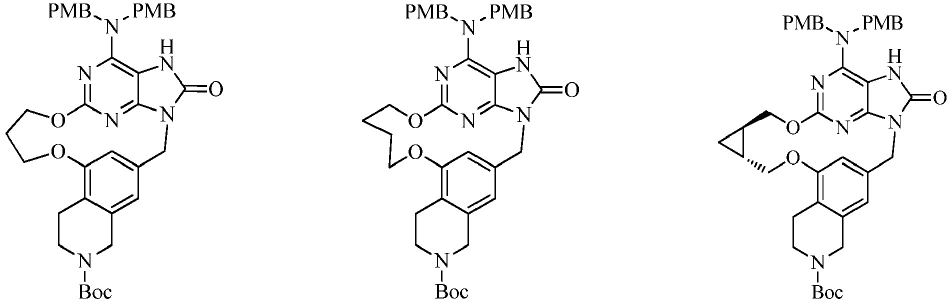

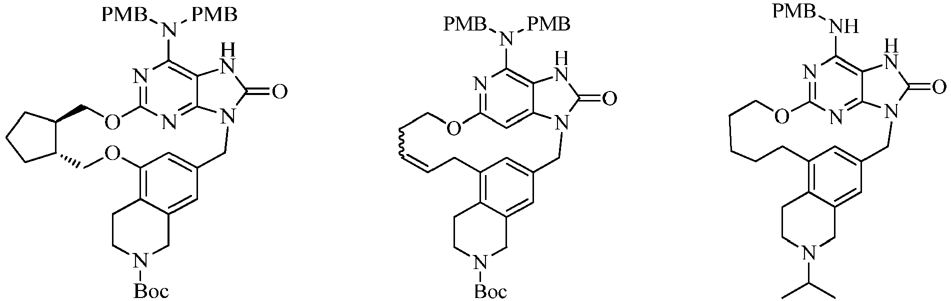

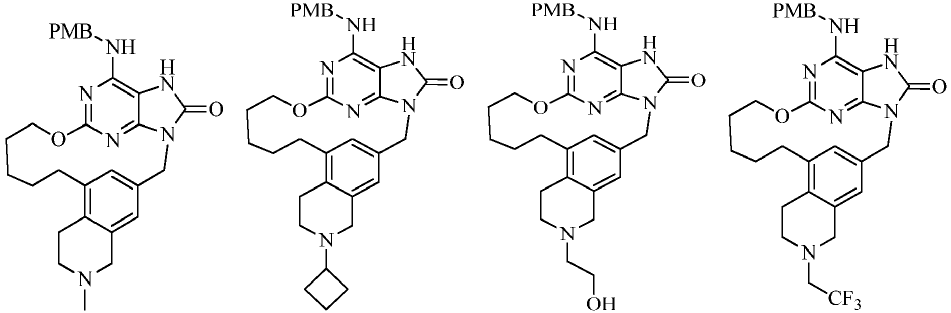

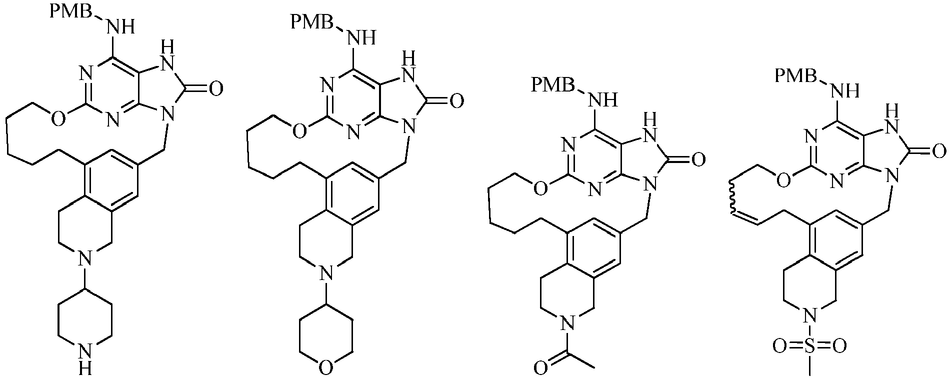

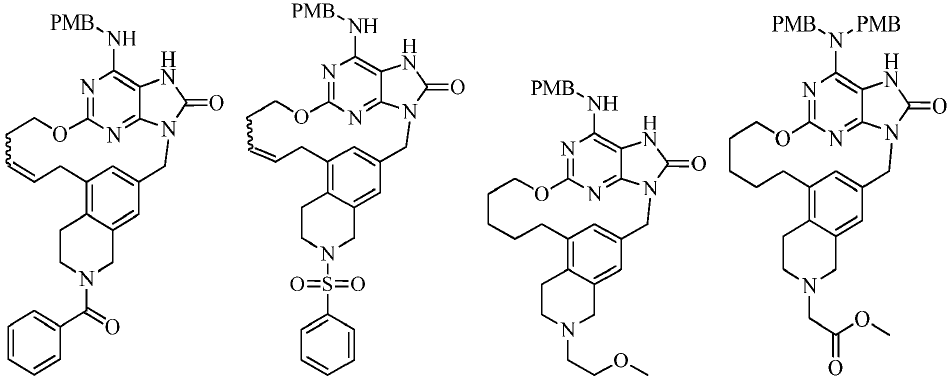

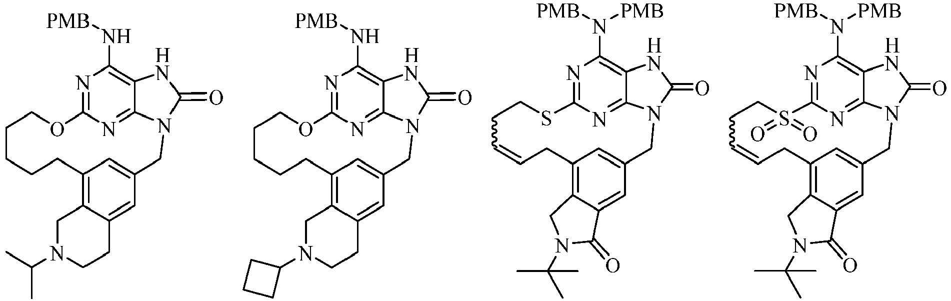

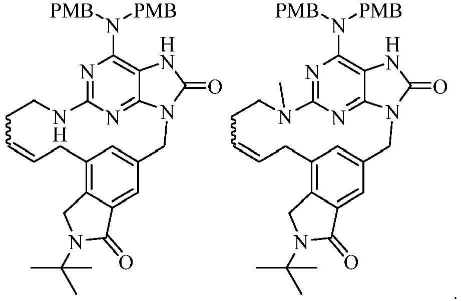

The invention also provides the preparation method of the compound as shown in formula **I**, which comprises the following method I. method II or method III:
The method I comprises the following steps: performing the following deprotection reaction on the compound as shown in formula **II** to obtain the compound as shown in formula **I**; 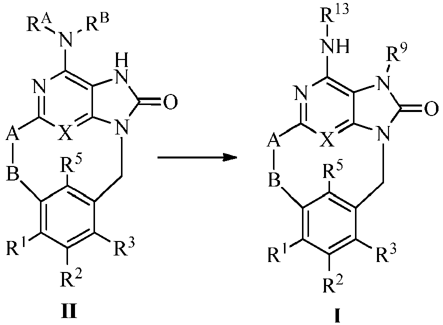
   In method I, R⁹and R¹³ are H, R^{A}, R^{B}, A, B, R¹, R², R³and R⁵ are defined as above;
The method II comprises the following steps: performing an acylation reaction of the compound of formula **I-A** and the compound of formula **III-A** to obtain the compound of formula **I**; 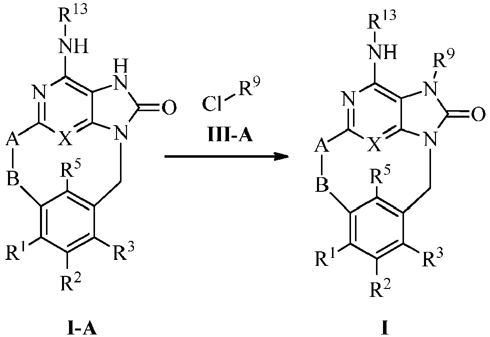
   In method II, R⁹is CONR¹⁰R¹¹or C(=O)R¹², R¹⁰, R¹¹, R¹², A, B, R¹, R², R³, R⁵and R¹³are defined as above;
The method III comprises the following steps: performing an acylation reaction of the compound of formula **I-B** and the compound of formula **III-B** to obtain the compound of formula **I**; 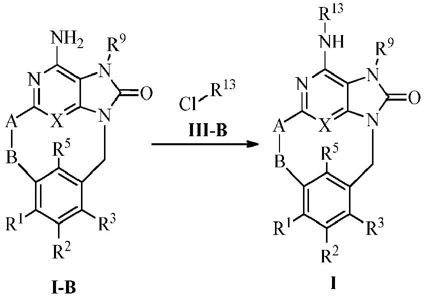
In method III, R¹³ is CONR¹⁴R¹⁵, C(=O)R¹⁶ or COOR¹⁷, R¹⁴, R¹⁵, R¹⁶, R¹⁷, A, B, R¹, R², R³, R⁵and R⁹ are defined as above.

In method I, the procedure and conditions of the deprotection reaction can be conventional in the art, such as heating in trifluoroacetic acid.

In method II or method III, the procedure and conditions of the acylation reaction can be conventional in the art, for example, in the presence of a base (such as pyridine and triethylamine, pyridine and DIPEA, DMAP and triethylamine or DMAP and DIPEA).

The preparation method of the compound as shown in formula **I** may further include the following steps: the compound as shown in formula **IV** performs the following intra-molecular ring forming reaction to obtain the compound as shown in formula **II**;

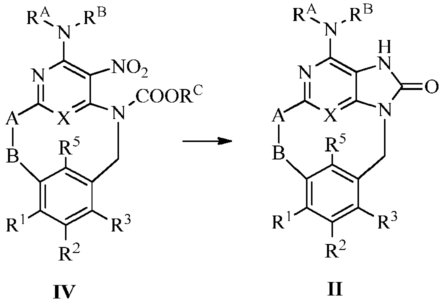

R^{C} is C₁-C₃alkyl, and R^{A}, R^{B}, A, B, R¹, R², R³, R⁵ and R⁹are defined as above.

The procedures and conditions for the intramolecular cyclisation reaction may be conventional in the art, for example in the presence of acetic acid and a metal (e. g. zinc and/or iron).

The present invention also provides a pharmaceutical composition comprising the compound of formula **I**, a solvate thereof, a prodrug thereof, a metabolite thereof, or a pharmaceutically acceptable salt thereof, and a pharmaceutically acceptable excipient.

The present invention also provides a use of the compound of formula **I**, a solvate thereof, a prodrug thereof, a metabolite thereof, or a pharmaceutically acceptable salt thereof, or the pharmaceutical composition in the preparation of a medicament. The medicament is for treating or preventing a tumor or an infection caused by a virus, preferably one or more of HBV, HCV, HIV and influenza virus.

The present invention also provides a use of the compound of formula **I**, a solvate thereof, a prodrug thereof, a metabolite thereof, or a pharmaceutically acceptable salt thereof, or the pharmaceutical composition in the preparation of a TLR7 agonist.

In the application, the TLR7 agonist can be used in mammalian organisms; it can also be used in vitro, mainly as experimental use, for example: as a standard sample or control to provide comparison, or according to the conventional method in the art to prepare a kit, to provide rapid detection of TLR7 inhibitory effect.

Unless otherwise specified, the terms used in the present invention have the following meanings:
Herein, the ethylenically linked " 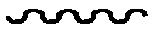 "means that the olefin is in the Z configuration, the E configuration, or a mixture of the two, for example, 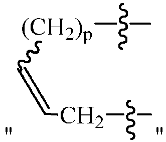 is a Z configuration olefin 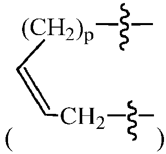 and/or an E configuration olefin 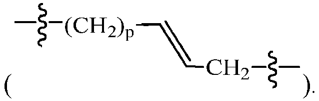 As used herein, the term may be preceded and/or followed by a single dash "-", or a double dash "=", indicating the bond order of the bond between the named substituent and the parent moiety; a single dash indicating a single bond and a double dash indicating a double bond. In the absence of a single or double dash, a single bond may be considered to be formed between a substituent and its parent moiety; furthermore, unless otherwise indicated, substituents are read as "left to right" or "top to bottom". For example, "-Z⁵-L²-" means that Z⁵ is connected to A, and L² is connected to B; 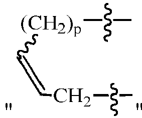 means "(CH₂)ₚ" is connected to A.

The term "pharmaceutically acceptable" means that the salts, solvents, excipients, and the like are generally non-toxic, safe, and suitable for patient use. The "patient" is preferably a mammal, more preferably a human.

The term "mammal" includes any mammal. Examples of mammals include, but are not limited to, cattle, horses, sheep, pigs, cats, dogs, mice, rats, rabbits, guinea pigs, monkeys, humans, and the like, most preferably humans.

The term "pharmaceutically acceptable salt" refers to a salt prepared from a compound of the present invention with a relatively non-toxic, pharmaceutically acceptable acid or base. When the compound of the present invention contains a relatively acidic functional group, a base addition salt can be obtained by contacting the neutral form of the compound with a sufficient amount of a pharmaceutically acceptable base in a pure solution or in a suitable inert solvent. Pharmaceutically acceptable base addition salts include, but are not limited to, lithium salt, sodium salt, potassium salt, calcium salt, aluminum salt, magnesium salt, zinc salt, bismuth salt, ammonium salt, diethanolamine salt. When the compounds of the present invention contain relatively basic functional groups, acid addition salts can be obtained by contacting the neutral form of such compounds with a sufficient amount of a pharmaceutically acceptable acid in neat solution or in a suitable inert solvent. The pharmaceutically acceptable acid includes inorganic acid, and the inorganic acid includes but is not limited to: hydrochloric acid, hydrobromic acid, hydroiodic acid, nitric acid, carbonic acid, phosphoric acid, phosphorous acid, sulfuric acid and the like. The pharmaceutically acceptable acid includes an organic acid, and the organic acid includes, but is not limited: acetic acid, propionic acid, oxalic acid, isobutyric acid, maleic acid, malonic acid, benzoic acid, succinic acid, suberic acid, fumaric acid, lactic acid, mandelic acid, phthalic acid, benzene sulfonic acid, p-toluenesulfonic acid, citric acid, salicylic acid, tartaric acid, methanesulfonic acid, isonicotinic acid, oleic acid, tannic acid, pantothenic acid, ascorbic acid, 4 '-methylene-bis (3-hydroxy-2-naphthoic acid)), amino acids (e. g., glutamic acid, arginine), etc. When the compound of the present invention contains relatively acidic and relatively basic functional groups, it can be converted into a base addition salt or an acid addition salt. See Berge et al., "Pharmaceutical Salts", Journal of Pharmaceutical Science 66: 1-19 (1977), or Handbook of Pharmaceutical Salts: Properties, Selection, and Use (P. Heinrich Stahl and Camille G. Wermuth, ed., Wiley-VCH, 2002).

The term "solvate" refers to a substance formed by combining a compound of the present invention with a stoichiometric or non-stoichiometric amount of a solvent. The solvent molecules in the solvate may be present in an ordered or unordered arrangement. The solvent includes, but is not limited to, water, methanol, ethanol and the like.

The term "prodrug" refers to a derivative of a compound of the invention which is converted to a compound of the invention (drug) when the derivative is administered to a warm-blooded animal (e. g., a human). Typical examples of prodrugs include compounds having a biologically labile protecting group on a functional portion of the active compound. Prodrugs include compounds that can produce an active compound by oxidation, reduction, amination, deamination, hydroxylation, dehydroxylation, hydrolysis, dehydration, alkylation, dealkylation, acylation, deacylation, phosphorylation, dephosphorylation.

The term "metabolite" refers to a degradation product of a compound of the invention by one or more metabolic processes, which exerts a desired biological activity.

The terms "compound", "solvate", "prodrug", "metabolite" and "pharmaceutically acceptable salt" may exist in crystalline or amorphous form. The term "crystal form" means that the ions or molecules therein are arranged strictly periodically in three-dimensional space in a certain manner, and have a regular pattern of periodic repetition at intervals of a certain distance; due to the different periodic arrangements, there may be multiple crystal forms, that is, polymorphic phenomena. The term "amorphous" means that the ions or molecules therein present a disordered distribution state, that is, the ions and molecules do not have periodic arrangement rules.

The terms "compounds", "solvates", "prodrugs", "metabolites" and "pharmaceutically acceptable salts", if stereoisomers exist, may exist as individual stereoisomers or as mixtures thereof (e. g., racemates). The term "stereoisomer" refers to cis-trans isomers or optical isomers. These stereoisomers can be separated, purified and enriched by asymmetric synthesis methods or chiral separation methods (including but not limited to thin layer chromatography, rotary chromatography, column chromatography, gas chromatography, high pressure liquid chromatography, etc.), and can also be obtained by chiral resolution by bonding (chemical combination, etc.) or salt formation (physical combination, etc.). The term "single stereoisomer" means that the mass content of one stereoisomer of the compound of the present invention relative to all stereoisomers of the compound is not less than 95%.

The terms "compound", "solvate", "prodrug", "metabolite" and "pharmaceutically acceptable salt", if tautomers exist, may exist in the form of a single tautomer or a mixture thereof, preferably in the form of the more stable tautomer. For example, 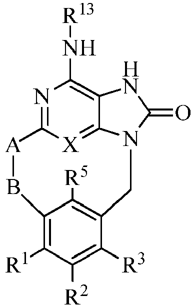 and 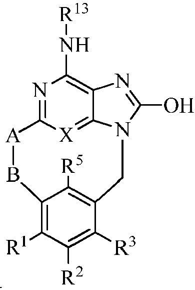 are tautomers.

The atoms in the terms "compound", "solvate", "prodrug", "metabolite", and "pharmaceutically acceptable salt" may be present in their natural or non-natural abundance. In the case of a hydrogen atom, its natural abundance form refers to about 99.985% of it being protium and about 0.015% of it being deuterium; its non-natural abundance form refers to about 95% of it being deuterium. That is, one or more of the atoms in the terms "compound, " "pharmaceutically acceptable salt, " "solvate, " and "solvate of a pharmaceutically acceptable salt" may be an atom present in a form that is not present in natural abundance.

When any variable (for example, R¹⁻¹) appears multiple times in the definition of a compound, the definition of each position of the variable has nothing to do with the definition of the other positions, and their meanings are independent of each other and do not affect each other. Therefore, if a group is substituted by 1, 2 or 3 R¹⁻¹groups, that is, the group may be substituted by up to 3 R¹⁻¹, the position R¹⁻¹The definition is independent of the definition of the other positions R¹⁻¹. In addition, combinations of substituents and/or variables are permissible only if the combination results in a stable compound.

The term "halogen" refers to fluorine, chlorine, bromine or iodine.

The term "alkyl" refers to a linear or branchedsaturated aliphatic hydrocarbon grouphaving a specified number of carbon atoms, and generally refers to a saturated alkyl. Examples of alkyls include methyl, ethyl, n-propyl, isopropyl, n-butyl, t-butyl, isobutyl, sec-butyl, n-pentyl, n-hexyl, n-heptyl, n-octyl, and similar alkyls.

The term "alkylene" refers to a divalent group of a linear or branchedsaturated aliphatic hydrocarbon grouphaving a specified number of carbon atoms. The two valents can be concentrated on the same atom, such as methylene (-CH₂-), ethylene (-CHCH₃-), and the two valents can also be connected to two atoms respectively, such as 1, 2-ethylene (-CH₂CH₂-).
the term "unsaturated" alkylene "refers to an alkyl having the specified number of carbon atoms, comprising one or more units of unsaturation of straight-chain or branched-chain ofaliphatic hydrocarbon radical of one to a divalent radical of for example-CH₂CH₂CH =CHCH₂-.

The term "alkoxy" refers to the group-O-R^{X}, where R^{X}is an alkyl as defined above.

The term "cycloalkyl" refers to a monovalent saturated cyclic alkyl, examples of which are cyclopropyl, cyclobutyl, cyclopentyl, or cyclohexyl.

The term "cycloalkylene" refers to a divalent group of a saturated cyclic alkylene, such as cycloalkylene 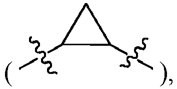 cyclobutylene (e. g., 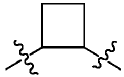 ), cyclopentylene (e. g., 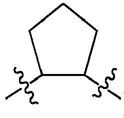 ), or cyclohexylene (e. g., 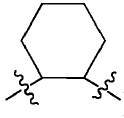 ), etc.

The term "heterocycloalkyl" refers to a saturated monocyclic group having a heteroatom. Examples of heterocycloalkyls are: tetrahydrofuryl, tetrahydropyranyl, tetrahydrothiophenyl, tetrahydropyridyl, tetrahydropyrrolyl, azacyclobutane, thiazolidinyl, azolyl, piperidinyl, morpholinyl, thiomorpholinyl, piperazinyl, azacycloheptanyl, diazacycloheptanyl, oxazacycloheptanyl, etc.

The term "heterocycloalkyl" refers to a divalent group of a saturated monocyclic group having a heteroatom. Examples of heterocycloalkyl are: sub-tetrahydrofuryl (e. g. 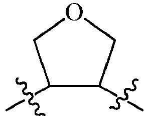 ), sub-tetrahydropyryl (e. g 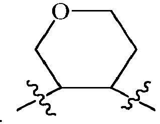 ), sub-tetrahydrothienyl (e. g. 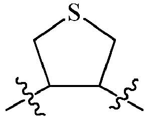 ), sub-tetrahydropyridyl (e. g. 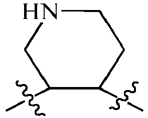 ), sub-tetrahydropyrrolyl (e. g. 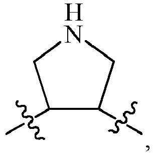 etc.

The term "aryl" refers to a C₆-C₁₀aryl, such as phenyl or naphthyl.

The term "heteroaryl" refers to an aromatic group containing heteroatoms, preferably containing 1, 2 or 3 aromatic 5-6-membered monocyclic or 9-10 membered bicyclic rings independently selected from nitrogen, oxygen and sulfur. When it is a bicyclic ring, at least one ring is aromatic, for example, furyl, pyridyl, pyridazinyl, pyrimidinyl, pyrazinyl, thienyl, isoazolyl, oxazolyl, diazolyl, imidazolyl, pyrrolyl, pyrazolyl, triazolyl, tetrazolyl, thiazolyl, isothiazolyl, thiadiazolyl, benzimidazolyl, indolyl, indazolyl, benzothiazolyl, benzoisothiazolyl, benzoazolyl, benzoisoazolyl, quinolyl, isoquinolyl, etc.

Theterm "heteroarylene" refers to a divalent group containing a heteroatom aromatic group, preferably containing 1, 2 or 3 aromatic 5-6-membered monocyclic or 9-10 membered bicyclic divalent groups independently selected from nitrogen, oxygen and sulfur. When it is a bicyclic ring, at least one ring has aromaticity, for example, sub-furyl (for example 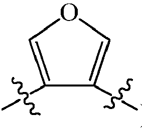 ), sub-pyridyl (for example 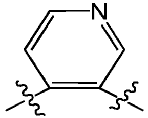 ), sub-pyridazinyl (for example 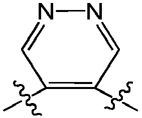 ), sub-pyrimidinyl (for example 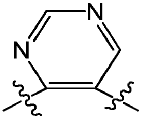 ), sub-pyrazinyl-based (for example 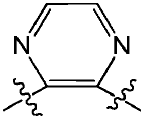 ), sub-thienyl (for example 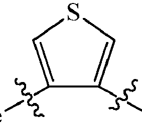 ).

The term "pharmaceutical excipients" refers to excipients and additives used in the production of pharmaceutical products and the formulation of prescriptions, and is all substances contained in pharmaceutical preparations except active ingredients. See the Pharmacopoeia of the People's Republic of China (2015 edition), or Handbook of Pharmaceutical Excipients (Raymond C Rowe, 2009 Sixth Edition)

The term "therapy" refers to therapeutic therapy. When a specific condition is involved, treatment means:(1) to alleviate one or more biological manifestations of the disease or condition, (2) to interfere with (a) one or more points in the biological cascade that causes or causes the disease or (B) One or more biological manifestations of the disease, (3) to improve one or more symptoms, effects or side effects related to the disease, or one or more symptoms, effects, or side effects associated with the condition or treatment thereof, or (4) slowing the development of one or more biological manifestations of the condition or condition.

The term "prevention" refers to a reduction in the risk of acquiring or developing a disease or disorder.

On the basis of not violating the common sense in the art, the above-mentioned preferred conditions can be combined arbitrarily, that is, each preferred example of the present invention can be obtained.

The reagents and raw materials used in the invention are commercially available.

The positive progress of the present invention is that the present invention provides a series of macrocyclic compounds with good TLR7 agonistic activity, which can be used totreat or prevent tumors or virus-caused infections.

### DETAILED DESCRIPTION OF EMBODIMENTS

Hereinafter, the present invention will be further described by way of examples, but the present invention is not limited to the scope of the described examples. The following embodiments do not indicate the specific conditions of the experimental method, in accordance with the conventional methods and conditions, or in accordance with the commodity instructions.

The compound of the invention is prepared by the following process:

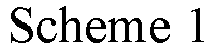

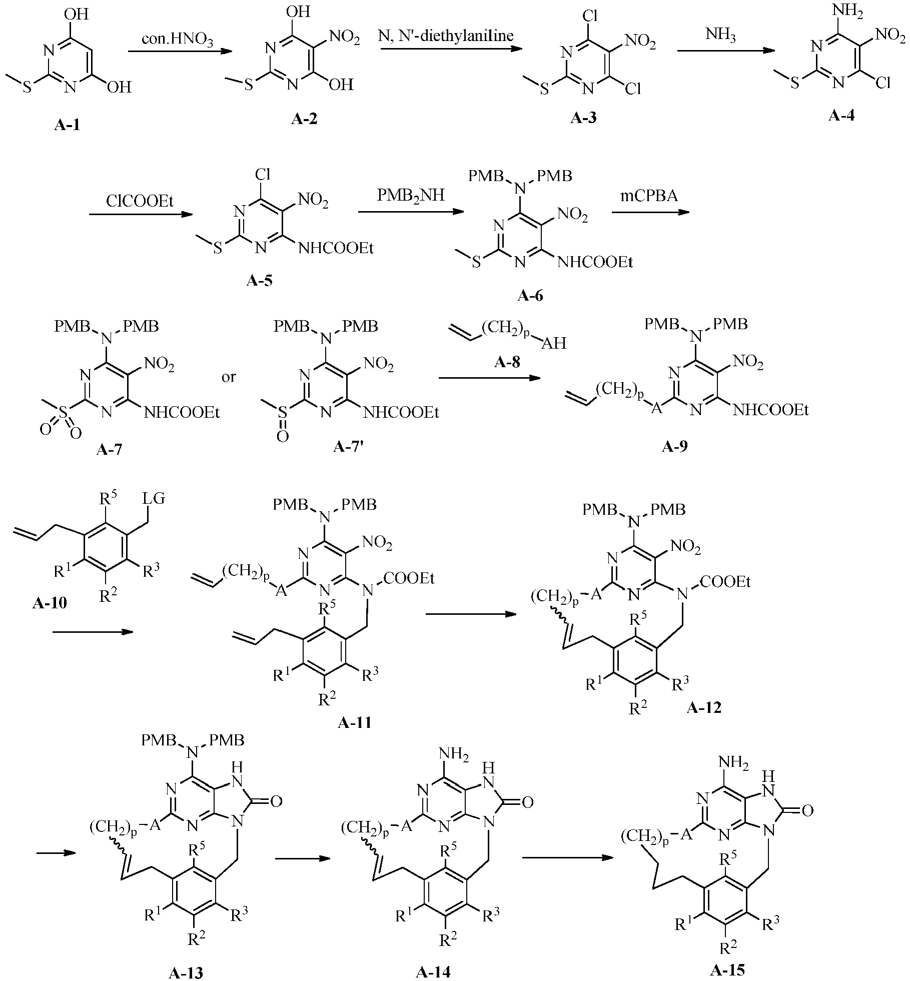

In scheme 1, the initial **compound A-1** is nitrated to obtain **compound A-2**; **A-2** is halogenated to obtain **compound A-3**; **A-3** is ammoniated to obtain **A-4**; **A-4** is acylated to obtain **A-5**; **A-5** is aminated to obtain **A-6**; **A-6** is oxidized to obtain **A-7** or **A-7'**; **A-9** is obtained by substitution reaction with **A-8**; **A-9** undergoes substitution reaction with **A-10** to form **A-11**; **A-11**undergoes olefin metathesis reaction to obtain macrocyclic compound **A-12**; **A-12** is closed in one step through nitro reduction to obtain **A-13**; **A-13** is deprotecting group to obtain**A-14**; **A-14** is catalytically hydrogenated to obtain **A-15**.

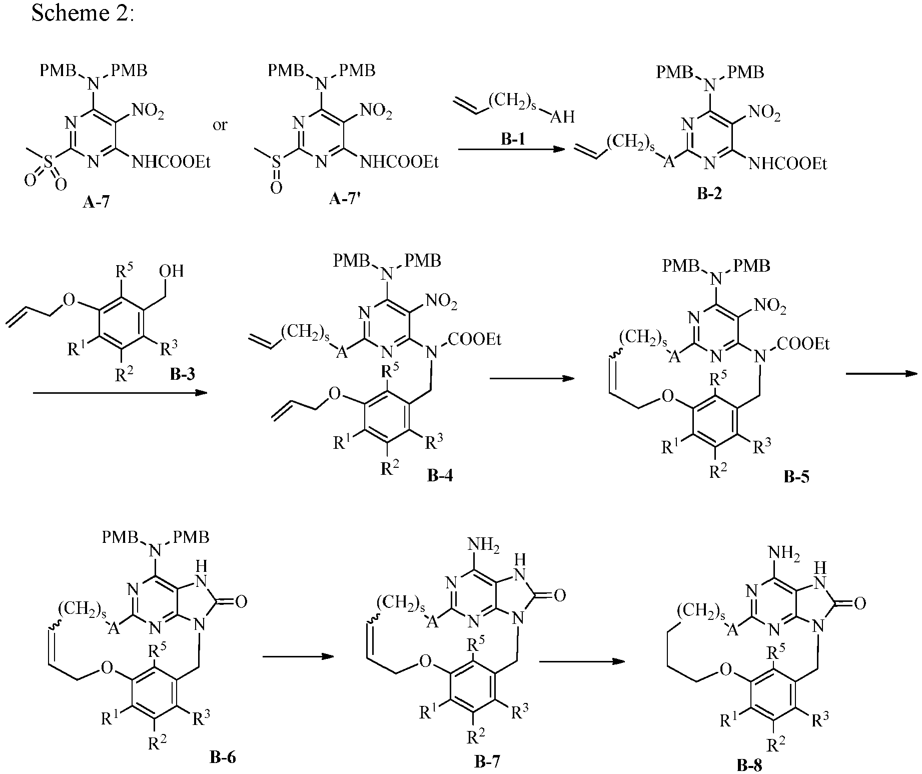

In scheme 2, the initial **compound A-7** or **A-7** ' and **B-1** are substituted to obtain the compound **B-2**; **B-2** and **B-3** are substituted to obtain the compound **B-4**; **B-4** is subjected to olefin metathesis to obtain the macrocyclic compound **B-5**; **B-5** is ring-closed by nitro reduction to obtain **B-6**; **B-6** is deprotected to obtain **B-7**; **B-7** is catalytic hydrogenated to obtain **B-8**.

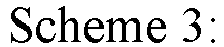

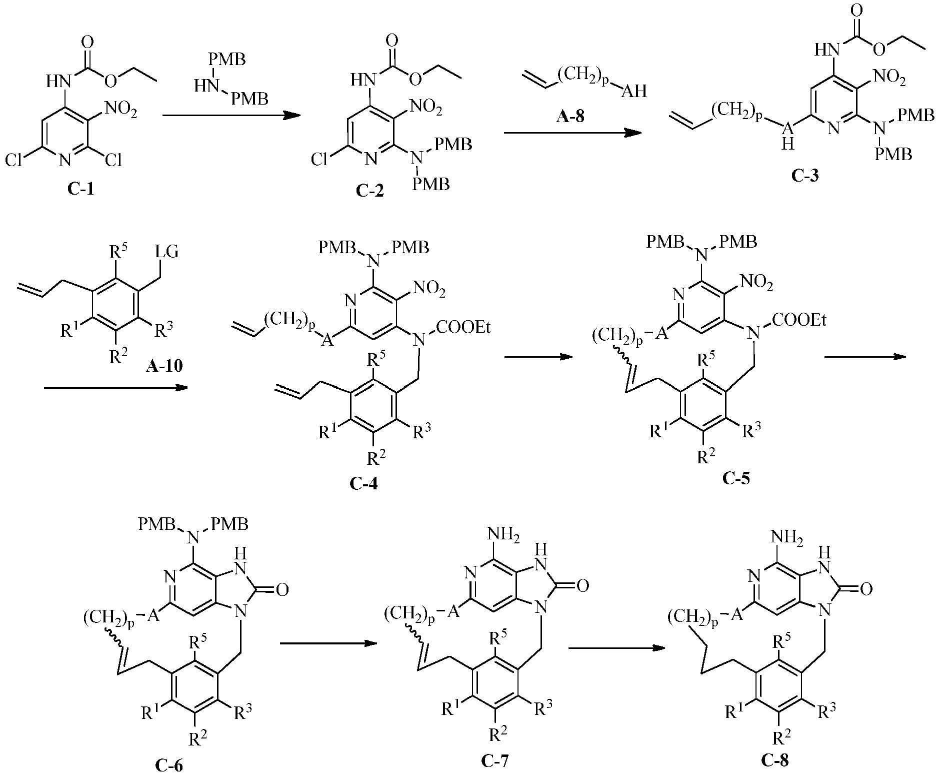

In scheme 3, the initial **compound C-1** is aminated to obtain compound **C-2**; **C-2** and **A-8** are substituted to obtain compound **C-3**; **C-3** and **A-10** are substituted to obtain compound **C-4**; **C-4** is subjected to olefin metathesis to obtain macrocyclic compound **B-5**; **C-5** is subjected to one-step nitro reduction to obtain**C-6**; **C-6** is deprotected to obtain**C-7**; **C-7** is catalytic hydrogenated to obtain **C-8.**

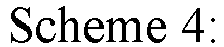

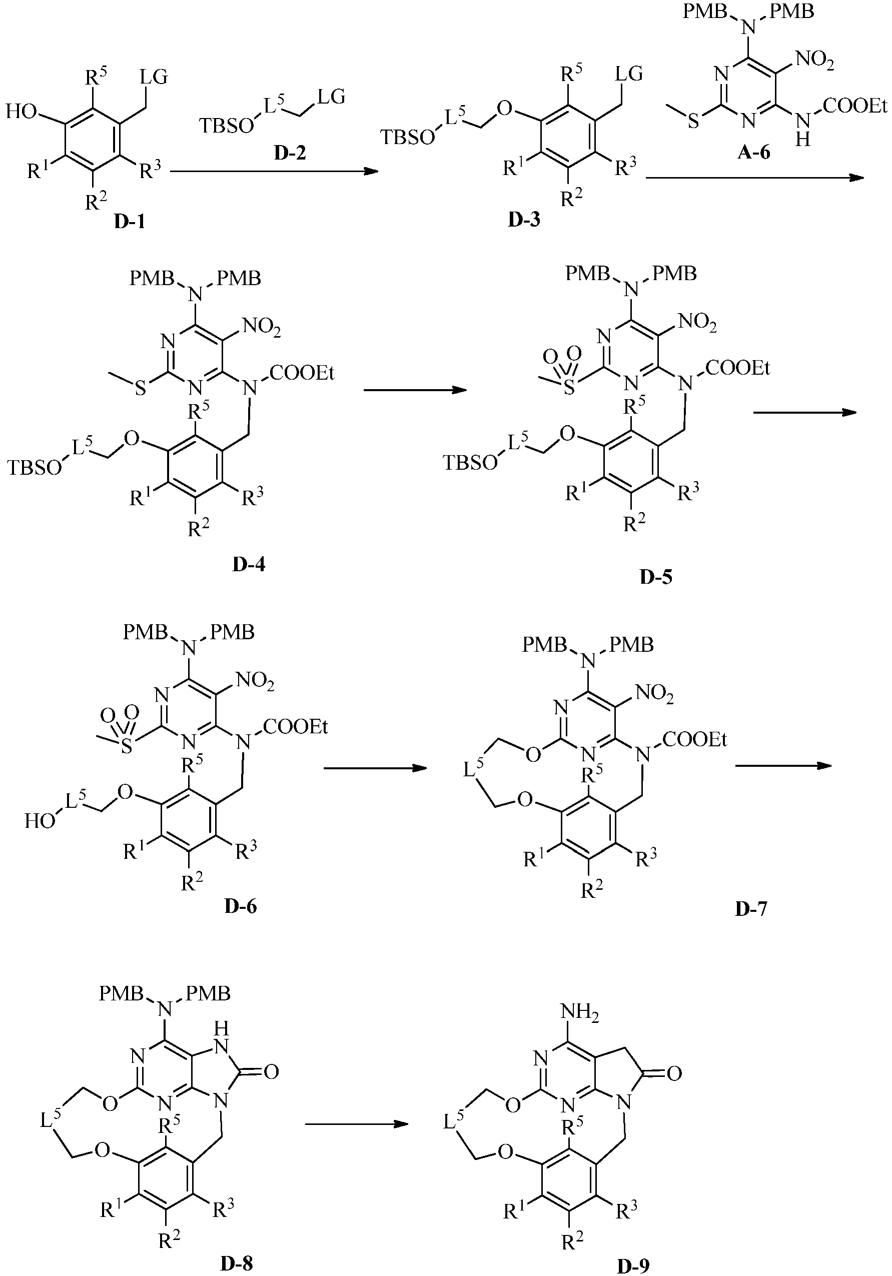

In scheme 4, the initial **compound D-1** and **compound D-2** are substituted to obtain compound **D-3**; **D-3** and **A-6** are substituted to obtain compound **D-4**; **D-4** is oxidized to obtain compound **D-5**; **D-5** is deprotected to obtain **compound D-6**; **D-6** undergoes substitution reaction to close the A-15 ring to obtain **D-7. D-7** closes the ring in one step through nitro reduction to obtain **D-8**;**D-9** is obtained by catalytic hydrogenation of **D-8.**

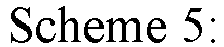

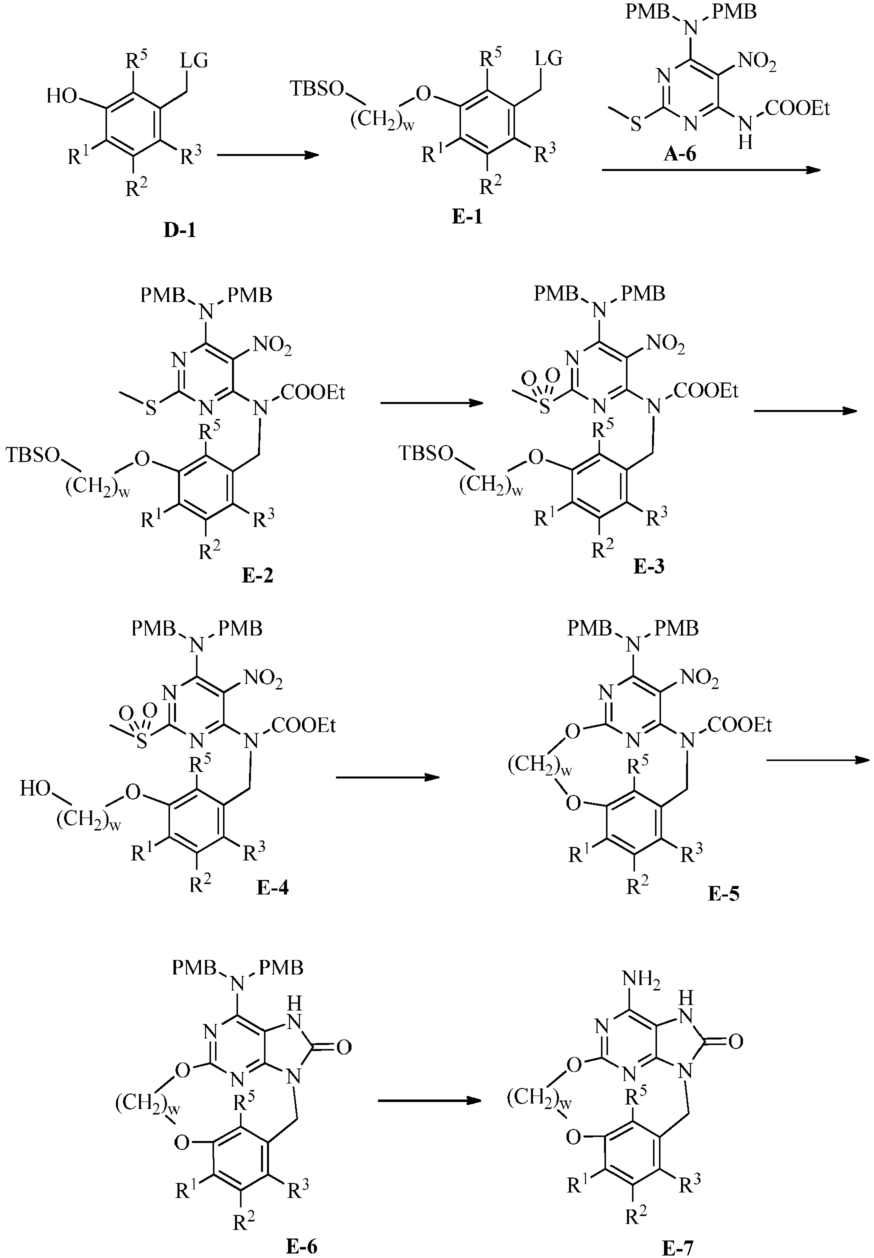

In scheme 5, the initial **compound D-1** is substituted to obtain compound **E-1**; **E-1** and **A-**6 are substituted to obtain compound **E-2**; **E-2** is oxidized to obtain compound **E-3**; **Compound E-4**is obtained by deprotecting **E-3**; **E-4** undergoes substitution reaction to close the ring to obtain **E-5**; **E-5** undergoes nitro reduction to close the ring to obtain **E-6**; **E-6**undergoes catalytic hydrogenation to obtain **E-7.**

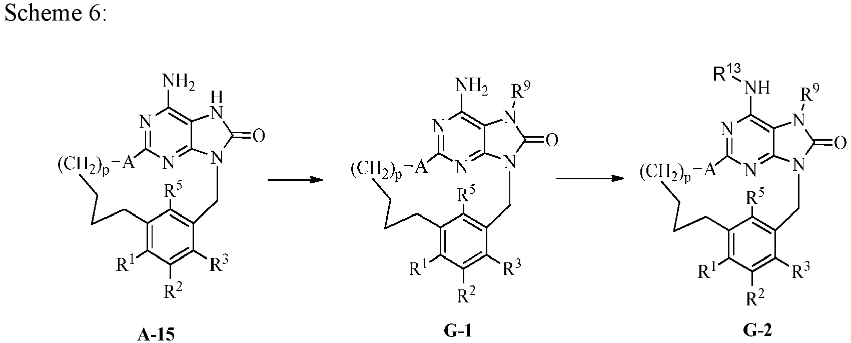

In scheme 6, **compound A-15** undergoes acylation reaction to generate compound **G-1**(the R⁹is CONR¹⁰R¹¹or C(=O)R¹²); **G-1** continue to undergo acylation reaction to generate compound **G-2** (R⁹is CONR¹⁰R¹¹or C(=O)R¹², R¹³is CONR¹⁴R¹⁵, C(=O)R¹⁶ or COOR¹⁷).

In the above schemes 1 to 6, LG is-OH, halogen, -OS(O)₂(C₁-C₄alkyl), and the definitions of each substituent in each compound are as described in the preceding items.

In the following examples, the structures of the compounds were determined by nuclear magnetic resonance (NMR) or/and mass spectrometry (MS). The NMR shift (δ) is given in units of 10⁻⁶(ppm). The NMR was determined by Bruker AVANCE-400 NMR. The solvents were deuterated dimethyl sulfoxide (DMSO-d₆), deuterated chloroform (CDCl₃), deuterated methanol (CD₃OD), and the internal standard was tetramethylsilane (TMS).

SHIMADZU LC system (column: Xselect O,R: CSH^{™} Prep-C18, 19* 150mm, liquid processor LH-40, pump LC-20AP, detector SPD-20A, system controller CBM-20A, solvent system: acetonitrile and 0.05% trifluoroacetic acid aqueous solution).

LC/MS spectra of the compounds were obtained using LC/MS(Agilent Technologies 1200 Series). The LC/MS conditions were as follows (10 min run time):
Acidic conditions: A:0.05% trifluoroacetic acid in water; B:0.05% trifluoroacetic acid in acetonitrile;
Alkaline conditions: A:0.05%NH₃•H₂O aqueous solution; B: acetonitrile
Neutral conditions: A: 10 mM NH₄OAC aqueous solution; B: acetonitrile

Unless otherwise specified, in the following examples, the intermediates and final compounds were purified using silica gel column chromatography, or using a XselectO,R-CSH^{™} Prep-C18(5 µm, OBD^{™} 19^{∗}150mm) column or using a XBridgeTM Prep Phenyl(5 µm, OBD^{™} 30* 100mm) on a reversed-phase chromatographic column by preparative HPLC.

Silica gel column chromatography generally uses Yantai Huanghai silica gel 200-300 mesh silica gel as the carrier.

The Combiflash Rf200(TELEDYNE ISCO) was used CombiFlash a rapid preparator.

Thin layer chromatography (TLC) silica gel plate uses Yantai Huanghai HSGF254 or Qingdao GF254 silica gel plate. The specifications of silica gel plate used for thin layer chromatography detection products are 0.15mm -0.2mm, and the specifications of thin layer chromatography separation and purification products are 0.4mm -0.5mm.

The known starting materials of the present invention can be synthesized using or following methods known in the art, or can be purchased from ABCR GmbH & Co.KG, Acros Organics, Aldrich Chemical Company, Shaoyuan Chemical Technology (Accela ChemBio Inc), Darry Chemicals and other companies.

### Abbreviations:

AczO: Acetic anhydride; AIBN: 2,2'-azobis(2-methylpropionitrile); BH₃: Borane; Boc₂O: tert-butyldicarbonate; CBr₄: Carbon tetrabromide; CH₃I (MeI): Iodomethane; con.H₂SO₄: con. sulfuric acid; con.HNO₃: con. nitric acid; Cs₂CO₃: Caesium carbonate; DCM: dichloromethane; DIAD: diisopropyl azodiformate; DIBAL-H: diisobutyl aluminium hydride; DIEA: N,N-diisopropylethylamine; DMAP: 4-Dimethylaminopyridine; DMF: N,N-dimethylformamide; DMSO: dimethyl sulfoxide; H₂SO₄: sulfuric acid; HOAc; Acetic Acid; K₂CO₃: Potassium carbonate; K₃PO₄: Tripotassium Orthophosphate; LiAlH₄: Lithium Aluminum Hydride; LiHMDS: Lithium bis(trimethylsilyl)amide; LiOH: Lithium hydroxide; mCPBA: 3-Chloroperbenzoic acid; MeOH: methanol; NaCNBH₃: Sodium cyanoborohydride; NaH: Sodium hydride; NaHCO₃: sodium bicarbonate; NBS: N-Bromosuccinimide; NIS: N-iodosuccinimide; PCy3: tricyclohexylphosphane; Pd(dppf)Cl₂: [1,1'-Bis(diphenylphosphino)ferrocene]dichloropalladium(II) ; Pd(OAc)₂: Palladium( II )acetate; Pd/C: Palladium 10% on Carbon; Pd₂(dba)₃: Tris(dibenzylideneacetone)dipalladium; POCl₃: phosphorus oxychloride; PPh₃: triphenylphosphine; SOCl₂: thionyl chloride; TBAF: Tetrabutylammonium fluoride; TBDPSCI: tert-Butyldiphenylchlorosilane; TBSCl: t-Butyldimethylchlorosilane; t-BuOK: potassium t-butoxide; TEA: triethylamine; TES: Triethylsilane; TFA: trifluoroacetic acid; TFAA: Trifluoroacetic anhydride; TfOH: Trifluoromethanesulfonic acid; THF: Tetrahydrofuran; TLC: thin layer chromatography; TMP: Trimethyl phosphate; XantPhos: 9,9-Dimethyl-4,5-bis(diphenylphosphino)xanthene; Zn: Zinc; ZnCl₂: Zinc chloride.

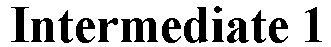

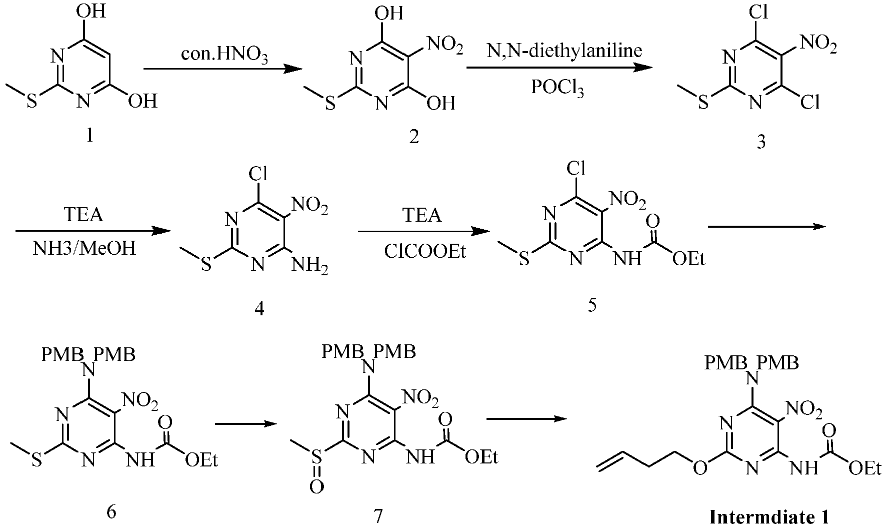

### Step 1: Preparation of compound 2

To a stirred solution of fuming nitric acid (70 mL) was added **compound 1** (25 g, 0.16 mol) at 0 °C, then the resulting mixture was stirred at 0 °C for 1 hour. After the reaction was completed, the reaction mixture was poured into ice-water and filtered, washed with water, ether and dried to afford **compound 2** (22 g, 68%) as a light brown solid. MS: 204.0 (M+H)⁺.

### Step 2: Preparation of compound 3

To a stirred suspension of **compound 2** (22 g, 0.11 mol) in POCl₃ (80 mL) was added N,N-diethylaniline (26mL) at 0 °C, then the resulting mixture was stirred at 110 °C for 1 hour. After the reaction was completed, the reaction mixture was concentrated in vacuo and the residue was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 3** (15 g, 58%) as a yellow oil.

### Step 3: Preparation of compound 4

To a stirred mixture solution of **compound 3** (15 g, 63 mmol) in THF (180 mL) was added TEA (19.8mL, 0.14 mol) and 7.0 M NH₃ solution in Methanol (9.9 mL, 69.3 mmol ) at 0 °C, then the resulting mixture was stirred at 20 °C for 3 hours. After the reaction was completed, the reaction mixture was concentrated in vacuo to afford **compound 4** (13 g). MS: 221.0 (M+H)⁺.

### Step 4: Preparation of compound 5

To a stirred mixture solution of **compound 4** (13 g, 590 mmol) and TEA (12g, 0.12 mol) in DCM (130 mL) was added Ethyl chloroformate (7.6 g, 71mmol) at 0 °C, then the resulting mixture was stirred at 20 °C for 6 hours. After the reaction was completed, the reaction mixture was concentrated in vacuo and the residue was extracted with DCM. The combined organic layers were washed with brine, dried over anhydrous Na₂SO₄, filtered and concentrated in vacuo to give a crude product, which was purified by silica gel flash chromatography to afford **compound 5** (15 g, 58%) as a yellow solid. MS: 293.2 (M+H)⁺. ¹HNMR (400 MHz, DMSO-*d₆*) δ 11.50 (s, 1H), 4.16 (q, J = 8 Hz, 2 H), 2.57 (s, 3H), 1.22 (t, J =8 Hz, 3H).

### Step 5: Preparation of compound 6

To a stirred mixture solution of **compound 5** (1 g, 3.42 mmol) in DCM (30 mL) was added TEA (0.69 g, 6.83 mmol) and bis (4-methoxybenzyl) amine (1.32 g, 5.12 mmol) at 0 °C, then the resulting mixture was stirred at 20 °C for 1 hour. After the reaction was completed, the reaction mixture was poured into ice-water and extracted with DCM. The combined organic layers were washed with sodium thiosulfate solution, brine, dried over anhydrous Na₂SO₄, filtered and concentrated in vacuo to give a crude product, which was purified by silica gel flash chromatography to afford **compound 6** (1.5 g, 91%) as a yellow oil. MS: 514.2 (M+H)⁺.

### Step 6: Preparation of compound 7

To a stirred mixture solution of **compound 6** (1.5 g, 2.92 mmol) in DCM (30 mL) was added mCPBA (0.75 g, 4.38 mmol) at 0 °C, then the resulting mixture was stirred at 20 °C for 1 hour. After the reaction was completed, the reaction mixture was quenched by adding ice-water and extracted with DCM. The combined organic layers were washed with a solution of NaHCO₃, brine, dried over anhydrous Na₂SO₄, filtered and concentrated in vacuo to give a crude product, which was purified by silica gel flash chromatography to afford **compound 7** (1.3 g, 82%) as a yellow oil. MS: 530.2 (M+H)⁺.

### Step 7: Preparation of Intermediate 1

To a stirred mixture solution of **compound 7** (700 mg, 1.28 mmol) in 3-butene-1-ol (5 mL) was added TEA (390mg, 3.85mmol) at 20 °C, then the resulting mixture was stirred at 80 °C for 1 hour. After the reaction was completed, the reaction mixture was concentrated in vacuo to give a crude product, which was purified by silica gel flash chromatography to afford **Intermediate 1** (500 mg, 74.4%) as a colorless oil. MS: 538.2 (M+H)⁺.

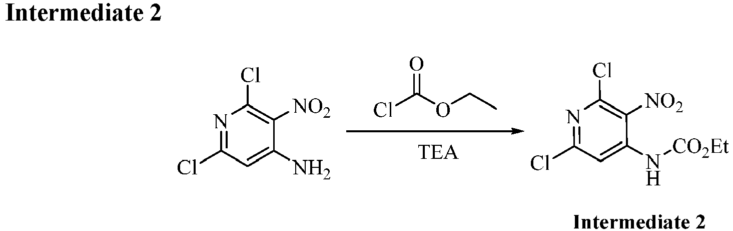

To a stirred mixture solution of 2,6-dichloro-3-nitropyridine-4-amine (10.0 g, 48.1 mmol) and TEA (7.30 g,72.1 mmol) in THF (30 mL) was added ethyl chloroformate (6.26 g, 57.7 mmol) at 0 °C, then the resulting mixture was stirred at 0 °C for 4 hours. After the reaction was completed, the reaction mixture was concentrated in vacuo and the residue was extracted with DCM. The combined organic layers were washed with brine, dried over anhydrous Na₂SO₄, filtered and concentrated in vacuo to give a crude product, which was purified by silica gel flash chromatography to afford **Intermediate 2** (10 g, 75%) as a yellow solid. MS: 280.1 (M+H)⁺.

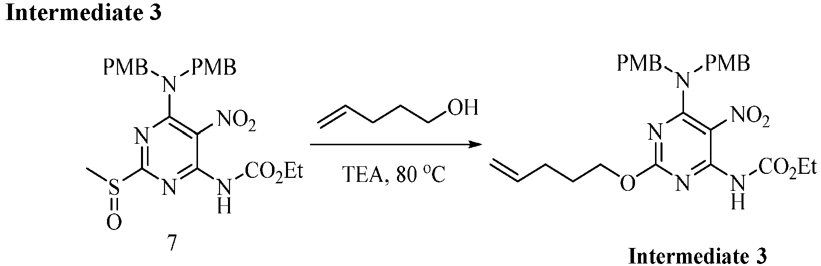

To a stirred mixture solution of **compound 7** (1.48 g, 2.8 mmol) in pent-4-en-1-ol (3 mL) was added TEA (565 mg, 5.6 mmol) at 20 °C, then the resulting mixture was stirred at 80 °C for 1 hour. After the reaction was completed, the reaction mixture was concentrated in vacuo to give a crude product, which was purified by silica gel flash chromatography to afford **Intermediate 3** (1.25 g, 81%) as a yellow oil. MS: 552.4 (M+H)⁺.

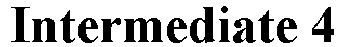

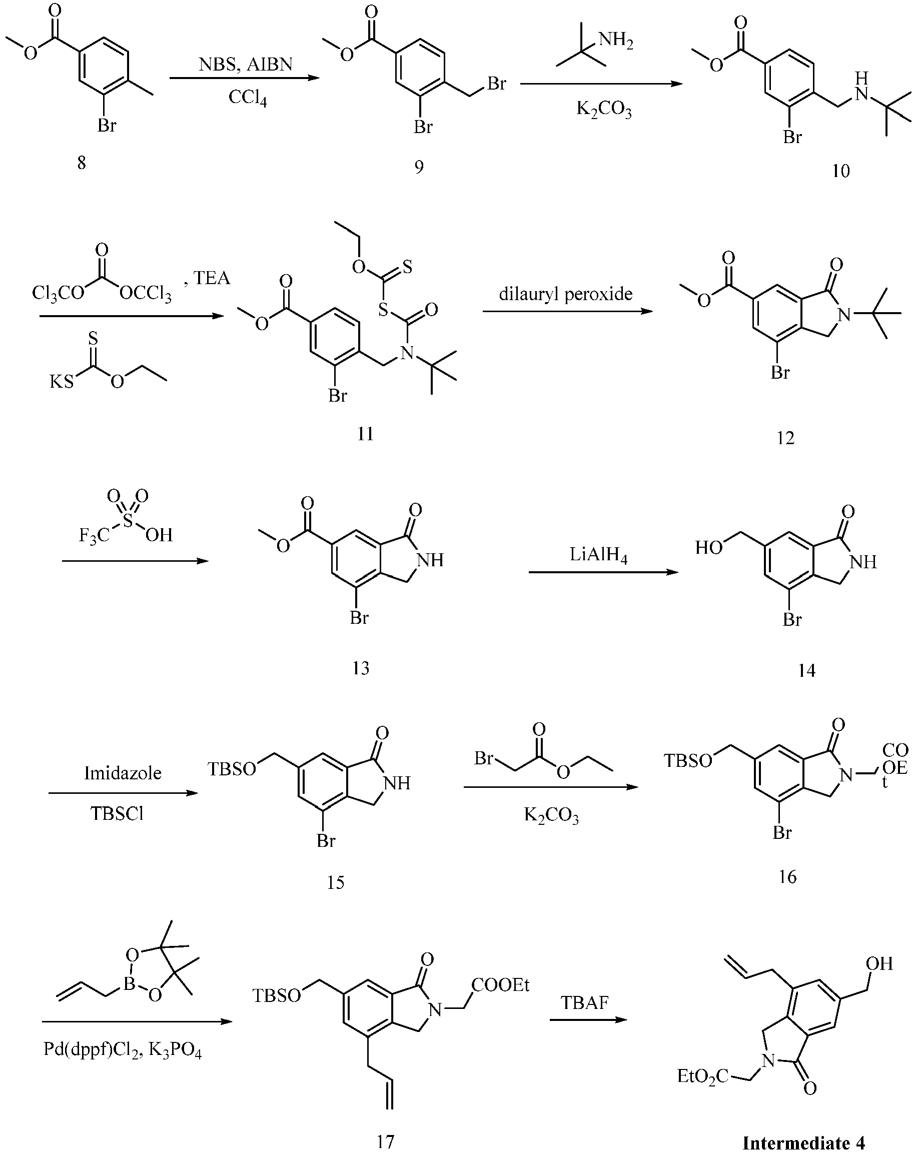

### Step 1: Preparation of compound 9

To a stirred mixture solution of **compound 8** (10 g, 43.7 mmol) in CCl₄ (250 mL) was added NBS (8.55 g, 48.0 mmol) and AIBN (1.43 g, 8.73 mmol) at 20 °C, then the resulting mixture was stirred at 80 °C for 14 hours. After the reaction was completed, the reaction mixture was concentrated in vacuo and the residue was extracted with DCM. The combined organic layers were washed with brine, dried over anhydrous Na₂SO₄, filtered and concentrated in vacuo to give a crude product, which was purified by silica gel flash chromatography to afford **compound 9** (7.5 g, 55.8%) as a white solid.

### Step 2: Preparation of compound 10

A mixture solution of **compound 9** (10 g, 32.5 mmol), 2-methylpropan-2-amine (4.75 g, 64.9 mmol) and K₂CO₃ (13.46 g, 97 mmol) in THF (100 mL) was stirred at 50 °C for 2 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 10** (6.6 g, 67.7%) as a yellow oil. MS: 300.1 (M+H)⁺.

### Step 3: Preparation of compound 11

To a stirred mixture solution of **compound 10** (0.70 g, 2.33 mmol) in DCM (15 mL) was added triphosgene (0.692 g, 2.33 mmol) and TEA (0.71 g, 7.00 mmol) at 0 °C, then the resulting mixture was stirred at 0 °C for 0.5 hour. After the reaction was completed, the reaction mixture was concentrated in vacuo and the residue was dissolved in ACN (15 mL) and potassium ethylxanthate (0.37 g, 2.33 mmol), then the resulting mixture was stirred at 20 °C for 1 hour . After the reaction was completed, the reaction mixture was quenched by adding ice-water and extracted with DCM. The combined organic layers were washed with brine, dried over anhydrous Na₂SO₄, filtered and concentrated in vacuo to give a crude product, which was purified by silica gel flash chromatography to afford **compound 11** (0.35 g, 33.5%) as a yellow solid.

### Step 4: Preparation of compound 12

A mixture solution of **compound 11** (2.7 g, 6.02 mmol) and dilauroyl peroxide (2.88 g, 7.23 mmol) in DCM (100 mL) was stirred at 50 °C for 4 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 12** (1.20 g, 61.1%) as a yellow solid. MS: 326.1 (M+H)⁺.

### Step 5: Preparation of compound 13

A mixture solution of **compound 12** (1.0 g, 3.1 mmol) in CF₃SO₃H (5.0 g, 33.3 mmol) was stirred at 50 °C for 2 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 13** (0.54 g, 62.2%) as a white solid. MS: 270.1 (M+H)⁺.

### Step 6: Preparation of compound 14

To a stirred mixture solution of **compound 13** (1.20 g, 4.44 mmol) in THF (10 mL) was added 2.5 M lithium aluminum tetrahydride solution in THF (1.76 mL, 4.4 mmol) at 0 °C, then the resulting mixture was stirred at 0 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 14** (0.7 g, 74.7%) as a white solid. MS: 242.1 (M+H)⁺.

### Step 7: Preparation of compound 15

To a stirred mixture solution of **compound 14** (1.70 g, 7.02 mmol) in DMF (20 mL) was added imidazole (1.43 g, 7.02 mmol) and TBSCl (2.12 g, 14.05 mmol) at 0 °C, then the resulting mixture was stirred at 20 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 15** (1.9 g, 76%) as a white solid. MS: 356.1 (M+H)⁺.

### Step 8: Preparation of compound 16

A mixture solution of **compound 15** (1.90 g, 5.33 mmol), ethyl 2-bromoacetate (1.34 g, 8.00 mmol) and K₂CO₃ (2.2 g, 16.0 mmol) in ACN (25 mL) was stirred at 50 °C for 4 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 16** (2.2 g, 93%) as a yellow solid. MS: 442.2 (M+H)⁺.

### Step 9: Preparation of compound 17

A mixture solution of **compound 16** (0.6 g, 1.36 mmol), water (3 mL), 4,4,5,5-tetramethyl-2-prop-2-enyl-1,3,2-dioxaborolane (0.68 g, 4.07 mmol), (dppf)Cl₂Pd (0.099 g, 0.14mmol) and potassium phosphate (0.86 g, 4.07 mmol) in Dioxane (15 mL) was stirred at 80 °C for 2 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 17** (0.4 g, 73.1%)as a white solid. MS: 404.3 (M+H)⁺.

### Step 10: Preparation of Intermediate 4

A mixture solution of **compound 17** (0.42 g, 1.04 mmol) and 1.0 M TBAF THF solution (1.04 mL, 1.04 mmol) in THF (10 mL) was stirred at 20 °C for 1 hour. After the reaction was completed, the reaction mixture was extracted with DCM. The combined organic layers were washed with brine, dried over anhydrous Na₂SO₄, filtered and concentrated in vacuo to give a crude product, which was purified by silica gel flash chromatography to afford **Intermediate 4** (0.22 g, 73.1%) as a white solid. MS: 290.2 (M+H)⁺.

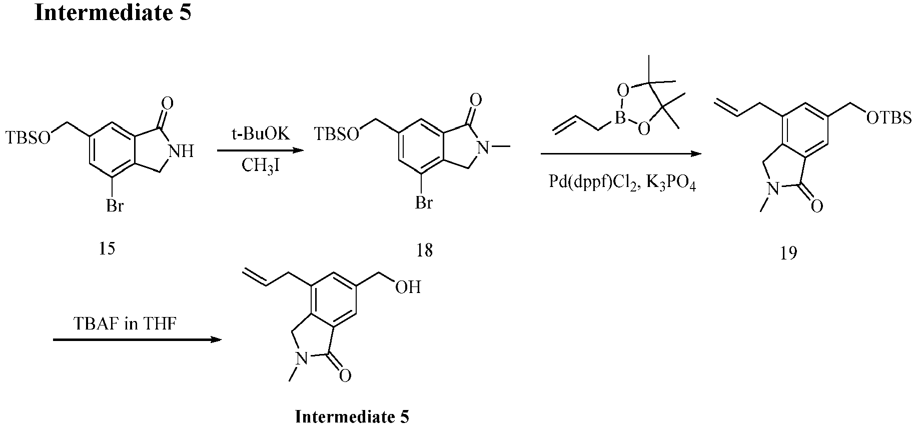

### Step 1: Preparation of compound 18

To a stirred mixture solution of **compound 15** (0.30 g, 1.11 mmol) in DMF (10 mL) was added t-BuOK (0.19 g, 1.67 mmol) at 0 °C, then the resulting mixture was stirred at 0 °C for 0.5 hour. Then CH₃I (0.32 g, 2.22mmol) was added to the above mixture solution and the resulting mixture was stirred at 20 °C for 2 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 18** (0.28 g, 89%) as a white solid. MS: 370.1 (M+H)⁺.

### Step 2: Preparation of compound 19

A mixture solution of **compound 18** (0.28 g, 0.76 mmol), water (1 mL), 4,4,5,5-tetramethyl-2-prop-2-enyl-1,3,2-dioxaborolane (0.38 g, 2.27 mmol), (dppf)Cl₂Pd (0.055 g, 0.076 mmol) and potassium phosphate (0.48 g, 2.27 mmol) in Dioxane (5 mL) was stirred at 80 °C for 2 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 19** (0.22 g, 88%)as a yellow solid. MS: 332.2 (M+H)⁺.

### Step 3: Preparation of Intermediate 5

A mixture solution of **compound 19** (0.22 g, 0.66 mmol) and 1.0 M TBAF THF solution (0.66 mL, 0.66 mmol) in THF (10 mL) was stirred at 20 °C for 1 hour. After the reaction was completed, the reaction mixture was extracted with DCM. The combined organic layers were washed with brine, dried over anhydrous Na₂SO₄, filtered and concentrated in vacuo to give a crude product, which was purified by silica gel flash chromatography to afford **Intermediate 5** (0.130g, 90%) as a yellow solid. MS: 218.2 (M+H)⁺.

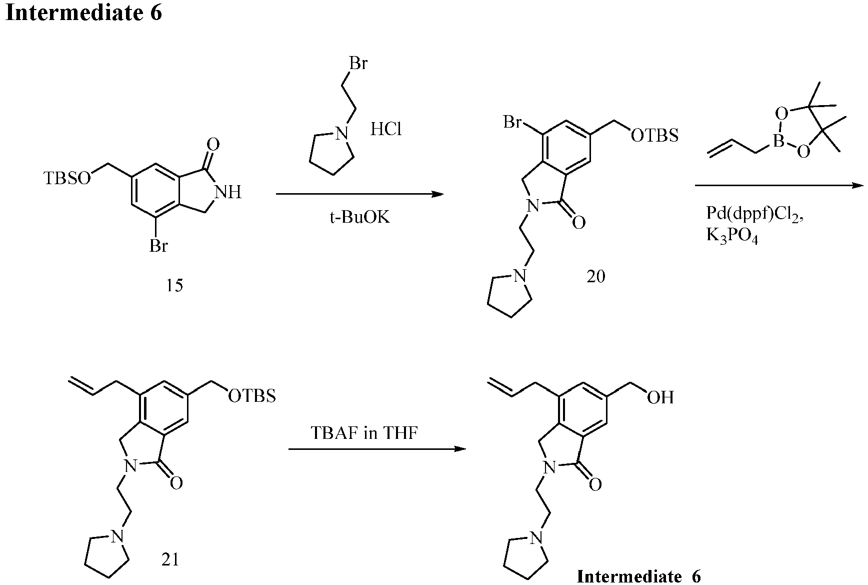

### Step 1: Preparation of compound 20

To a stirred mixture solution of **compound 15** (0.65 g, 1.82 mmol) in DMF (15 mL) was added t-BuOK (0.31 g, 2.74 mmol) at 0 °C, then the resulting mixture was stirred at 0 °C for 0.5 hour. Then 1-(2-bromoethyl)pyrrolidine (0.59 g, 2.74 mmol) was added to the above mixture solution and the resulting mixture was stirred at 20 °C for 2 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 20** (0.25 g, 30.2%) as a yellow solid. MS: 453.2 (M+H)⁺.

### Step 2: Preparation of compound 21

A mixture solution of **compound 20** (0.21 g, 0.46 mmol), water (2 mL), 4,4,5,5-tetramethyl-2-prop-2-enyl-1,3,2-dioxaborolane (233 mg, 1.39 mmol), (dppf)Cl₂Pd (33.9 mg, 0.046 mmol) and potassium phosphate (295 mg, 1.39 mmol) in Dioxane (10 mL) was stirred at 80 °C for 2 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 21** (0.13 g, 67.7%) as a yellow solid. MS: 415.3 (M+H)⁺.

### Step 3: Preparation of Intermediate 6

A mixture solution of **compound 21** (200 mg, 0.48 mmol) and 1.0 M TBAF THF solution (0.48mL, 0.48mmol) in THF (5 mL) was stirred at 20 °C for 1 hour. After the reaction was completed, the reaction mixture was extracted with DCM. The combined organic layers were washed with brine, dried over anhydrous Na₂SO₄, filtered and concentrated in vacuo to give a crude product, which was purified by silica gel flash chromatography to afford **Intermediate 6** (0.081 g, 55.2%) as a yellow solid. MS: 301.2 (M+H)⁺.

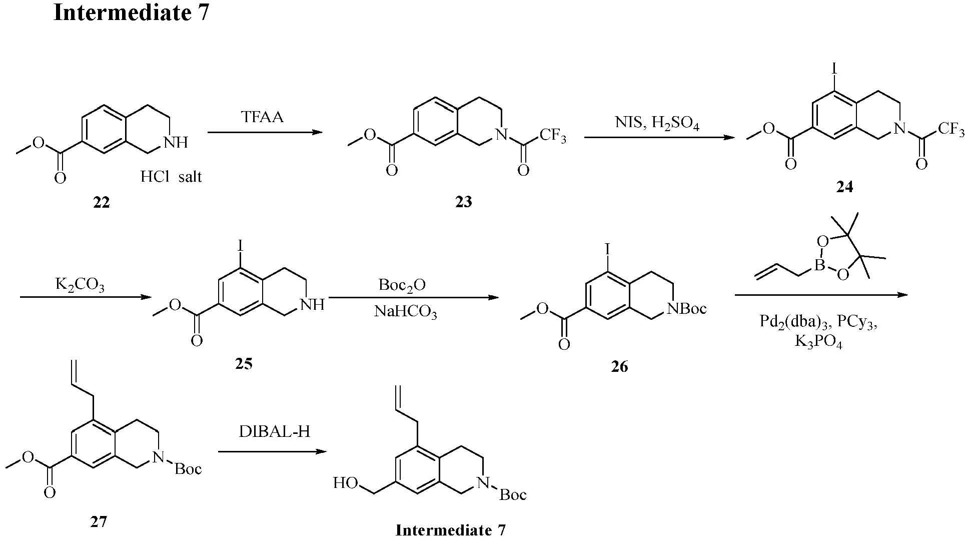

### Step 1: Preparation of compound 23

To a stirred mixture solution of **compound 22** (3.30 g, 14.49 mmol) in DCM (15 mL) was added TFAA (6.09 g, 29.0 mmol) at 0 °C, then the resulting mixture was stirred at 20 °C for 3 hours. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by silica gel flash chromatography to afford **compound 23** (4.0 g, 96%) as a colorless oil. MS: 288.1 (M+H)⁺.

### Step 2: Preparation of compound 24

To a stirred mixture solution of **compound 23** (5.01 g, 17.41 mmol) in TFA (40 mL) was added NIS (7.8 g, 34.8 mmol) and H₂SO₄ (0.368 g, 3.75 mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 24** (3.7 g, 51%) as a white solid. MS: 414.5 (M+H)⁺.

### Step 3: Preparation of compound 25

To a stirred suspension of **compound 24** (1.70 g, 4.11 mmol) in MeOH (20 mL) was added K₂CO₃ (1.37 g) and H₂O (2.5 mL) at 0 °C, then the resulting mixture was stirred at 20 °C for 3 hours. After the reaction was completed, the reaction mixture was poured into ice-water and extracted with DCM. The combined organic layers were washed with brine, dried over anhydrous Na₂SO₄, filtered and concentrated in vacuo to afford **compound 25**.

### Step 4: Preparation of compound 26

To a stirred mixture solution of **compound 25** (1.3 g, 4.11 mmol) in EA (20 mL) and H₂O (20 mL) was added a solution of BoczO (1.08 g, 4.93 mmol) and NaHCO₃ (464 mg, 5.1 mmol) in EA (20 mL) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 26** (1.5 g, 87%) as a colorless oil.

### Step 5: Preparation of compound 27

A mixture solution of **compound 26** (1.0 g, 2.4 mmol)), 4,4,5,5-tetramethyl-2-prop-2-enyl-1,3,2-dioxaborolane (4.03 g, 23.97 mmol), tris(dibenzylideneacetone)dipalladium (0.44 g, 0.48 mmol), tricyclohexylphosphane (0.13 g, 0.48 mmol) and potassium metaphosphate (1.5 g, 7.3 mmol) in DMF (10 mL) was stirred at 80 °C for 2 hours under N₂ protected. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 27** (700 mg, 88%) as a yellow oil.

### Step 6: Preparation of Intermediate 7

To a stirred mixture solution of **compound 27** (600 mg, 1.81 mmol) in THF (15 mL) was added 1.5 M DIBAL-H solution in toluene (3.62 mL, 5.43 mmol) at 0 °C, then the resulting mixture was stirred at 0 °C for 2 hours. After the reaction was completed, the reaction mixture was quenched by adding MeOH and extracted with EA. The combined organic layers were washed with brine, dried over anhydrous Na₂SO₄, filtered and concentrated in vacuo to give a crude product, which was purified by silica gel flash chromatography to afford **Intermediate 7** (130 mg, 23.6%) as a yellow oil.

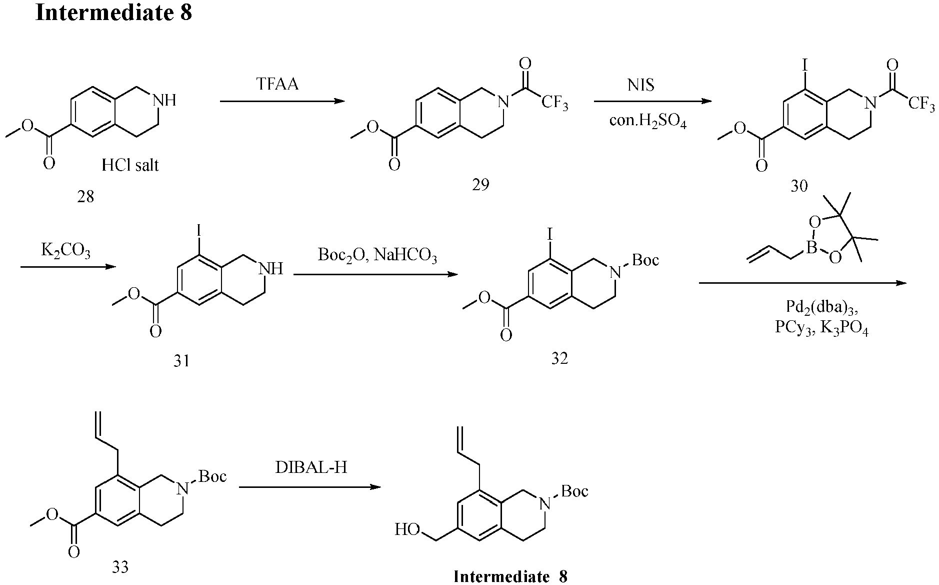

### Step 1: Preparation of compound 29

To a stirred mixture solution of **compound 28** (5.0 g, 22 mmol) in DCM (120 mL) was added TFAA (6.1 mL, 43.9 mmol) at 0 °C, then the resulting mixture was stirred at 20 °C for 12 hours. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by silica gel flash chromatography to afford **compound 29** (5.9 g, 93%) as a white solid. MS: 288.1 (M+H)⁺. ¹H-NMR (CDCl₃, 400 MHz): δ 7.91-7.86 (m, 2H), 7.24-7.19 (m, 1H), 4.82 (d, J = 20 Hz, 2H), 3.92 (s, 3H), 3.87 (t, J = 6.0 Hz, 2H), 3.03-2.99 (m, 2H).

### Step 2: Preparation of compound 30

To a stirred mixture solution of **compound 29** (5.89 g, 20.51 mmol) in TFA (40 mL) was added NIS (7.8 g, 34.8 mmol) and H₂SO₄ (4 mL) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 30** (7 g, 83%) as a white solid. ¹H-NMR (CDCl₃, 400 MHz): δ 8.39 (s, 1H), 7.83 (s, 1H), 4.69-4.68 (m, 2H), 3.92 (s, 3H), 3.90-3.82 (m, 2H), 3.01-2.95 (m, 2H).

### Step 3: Preparation of compound 31

To a stirred suspension of **compound 30** (7.0 g, 16.9 mmol) in MeOH (20 mL) was added K₂CO₃ (4.7 g) and H₂O (10 mL) at 0 °C, then the resulting mixture was stirred at 20 °C for 3 hours. After the reaction was completed, the reaction mixture was poured into ice-water and extracted with DCM. The combined organic layers were washed with brine, dried over anhydrous Na₂SO₄, filtered and concentrated in vacuo to afford **compound 31.** MS: 318.1 (M+H)⁺.

### Step 4: Preparation of compound 32

To a stirred mixture solution of **compound 31** (5.4 g, 16.9 mmol) in EA (60 mL) and H₂O (60 mL) was added a solution of BoczO (4.5 g, 20.3 mmol) and NaHCO₃ (1.7 g, 20 mmol) in EA (20 mL) at 0 °C, then the resulting mixture was stirred at 20 °C for 12 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 32** (5.8 g, 82%) as a colorless oil. MS: 403.1 (M-55+41)⁺. ¹H NMR (CDCl₃, 400 MHz) δ 8.34 (s, 1H) 7.79 (s, 1H), 4.47 (s, 2H), 3.91 (s, 3H), 3.63 (t, J = 5.6 Hz, 2H), 2.85 (t, J = 5.6 Hz, 2H), 1.50 (s, 9H).

### Step 5: Preparation of compound 33

A mixture solution of **compound 32** (1.0 g, 2.4 mmol), 4,4,5,5-tetramethyl-2-prop-2-enyl-1,3,2-dioxaborolane (4.0 g, 24 mmol), tris(dibenzylideneacetone)dipalladium (0.44 g, 0.48 mmol), tricyclohexylphosphane (0.13 g, 0.48 mmol) and potassium metaphosphate (1.5 g, 7.3 mmol) in DMF (15 mL) was stirred at 80 °C for 2 hours under N₂ protected. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 33** (608 mg, 77%) as a yellow oil. MS: 317.2 (M-55+41)⁺.

### Step 6: Preparation of Intermediate 8

To a stirred mixture solution of **compound 33** (1.1 g, 3.2 mmol) in THF (40 mL) was added 1.5 M DIBAL-H solution in toluene (6.4 mL, 9.6 mmol) at -10 °C, then the resulting mixture was stirred at -10 °C for 0.5 hour. After the reaction was completed, the reaction mixture was quenched by adding a solution of NH₄Cl and extracted with EA. The combined organic layers were washed with brine, dried over anhydrous Na₂SO₄, filtered and concentrated in vacuo to give a crude product, which was purified by silica gel flash chromatography to afford **Intermediate 8** (480 mg, 49%) as a colorless oil. ¹H NMR (CDCl₃, 400 MHz) δ 7.03 (s, 2H), 5.97-5.87 (m, 1H), 5.11-5.03 (m, 2H), 4.63 (s, 2H), 4.53 (s, 2H), 3.61 (t, J = 5.6 Hz, 2H), 3.33 (d, J = 5.6 Hz, 2H), 2.84 (t, J = 5.6 Hz, 2H), 1.48 (s, 9H).

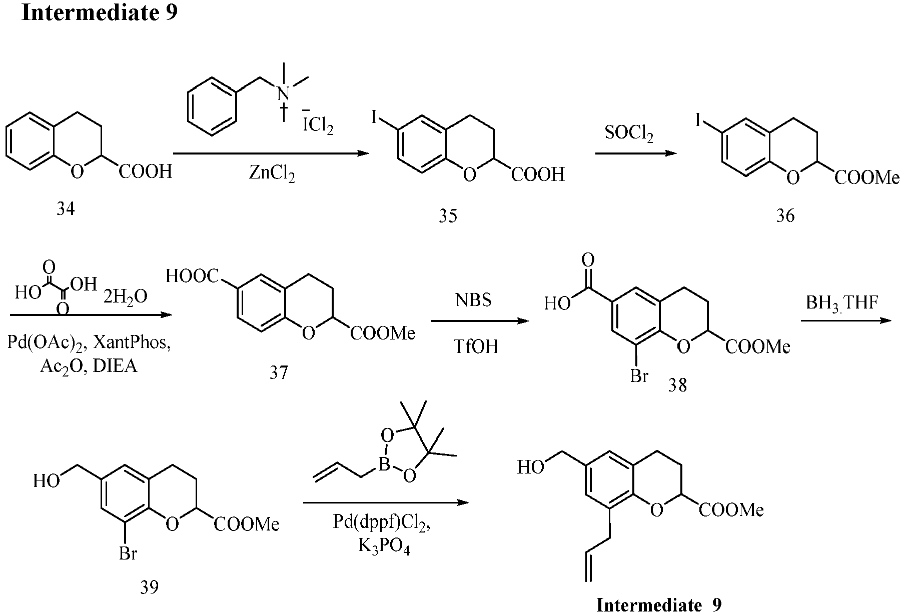

### Step 1: Preparation of compound 35

To a stirred mixture solution of **compound 34** (5.0 g, 28.1 mmol) in AcOH (100 mL) was added benzyltrimethylammonium dichloroiodate (11.72 g, 33.7 mmol) and ZnCh (4.59 g, 33.7 mmol) at 0 °C, then the resulting mixture was stirred at 20 °C for 15 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 35** (5.8 g) as a yellow solid. MS: 303.0 (M-H)⁻.

### Step 2: Preparation of compound 36

To a stirred mixture solution of **compound 35** (5.8 g, 19.07 mmol) in MeOH (50 mL) was added SOCl₂ (4.54 g, 38.1 mmol) at 0 °C, then the resulting mixture was stirred at 20 °C for 1 hour. After the reaction was completed, the reaction mixture was concentrated in vacuo and extracted with DCM. The combined organic layers were washed with brine, dried over anhydrous Na₂SO₄, filtered and concentrated in vacuo to give a crude product, which was purified by silica gel flash chromatography to afford **compound 36** (5.2 g, 86%) as a white solid. ¹H NMR (400 MHz, DMSO-*d₆*) δ 7.43-7.34 (m, 2H), 6.65 (d, J = 8.5 Hz, 1H), 4.92 (dd, J = 6.4, 4.0 Hz, 1H), 3.66 (s, 3H), 2.82-2.72 (m, 1H), 2.64-2.54 (m, 1H), 2.19-1.96 (m, 2H).

### Step 3: Preparation of compound 37

In a sealed tube, to a stirred mixture solution of **compound 36** (4 g, 12.57 mmol), oxalic acid (3.2 g, 12.57 mmol), Pd(OAc)₂ (282 mg, 1.26 mmol) and Xantphos (28 mg, 1.26 mmol) in DMF (20 mL) was added DIEA (3.25 g, 25.1 mmol) and AczO (2.57 g, 25.1 mmol) at 0 °C, then the resulting mixture was stirred at 100 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 37** (2.5 g, 84%) as a white solid. ¹H NMR (400 MHz, DMSO-*d₆*) δ 12.54 (s, 1H), 7.67 (dt, J = 4.9, 2.1 Hz, 2H), 6.89 (d, J = 9.0 Hz, 1H), 5.02 (dd, J = 6.3, 4.1 Hz, 1H), 3.69 (s, 3H), 2.83 (dt, J = 16.8, 6.2 Hz, 1H), 2.65 (dt, J = 16.3, 7.1 Hz, 1H), 2.22-2.01 (m, 2H).

### Step 4: Preparation of compound 38

To a stirred mixture solution of **compound 37** (3.8 g, 16.09 mmol) in TFA (50 mL) was added NBS (1.1 g, 19.3 mmol) and CF₃SO₃H (0.24 g, 1.61 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 38** (3.0 g, 59.2%) as a yellow solid. ¹H NMR (400 MHz, DMSO-*d₆*) δ 12.88 (s, 1H), 7.87 (d, J = 2.1 Hz, 1H), 7.65 (s, 1H), 5.20-5.16 (m, 1H), 3.68 (s, 3H), 2.85 (dt, J = 17.0, 5.8 Hz, 1H), 2.65 (dt, J = 16.2, 7.5 Hz, 1H), 2.10-2.20 (m, , 2H).

### Step 5: Preparation of compound 39

To a stirred mixture solution of **compound 38** (1 g, 3.17 mmol) in THF (10 mL) was added 1.0 M BH₃-THF solution (9.5mL, 9.52mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 39** (600 mg, 62.8%) as a colorless oil. ¹H NMR (400 MHz, DMSO-*d₆*) δ 7.32 (s, 1H), 6.99 (s, 1H), 5.13 (t, J = 5.7 Hz, 1H), 5.07 (t, J = 5.0 Hz, 1H), 4.36 (d, J = 5.7 Hz, 2H), 3.36 (s, 3H), 2.79 (dt, J = 16.9, 5.8 Hz, 1H), 2.60 (m, 1H), 2.22-2.03 (m, 2H).

### Step 6: Preparation of Intermediate 9

A mixture solution of **compound 39** (1.8 g, 5.98 mmol), 4,4,5,5-tetramethyl-2-prop-2-enyl-1,3,2-dioxaborolane (3.0 g, 17.9 mmol), Pd(dppf)Cl₂ (0.44 g, 0.6 mmol) and potassium metaphosphate (3.81 g, 17.93 mmol) in Dioxane (16 mL) and water (3.2 mL) was stirred at 80 °C for 4 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **Intermediate 9** (1.2 g, 77%) as a yellow oil. ¹H NMR (400 MHz, DMSO-*d₆*) δ 6.88 (s, 1H), 6.84 (s, 1H), 6.05-5.89 (m, 1H), 5.10-4.89 (m, 3H), 4.33 (s, 2H), 4.32-4.30 (m, 1H), 3.68 (s, 3H), 3.29 (q, J = 7.6, 6.7 Hz, 2H), 2.75 (dt, J = 16.6, 6.1 Hz, 1H), 2.50-2.60 (m, , 1H), 2.20-1.98 (m, 2H).

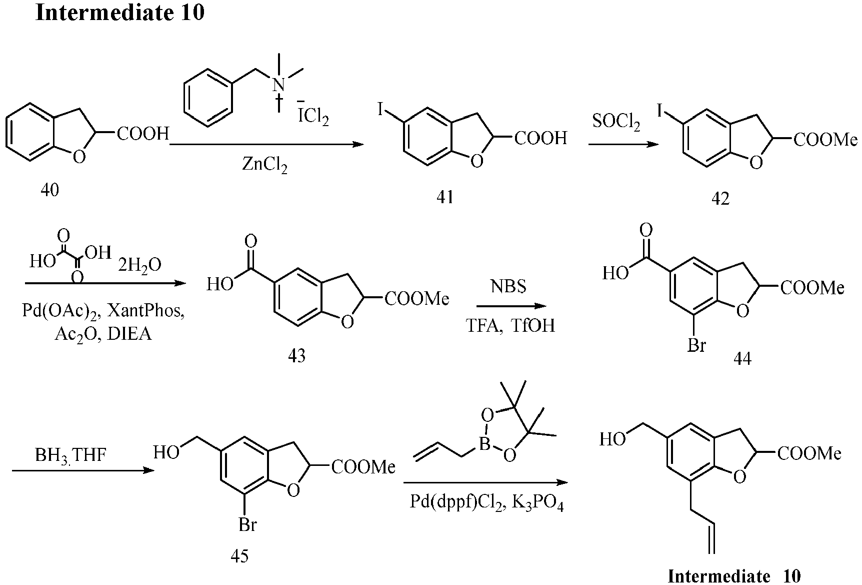

### Step 1: Preparation of compound 41

To a stirred mixture solution of **compound 40** (5.0 g, 30.5 mmol) in AcOH (50 mL) was added benzyltrimethylammonium dichloroiodate (12.7 g, 36.5 mmol) and ZnCh (4.98 g, 36.5 mmol) at 0 °C, then the resulting mixture was stirred at 20 °C for 15 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 41** (6.5 g) as a yellow solid. MS: 289.0 (M-H)⁻.

### Step 2: Preparation of compound 42

To a stirred mixture solution of **compound 41** (1 g, 3.45 mmol) in MeOH (10 mL) was added SOCl₂ (1.23 g, 10.34 mmol) at 0 °C, then the resulting mixture was stirred at 20 °C for 1 hour. After the reaction was completed, the reaction mixture was concentrated in vacuo and extracted with DCM. The combined organic layers were washed with brine, dried over anhydrous Na₂SO₄, filtered and concentrated in vacuo to give a crude product, which was purified by silica gel flash chromatography to afford **compound 42** (0.9 g, 86%) as a white solid. ¹H NMR (400 MHz, DMSO-*d₆*) δ 7.54 (s, 1H), 7.43 (d, J = 8.4 Hz, 1H), 6.72 (d, J = 8.5 Hz, 1H), 5.36 (dd, J = 10.7, 6.0 Hz, 1H), 3.69 (s, 3H), 3.60-3.53 (m, 1H), 3.30 (d, J = 6.6 Hz, 1H).

### Step 3: Preparation of compound 43

In a sealed tube, to a stirred mixture solution of **compound 42** (4.8 g, 15.8 mmol), oxalic acid (2.84 g, 31.6 mmol), Pd(OAc)₂ (0.18 g, 0.79 mmol) and Xantphos (0.91 g, 1.58 mmol) in DMF (50 mL) was added DIEA (4.1 g, 31.6 mmol) and AczO (3.22 g, 31.6 mmol) at 0 °C, then the resulting mixture was stirred at 100 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 43** (3.0 g, 86%) as a yellow solid.

### Step 4: Preparation of compound 44

To a stirred mixture solution of **compound 43** (3.1 g, 13.95 mmol) in TFA (50 mL) was added NBS (1.1 g, 19.3 mmol) and CF₃SO₃H (2.09 g, 13.95 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 44** (2.4 g, 57.1%) as a yellow solid. ¹H NMR (400 MHz, DMSO-*d₆*) δ 12.93 (s, 1H), 7.88 (s, 1H), 7.77 (s, 1H), 5.57 (dd, J = 10.8, 6.3 Hz, 1H), 3.76-3.69 (m, 4H), 3.45 (dd, J = 16.5, 6.4 Hz, 1H).

### Step 5: Preparation of compound 45

To a stirred mixture solution of **compound 44** (2.4 g, 7.97 mmol) in THF (20 mL) was added 1.0 M BH₃-THF solution (15.9 mL, 15.94 mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 45** (1.1 g, 63.1%) as a colorless oil. ¹H NMR (400 MHz, DMSO-*d₆*) δ 7.25 (s, 1H), 7.14 (s, 1H), 5.47-5.39 (m, 1H), 5.15 (td, J = 5.8, 1.8 Hz, 1H), 4.38 (d, J = 5.8 Hz, 2H), 3.71 (s, 3H), 3.69-3.61 (m, 1H), 3.37 (dd, J = 16.3, 6.2 Hz, 1H).

### Step 6: Preparation of Intermediate 10

A mixture solution of **compound 45** (1.1 g, 3.83 mmol), 4,4,5,5-tetramethyl-2-prop-2-enyl-1,3,2-dioxaborolane (1.93 g, 11.49 mmol), Pd(dppf)Cl₂ (0.28 g, 0.38 mmol) and potassium metaphosphate (2.44 g, 11.49 mmol) in Dioxane (10 mL) and water (2 mL) was stirred at 80 °C for 4 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **Intermediate 10** (0.6 g, 77%) as a yellow oil. ¹H NMR (400 MHz, DMSO-*d₆*) δ 7.01 (s, 1H), 6.88 (s, 1H), 5.94 (ddt, J = 16.9, 10.1, 6.7 Hz, 1H), 5.33 (dd, J = 10.6, 5.7 Hz, 1H), 5.09-4.98 (m, 3H), 4.36 (d, J = 4.8 Hz, 2H), 3.68 (d, J = 1.7 Hz, 3H), 3.54 (dd, J = 16.1, 10.6 Hz, 1H), 3.27 (d, J = 6.5 Hz, 3H).

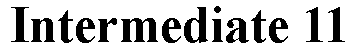

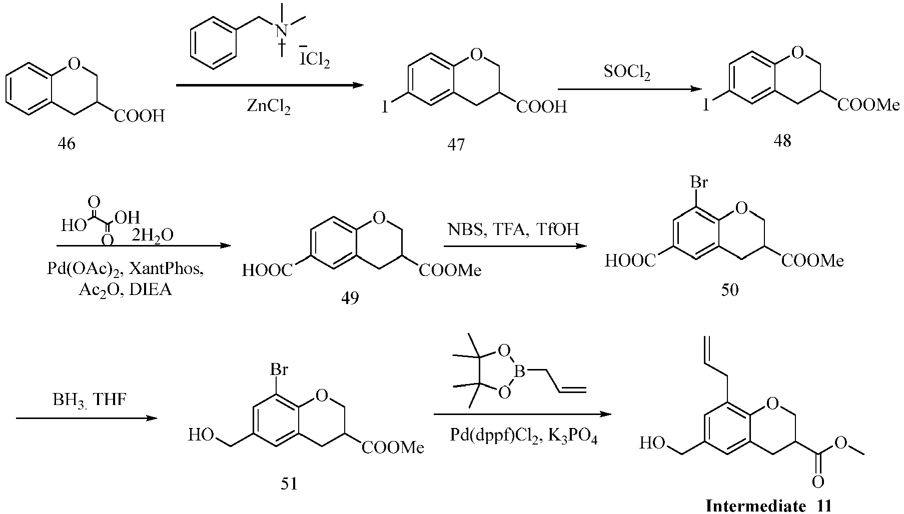

### Step 1: Preparation of compound 47

To a stirred mixture solution of **compound 46** (4.50 g, 25.3 mmol) in AcOH (100 mL) was added benzyltrimethylammonium dichloroiodate (10.57 g, 30.4 mmol) and ZnCh (4.13 g, 30.4 mmol) at 0 °C, then the resulting mixture was stirred at 20 °C for 15 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 47** (5.0 g) as a yellow solid. MS: 303.0 (M-H)⁻.

### Step 2: Preparation of compound 48

To a stirred mixture solution of **compound 47** (5.0 g, 16.45 mmol) in MeOH (50 mL) was added SOCl₂ (3.74 g, 32.89 mmol) at 0 °C, then the resulting mixture was stirred at 20 °C for 1 hour. After the reaction was completed, the reaction mixture was concentrated in vacuo and extracted with DCM. The combined organic layers were washed with brine, dried over anhydrous Na₂SO₄, filtered and concentrated in vacuo to give a crude product, which was purified by silica gel flash chromatography to afford **compound 48** (4.5 g, 86%) as a white solid.

### Step 3: Preparation of compound 49

In a sealed tube, to a stirred mixture solution of **compound 48** (4.5 g, 14.15 mmol), oxalic acid (3.56 g, 28.3 mmol), Pd(OAc)₂ (317 mg, 1.4 mmol) and Xantphos (816 mg, 1.4 mmol) in DMF (20 mL) was added DIEA (2.7 g, 21.22 mmol) and AczO (2.2 g, 21.22 mmol) at 0 °C, then the resulting mixture was stirred at 100 °C for 1 hour. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 49** (3.3 g, 98%) as a white solid.

### Step 4: Preparation of compound 50

To a stirred mixture solution of **compound 49** (3.3 g, 13.98 mmol) in TFA (50 mL) was added NBS (2.7 g, 15.38 mmol) and CF₃SO₃H (0.21 g, 1.4 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 50** (3.7 g, 76%) as a yellow solid.

### Step 5: Preparation of compound 51

To a stirred mixture solution of **compound 50** (2 g, 6.35 mmol) in THF (10 mL) was added 1.0 M BH₃-THF solution (12.7 mL, 12.69 mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 51** (740 mg, 38.7%) as a colorless oil. MS: 283.1 (M-H₂O)⁺.

### Step 6: Preparation of Intermediate 11

A mixture solution of **compound 51** (740 mg, 2.46 mmol), 4,4,5,5-tetramethyl-2-prop-2-enyl-1,3,2-dioxaborolane (2.07 g, 12.30 mmol), Pd(dppf)Cl₂ (0.18 g, 0.246 mmol) and potassium metaphosphate (1.04 g, 4.92 mmol) in Dioxane (10 mL) and water (2 mL) was stirred at 80 °C for 4 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **Intermediate 11** (0.61 g, 95%) as a colorless oil. MS: 245.2 (M-H₂O)⁺.

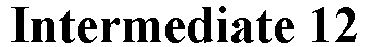

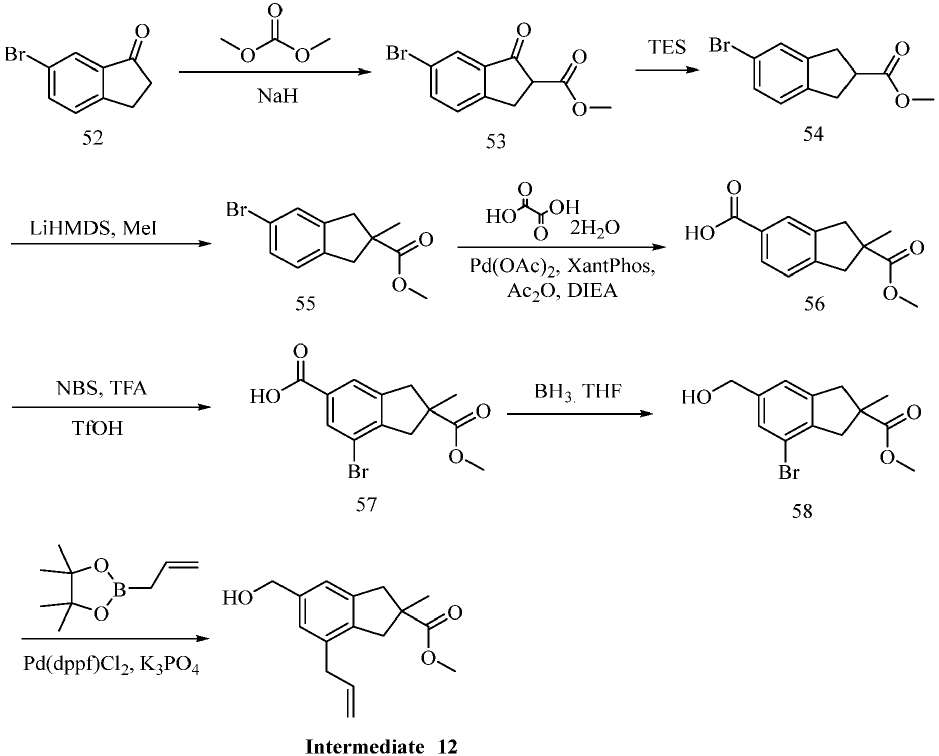

### Step 1: Preparation of compound 53

A mixture solution of **compound 52** (10.0 g, 47.4 mmol), NaH (5.7 g, 142.2 mmol) anddimethyl carbonate (6.4 g, 71.1 mmol) in THF (80 mL) was stirred at 65 °C for 1 hour under N₂ protected. After the reaction was completed, the reaction mixture was poured into ice-water and extracted with EA. The combined organic layers were washed with 3M HCl solution, brine, dried over anhydrous Na₂SO₄, filtered and concentrated in vacuo to give a crude product, which was purified by silica gel flash chromatography to afford **compound 53** (9.5 g, 74%).

### Step 2: Preparation of compound 54

A mixture solution of **compound 53** (4.5 g, 16.8 mmol) and Et₃SiH (11.7 g, 100.7 mmol) in TFA (70 mL) was stirred at 80 °C for 1 hour under N₂ protected. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by silica gel flash chromatography to afford **compound 54** (2.5 g, 58.8%) as a light brown solid. MS: 255.2 (M+H)⁺.

### Step 3: Preparation of compound 55

To a stirred mixture solution of **compound 54** (2.5 g, 9.33 mmol) in THF (40 mL) was added LiHMDS (1.0 M, 18.66 mL, 18.7 mmol) at -78 °C, then the resulting mixture was stirred at -40°C for 1 hour. CH₃I (10 mmol) was added to the above mixture solution at -78 °C and the resulting mixture was stirred at -40°C for 1 hour. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 55** (1.5 g, 63 %) as a white solid. MS: 270.1 (M+H)⁺

### Step 4: Preparation of compound 56

In a sealed tube, to a stirred mixture solution of **compound 55** (2.0 g, 7.46 mmol), oxalic acid (1.88 g, 14.93 mmol), Pd(OAc)₂ (167 mg, 0.75 mmol) and Xantphos (432 mg, 0.75 mmol) in DMF (35 mL) was added DIEA (1.45 g, 11.19 mmol) and AczO (1.14 g, 11.19 mmol) at 0 °C, then the resulting mixture was stirred at 100 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 56** (1.15 g, 66%) as a white solid. MS: 235.2 (M+H)⁺.

### Step 5: Preparation of compound 57

To a stirred mixture solution of **compound 56** (800 mg, 3.42 mmol) in TFA (10 mL) was added NBS (730 mg, 4.1 mmol) and CF₃SO₃H (0.05 mL) at 0 °C, then the resulting mixture was stirred at 10 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 57** (750 mg, 70%). MS: 314.3 (M+H)⁺.

### Step 6: Preparation of compound 58

To a stirred mixture solution of **compound 57** (750 mg, 2.4 mmol) in THF (10 mL) was added 1.0 M BH₃-THF solution (12 mL, 12 mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 58** (650 mg) as a colorless oil.

### Step 7: Preparation of Intermediate 12

A mixture solution of **compound 58** (650 mg, 2.17 mmol), 4,4,5,5-tetramethyl-2-prop-2-enyl-1,3,2-dioxaborolane (730 mg, 4.35 mmol), Pd(dppf)Cl₂ (159 mg, 0.22 mmol) and potassium metaphosphate (923 mg, 4.35 mmol) in Dioxane (20 mL) and water (2 mL) was stirred at 100 °C for 2 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **Intermediate 12** (500 mg, 88%) as a colorless oil. MS: 261.2 (M+H)⁺.

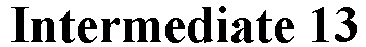

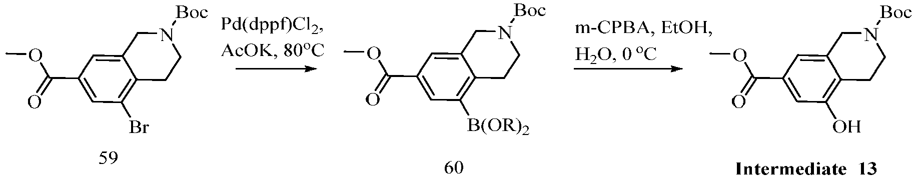

### Step 1: Preparation of compound 60

A mixture solution of **compound 59** (10 g, 27.0 mmol), bis(pinacolato)diboron (13.72 g, 54.0mmol), Pd(dppf)Cl₂ (1.98 g, 2.70 mmol) and potassium metaphosphate (7.95 g, 81 mmol) in Dioxane (200 mL) was stirred at 80 °C for 2 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 60** (10.5 g, 93%) as a yellow solid. MS: 418.3 (M+H)⁺.

### Step 2: Preparation of Intermediate 13

To a stirred mixture solution of **compound 60** (10.5 g, 25.2 mmol) in Ethanol (50 mL) and water (25 mL) was added mCPBA (5.2 g, 30.2 mmol) at 0 °C, then the resulting mixture was stirred at 20 °C for 1 hour. After the reaction was completed, the reaction mixture was quenched by adding ice-water and extracted with DCM. The combined organic layers were washed with a solution of NaHCO₃, brine, dried over anhydrous Na₂SO₄, filtered and concentrated in vacuo to give a crude product, which was purified by silica gel flash chromatography to afford **Intermediate 13** (7.5 g, 97%) as a white solid. MS: 308.2 (M+H)⁺.

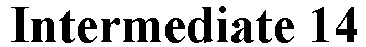

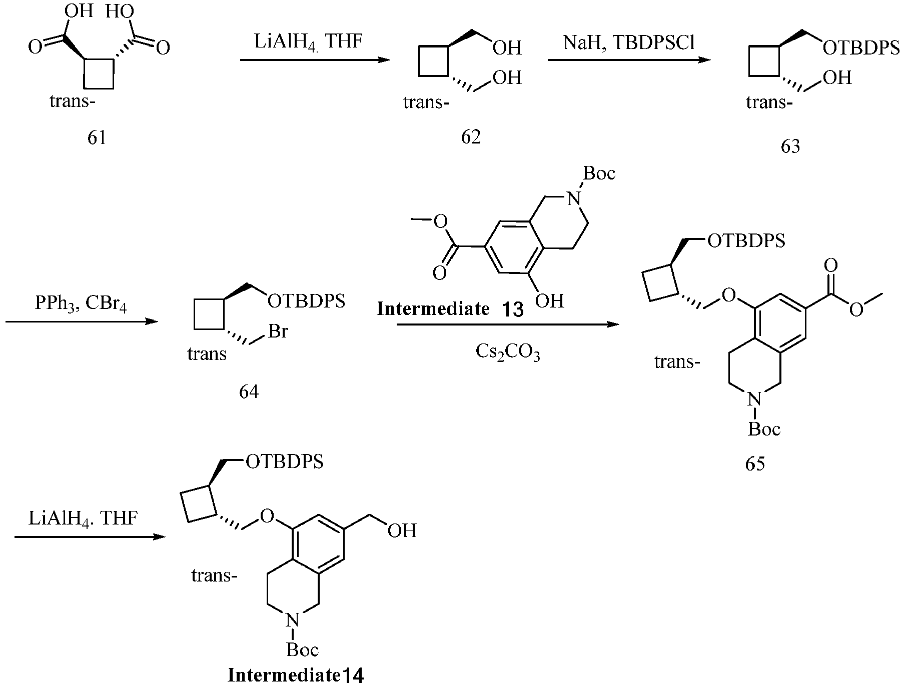

### Step 1: Preparation of compound 62

To a stirred mixture solution of **compound 61** *(Trans-,* 5.0 g, 4.7 mmol) in THF (50 mL) was added 2.5 M lithium aluminum tetrahydride solution in THF (69.2 mL, 173 mmol) at 0 °C, then the resulting mixture was stirred at 20 °C for 12 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 62** (2 g, 49.6%) as a colorless oil.

### Step 2: Preparation of compound 63

To a stirred mixture solution of **compound 62** (2.0 g) in THF (15 mL) was added NaH (124 mg, 5.2 mmol) at 0 °C, then the resulting mixture was stirred at 0 °C for 0.5 hour. Then TBDPSCl (1.2 g, 4.3 mmol) was added to the above mixture solution at 0 °C and the resulting mixture was stirred at 20 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 63** (110 mg, 72.1%) as a colorless oil.

### Step 3: Preparation of compound 64

To a stirred mixture solution of **compound 63** (3 g, 8.5 mmol) in DCM (50 mL) was added PPh₃ (3.3 g, 12.7 mmol) and CBr₄ (4.2 g, 12.7 mmol), then the resulting mixture was stirred at 20 °C for 2 hours. After the reaction was completed, the reaction mixture was quenched by adding ice-water and extracted with DCM. The combined organic layers were washed with brine, dried over anhydrous Na₂SO₄, filtered and concentrated in vacuo to give a crude product, which was purified by silica gel flash chromatography to afford **compound 64** (3.2 g, 91%) as a colorless oil. ¹H NMR (400 MHz, CDCl₃) δ 7.66 (d, J = 6.9 Hz, 4H), 7.41 (dd, J = 10.7, 7.0 Hz, 6H), 3.60-3.70(m, 2H), 3.49 (dd, J = 9.7, 6.5 Hz, 1H), 3.38 (t, J = 8.8 Hz, 1H), 2.56 (h, J = 8.0 Hz, 1H), 2.25 (d, J = 7.0 Hz, 1H), 2.03 (q, J = 11.1, 9.9 Hz, 1H), 1.85 (q, J = 10.0, 9.2 Hz, 1H), 1.60-1.80 (m, , 2H), 1.06 (s, 9H).

### Step 4: Preparation of compound 65

A mixture solution of **Intermdiate 13** (1 g, 3.25 mmol), **compound 64** (1.4 g, 3.3 mmol) and Cs₂CO₃ (2.1 g, 6.5 mmol) in DMF (10 mL) was stirred at 100 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 65** (1.4 g, 66.8%) as a colorless oil. MS: 644.4 (M+H)⁺.

### Step 5: Preparation of Intermediate 14

To a stirred mixture solution of **compound 65** (1.4 g, 2.2 mmol) in THF (20 mL) was added 2.5 M lithium aluminum tetrahydride solution in THF (0.87 mL, 2.2 mmol) at 0 °C, then the resulting mixture was stirred at 0 °C for 0.5 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **Intermdiate 14** (*Trans-,* 1.1 g, 82%) as a colorless oil. MS: 616.4 (M+H)⁺.

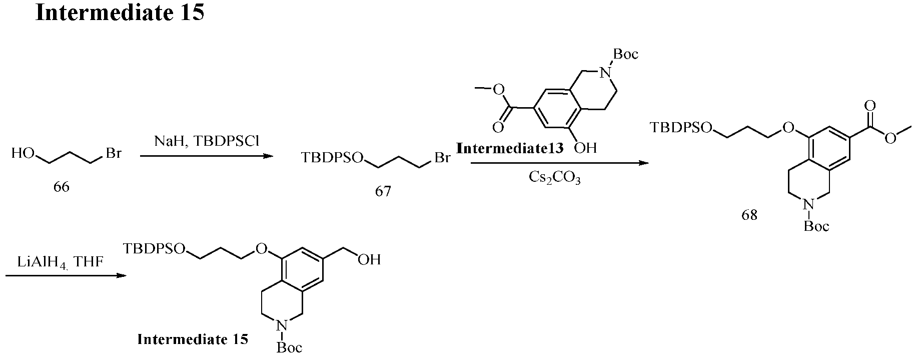

### Step 1: Preparation of compound 67

To a stirred mixture solution of **compound 66** (5.0 g, 36.0 mmol) in DMF (80 mL) was added NaH (1.72 g, 43.2 mmol) at 0 °C, then the resulting mixture was stirred at 20 °C for 0.5 hour. Then TBDPSCl (11.87 g, 43.2 mmol) was added to the above mixture solution at 0 °C and the resulting mixture was stirred at 20 °C for 2 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 67** (3.5 g, 25.8%) as a colorless oil.

### Step 2: Preparation of compound 68

A mixture solution of **Intermdiate B13** (1 g, 3.25 mmol), **compound 67** (1.2 g, 3.3 mmol) and Cs₂CO₃ (2.1 g, 6.5 mmol) in DMF (10 mL) was stirred at 100 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 68** (1.4 g, 73%) as a colorless oil. MS: 604.4 (M+H)⁺.

### Step 3: Preparation of Intermediate 15

To a stirred mixture solution of **compound 68** (1.4 g, 2.34 mmol) in THF (20 mL) was added 2.5 M lithium aluminum tetrahydride solution in THF (0.92 mL, 2.3 mmol) at 0 °C, then the resulting mixture was stirred at 0 °C for 0.5 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **Intermediate 15** (1.05 g, 82%) as a colorless oil. MS: 576.3 (M+H)⁺.

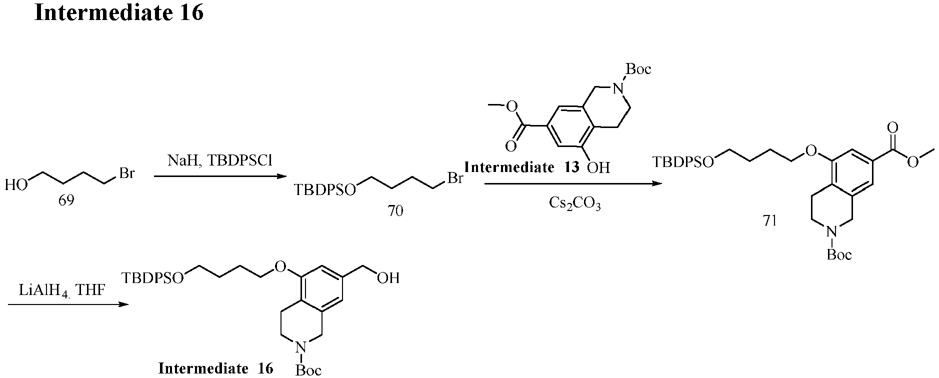

### Step 1: Preparation of compound 70

To a stirred mixture solution of **compound 69** (5 g, 32.3 mmol) in DMF (80 mL) was added NaH (1.6 g, 39.5 mmol) at 0 °C, then the resulting mixture was stirred at 20 °C for 0.5 hour. Then TBDPSCl (10.9 g, 39.5 mmol) was added to the above mixture solution at 0 °C and the resulting mixture was stirred at 20 °C for 2 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 70** (3.0 g, 22.4%) as a colorless oil.

### Step 2: Preparation of compound 71

A mixture solution of **Intermdiate B13** (1 g, 3.25 mmol), **compound 70** (1.2 g, 3.3 mmol) and Cs₂CO₃ (2.1 g, 6.5 mmol) in DMF (10 mL) was stirred at 100 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 71** (1.5 g, 75%) as a colorless oil. MS: 618.4 (M+H)⁺.

### Step 3: Preparation of Intermediate 16

To a stirred mixture solution of **compound 71** (0.7 g, 1.2 mmol) in THF (20 mL) was added 2.5 M lithium aluminum tetrahydride solution in THF (0.92 mL, 2.3 mmol) at 0 °C, then the resulting mixture was stirred at 0 °C for 0.5 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **Intermdiate 16** (0.55 g, 82%) as a colorless oil. MS: 590.4 (M+H)⁺.

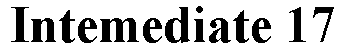

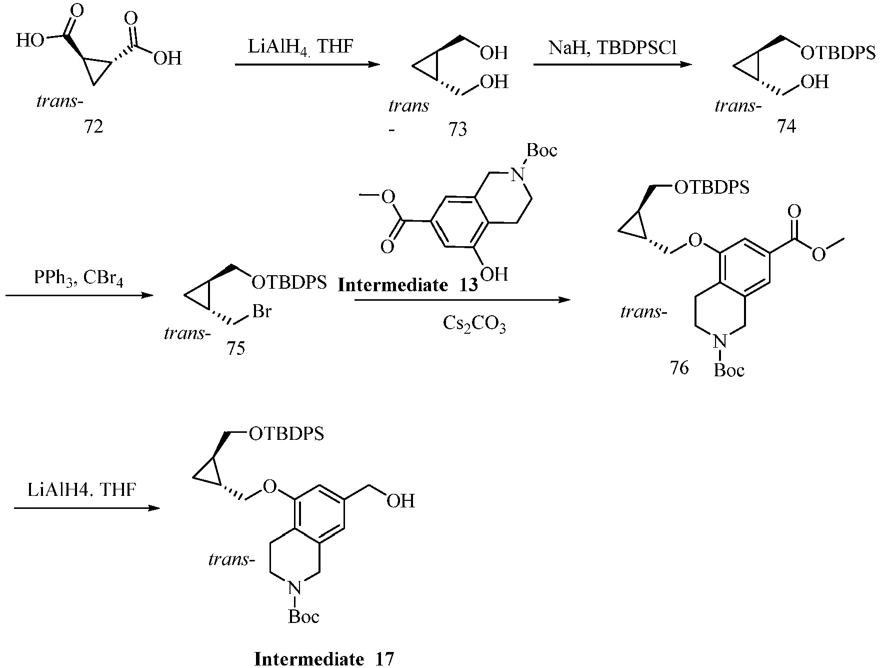

### Step 1: Preparation of compound 73

To a stirred mixture solution of **compound 72** (*Trans-,* 5.0 g, 31.6 mmol) in THF (50 mL) was added 2.5 M lithium aluminum tetrahydride solution in THF (50.56 mL, 126.4 mmol) at 0 °C, then the resulting mixture was stirred at 20 °C for 15 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 73** (500 mg, 15.5%) as a colorless oil.

### Step 2: Preparation of compound 74

To a stirred mixture solution of **compound 73** (500 mg, 4.9 mmol) in DMF (10 mL) was added NaH (235 mg, 5.87 mmol) at 0 °C, then the resulting mixture was stirred at 0 °C for 1 hour. Then TBDPSCl (1.61 g, 5.87 mmol) was added to the above mixture solution at 0 °C and the resulting mixture was stirred at 20 °C for 2 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 74** (400 mg, 23.9%) as a colorless oil. ¹H NMR (400 MHz, DMSO-*d₆*) δ 7.64-7.58 (m, 4H), 7.49-7.38 (m, 6H), 4.40 (t, *J=* 5.6 Hz, 1H), 3.59 (dd, *J=* 10.7, 5.9 Hz, 1H), 3.50 (dd, J = 10.7, 6.3 Hz, 1H), 3.27 (t, J = 5.6 Hz, 1H), 3.18 (dt, *J=* 11.2, 6.0 Hz, 1H), 0.98 (s, 9H), 0.90 (dt, *J=* 11.4, 5.7 Hz, 1H), 0.84-0.76 (m, 1H), 0.25-0.35 (m, 2H).

### Step 3: Preparation of compound 75

To a stirred mixture solution of **compound 74** (400 mg, 1.18 mmol) in DCM (10 mL) was added PPh₃ (462 mg, 1.76 mmol) and CBr₄ (584 mg, 1.76 mmol), then the resulting mixture was stirred at 20 °C for 2 hours. After the reaction was completed, the reaction mixture was quenched by adding ice-water and extracted with DCM. The combined organic layers were washed with brine, dried over anhydrous Na₂SO₄, filtered and concentrated in vacuo to give a crude product, which was purified by silica gel flash chromatography to afford **compound 75** (250 mg, 52.8%) as a colorless oil.

### Step 4: Preparation of compound 76

A mixturesolution of **Intermdiate B13** (190 mg, 0.62 mmol), **compound 75** (249 mg, 0.618 mmol) and Cs₂CO₃ (604 mg, 1.86 mmol) in DMF (5 mL) was stirred at 100 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 76** (320 mg, 82%) as a white solid. MS: 630.4 (M+H)⁺.

### Step 5: Preparation of Intermediate 17

To a stirred mixture solution of **compound 76** (320 mg, 0.51 mmol) in THF (5 mL) was added 2.5 M lithium aluminum tetrahydride solution in THF (0.5mL, 0.51mmol) at 0 °C, then the resulting mixture was stirred at 0 °C for 0.5 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **Intermdiate 17** (250 mg, 82%) as a white solid. MS: 602.4 (M+H)⁺.

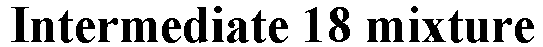

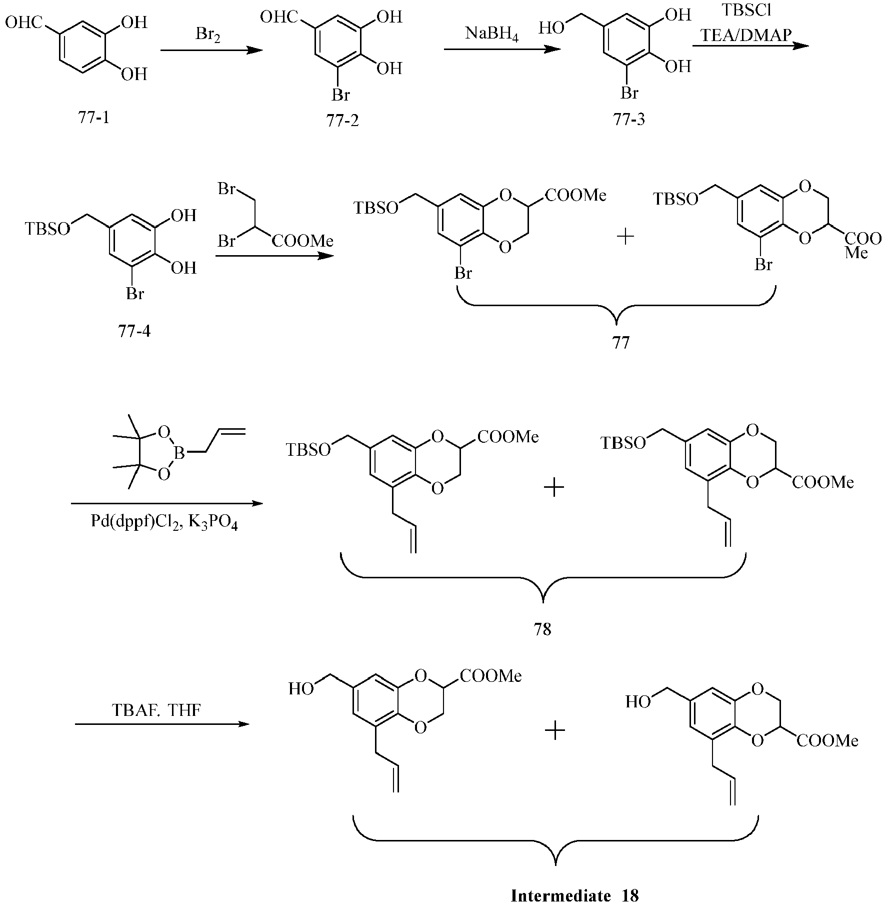

### Step 1: Preparation of compound 77-2

To a stirred mixture solution of **compound 77-1** (20 g, 144.8 mmol) in MeOH (200 mL) was added a solution of Br₂ (31.28 g, 173.76 mmol) in MeOH (100 mL) at 0 °C, then the resulting mixture was stirred at 20 °C for 2 hours. After the reaction was completed, the reaction mixture was quenched by adding ice-water, filtered and purified by silica gel flash chromatography to afford **compound 77-2** (20 g, 63.65%) as a brown solid. MS: 219.0 (M+H)⁺.

### Step 2: Preparation of compound 77-3

To a stirred mixture solution of **compound 77-2** (10 g, 46.08 mmol) in MeOH (100 mL) was added NaBH₄ (2.63 g, 69.12 mmol) at 0 °C, then the resulting mixture was stirred at 20 °C for 2 hour2. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 77-3** (7.9 g, 78.27%) as a yellow solid.

### Step 3: Preparation of compound 77-4

To a stirred mixture solution of **compound 77-3** (7.9 g, 36.1 mmol), DMAP (4.41 g, 36.07 mmol) and imidazole (4.91 g, 72.14 mmol) in DCM (150 mL) was added TBDPSCl (8.91g, 108.20mmol) at 0 °C, then the resulting mixture was stirred at 20 °C for 10 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 77-4** (2.2 g, 18.30%) as a yellow solid.

### Step 4: Preparation of compound 77

A suspension of **compound 77-4** (2.2 g, 6.6 mmol), methyl 2,3-dibromopropanoate (1.79 g, 7.28 mmol) and K₂CO₃ (2.74 g, 19.8 mmol) in DMF (33 mL) was stirred at 50 °C for 5 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 77** (2.10 g, 76.2%) as a white solid. MS: 379.5 (M+H)⁺.

### Step 5: Preparation of compound 78

A mixture solution of **compound 77** (1.5 g, 3.59 mmol), 4,4,5,5-tetramethyl-2-prop-2-enyl-1,3,2-dioxaborolane (1.81 g, 10.78 mmol), Pd(dppf)Cl₂ (260 mg, 0.32 mmol) and potassium metaphosphate (2.29 g, 10.78 mmol) in Dioxane (5 mL) and water (1 mL) was stirred at 80 °C for 2 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 78** (1.2 g, 88%) as a colorless oil.

### Step 6: Preparation of Intermediate 18 mixture

A mixture solution of **compound 78** (1.2 g, 3.17 mmol), 1.0 M TBAF solution in THF (3.17 mL, 3.17 mmol) in THF (15 mL) was stirred at 25 °C for 2 hours. After the reaction was completed, the reaction mixture was extracted with EA. The combined organic layers were washed with brine, dried over anhydrous Na₂SO₄, filtered and concentrated in vacuo to give a crude product, which was purified by silica gel flash chromatography to afford **Intermdiate 18 mixture** (600 mg, 71.6%) as a colorless oil. ¹H NMR (400 MHz, DMSO-*d₆*) δ 6.71 (d, *J=* 31.7 Hz, 1H), 6.64 (s, 1H), 6.01-5.81 (m, 1H), 5.15 (d, *J* = 2.8 Hz, 1H), 5.10-4.97 (m, 2H), 4.40-4.50 (m 1H), 4.33 (d, *J* = 9.6 Hz, 2H), 4.23 (dd, *J* = 11.8, 2.6 Hz, 1H), 3.90-1.05 (m, 1H), 3.31 (t, *J* = 6.9 Hz, 1H), 3.24 (t, *J* = 5.6 Hz, 1H).

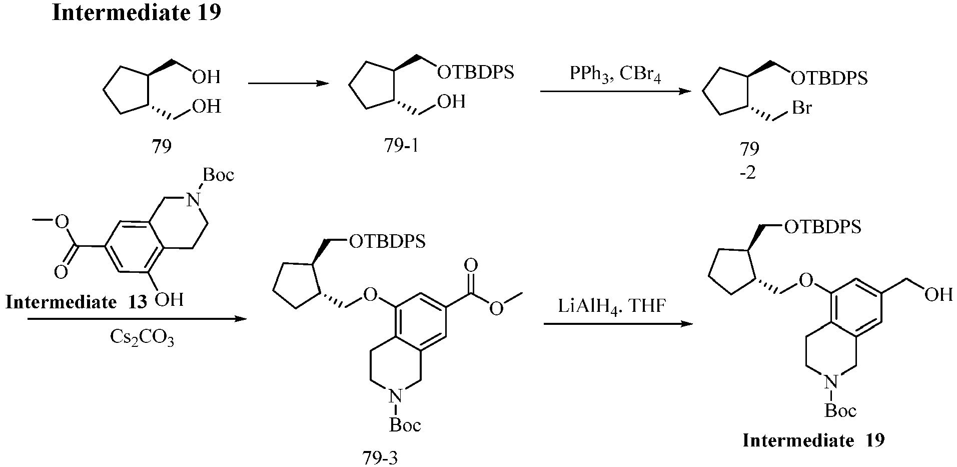

### Step 1: Preparation of compound 79-1

To a stirred mixture solution of **compound 79** (641 mg, 4.92 mmol) in THF (15 mL) was added NaH (124 mg, 5.2 mmol) at 0 °C, then the resulting mixture was stirred at 0 °C for 0.5 hour. Then TBDPSCl (1.2 g, 4.3 mmol) was added to the above mixture solution at 0 °C and the resulting mixture was stirred at 20 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 79-1** (1.31 g, 72.1%) as a colorless oil.

### Step 2: Preparation of compound 79-2

To a stirred mixture solution of **compound 79-1** (3.11 g, 8.46 mmol) in DCM (50 mL) was added PPh₃ (3.3 g, 12.7 mmol) and CBr₄ (4.2 g, 12.7 mmol), then the resulting mixture was stirred at 20 °C for 2 hours. After the reaction was completed, the reaction mixture was quenched by adding ice-water and extracted with DCM. The combined organic layers were washed with brine, dried over anhydrous Na₂SO₄, filtered and concentrated in vacuo to give a crude product, which was purified by silica gel flash chromatography to afford **compound 79-2** (3.31 g, 91%) as a colorless oil.

### Step 3: Preparation of compound 79-3

A mixture solution of **Intermdiate B13** (1 g, 3.25 mmol), **compound 79-2** (1.4 g, 3.3 mmol) and Cs₂CO₃ (2.1 g, 6.5 mmol) in DMF (10 mL) was stirred at 100 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 79-3** (1.4 g, 67%) as a colorless oil. MS: 658.5 (M+H)⁺.

### Step 4: Preparation of Intermediate 19

To a stirred mixture solution of **compound 79-3** (1.43 g, 2.17 mmol) in THF (20 mL) was added 2.5 M lithium aluminum tetrahydride solution in THF (0.87 mL, 2.2 mmol) at 0 °C, then the resulting mixture was stirred at 0 °C for 0.5 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **Intermediate 19** (1.12 g, 82%) as a colorless oil. MS: 630.8 (M+H)⁺.

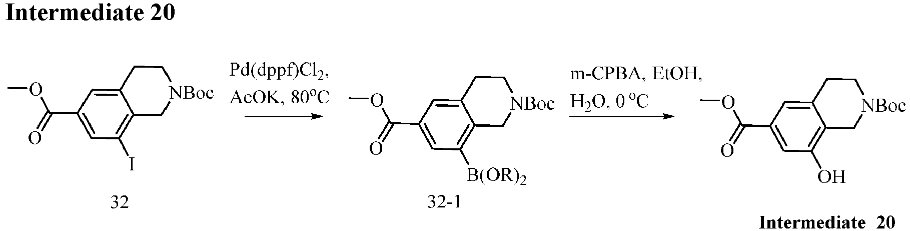

The title compound **Intermdiate 20** was prepared in analogy to the preparation of **Intermediate 13** by using **compound 32** as a start material..

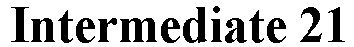

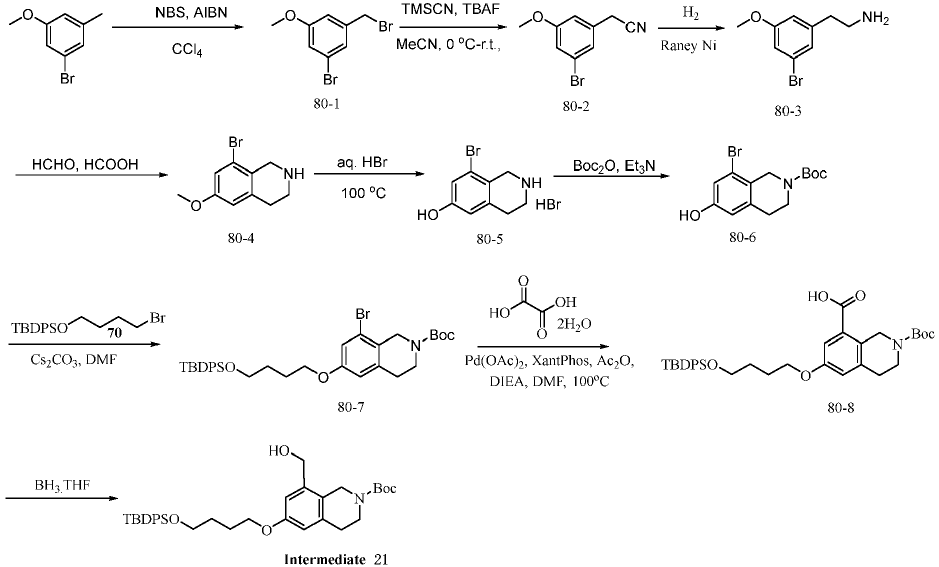

### Step 1: Preparation of compound 80-1

To a stirred mixture solution of 1-bromo-3-methoxy-5-toluene (40.2 g, 200 mmol) in CCl₄ (300 mL) was added NBS (39.1 g, 220 mmol) and AIBN (3.28 g, 20 mmol), then the resulting mixture was stirred at 80 °C for 13 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 80-**1 (54 g, 96%) as a white solid. ¹H NMR (CDCl₃, 400 MHz) *δ* (ppm) 7.27-6.52 (m, 3H), 4.38 (s, 2H), 3.80 (s, 3H).

### Step 2: Preparation of compound 80-2

To a stirred mixture solution of **compound 80-1** (20.0 g, 71.4 mmol) in ACN (150 mL) was added trimethylcyanosilane (108 mmol) at 0 °C and the resulting mixture was stirred at 20 °C for 0.5 hour. Then 1.0 M TBAF solution in THF (108 mL, 108 mmol) was stirred at 50 °C for 8 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 80-2** (12.54 g, 78%) as a yellow oil. ¹H-NMR (CDCl₃, 400 MHz): *δ* (ppm) 7.04 (s, 1H), 7.00 (s, 1H), 6.79 (s, 1H), 3.793 (s, 3H), 3.68 (s, 2H).

### Step 3: Preparation of compound 80-3

A mixture solution of **compound 80-2** (1.2 g, 5.31 mmol), Raney Nickel(1.0g) and NH₃HO₂ (8 mL) in MeOH (8 mL) was stirred at 20 °C for 13 hours under H₂ 50 psi pressure. After the reaction was completed, the reaction mixture was filtered and concentrated in vacuo to afford **compound 80-3** (420 mg, 34.4%) as a yellow oil. MS: 230.0 (M+H)⁺.

### Step 4: Preparation of compound 80-4

To a stirred mixture solution of **compound 80-3** (420 mg, 1.83 mmol) in formic acid (6 mL) was added paraformaldehyde (66 mg, 0.733 mmol) at 20 °C and the resulting mixture was stirred at 50 °C for 16 hours. After the reaction was completed, the reaction mixture was concentrated in vacuo and extracted with DCM. The combined organic layers were washed with brine, dried over anhydrous Na₂SO₄, filtered and concentrated in vacuo to give a crude product, which was purified by silica gel flash chromatography to afford **compound 80-4** (177 mg, 40.1%) as a red oil. MS:256, 258 (M+H)⁺. ¹H-NMR (CDCl₃, 400 MHz): *δ* (ppm) 6.95 (s, 1H), 6.61 (s, 1H), 3.75 (s, 3H), 3.48 (s, 2H), 2.88 (t, J = 5.6 Hz, 2H), 2.62 (t, J = 5.6 Hz, 2H), 2.48 (s, 3H).

### Step 5: Preparation of compound 80-5

A mixture solution of **compound 80-4** (1.65 g, 6.82 mmol) in 40% HBr solution (25 mL) was stirred at 100 °C for 36 hours. After the reaction was completed, the reaction mixture was concentrated in vacuo, cooled and filtered to afford **compound 80-5** (2.11 g) as a yellow solid. MS: 228.3 (M+H)⁺.

### Step 6: Preparation of compound 80-6

To a stirred mixture solution of **compound 80-5** (15.61 g, 50.51 mmol) in THF (150 mL) and H₂O (150 mL) was added BoczO (11.02 g, 50.5 mmol) and TEA (24.85 mL, 177 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 80-6** (10.64 g, 64.2%) as a yellow solid.

### Step 7: Preparation of compound 80-7

A mixture solution of **compound 80-6** (1.0 g, 3.05 mmol), **compound 70** (1.19 g, 3.05 mmol) and Cs₂CO₃ (1.49 g, 4.57 mmol) in DMF (10 mL) was stirred at 100 °C for 1 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 80-7** (1.3 g, 66.8%) as a colorless oil. MS: 638.3 (M+H)⁺.

### Step 8: Preparation of compound 80-8

In a sealed tube, to a stirred mixture solution of **compound 80-7** (1.3 g, 2.03 mmol), oxalic acid (0.37 g, 4.07 mmol), Pd(OAc)₂ (0.046 g, 0.204 mmol) and Xantphos (0.12 g, 0.20 mmol) in DMF (10 mL) was added DIEA (0.26 g, 2.04 mmol) and AczO (0.42 g, 4.07 mmol) at 0 °C, then the resulting mixture was stirred at 100 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 80-8** (77.6 mg, 63.3%) as a colorless oil. MS: 602.2 (M+H)⁺.

### Step 9: Preparation of Intermediate 21

To a stirred mixture solution of **compound 80-8** (1.2 g, 1.99 mmol) in THF (10 mL) was added 1.0 M BH₃-THF solution (3.0 mL, 3.0 mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **Intermediate 21** (1.0 g, 85%) as a colorless oil. MS: 590.4 (M+H)⁺.

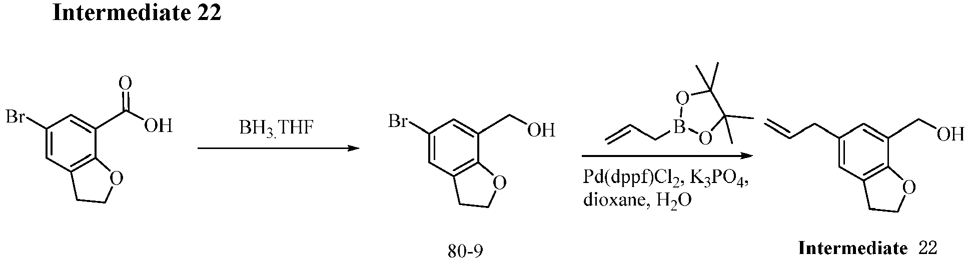

### Step 1: Preparation of compound 80-9

To a stirred mixture solution of 5-bromo-2,3-dihydrobenzofuran-7-carboxylic acid (2.5 g, 10.29 mmol) in THF (30 mL) was added 1.0 M BH₃-THF solution (15.4 mL, 15.4 mmol) at 0 °C, then the resulting mixture was stirred at 20 °C for 12 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 80-9** (2.2 g, 93%) as a colorless oil.

### Step 2: Preparation of Intermediate 22

A mixture solution of **compound 80-9** (2.2 g, 9.60 mmol), 4,4,5,5-tetramethyl-2-prop-2-enyl-1,3,2-dioxaborolane (4.84 g, 28.8 mmol), Pd(dppf)Cl₂ (0.7 g, 0.96 mmol) and potassium metaphosphate (6.12 g, 28.8 mmol) in Dioxane (15 mL) and water (3 mL) was stirred at 80 °C for 2 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **Intermediate 22** (1.5 g, 82%) as a colorless oil.

### Example 1

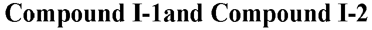

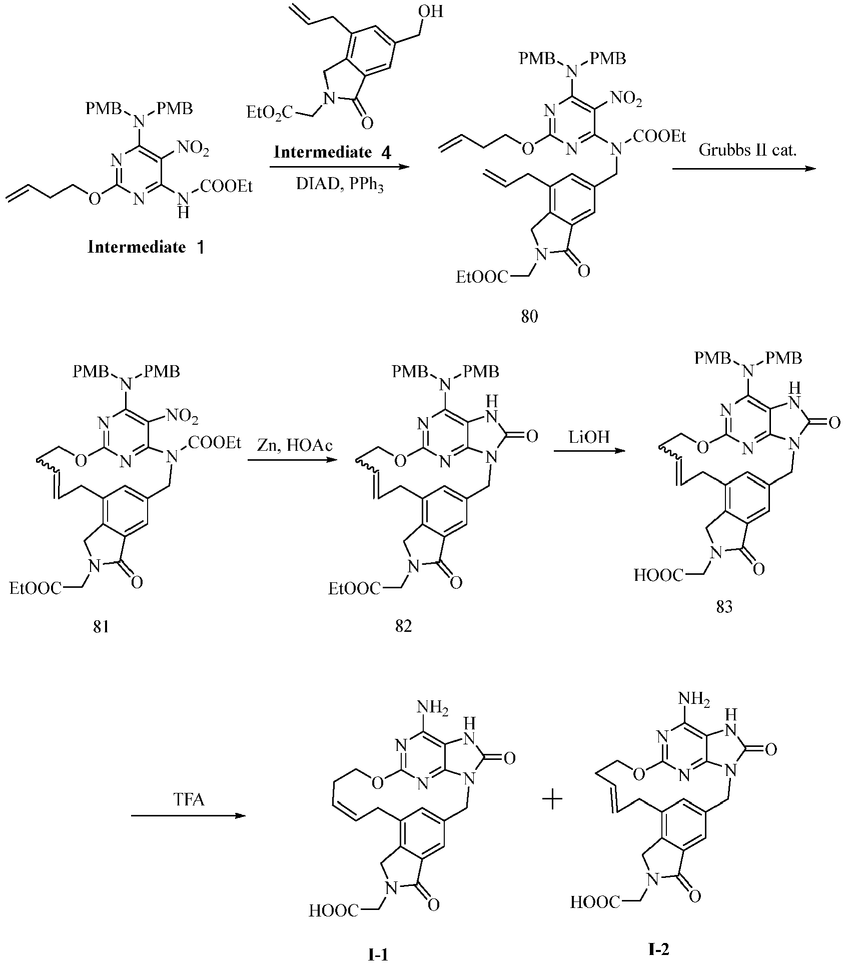

### Step 1: Preparation of compound 80

To a stirred mixture solution of **Intermediate 1** (450 mg, 0.84 mmol) and **Intermediate 4** (242 mg, 0.84 mmol) in THF (10 mL) was added PPh₃ (329 mg, 1.26 mmol) and DIAD (254 mg, 1.26 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 80** (600 mg, 89%) as a yellow oil. MS: 809.4 (M+H)⁺.

### Step 2: Preparation of compound 81

To a stirred mixture solution of **compound 80** (520 mg, 0.643 mmol) in DCM (200 mL) was added Grubbs'II reagent (109mg, 0. 13mmol) under N₂ protected, then the resulting mixture was stirred at 20 °C for 12 hours. After the reaction was completed, the reaction mixture was concentrated in vacuo to give a crude product, which was purified by silica gel flash chromatography to afford **compound 81** (401 mg, 80%) as a yellow solid. MS: 781.4 (M+H)⁺.

### Step 3: Preparation of compound 82

To a stirred mixture solution of **compound 81** (220 mg, 0.28 mmol) in AcOH (5 mL) was added Zn (92 mg, 1.41 mmol) under N₂ protected, then the resulting mixture was stirred at 70 °C for 0.5 hour. After the reaction was completed, the reaction mixture was filtered and concentrated in vacuo to give a crude product, which was purified by silica gel flash chromatography to afford **compound 82** (130 mg, 65.5%) as a white solid. MS: 704.4 (M+H)⁺.

### Step 4: Preparation of compound 83

A mixture solution of **compound 82** (130 mg, 0.18 mmol) and LiOH (37 mg, 0.9 mmol) in THF (1 mL) and water (1 mL) was stirred at 20 °C for1 hour. After the reaction was completed, the reaction mixture was adjusted pH to 5, filtered and dried to afford **compound 83** (100 mg, 76.9%) as a white solid. MS: 677.3 (M+H)⁺.

### Step 5: Preparation of Compound I-1 and Compound I-2

A mixture solution of **compound 83** (100 mg, 0.15 mmol) in TFA (5 mL) was stirred at 70 °C for 0.5 hour. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to give a crude product, which was purified by pre-HPLC to afford **Compound I-1** and **Compound I-2**.

**Compound I-1** (white solid, 20 mg, 31%). MS: 436.9 (M+H)⁺. ¹H NMR (DMSO-*d₆*, 400 MHz): δ 12.89 (s, 1H), 9.88 (s, 1H), 8.08 (s, 1H), 7.44 (s, 1H), 6.40 (s, 2H), 5.78-5.68 (m, 1H), 5.62 (d, *J* = 9.0 Hz, 1H), 4.92 (br, 2H), 4.50-4.42 (m, 4H), 4.23 (br, 2H), 3.38 (br, 2H), 2.34 (br, 2H).

**Compound I-2** (white solid, 20 mg, 31%). MS: 436.9 (M+H)⁺. ¹H NMR (DMSO-d6, 400 MHz): δ 12.89 (s, 1H), 9.88 (s, 1H), 8.35 (s, 1H), 7.45 (s, 1H), 6.40 (s, 2H), 5.75 (dt, J = 14.8, 7.1 Hz, 1H), 5.51 (dt, J = 15.3, 7.4 Hz, 1H), 4.93 (br, 2H), 4.72 (d, J = 5.7 Hz, 2H), 4.40 (br, 2H), 4.25 (br, 2H), 3.24 (d, J = 7.1 Hz, 2H), 2.44 (br, 2H).

### Example 2

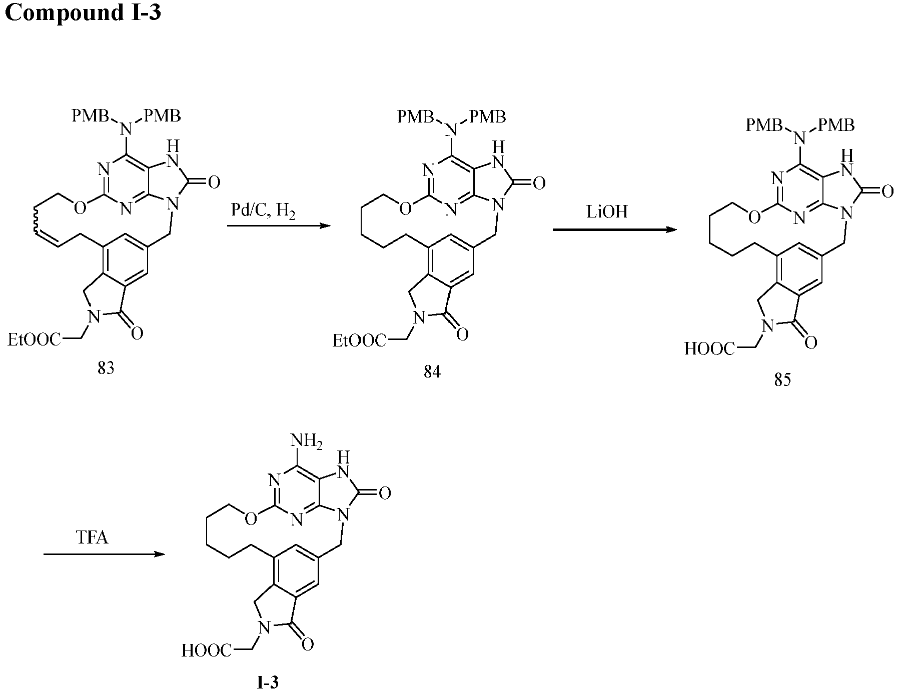

### Step 1: Preparation of compound 84

A mixture solution of **compound 83** (250 mg, 0.36 mmol) and Pd/C (100 mg) in EA (10 mL) under H₂ atmosphere was stirred at 25 °C for 4 hours. After the reaction was completed, the reaction mixture was filtered and concentrated in vacuo to afford **compound 84** (240 mg, 96%) as a white solid. MS: 707.4 (M+H)⁺.

### Step 2: Preparation of compound 85

A mixture solution of **compound 84** (120 mg, 0.18 mmol) and LiOH (37 mg, 0.9 mmol) in THF (2 mL) and water (2 mL) was stirred at 20 °C for1 hour. After the reaction was completed, the reaction mixture was adjusted pH to 5, filtered and dried to afford **compound 85** (70 mg, 60.8%) as a white solid. MS: 679.3 (M+H)⁺.

### Step 3: Preparation of Compound I-3

A mixture solution of **compound 84** (70 mg, 0.1 mmol) in TFA (5 mL) was stirred at 70 °C for 0.5 hour. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to give a crude product, which was purified by pre-HPLC to afford **Compound I-3** (white solid, 21 mg, 46.7%). MS: 438.9 (M+H)⁺.¹H NMR (400 MHz, DMSO-*d₆*) δ 12.87 (s, 1H), 9.95 (s, 1H), 7.89 (s, 1H), 7.27 (s, 1H), 6.35 (s, 2H), 4.94 (br, 2H), 4.42 (br, 2H), 4.23 (d, *J* = 5.2 Hz, 4H), 2.71 (d, *J* = 7.3 Hz, 2H), 1.74 (br, 2H), 1.35-1.29 (m, 4H).

### Example 3

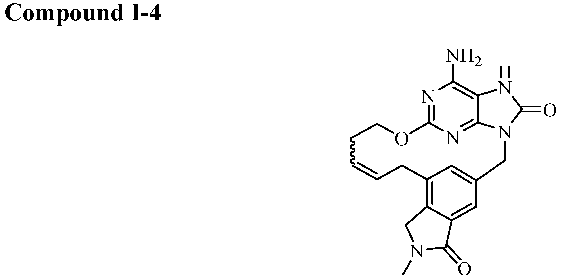

The title **Compound I-4** was prepared in analogy to the preparation of **Example 1** by using **Intermediate 1** and **Intermediate 5** as a start material. MS: 393.0 (M+H)⁺. ¹H NMR (400 MHz, DMSO-*d₆*) δ 9.88 (s, 1H), 8.30 (s, 1H), 7.40 (s, 1H), 6.42 (s, 2H), 5.79-5.69 (m, 1H), 5.56-5.45 (m, 1H), 4.91 (s, 2H), 4.72 (s, 2H), 4.34 (s, 2H), 3.22 (d, J = 7.1 Hz, 2H), 3.04 (s, 3H), 2.44 (s, 2H).

### Example 4

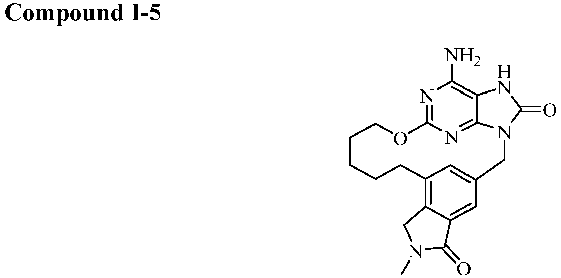

The title **Compound I-5** was prepared in analogy to the preparation of **Compound I-3.** MS: 395.2 (M+H)⁺. ¹H NMR (400 MHz, DMSO-*d₆*) δ 9.98 (s, 1H), 7.82 (s, 1H), 7.20 (s, 1H), 6.43 (s, 2H), 4.93 (s, 2H), 4.37 (s, 2H), 4.23 (d, J = 7.1 Hz, 2H), 3.02 (s, 3H), 2.70 (br, 2H), 1.75 (br, 2H), 1.31 (br, 4H).

### Example 5

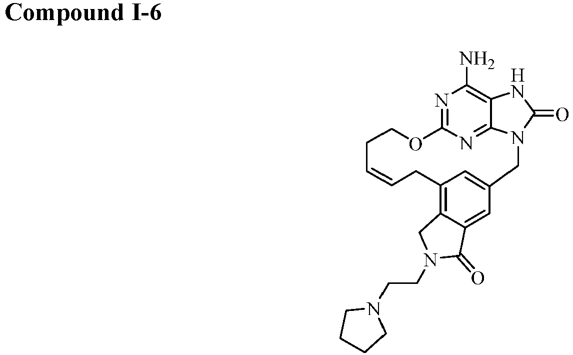

The title **Compound I-6** was prepared in analogy to the preparation of **Example 1** by using **Intermediate 1** and **Intermediate 6** as a start material. MS: 475.9 (M+H)⁺.

### Example 6

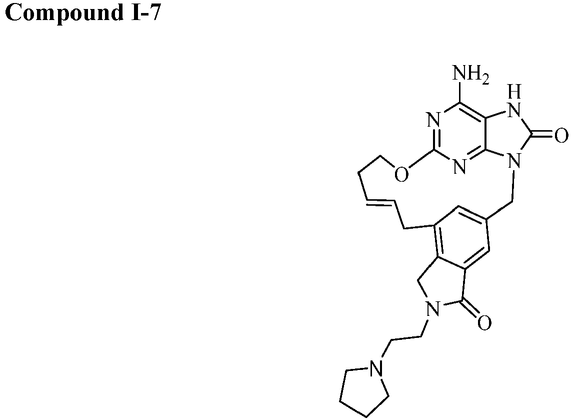

The title **Compound I-6** was prepared in analogy to the preparation of **Compound I-2** by using **Intermediate 1** and **Intermediate 6** as a start material. MS: 475.9 (M+H)⁺. ¹H NMR (400 MHz, DMSO-*d₆*) δ 9.90 (s, 1H), 7.46 (s, 1H), 6.40 (s, 2H), 5.78 (dd, J = 15.4, 7.5 Hz, 1H), 5.56-5.46 (m, 1H), 4.93 (s, 2H), 4.71 (br, 2H), 4.41 (br, 2H), 3.86 (t, J = 5.8 Hz, 2H), 3.56 (br, 2H), 3.45 (d, J = 6.0 Hz, 2H), 3.24 (d, J = 6.9 Hz, 2H), 3.05 (br, 2H), 2.44 (br, 2H), 1.98 (br, 2H), 1.75-1.85 (m, 2H).

### Example 7

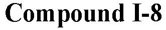

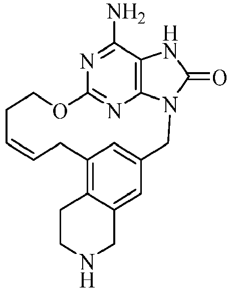

The title **Compound I-8** was prepared in analogy to the preparation of **Example 1** by using **Intermediate 1** and **Intermediate 7** as a start material. MS: 379.1 (M+H)⁺. ¹H NMR (400 MHz, DMSO-*d₆*) δ 9.96 (s, 1H), 7.73 (s, 1H), 7.00 (s, 1H), 6.41 (s, 2H), 5.69 (t, J = 8.6 Hz, 1H), 5.60 (dt, J = 10.7, 6.6 Hz, 1H), 4.79 (br, 2H), 4.41 (t, J = 8.5 Hz, 2H), 4.22 (br, 2H), 3.28 (d, J = 6.6 Hz, 2H), 3.21 (d, J = 7.6 Hz, 2H), 2.84 (t, J = 6.2 Hz, 2H), 2.26 (d, J = 8.4 Hz, 2H).

### Example 8

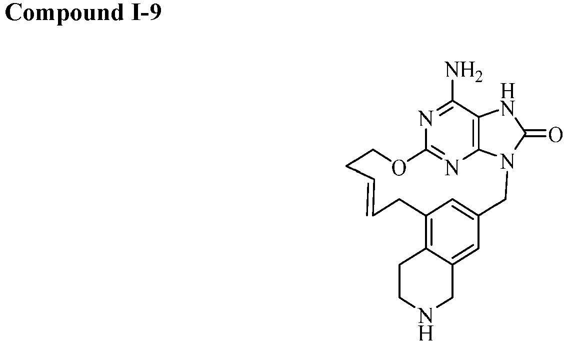

The title **Compound I-9** was prepared in analogy to the preparation of **Example 1** by using **Intermediate 1** and **Intermediate 7** as a start material. MS: 379.1 (M+H)⁺. ¹H NMR (400 MHz, DMSO-*d₆*) δ 9.93 (s, 1H), 8.04 (s, 1H), 7.01 (s, 1H), 6.43 (s, 2H), 5.74 (dt, J = 15.0, 7.2 Hz, 1H), 5.43 (dt, J = 15.2, 7.4 Hz, 1H), 4.79 (s, 2H), 4.71 (d, J = 6.0 Hz, 2H), 4.24 (d, J = 5.1 Hz, 2H), 3.36 (d, J = 6.4 Hz, 2H), 3.09 (d, J = 7.1 Hz, 2H), 2.78 (t, J = 6.4 Hz, 2H), 2.43 (d, J = 5.7 Hz, 2H).

### Example 9

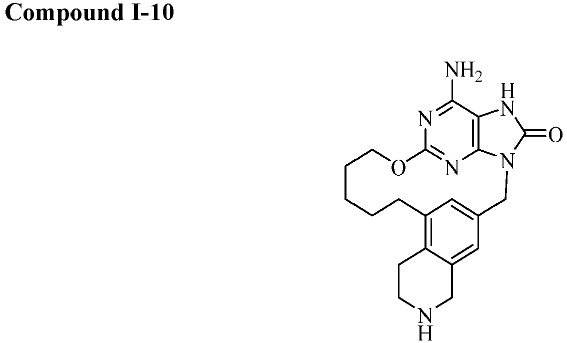

The title **Compound I-10** was prepared in analogy to the preparation of **Compound I-3.** MS: 381.0 (M+H)⁺. ¹H NMR (400 MHz, DMSO-*d₆*) δ 10.03 (s, 1H), 7.57 (s, 1H), 6.86 (s, 1H), 6.39 (s, 2H), 4.80 (d, J = 5.5 Hz, 2H), 4.21 (d, J = 10.9 Hz, 4H), 3.43 (d, J = 3.6 Hz, 2H), 2.84 (t, J = 6.5 Hz, 2H), 2.63 (br, 2H), 1.68 (br, 2H), 1.46-1.24 (m, 4H).

### Example 10

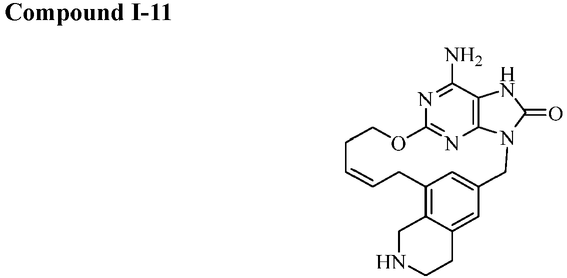

The title **Compound I-11** was prepared in analogy to the preparation of **Example 1** by using **Intermediate 1** and **Intermediate 8** as a start material. MS: 379.10 (M+H)⁺. ¹H NMR (400 MHz, DMSO-*d₆*) δ 9.95 (s, 1H), 7.70 (s, 1H), 7.01 (s, 1H), 6.42 (s, 2H), 5.73-5.57 (m, 2H), 4.79 (s, 2H), 4.42 (t, J = 7.2 Hz, 2H), 4.18 (s, 2H), 3.30-3.27 (m, 2H), 3.18-3.17 (m, 2H), 2.94 (t, J = 6.8 Hz, 2H), 2.32-2.24 (m, 2H).

### Example 11

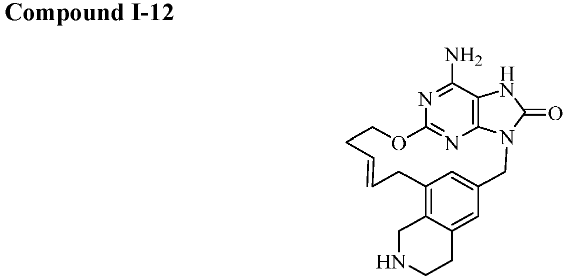

The title **Compound I-12** was prepared in analogy to the preparation of **Example 1** by using **Intermediate 1** and **Intermediate 8** as a start material. MS: 379.10 (M+H)⁺. ¹H NMR (400 MHz, DMSO-*d₆*) δ 9.93 (s, 1H), 8.01 (s, 1H), 7.01 (s, 1H), 6.42 (s, 2H), 5.77-5.69 (m, 1H), 5.47-5.39 (m, 1H), 4.78 (s, 2H), 4.72 (s, 2H), 4.12 (br, 2H), 3.28 (s, 2H), 3.05 (d, J = 6.8 Hz, 2H), 2.94 (t, J = 5.6 Hz, 2H), 2.46-2.39 (m, 2H).

### Example 12

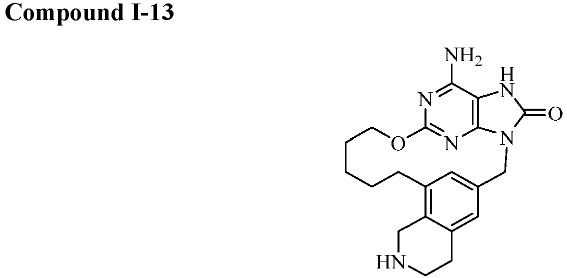

The title **Compound I-13** was prepared in analogy to the preparation of **Compound I-3.** MS: 381.00 (M+H)⁺. ¹H NMR (400 MHz, DMSO-*d₆*) δ 10.05 (s, 1H), 7.55 (s, 1H), 6.88 (s, 1H), 6.46 (s, 2H), 4.81 (s, 2H), 4.25 (t, J = 6.4 Hz, 2H), 4.17 (s, 2H), 3.28-3.27 (m, 2H), 2.92 (t, J = 5.6 Hz, 2H), 2.59-2.56 (m, 2H), 1.69-1.67 (m, 2H), 1.41-1.68 (m, 4H).

### Example13

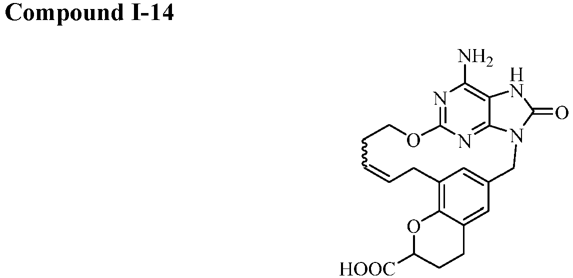

The title **Compound I-14** was prepared in analogy to the preparation of **Example 1** by using **Intermediate 1** and **Intermediate 9** as a start material. MS: 423.70 (M+H)⁺. ¹H NMR (400 MHz, DMSO-*d₆*) δ 9.84 (s, 1H), 7.89 (s, 1H), 6.80 (s, 1H), 6.42 (s, 2H), 5.64 (s, 1H), 5.35-5.045 (m, 1H), 4.75-4.43 (m, 5H), 3.05 (d, J = 7.2 Hz, 2H), 2.70-2.50 (m, 2H), 2.39 (s, 2H), 2.05-1.92 (m, 3H).

### Example 14

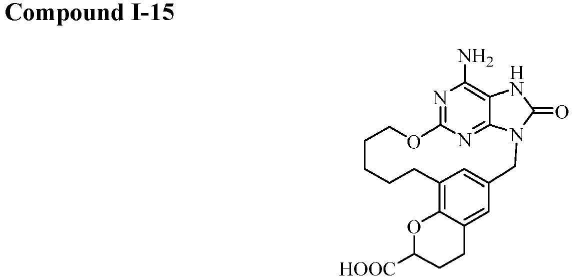

The title **Compound I-15** was prepared in analogy to the preparation of **Compound I-3.** MS: 425.70 (M+H)⁺. ¹H NMR (400 MHz, DMSO-*d₆*) δ 9.90 (s, 1H), 7.38 (s, 1H), 6.66 (s, 1H), 6.36 (s, 2H), 4.74 (d, J = 16.2 Hz, 3H), 4.21 (t, J = 7.0 Hz, 2H), 2.67 (d, J = 16.5 Hz, 1H), 2.58 (br, 2H), 2.54 (d, J = 7.7 Hz, 1H), 1.95-2.05(m, 2H), 1.73 (br, 1H), 1.62 (br, 1H), 1.30-1.42 (m, 2H), 1.28 (br, 2H).

### Example 15

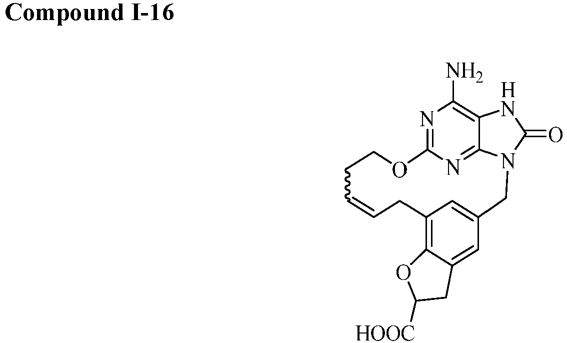

The title **Compound I-16** was prepared in analogy to the preparation of **Example 1** by using **Intermediate 1** and **Intermediate 10** as a start material. MS: 410.10 (M+H)⁺. ¹H NMR (400 MHz, DMSO-*d₆*) δ 13.02 (s, 1H), 9.84 (s, 1H), 7.93 (d, J = 10.7 Hz, 1H), 6.98 (s, 1H), 6.37 (s, 2H), 5.60-5.75 (dm, J = 14.6, 7.1 Hz, 1H), 5.40-5.50 (m, , 1H), 5.17 (dd, J = 10.7, 6.4 Hz, 1H), 4.72 (br, 3H), 4.69-4.65 (m, 1H), 3.49-3.44 (m, 2H), 3.20-3.10 (m, 2H), 2.88-2.57 (m, 1H), 2.35-2.45 (m, J = 5.9 Hz, 1H).

### Example 16

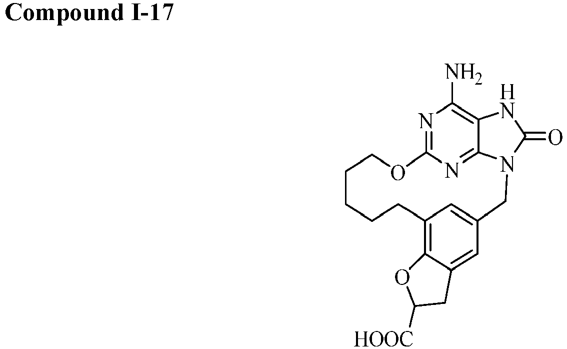

The title **Compound I-17** was prepared in analogy to the preparation of **Compound I-3.** MS: 412.10 (M+H)⁺. ¹H NMR (400 MHz, DMSO-*d₆*) δ 9.90 (s, 1H), 7.39 (s, 1H), 6.81 (s, 1H), 6.34 (s, 2H), 5.14 (dd, J = 10.6, 6.2 Hz, 1H), 4.75 (s, 2H), 4.15-4.25 (m, 2H), 3.14 (dd, J = 16.3, 6.1 Hz, 1H), 2.58 (d, J = 5.8 Hz, 2H), 1.65-1.75 (m, 2H), 1.41-1.26 (m, 5H).

### Example 17

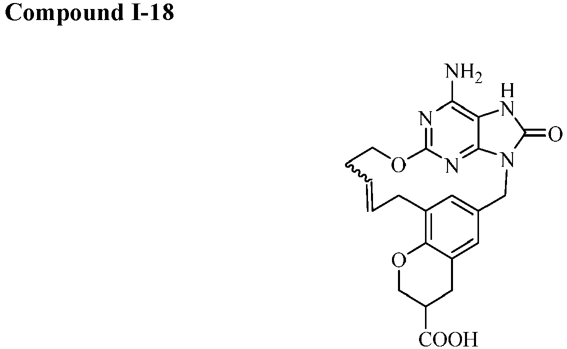

The title **Compound I-18** was prepared in analogy to the preparation of **Example 1** by using **Intermediate 1** and **Intermediate 11** as a start material. MS: 424.30 (M+H)⁺. ¹H NMR (400 MHz, DMSO-*d₆*) δ 9.85 (s, 1H), 7.91 (s, 1H), 6.88 (s, 1H), 6.37 (s, 1H), 5.68-5.61 (m, 1H), 5.39 (dd, J = 15.2, 7.4 Hz, 1H), 4.70 (s, 2H), 4.28 (d, J = 10.7 Hz, 1H), 4.10 (dd, J = 10.5, 6.7 Hz, 1H), 3.49 (d, J = 5.7 Hz, 2H), 3.28 (br, 2H), 3.01 (d, J = 7.2 Hz, 1H), 2.89 (br, 2H), 2.53 (br, 2H), 2.41 (d, J = 7.6 Hz, 2H).

### Example 18

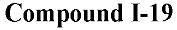

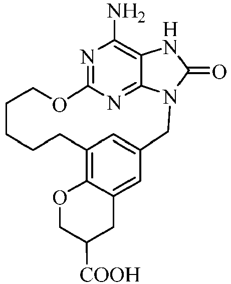

The title **Compound I-19** was prepared in analogy to the preparation of **Example 3** by using **Compound I-18** as a start material. MS: 426.30 (M+H)⁺. ¹H NMR (400 MHz, DMSO-*d₆*) δ 12.57 (s, 1H), 9.88 (s, 1H), 7.40 (s, 1H), 6.74 (s, 1H), 6.33 (s, 2H), 4.72 (q, J = 14.2 Hz, 2H), 4.28 (d, J = 10.3 Hz, 2H), 4.22 (br, 1H), 4.04 (br, 1H), 2.86 (br, 3H), 2.54 (d, J = 10.4 Hz, 2H), 1.73-1.21 (m, 6H).

### Example 19

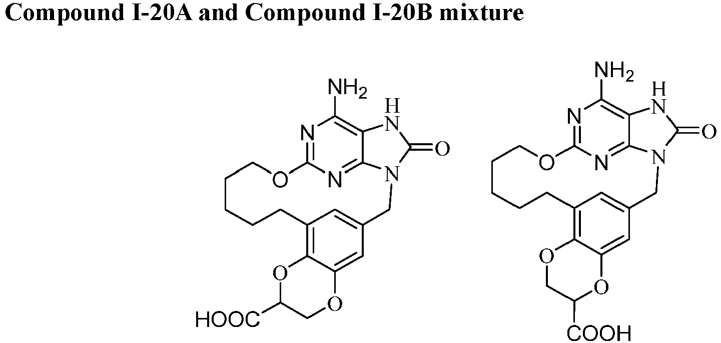

The title **Compound I-20A** and **Compound I-20B** mixture was prepared in analogy to the preparation of **Compound I-1** and **Compound I-3** by using **Intermediate 1** and **Intermediate 18** as a start material. MS: 428.20 (M+H)⁺. ¹H NMR (400 MHz, DMSO-*d₆*) δ 9.93 (d, J = 5.8 Hz, 1H), 7.14 (s, 1H), 6.49 (s, 1H), 6.39 (s, 2H), 4.97 (d, J = 38.1 Hz, 1H), 4.82-4.71 (m, 1H), 4.36 (d, J = 11.8 Hz, 1H), 4.16 (d, J = 15.0 Hz, 3H), 2.61 (d, J = 18.6 Hz, 2H), 1.75-1.62 (m , 3H), 1.34-1.22 (m, 5H).

### Example 20

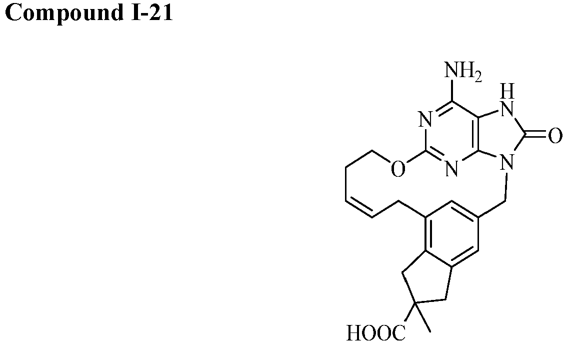

The title **Compound I-21** was prepared in analogy to the preparation of **Example 1** by using **Intermediate 1** and **Intermediate 12** as a start material. MS: 437.00 (M+H)⁺. ¹H NMR (400 MHz, DMSO-*d₆*) δ 12.30 (s, 1H), 9.84 (s, 1H), 7.93 (s, 1H), 6.95 (s, 1H), 6.37 (s, 2H), 5.65-5.75(m, , 1H), 5.35-5.48 (m, 1H), 4.75 (s, 2H), 4.70 (s, 2H), 3.19 (d, J = 16.2 Hz, 1H), 3.07 (t, J = 7.0 Hz, 2H), 2.71 (d, J = 16.4 Hz, 1H), 2.62 (d, J = 16.3 Hz, 1H), 2.41 (d, J = 6.1 Hz, 2H), 1.23 (s, 3H).

### Example 21

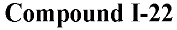

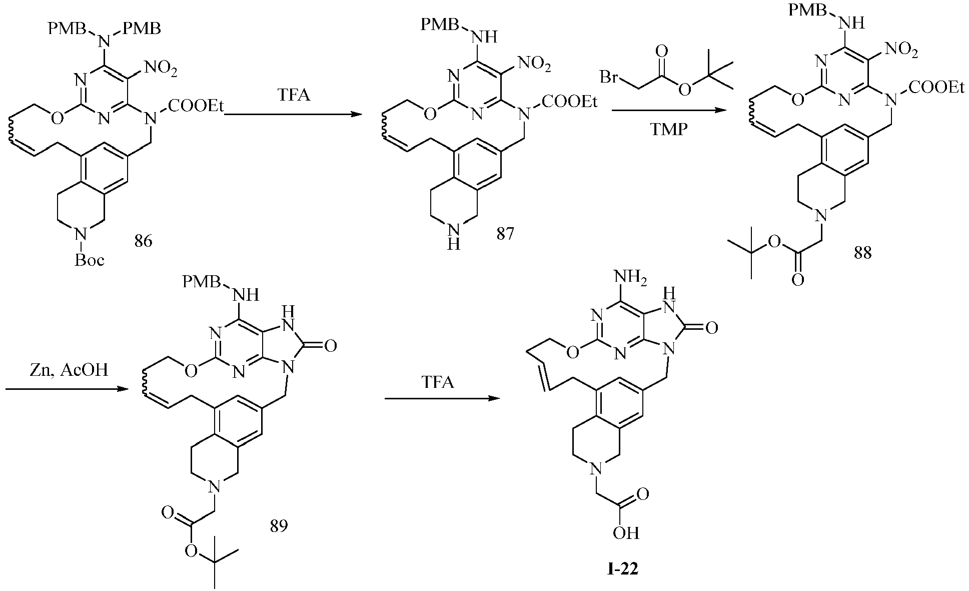

### Step 1: Preparation of compound 86

The title **compound 86** was prepared in analogy to the preparation of **compound 81** by using **Intermediate 1** and **Intermediate 7** as a start material.

### Step 2: Preparation of compound 87

A mixture solution of **compound 86** (500 mg, 629 mmol) in TFA (10 mL) was stirred at 20 °C for 2 hours. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to afford **compound 87** (300 mg) as a solid.

### Step 3: Preparation of compound 88

A mixture solution of **compound 87** (300 mg, 0.44 mmol), tert-butyl 2-bromoacetate (85 mg, 0.44 mmol) and 2,2,6,6-tetramethylpiperidine (246 mg, 1.74 mmol) in DMF (5 mL) was stirred at 25 °C for 12 hours. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 88** (120 mg, 40.0%) as a yellow solid. MS: 689.0 (M+H)⁺.

### Step 4: Preparation of compound 89

To a stirred mixture solution of **compound 88** (110 mg, 0.16 mmol) in AcOH (7.5 mL) was added Zn (52.2 mg, 0.80 mmol) under N₂ protected, then the resulting mixture was stirred at 80 °C for 0.5 hour. After the reaction was completed, the reaction mixture was filtered and concentrated in vacuo to give a crude product, which was purified by silica gel flash chromatography to afford **compound 89** (52 mg, 53.1%) as a colorless oil. MS: 613.0 (M+H)⁺.

### Step 5: Preparation of Compound I-22

A mixture solution of **compound 89** (52 mg) in TFA (5 mL) was stirred at 70 °C for 1 hour. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to give a crude product, which was purified by pre-HPLC to afford **Compound I-22** (7.28 mg, 19.5%) as a white solid. MS: 437.0 (M+H)⁺. ¹H NMR (400 MHz, DMSO-*d₆*)) δ 9.92 (s, 1H), 8.08 (s, 1H), 6.99 (s, 1H), 6.42 (s, 2H), 5.70-7.80 (m, 1H), 5.40-4.50 (m, 1H), 4.80 (nr, 2H), 4.73 (br, 2H), 4.36 (br, 2H), 4.11 (br, 2H), 3.47 (br, 2H), 3.10 (d, J = 7.2 Hz, 2H), 2.89 (d, J = 6.3 Hz, 2H), 2.44 (d, J = 6.0 Hz, 2H).

### Example 22

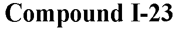

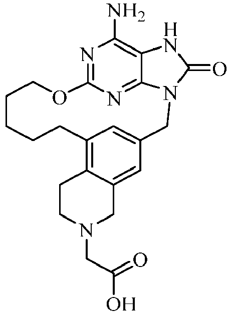

The title **Compound I-23** was prepared in analogy to the preparation of **Compound I-3** by using **compound 89** as a start material. MS: 439.0 (M+H)⁺. ¹H NMR (400 MHz, DMSO-*d₆*) δ 9.95 (s, 1H), 7.58 (s, 1H), 6.83 (s, 1H), 6.36 (s, 2H), 4.81 (s, 2H), 4.32 (s, 2H), 4.23 (t, J = 6.6 Hz, 2H), 4.07 (s, 2H), 3.50-3.60(m, 2H), 2.94 (d, J = 6.5 Hz, 2H), 2.62 (d, J = 6.1 Hz, 2H), 2.38-2.50 (m, 1H), 1.69 (br, 2H), 1.38 (t, J = 7.2 Hz, 2H), 1.31 (br, 2H).

### Example 23

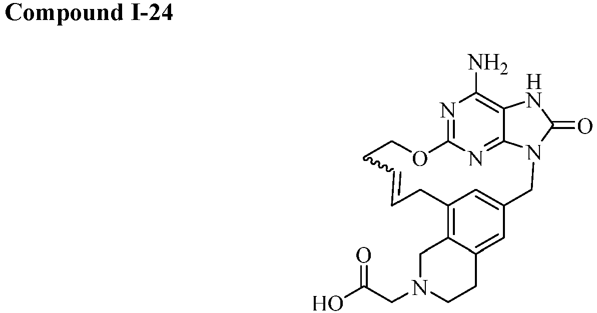

The title **Compound I-24** was prepared in analogy to the preparation of **Compound I-1** and **Compound I-22** by using **Intermediate 1** and **Intermediate 8** as a start material. MS: 437.2 (M+H)⁺.

### Example 24

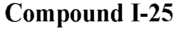

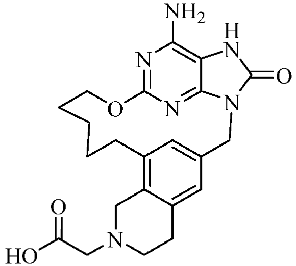

The title **Compound I-25** was prepared in analogy to the preparation of **Compound I-23** by using **Intermediate 1** and **Intermediate 8** as a start material. MS: 439.2 (M+H)⁺. ¹H NMR (400 MHz, DMSO-*d₆*) δ 9.91 (s, 1H), 7.43 (s, 1H), 6.76 (s, 1H), 6.35 (s, 2H), 4.77 (s, 2H), 4.28-4.20 (m, 2H), 3.65-3.75 (m, 2H), 3.35-3.30(m, 2H), 2.83-2.73 (m, 4H), 2.56-2.51 (m, 2H), 1.65 (br, 2H), 1.43-1.25 (m, 4H).

### Example 25

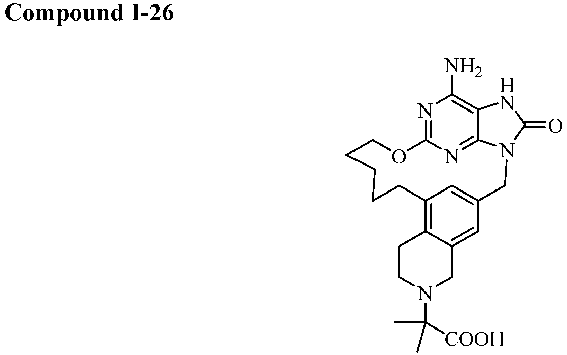

The title **Compound I-26** was prepared in analogy to the preparation of **Compound I-22** and **Compound I-3** by using **compound 87** and ethyl 2-bromo-2-methylpropanoate as a start material. MS: 467.5 (M+H)⁺. ¹H NMR (400 MHz, DMSO-*d₆*) δ 9.96 (s, 1H), 7.57 (s, 1H), 6.91 (s, 1H), 6.37 (s, 2H), 4.82 (, 2H), 4.32 (s, 2H), 4.15-4.25 (m, 2H), 3.30 (m, 2H), 2.95 (m, 2H), 2.66 (m, 2H), 1.69 (br, 2H), 1.51 (s, 6H), 1.35 (m, 4H).

### Example 26

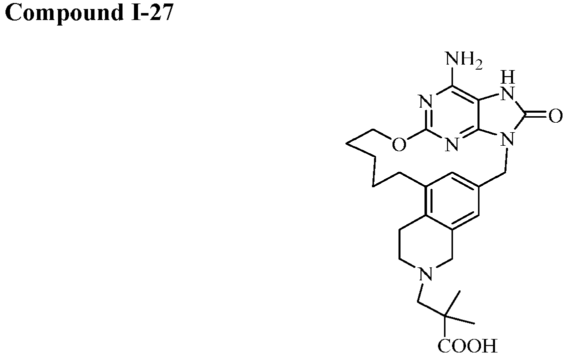

The title **Compound I-27** was prepared in analogy to the preparation of **Compound I-22** and **Compound I-3** by using **compound 87** and ethyl 2,2 '- dimethyl-3-bromopropionate as a start material. MS: 481.2 (M+H)⁺. ¹H NMR (400 MHz, DMSO-*d₆*) δ 9.96 (s, 1H), 7.67 (s, 1H), 6.87 (s, 1H), 6.38 (s, 2H), 4.82 (m, 2H), 4.26-4.32 (m, 4H), 3.60 (m, 2H), 3.26 (br, 2H), 2.95 (br, 2H), 2.49-2.62 (m, 2H), 1.70 (br, 2H), 1.31-1.42 (m, 4H), 1.21 (s, 6H).

### Example 27

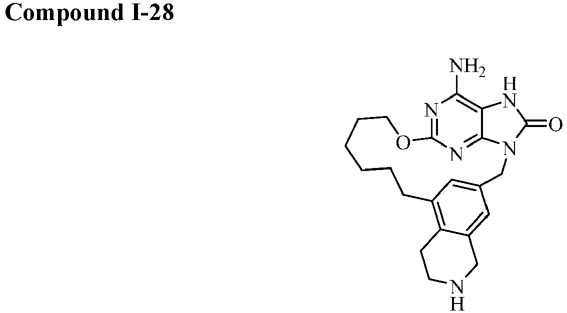

The title **Compound I-28** was prepared in analogy to the preparation of **Compound I-1** and **Compound I-3** by using **Intermediate 3** and **Intermediate 7** as a start material. MS: 383.2 (M+H)⁺. ¹H NMR (400 MHz, DMSO-*d₆*) δ 9.98 (s, 1H), 8.50 (br, 1H), 7.37 (s, 1H), 6.43 (s, 2H), 4.80 (s, 2H), 4.41 (t, J = 6.4 Hz, 2H), 4.23 (s, 2H), 3.34 (br, J = 6.1 Hz, 2H), 2.83 (br, 2H), 2.61-2.53 (m, 2H), 1.56-1.48 (m, 4H), 1.31 (s, 4H).

### Example 28

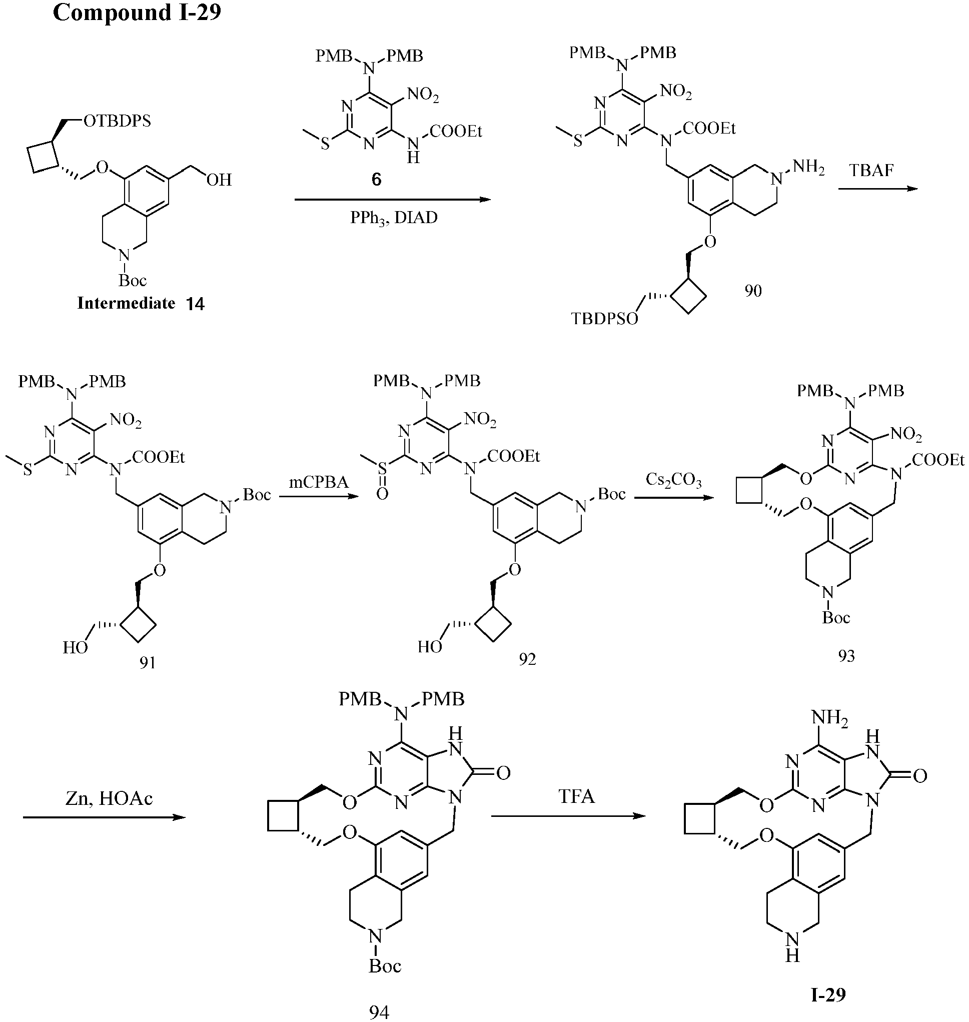

### Step 1: Preparation of compound 90

To **a** stirred mixture solution of **compound 6 (0.5** g, 0.97 mmol) and **Intermediate 14** (0.61 g, 0.97 mmol) in THF (15 mL) was added PPh₃ (0.38 g, 1.46 mmol) and DIAD (295 mg, 1.46 mmol) at 0 °C, then the resulting mixture was stirred at 20 °C for 2 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 90** (0.85 g, 79%) as a yellow solid.

### Step 2: Preparation of compound 91

A mixture solution of **compound 90** (0.85 g, 0.77 mmol), 1.0 M TBAF solution in THF (0.77 mmol, 0.76 mL) in THF (10 mL) was stirred at 25 °C for 12 hours. After the reaction was completed, the reaction mixture was extracted with EA. The combined organic layers were washed with brine, dried over anhydrous Na₂SO₄, filtered and concentrated in vacuo to give a crude product, which was purified by silica gel flash chromatography to afford **compound 91** (0.55 g, 82%) as a yellow solid. MS: 872.4 (M+H)⁺.

### Step 3: Preparation of compound 92

To a stirred mixture solution of **compound 91** (0.55 g, 0.63 mmol) in DCM (20 mL) was added mCPBA (0.13 g, 0.76 mmol) at 0 °C, then the resulting mixture was stirred at 5 °C for 1 hour. After the reaction was completed, the reaction mixture was quenched by adding ice-water and extracted with DCM. The combined organic layers were washed with a solution of NaHCO₃, brine, dried over anhydrous Na₂SO₄, filtered and concentrated in vacuo to give a crude product, which was purified by silica gel flash chromatography to afford **compound 92** (0.53 g) as a yellow solid. MS: 888.4 (M+H)⁺.

### Step 4: Preparation of compound 93

Amixture solution of **compound 92** (0.55 g, 0.63 mmol) and Cs₂CO₃ (606 mg, 1.86 mmol) in THF (10 mL) was stirred at 25 °C for 2 hours. After the reaction was completed, the reaction mixture was quenched by adding ice-water and extracted with DCM. The combined organic layers were washed with a solution of NaHCO₃, brine, dried over anhydrous Na₂SO₄, filtered and concentrated in vacuo to give a crude product, which was purified by silica gel flash chromatography to afford **compound 93** (0.16 g, 31.3%) as a yellow solid. MS: 825.4 (M+H)⁺.

### Step 5: Preparation of compound 94

To a stirred mixture solution of **compound 93** (160 mg, 0.19 mmol) in AcOH (5 mL) was added Zn (63.4 mg, 0.97 mmol) under N₂ protected, then the resulting mixture was stirred at 70 °C for 0.5 hour. After the reaction was completed, the reaction mixture was filtered and concentrated in vacuo to give a crude product, which was purified by silica gel flash chromatography to afford **compound 94** (50 mg, 34.4%) as a white solid. MS: 749.4 (M+H)⁺.

### Step 6: Preparation of Compound I-29

A mixture solution of **compound 94** (50 mg, 0.067 mmol) in TFA (5 mL) was stirred at 70 °C for 1 hour. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to give a crude product, which was purified by pre-HPLC to afford **Compound I-29** (13.45 mg, 49.3%) as a white solid. MS: 507.2 (M+H)⁺. ¹H NMR (400 MHz, DMSO-*d₆*) δ 10.02 (s, 1H), 7.50 (s, 1H), 6.68 (s, 1H), 6.43 (s, 2H), 4.94 (d, J= 13.8 Hz, 1H), 4.77 (br, 1H), 4.58 (dd, *J=* 21.7, 13.5 Hz, 2H), 4.19 (br, 1H), 4.19 (d, *J* = 9.2 Hz, 1H), 4.04 (d, *J* = 13.6 Hz, 1H), 3.93 (br, 1H), 3.30-3.38 (m, 2H), 2.70-2.85 (m, 2H), 2.30-2.40 (m, 8.9 Hz, 2H), 1.89 (q, J= 9.4, 8.7 Hz, 1H), 1.85-1.73 (m, 1H), 1.66 (d, *J=* 8.9 Hz, 2H).

### Example 29

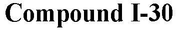

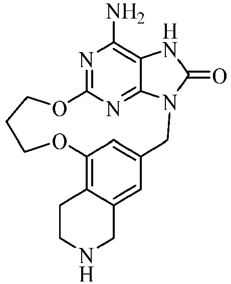

The title **Compound I-30** was prepared in analogy to the preparation of **Compound I-29** by using **Intermediate 6** and **Intermediate 15** as a start material. MS: 369.2 (M+H)⁺. ¹H NMR (400 MHz, DMSO-*d₆*) δ 10.01 (s, 1H), 7.83 (s, 1H), 6.72 (s, 1H), 6.45 (s, 2H), 4.85 (s, 2H), 4.44 (s, 2H), 4.34 (t, J = 6.9 Hz, 2H), 4.18 (s, 1H), 4.18 (d, J = 9.0 Hz, 1H), 3.31 (s, 2H), 2.71 (t, J = 6.4 Hz, 2H), 1.86 (s, 2H).

### Example 30

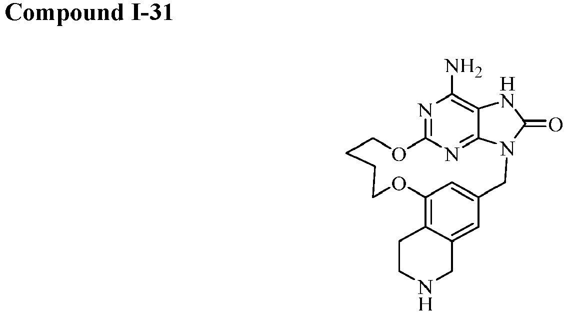

The title **Compound I-31** was prepared in analogy to the preparation of **Compound I-29** by using **Intermediate 6** and **Intermediate 16** as a start material. MS: 383.2 (M+H)⁺. ¹H NMR (400 MHz, DMSO-*d₆*) δ 9.99 (s, 1H), 7.82 (s, 1H), 6.69 (s, 1H), 6.42 (s, 2H), 4.80 (s, 2H), 4.56 (d, J = 6.8 Hz, 2H), 4.35 (t, J = 6.7 Hz, 2H), 4.18 (d, J = 4.9 Hz, 2H), 3.31 (d, J = 6.7 Hz, 2H), 2.72 (t, J = 6.4 Hz, 2H), 1.63 (d, J = 7.5 Hz, 2H), 1.53 (d, J = 7.5 Hz, 2H).

### Example 31

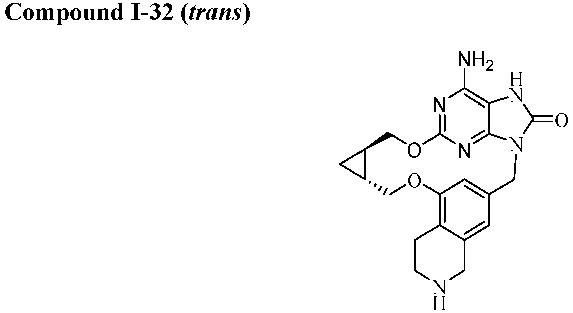

The title **Compound I-32** was prepared in analogy to the preparation of **Compound I-29** by using **Intermediate** 6 and **Intermediate 17** as a start material. MS: 394.2 (M+H)⁺. ¹H NMR (400 MHz, DMSO-*d₆*) δ 9.98 (s, 1H), 7.52 (s, 1H), 6.73 (s, 1H), 6.42 (s, 2H), 4.97 (d, J = 13.9 Hz, 1H), 4.88 (d, J = 11.4 Hz, 1H), 4.55-4.67 (m, 2H), 4.17 (br, 2H), 3.90-4.00(m, J = 12.8, 4.4 Hz, 1H), 3.53-3.45 (m, 1H), 3.32-3.13 (m, 2H), 2.65-2.75 (m, 2H), 1.17 (br, 1H), 0.94 (br, 1H), 0.58-0.40 (m, 2H).

### Example 32

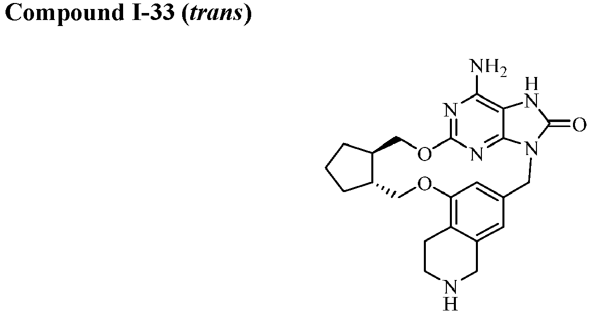

The title **Compound I-33** was prepared in analogy to the preparation of **Compound I-29** by using **Intermediate 6** and **Intermediate 19** as a start material. MS: 422.5 (M+H)⁺. ¹H NMR (400 MHz, MeOH-*d₄*) δ 7.76 (s, 1H), 6.80 (s, 1H), 4.94 (s, 2H), 4.82-4.78 (m, 1H), 4.45-4.38 (m, 1H), 4.26 (br, 3H), 3.50-3.40 (m, 2H), 2.91 (t, J = 6.5 Hz, 2H), 2.12 (br, 1H), 1.93 (br, 1H), 1.80-1.45 (m, 7H).

### Example 33

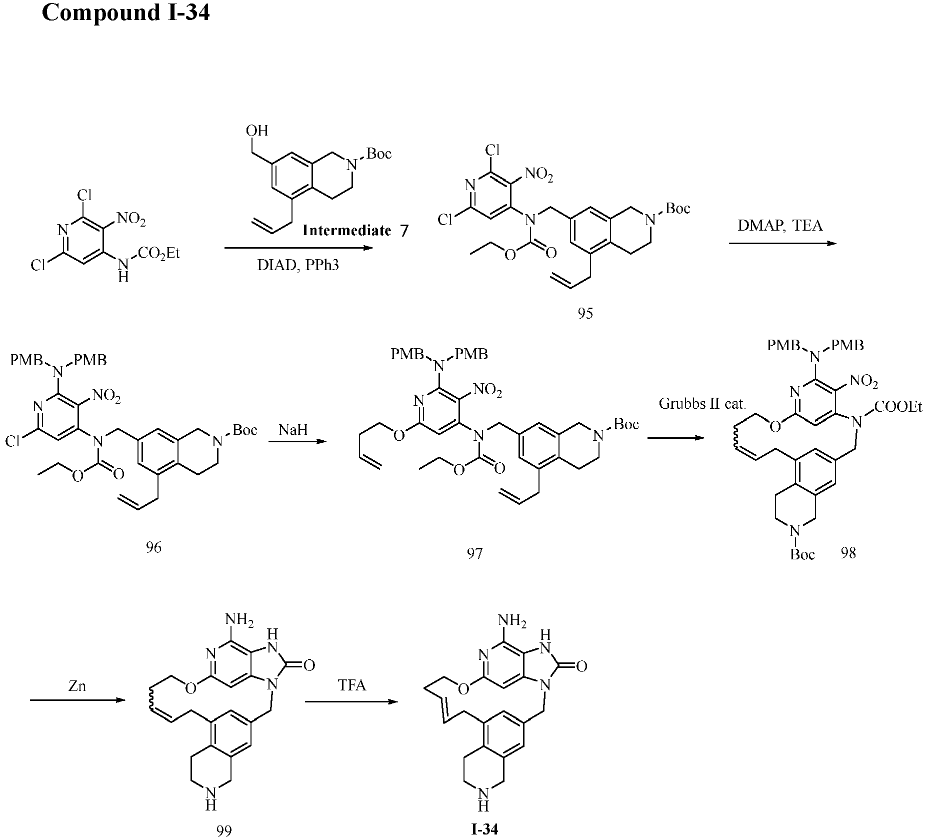

### Step 1: Preparation of compound 95

To a stirred mixture solution of **Intermediate** 2 (1.25 g, 4.45 mmol) and **Intermediate 7** (0.9 g, 2.97 mmol) in THF (30 mL) was added PPh₃ (1.56 g, 5.93 mmol) and DIAD (0.90 g, 4.45 mmol) at 0 °C, then the resulting mixture was stirred at 20 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 95** (1.3 g, 78%) as a yellow oil. MS: 509.1 (M+H)⁺.

### Step 2: Preparation of compound 96

To a stirred mixture solution of **compound 95** (1.3 g, 2.3 mmol), DMAP (12 g, 98.2 mmol) and TEA (0.465 g, 4.60 mmol) in DCM (10 mL) was added bis (4-methoxybenzyl) amine (0.65 g, 2.53 mmol) at 0 °C, then the resulting mixture was stirred at 20 °C for 7 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 96** (330 mg, 18.3%) as a yellow oil. MS: 786.1 (M+H)⁺.

### Step 3: Preparation of compound 97

To a stirred mixture solution of butyl-3-ene-1-ol (605 mg, 8.39 mmol) in THF (3 mL) was added NaH (50.4 mg, 1.26 mmol) at 25 °C, then the resulting mixture was stirred at 25 °C for 35 mins. A solution of **compound 96** (330 mg, 0.42 mmol) in THF (3 mL) was added to the above mixture solution at 0 °C, then the resulting mixture was stirred at 25 °C for 2 hours. After the reaction was completed, the reaction mixture was poured into ice-water and extracted with EA. The combined organic layers were washed with brine, dried over anhydrous Na₂SO₄, filtered and concentrated in vacuo to give a crude product, which was purified by silica gel flash chromatography to afford **compound 97** (320 mg, 93%) as a yellow oil. MS: 822.1 (M+H)⁺.

### Step 4: Preparation of compound 98

To a stirred mixture solution of **compound 97** (320 mg, 0.389 mmol) in DCM (100 mL) was added Grubbs'II reagent (66.1 mg, 0.078 mmol) under N₂ protected, then the resulting mixture was stirred at 20 °C for 3 hours. After the reaction was completed, the reaction mixture was concentrated in vacuo to give a crude product, which was purified by silica gel flash chromatography to afford **compound 98** (190 mg, 61.5%) as a yellow oil. MS: 794.4 (M+H)⁺.

### Step 5: Preparation of compound 99

To a stirred mixture solution of **compound 98** (190 mg, 0.24 mmol) in AcOH (10 mL) and water (1 mL) was added Zn (78 mg, 1.2 mmol) under N₂ protected, then the resulting mixture was stirred at 70 °C for 3 hours. After the reaction was completed, the reaction mixture was filtered and concentrated in vacuo to give a crude product, which was purified by silica gel flash chromatography to afford **compound 99** (50 mg, 29.1%) as a yellow oil. MS: 718.5 (M+H)⁺.

### Step 6: Preparation of Compound I-34

A mixture solution of **compound 99** (50 mg, 0.07 mmol) in TFA (5 mL) was stirred at 80 °C for 3 hours. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to give a crude product, which was purified by pre-HPLC to afford **Compound I-34** (19 mg, 71.9%) as a white solid. MS: 378.1 (M+H)⁺. ¹H NMR (400 MHz, DMSO-*d₆*) δ 10.41 (s, 1H), 8.94 (s, 2H), 7.22 (s, 1H), 6.96 (d, *J=* 5.2 Hz, 1H), 6.09 (s, 1H), 5.69 (dd, *J=* 15.2, 6.8 Hz, 1H), 4.95-5010 (m, 1H), 4.92 (d, *J*= 5.3 Hz, 2H), 4.34 (br, 2H), 4.22 (br, 2H), 3.19 (br, , 4H), 2.83 (d, *J*= 6.4 Hz, 2H), 2.37 (br, 2H).

### Example 34

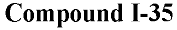

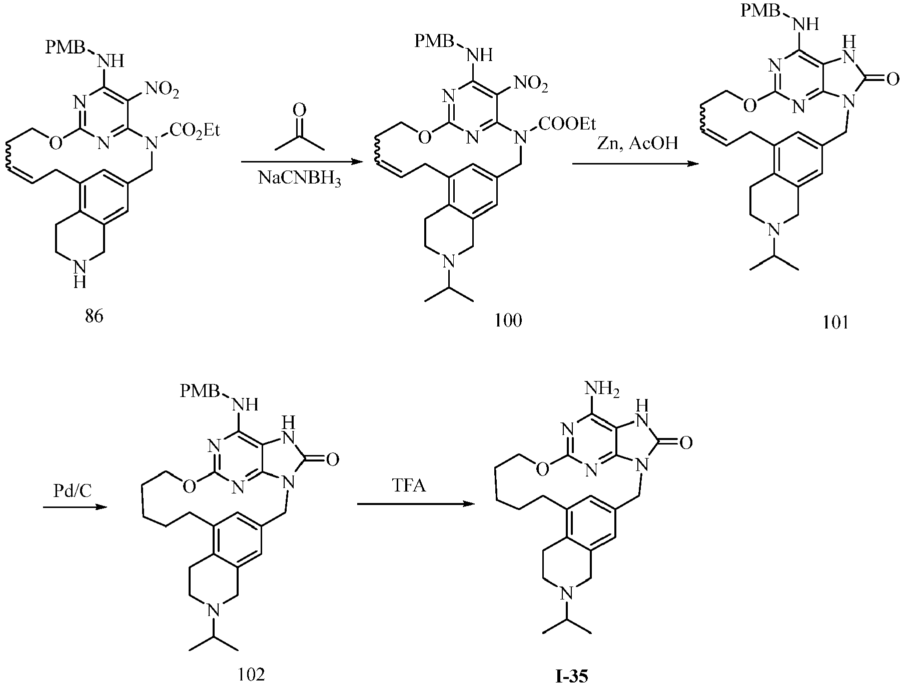

### Step 1: Preparation of compound 100

To a stirred mixture solution of **compound 99** (350 mg, 0.51 mmol l) in MeOH (10 mL) was added acetone (590 mg, 10.16 mmol) at 20 °C, then the resulting mixture was stirred at 25 °C for 1 hour. Then NaCNBH₃ (63.9 mg, 1.02 mmol) was added to the above mixture solution and the resulting mixture was stirred at 25 °C for 13 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **compound 100** (270 mg) as a yellow oil. MS: 617.0 (M+H)⁺.

### Step 2: Preparation of compound 101

To a stirred mixture solution of **compound 100** (70 mg, 0.44 mmol) in AcOH (7.5 mL) was added Zn (143 mg, 2.19 mmol) under N₂ protected, then the resulting mixture was stirred at 80 °C for 0.5 hour. After the reaction was completed, the reaction mixture was filtered and concentrated in vacuo to give a crude product, which was purified by silica gel flash chromatography to afford **compound 101** (90 mg, 27%) as a yellow oil. MS: 541.0 (M+H)⁺.

### Step 3: Preparation of compound 102

A mixture solution of **compound 101** (90 mg, 0.12 mmol) and Pd/C (126 mg) in MeOH (5 mL) and EA (5 mL) under H₂ atmosphere was stirred at 25 °C for 14 hours. After the reaction was completed, the reaction mixture was filtered and concentrated in vacuo to afford **compound 102** (39 mg, 60.8%) as a yellow solid. MS: 543.0 (M+H)⁺.

### Step 4: Preparation of Compound I-35

A mixture solution of **compound 102** (39 mg, 0.072 mmol) in TFA (7.5 mL) was stirred at 80 °C for 1 hour. After the reaction was completed, the reaction mixture was concentrated in vacuo and extracted with EA. The combined organic layers were washed with solution of NaHCO₃, brine, dried over anhydrous Na₂SO₄, filtered and concentrated in vacuo to give a crude product, which was purified by pre-HPLC to afford **Compound I-35** (12.49 mg, 39.7%) as a white solid. MS: 423.0 (M+H)⁺. ¹H NMR (400 MHz, DMSO-*d₆*) δ 10.04 (s, 1H), 7.59 (s, 1H), 6.89 (s, 1H), 6.41 (s, 2H), 4.85 (d, J = 14.3 Hz, 1H), 4.79 (d, J = 14.4 Hz, 1H), 4.31 (d, J = 5.8 Hz, 2H), 4.27-4.14 (m, 2H), 3.68-3.50 (m, 3H), 3.35-3.11 (m, 3H), 3.04-2.85 (m, 2H), 2.64 (br 2H), 1.68 (br, 2H), 1.42-1.20 (m, 7H).

### Example 35

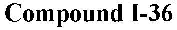

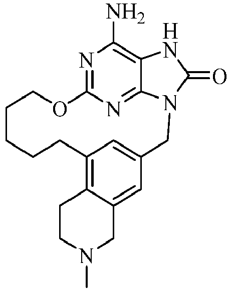

The title **Compound I-36** was prepared in analogy to the preparation of **Compound I-35** by using **compound 86** and paraformaldehyde as a start material. MS: 395.5 (M+H)⁺.

### Example 36

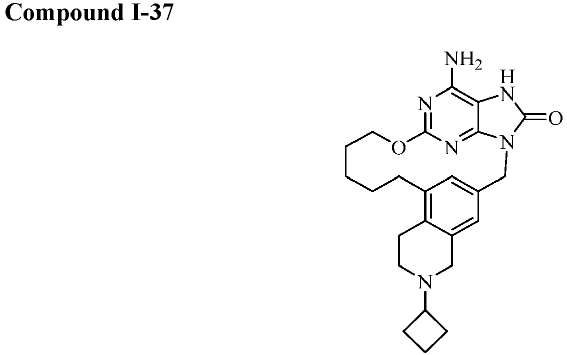

The title **Compound I-37** was prepared in analogy to the preparation of **Compound I-35** by using **compound 86** and cyclobutanone as a start material. MS: 435.2 (M+H)⁺. ¹H NMR (400 MHz, DMSO-*d₆*) δ 9.82 (s, 1H), 7.59 (s, 1H), 6.89 (s, 1H), 6.40 (s, 2H), 4.87 (d, J = 14.4 Hz, 1H), 4.75 (d, J = 14.5 Hz, 1H), 4.40 (d, J = 15.4 Hz, 1H), 4.26 (br, 1H), 4.15 (d, J = 9.5 Hz, 1H), 4.10-4.02 (m, 1H), 3.74-3.68 (m, 1H), 3.60-3.50 (m, 1H), 3.08 (d, J = 10.8 Hz, 1H), 2.98-2.83 (m, 2H), 2.62 (br, 2H), 2.28-2.13 (m, 4H), 1.69 (d, J = 40.2 Hz, 4H), 1.42-1.26 (m, 4H).

### Example 37

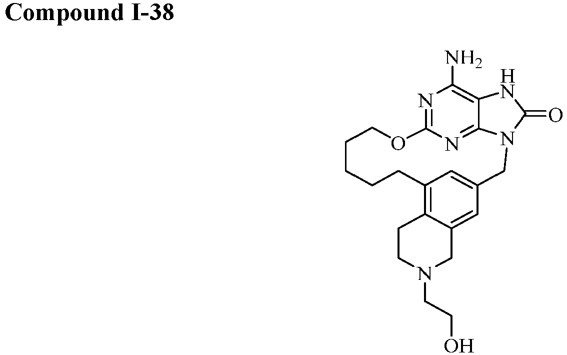

The title **Compound I-38** was prepared in analogy to the preparation of **Compound I-35** by using **compound 86** and 2-hydroxyacetaldehyde as a start material. MS: 425.5 (M+H)⁺. ¹H NMR (400 MHz, DMSO-*d₆*) δ 10.06 (s, 1H), 7.59 (s, 1H), 6.84 (s, 1H), 6.45 (s, 2H), 4.87 (d, J = 14.4 Hz, 1H), 4.76 (dd, J = 11.7, 5.2 Hz, 3H), 4.40-4.50 (m, 1H), 4.29 (d, J = 9.9 Hz, 2H), 4.17 (d, J = 8.7 Hz, 1H), 3.70-3.80 (m, 2H), 3.32-3.15 (m, 3H), 2.94 (br, 2H), 2.68-2.57 (m, 2H), 1.68 (d, J = 33.6 Hz, 2H), 1.40-1.29 (m, 4H).

### Example 38

### Compound I-39

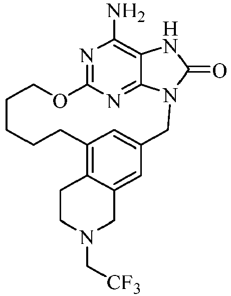

The title **Compound I-39** was prepared in analogy to the preparation of **Compound I-35** by using **compound 86** and 2,2,2-trifluoroacetaldehyde as a start material. MS: 463.3 (M+H)⁺. ¹H NMR (400 MHz, DMSO-*d₆*) δ 9.94 (s, 1H), 7.47 (s, 1H), 6.69 (s, 1H), 6.43 (s, 2H), 4.77 (s, 2H), 4.20-4.30 (m, 2H), 3.74 (br, 2H), 3.28 (d, J = 10.1 Hz, 2H), 2.89 (d, J = 6.0 Hz, 2H), 2.67 (d, J = 6.3 Hz, 2H), 2.60-2.54 (m, 2H), 1.67 (br, 2H), 1.44-1.37 (m, 2H), 1.29 (d, J = 7.9 Hz, 2H).

### Example 39

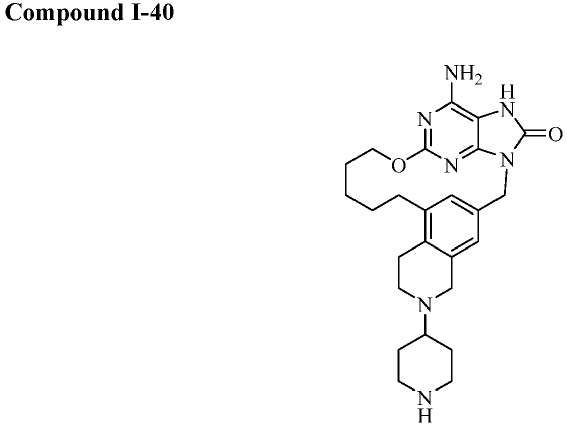

The title **Compound I-40** was prepared in analogy to the preparation of **Compound I-35** by using **compound 86** and piperidin-4-one as a start material. MS: 464.4 (M+H)⁺. ¹H NMR (400 MHz, DMSO-*d₆*) δ 10.04 (s, 1H), 7.61 (s, 1H), 6.91 (s, 1H), 6.42 (s, 2H), 4.87 (d, J = 14.2 Hz, 1H), 4.78 (d, J = 14.4 Hz, 1H), 4.38 (br, 1H), 4.25 (br, 1H), 4.19 (br 1H), 3.52-3.40 (m, 3H), 3.30 (br, 1H), 2.85-2.96 (m, 4H), 2.65 (br, 2H), 2.26 (br, 2H), 1.81 (d, J = 12.1 Hz, 2H), 1.73 (br, 2H), 1.66 (br, 1H), 1.30-1.38 (m, 5H).

### Example 40

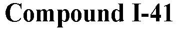

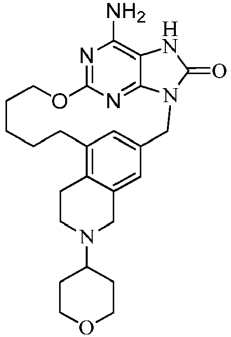

The title **Compound I-41** was prepared in analogy to the preparation of **Compound I-35** by using **compound 86** and dihydro-2H-pyran-4(3H)-one as a start material. MS: 465.4 (M+H)⁺. ¹H NMR (400 MHz, DMSO-*d₆*) δ 10.00 (s, 1H),7.60 (s, 1H), 6.91 (s, 1H), 6.41 (s, 2H), 4.87 (d, J = 14.4 Hz, 1H), 4.78 (d, J = 14.4 Hz, 1H), 4.45 (d, J = 15.1 Hz, 1H), 4.25 (br, 1H), 4.19 (br, 1H), 3.97 (d, J = 11.0 Hz, 2H), 3.75 (br, 1H), 3.25-3.35 (m, J = 18.0, 11.7 Hz, 4H), 3.02 (d, J = 17.8 Hz, 1H), 2.96-2.84 (m, 1H), 2.66 (br, 2H), 2.08-1.94 (m, 2H), 1.77-1.60 (m, 4H), 1.25-1.35 (m, 5H).

### Example 41

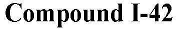

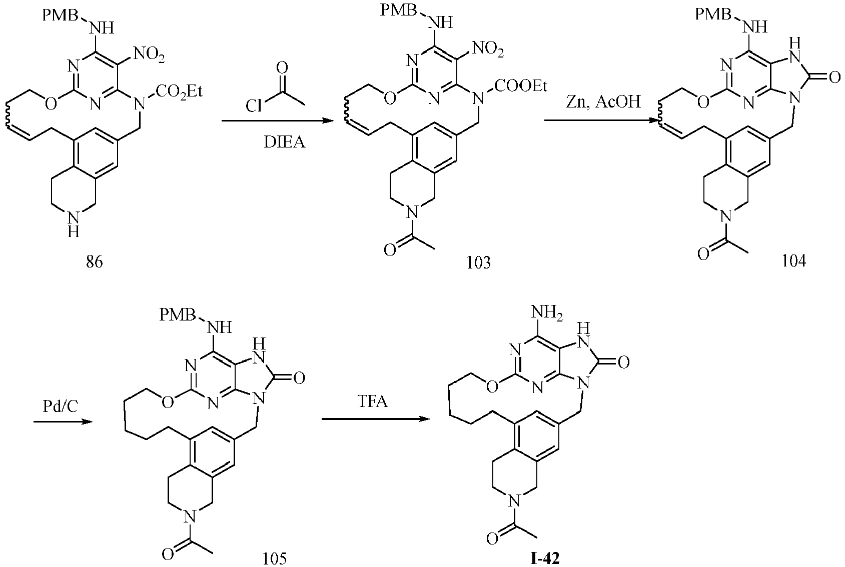

### Step 1: Preparation of compound 103

To a stirred mixture solution of **compound 86** (300 mg, 0.44 mmol) and DIEA (225 mg, 1.743 mmol) in DCM (5 mL) was added AcCl (35.0 mg, 0.45 mmol) at 0 °C, then the resulting mixture was stirred at 25 °C for 12 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **compound 103** (110 mg, 36.4%) as a yellow solid. MS: 617.0 (M+H)⁺.

### Step 2: Preparation of compound 104

To a stirred mixture solution of **compound 103** (100 mg, 0.16 mmol) in AcOH (7.5 mL) was added Zn (53.0 mg, 0.81 mmol) under N₂ protected, then the resulting mixture was stirred at 80 °C for 0.5 hour. After the reaction was completed, the reaction mixture was filtered and concentrated in vacuo to give a crude product, which was purified by silica gel flash chromatography to afford **compound 104** (110 mg) as a black oil. MS: 541.0 (M+H)⁺.

### Step 3: Preparation of compound 105

A mixture solution of **compound 104** (110 mg) and Pd/C (20 mg) in EA (10 mL) under H₂ atmosphere was stirred at 25 °C for 14 hours. After the reaction was completed, the reaction mixture was filtered and concentrated in vacuo to afford **compound 105** (0.11 g) as a yellow oil.

### Step 4: Preparation of Compound I-42

A mixture solution of **compound 105** (110 mg, 0.2 mmol) in TFA (7.5 mL) was stirred at 70 °C for 0.5 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-42** (7.75 mg) as a yellow solid. MS: 421.0 (M+H)⁺. ¹H NMR (400 MHz, DMSO-*d₆*) δ 9.88, (s, 1H), 7.96 (s, 1H), 6.97 (s, 1H), 6.44 (s, 2H), 5.81-5.32 (m, 2H), 4.85-4.68 (m, 4H), 4.58, (br, 2H), 3.67-3.54 (m, 2H), 3.21 (d, J = 7.7 Hz, 2H), 3.09 (d, J = 7.2 Hz, 2H), 2.71-2.54 (m, 2H), 2.45-2.35 (m, 2H), 2.04 (s, 3H).

### Example 42

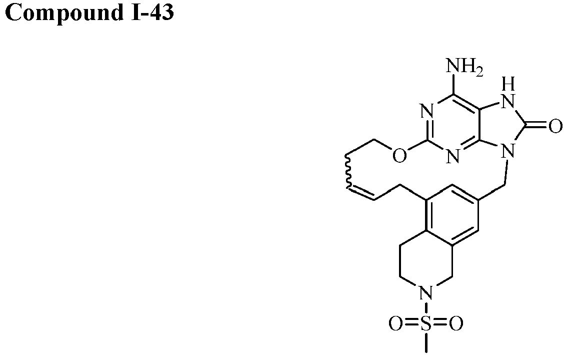

The title **Compound I-43** was prepared in analogy to the preparation of **Compound I-42** by using **compound 86** and methanesulfonyl chloride as a start material. MS: 457.0 (M+H)⁺. ¹H NMR (400 MHz, DMSO-*d₆*)δ 9.86 (s, 1H), 7.97 (s, 1H), 7.69 (s, 1H), 6.39 (s, 2H), 5.75-5.85(m, 1H), 5.305.40 (m, 1H), 4.77 (s, 2H), 4.72 (br, 2H), 4.29 (br, 2H), 3.21(d, J = 7.7 Hz, 2H), 3.08 (d, J = J = 7.2 Hz, 2H), 2.85-2.95 (m, 3H), 2.75-2.67 (m, 2H), 2.45-2.36 (m, 2H).

### Example 43

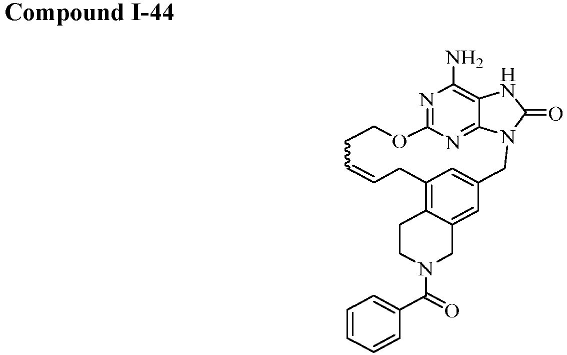

The title **Compound I-44** was prepared in analogy to the preparation of **Compound I-42** by using **compound 86** and benzoyl chloride as a start material. MS: 483.0 (M+H)⁺. ¹H NMR (400 MHz, DMSO-*d₆*) δ 9.83 (s, 1H), 7.98 (s, 1H), 7.55-7.22 (m, 5H), 7.67-6.68 (m, 1H), 6.37 (s, 2H), 5.56 (m, 2H), 4.99-4.32 (m, 6H), 3.84-3.48 (m, 2H), 3.07 (d, J = 6.9 Hz, 2H), 2.65 (br, 2H), 2.42 (d, J = 6.8 Hz, 2H).

### Example 44

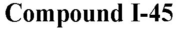

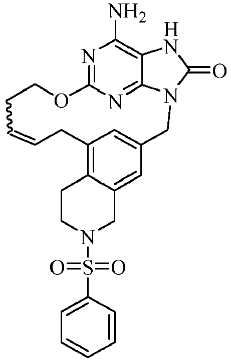

The title **Compound I-45** was prepared in analogy to the preparation of **Compound I-42** by using **compound 86** and benzenesulfonyl chloride as a start material. MS: 519.0 (M+H)⁺. ¹H NMR (400 MHz, DMSO-*d₆*) δ 9.82 (s, 1H), 7.93 (s, 1H), 7.80 (d, J = 7.8 Hz, 2H), 7.70-7.47 (m, 3H), 6.93 (s, 1H), 6.36 (s, 2H), 5.80-5.27 (m, 2H), 4.86-4.51 (m, 4H), 4.11 (br, 2H), 3.24 (t, J = 6.3 Hz, 2H), 3.14 -2.93 (m, 2H), 2.64 (t, J = 6.2 Hz, 2H), 2.45-2.35 (m, 2H).

### Example 45

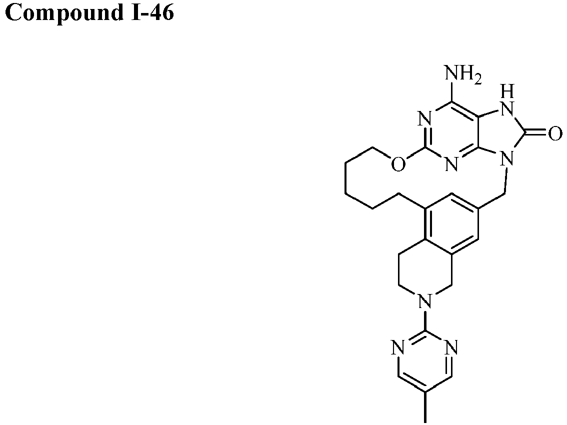

A mixture solution of **Compound I-10** (40 mg, 0.081 mmol) and 2-chloro-5-methylpyrimidine (20.80 mg, 0.16 mmol) in DMSO (1 mL) was stirred at 120 °C for 1 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-46** (3.34 mg, 7.8%) as a yellow solid. MS: 473.0 (M+H)⁺. ¹H NMR (400 MHz, DMSO-*d₆*) δ 9.95 (s, 1H), 7.48 (s, 1H), 6.86 (s, 1H), 6.45 (s, 2H), 4.80 (br, 2H), 4.73 (br, 2H), 4.20-4.30 (m, , 2H), 3.85-3.95 (m, 2H), 2.65-2.75 (m, 2H), 2.64-2.58 (m, 2H), 2.06 (br, 3H), 1.67 (br, 2H), 1.40 (t, J = 7.2 Hz, 2H), 1.28 (d, J = 8.0 Hz, 2H).

### Example 46

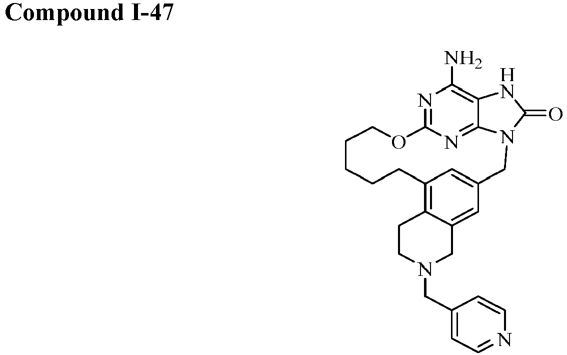

A mixture solution of **Compound I-10** (40 mg, 0.081 mmol), 4-(bromomethyl)pyridine (20.46 mg, 0.081 mmol) and DIEA (20.91 mg, 0.162 mmol) in DMF (1 mL) was stirred at 20 °C for 1 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-47** (10.23 mg, 26.5%) as a white solid. MS: 472.0 (M+H)⁺. ¹H NMR (400 MHz, DMSO-*d₆*) δ 10.00 (s, 1H), 8.71 (d, J = 5.2 Hz, 2H), 7.65-7.48 (m, 3H), 6.84 (s, 1H), 6.43 (s, 2H), 4.80 (br, 2H), 4.42 (br, 2H), 4.33-4.12 (m, 4H), 3.33 (br, 2H), 2.94 (br, 2H), 2.63 (br, 2H), 1.68 (br, 2H), 1.42-1.18 (m, 4H).

### Example 47

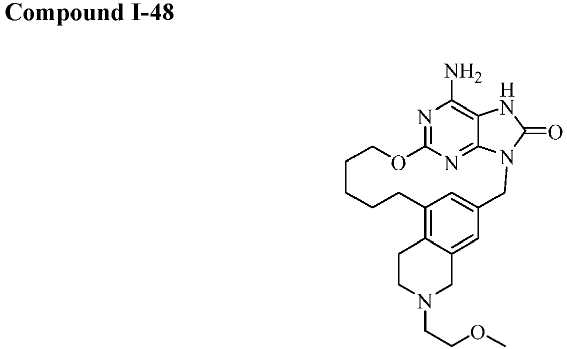

The title **Compound I-47** was prepared in analogy to the preparation of **Compound I-35** by using **compound 86** and 2-methoxyacetaldehyde as a start material. MS: 439.3 (M+H)⁺. ¹H NMR (400 MHz, DMSO-*d₆*) δ 10.04 (s, 1H), 7.59 (s, 1H), 6.85 (s, 1H), 6.42 (s, 2H), 4.89-4.74 (m, 2H), 4.46 (d, J = 15.5 Hz, 1H), 4.31-4.15 (m, 3H), 3.60-3.70 (m, , 2H), 3.37 (br, 2H), 3.30 (br, 5H), 2.94 (d, J = 5.8 Hz, 2H), 2.63 (br, 2H), 1.68 (m, 2H), 1.35 (m, 4H).

### Example 48

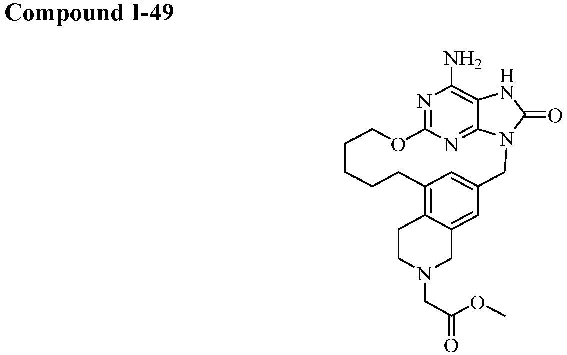

The title **Compound I-49** was prepared in analogy to the preparation of **Compound I-25**. MS: 453.3 (M+H)⁺. ¹H NMR (400 MHz, DMSO-*d₆*) δ 9.99 (s, 1H), 7.60 (s, 1H), 6.83 (s, 1H), 6.41 (s, 2H), 4.82 (br, 2H), 4.36 (br, 2H), 4.15-4.27 (m, 4H), 3.76 (br, 3H), 2.95 (br 2H), 2.63 (s, 3H), 1.69 (br, 2H), 1.38 (br, 2H), 1.35-1.28 (m, 3H).

### Example 49

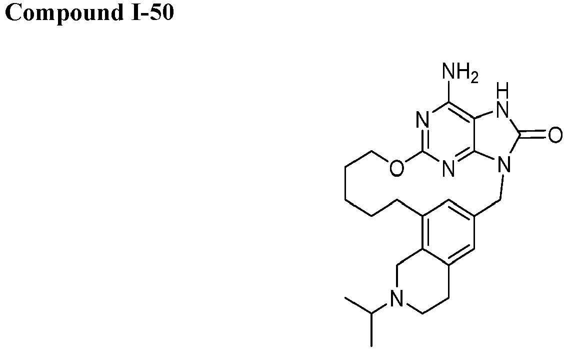

The title **Compound I-50** was prepared in analogy to the preparation of **Compound I-35** by using **Compound I-13** and acetone as a start material. MS: 422.5 (M+H)⁺. ¹H NMR (400 MHz, DMSO-*d₆*) δ 10.14 (s, 1H), 7.52 (s, 1H), 6.85 (s, 1H), 6.47 (s, 2H), 4.79 (br, 2H), 4.25 (br, 3H), 3.72-3.48 (m, 1H), 2.94 (br, 3H), 2.60 (br, 2H), 2.47-2.42 (m, 1H), 1.69 (br, 2H), 1.45-1.19 (m, 10H).

### Example 50

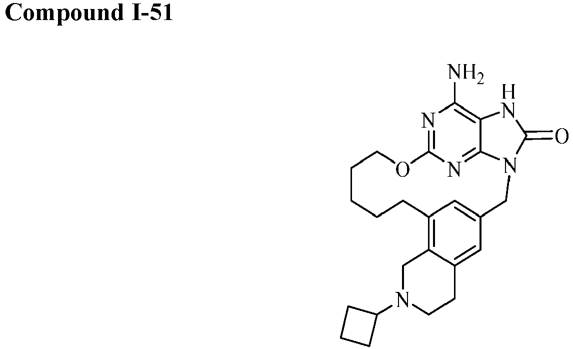

The title **Compound I-51** was prepared in analogy to the preparation of **Compound I-35** by using **Compound I-13** and cyclobutanone as a start material. MS: 434.5 (M+H)⁺. ¹H NMR (400 MHz, DMSO-*d₆*) δ 9.90 (s, 1H), 7.54 (s, 1H), 6.88 (s, 1H), 6.36 (s, 2H), 4.80 (br 2H), 4.25 (m, 3H), 3.80-3.90 (m, 1H), 2.90-3.00 (m, 3H), 2.40-2.50 (m, 1H), 2.22 (br, 4H), 1.70 (br, 4H), 1.34 (d, J = 35.6 Hz, 4H).

### Example 51

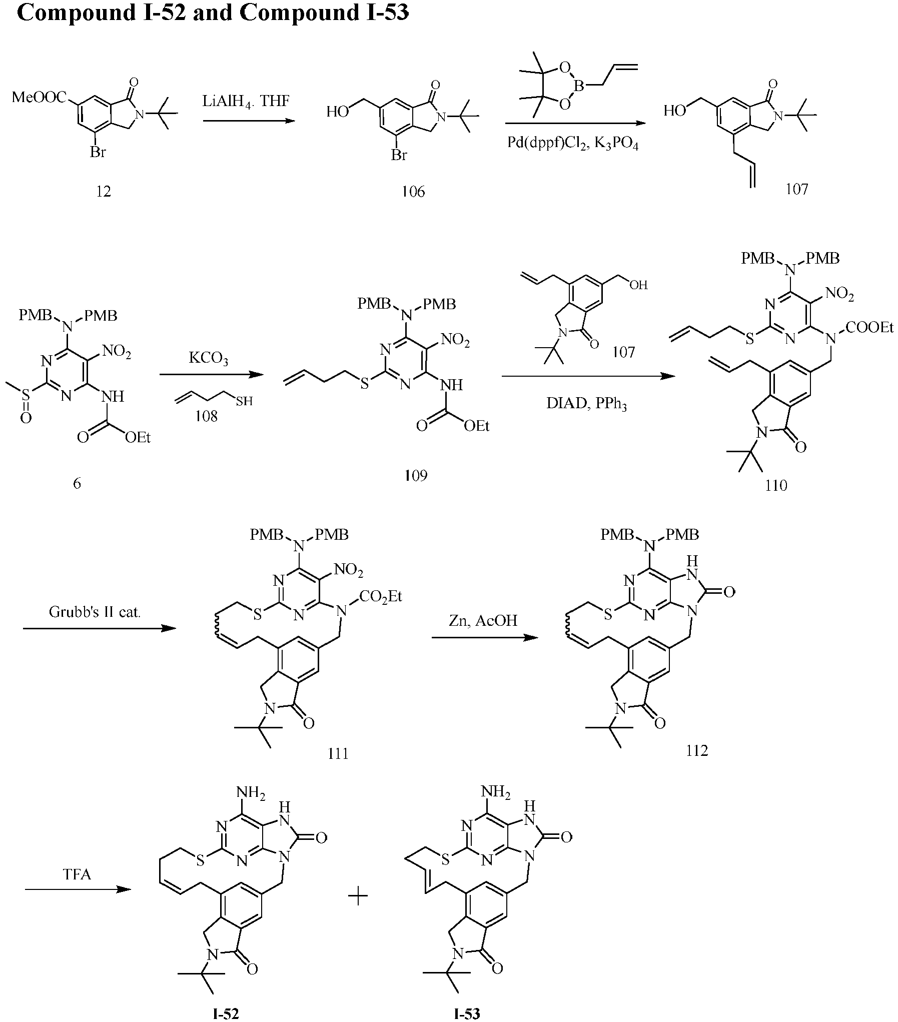

### Step 1: Preparation of compound 106

To a stirred mixture solution of **compound 12** (3.6 g, 11.04 mmol) in THF (30 mL) was added lithium aluminum tetrahydride (837.7 mg, 22.1 mmol) solution in THF (30 mL) at 0 °C, then the resulting mixture was stirred at 0 °C for 0.5 hour. After the reaction was completed, the reaction mixture was quenched by adding ice-water and extracted with EA. The combined organic layers were washed with brine, dried over anhydrous Na₂SO₄, filtered and concentrated in vacuo to give a crude product, which was purified by silica gel flash chromatography to afford **compound 106** (2.6 g, 79%) as a white solid. MS: 300.0 (M+H)⁺.

### Step 2: Preparation of compound 107

A mixture solution of **compound 106** (2.1 g, 7.04 mmol), 4,4,5,5-tetramethyl-2-prop-2-enyl-1,3,2-dioxaborolane (1.78 g, 10.56 mmol), Pd(dppf)Cl₂ (514.83 mg, 704.28 mmol) and potassium metaphosphate (2.99 g, 14.09 mmol) in Dioxane (20 mL) and water (5 mL) was stirred at 80 °C for 5 hours under N₂ protected. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 107** (500 mg, 88%) as a white solid. MS: 300.0 (M+3H)⁺. ¹H NMR (400 MHz, CDCl₃) δ 7.63 (s, 1H), 7.42 (s, 1H), 6.00-5.87 (m, 1H), 5.15-5.05 (m, 2H), 4.75 (d, J = 5.2 Hz, 2H), 4.36 (s, 2H), 3.42 (d, J = 6.5 Hz, 2H), 1.96 (t, J = 5.8 Hz, 1H), 1.56 (s, 9H).

### Step 3: Preparation of compound 109

A mixture solution of **compound 108** (499.49 mg, 5.67 mmol), K₂CO₃ (1.78 g, 10.56 mmol) and **compound 6** (2 g, 3.78 mmol) in THF (20 mL) was stirred at 20 °C for 5 mins. After the reaction was completed, the reaction mixture was filtered and concentrated in vacuo to give a crude product, which was purified by silica gel flash chromatography to afford **compound 109** (1.73 g, 83%) as a yellow oil. MS: 554.0 (M+H)⁺.

### Step 4: Preparation of compound 110

To a stirred mixture solution of **compound 109** (0.2 g, 0.36 mmol) and **compound 107** (93.69 mg, 0.36 mmol) in THF (5 mL) was added PPh₃ (142.13 mg, 0.54 mmol) and DIAD (109.57 mg, 0.54 mmol) at 0 °C, then the resulting mixture was stirred at 65 °C for 16 hours. After the reaction was completed, the reaction mixture was poured into ice-water and extracted with DCM. The combined organic layers were washed with brine, dried over anhydrous Na₂SO₄, filtered and concentrated in vacuo to give a crude product, which was purified by silica gel flash chromatography to afford **compound 110** (0.28 g, 97.5%) as a yellow solid. MS: 795.1 (M+H)⁺.

### Step 5: Preparation of compound 111

To a stirred mixture solution of **compound 110** (0.3 g, 0.38 mmol) in DCM (400 mL) was added Grubbs'II reagent (0.38 mmol) under N₂ protected, then the resulting mixture was stirred at 20 °C for 24 hours. After the reaction was completed, the reaction mixture was concentrated in vacuo to give a crude product, which was purified by silica gel flash chromatography to afford **compound 111** (0.13 g, 17.3%) as a yellow solid. MS: 767.4 (M+H)⁺.

### Step 6: Preparation of compound 112

To a stirred mixture solution of **compound 111** (68 mg, 0.09 mmol) in AcOH (5 mL) was added Zn (38.1 mg) under N₂ protected, then the resulting mixture was stirred at 70 °C for 1 hour. After the reaction was completed, the reaction mixture was filtered and concentrated in vacuo to give a crude product, which was purified by silica gel flash chromatography to afford **compound 112** (56 mg) as a white solid. MS: 691.0 (M+H)⁺.

### Step 7: Preparation of Compound I-52 and Compound I-53

A mixture solution of **compound 112** (56 mg) in TFA (2 mL) was stirred at 70 °C for 1 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-52** and **Compound I**-**53.**

**Compound I-52** (white solid, 2.8mg). MS: 451.0 (M+H)⁺. ¹H NMR (400 MHz, DMSO-*d₆*) δ 10.12 (s, 1H), 7.86 (s, 1H), 7.40 (s, 1H), 6.53 (s, 2H), 5.81-5.60 (m, 2H), 4.93 (s, 2H), 4.52 (s, 2H), 2.39 (s, 2H), 2.05-1.90 (m, 2H), 1.47 (s, 9H).

**Compound 1-53** (white solid, 3.0mg). MS: 451.0 (M+H)⁺. ¹H NMR (400 MHz, DMSO-*d₆*) δ 10.05 (s, 1H), 8.18 (s, 1H), 7.40 (s, 1H), 6.51 (s, 2H), 5.80-5.70 (m, 2H), 4.93 (s, 2H), 4.47 (s, 2H), 3.51 (s, 2H), 3.24 (d, J = 5.1 Hz, 2H), 2.49-2.44 (m, 2H), 1.47 (s, 9H).

### Example 52

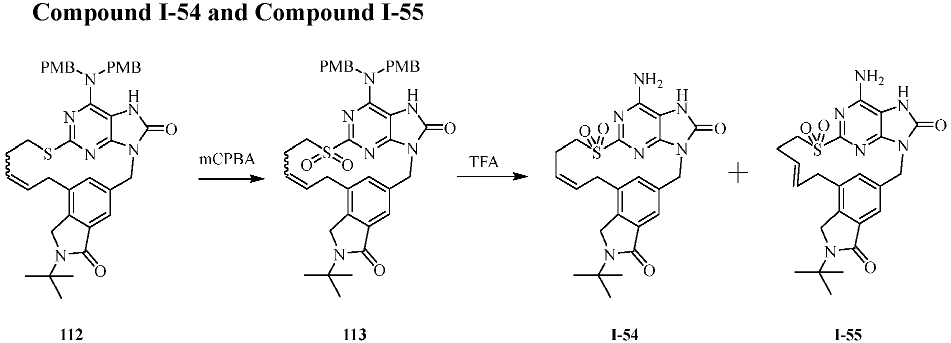

### Step 1: Preparation of compound 113

To a stirred mixture solution of **compound 112** (0.035 g, 0.05 mmol) in DCM (4 mL) was added mCPBA (17.49 mg, 0.10 mmol) at 0 °C, then the resulting mixture was stirred at 25 °C for 4 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **compound 113** (36 mg). MS: 723.0 (M+H)⁺.

### Step 2: Preparation of Compound I-54 and Compound I-55

A mixture solution of **compound 113** (36 mg) in TFA (2 mL) was stirred at 70 °C for 1 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-54** and **Compound I**-**55.**

**Compound 1-54** (white solid, 14.8 mg). MS: 483.0 (M+H)⁺. ¹H NMR (400 MHz, DMSO-*d₆*) δ 7.65 (s, 1H), 7.44 (s, 1H), 7.15 (s, 2H), 5.82-5.69 (m, 1H), 5.69-5.56 (m, 1H), 5.04 (s, 2H), 4.51 (s, 2H), 3.83-3.71 (m, 2H), 3.29 (s, 2H), 2.46 (s, 2H), 2.04-1.93 (m, 1H), 1.47 (s, 9H).

**Compound 1-55** (white solid, 14.8 mg). MS: 483.0 (M+H)⁺. ¹H NMR (400 MHz, DMSO-*d₆*) δ 7.71 (s, 1H), 7.46 (s, 1H), 7.19 (s, 2H), 5.57-5.46 (m, 1H), 5.46-5.37 (m, 1H), 5.02 (s, 2H), 4.47 (s, 2H), 3.86-3.75 (m, 2H), 3.20 (d, *J=* 6.6 Hz, 2H), 2.51 (s, 2H), 2.04-1.93 (m, 1H), 1.47 (s, 9H).

### Example 53

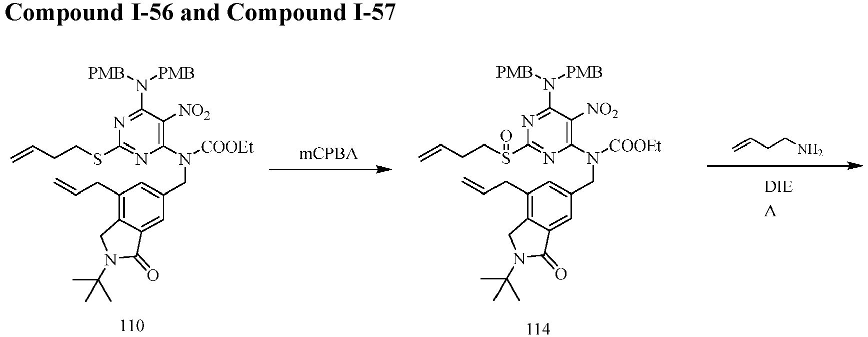

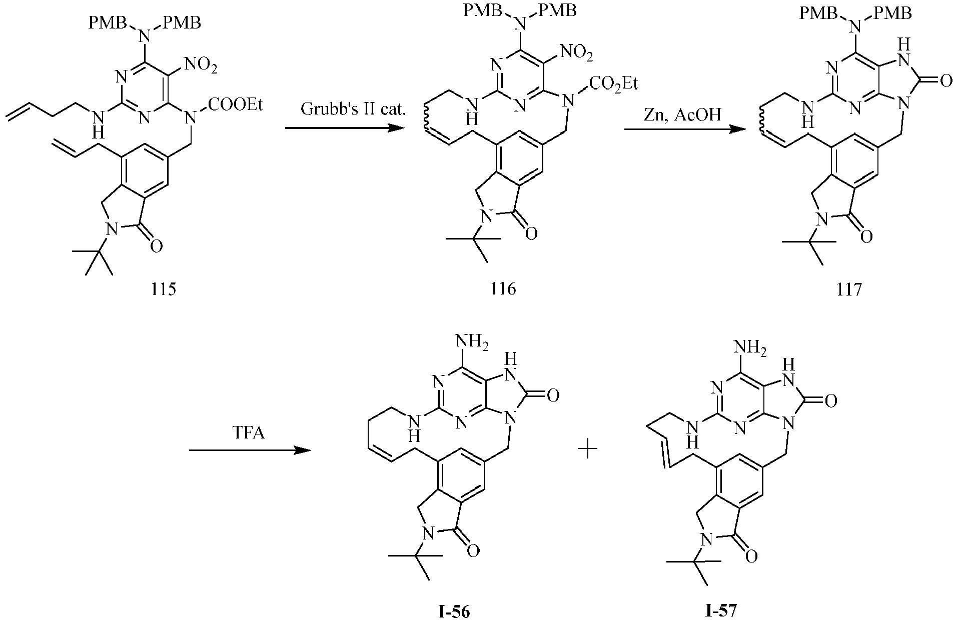

### Step 1: Preparation of compound 114

To a stirred mixture solution of **compound 110** (440 mg, 0.55 mmol) in DCM (3 mL) was added a solution of mCPBA (63.68 mg, 0.37 mmol) in DCM (3 mL) at 0 °C, then the resulting mixture was stirred at 0 °C for 2 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by silica gel flash chromatography to afford **compound 114** (450 mg). MS: 811.2 (M+H)⁺.

### Step 2: Preparation of compound 115

A mixture solution of **compound 114** (200 mg, 0.25 mmol), 3-butene-1-amine ((35.08 mg, 493.2 umol) and DIEA(159.37 mg, 1.23 mmol) in DCM (2 mL) and THF (4 mL) was stirred at 20 °C for 4 hours. After the reaction was completed, the reaction mixture was poured into ice-water and extracted with EtOAc. The combined organic layers were washed with brine, dried over anhydrous Na₂SO₄, filtered and concentrated in vacuo to give a crude product, which was purified by silica gel flash chromatography to afford **compound 115** (110 mg, 57%) as a white solid. MS: 778.4 (M+H)⁺.

### Step 3: Preparation of compound 116

To a stirred mixture solution of **compound 115** (110 mg, 0.14 mmol) in DCM (200 mL) was added Grubbs'II reagent (30.01 mg, 35.35 umol) under N₂ protected, then the resulting mixture was stirred at 20 °C for 24 hours. After the reaction was completed, the reaction mixture was concentrated in vacuo to give a crude product, which was purified by silica gel flash chromatography to afford **compound 116** (105 mg) as a yellow solid. MS: 750.4 (M+H)⁺.

### Step 4: Preparation of compound 117

To a stirred mixture solution of **compound 116** (60 mg, 0.080 umol) in AcOH (2 mL) was added Zn (26.16 mg, 0.4 mmol) under N₂ protected, then the resulting mixture was stirred at 70 °C for 4 hours. After the reaction was completed, the reaction mixture was filtered and concentrated in vacuo to give a crude product, which was purified by silica gel flash chromatography to afford **compound 117** (30 mg). MS: 674.2 (M+H)⁺.

### Step 5: Preparation of Compound I-56 and Compound I-57

A mixture solution of **compound 117** (30 mg, 44.52 umol) in TFA (2 mL) was stirred at 70 °C for 1 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-56** and **Compound 1-57.**

**Compound 1-56** (yellow solid, 10.22 mg). MS: 434.3 (M+H)⁺. ¹H NMR (400 MHz, DMSO-*d₆*) δ 9.91 (s, 1H), 8.27 (s, 1H), 7.36 (s, 1H), 6.99 (s, 2H), 5.75 (dt, *J*= 14.5, 7.1 Hz, 1H), 5.66-5.54 (m, 1H), 4.87 (s, 2H), 4.47 (s, 2H), 3.37-3.37 (m, 1H), 3.34-3.31 (m, 2H), 3.26 (d, *J*= 6.4 Hz, 2H), 2.28 (s, 2H), 1.47 (s, 9H).

**Compound I-57** (yellow solid, 9.64 mg). MS: 434.3 (M+H)⁺. ¹H NMR (400 MHz, DMSO-*d₆*) δ 9.87 (s, 1H), 8.07 (s, 1H), 7.37 (s, 1H), 6.66 (s, 2H), 5.68 (s, 1H), 5.33 (d, *J*= 4.7 Hz, 1H), 4.85 (s, 2H), 4.50 (s, 2H), 3.34-3.34 (m, 1H), 3.33-3.33 (m, 2H), 3.31-3.31 (m, 2H), 2.02-1.96 (m, 2H), 1.47 (s, 9H).

### Example 54

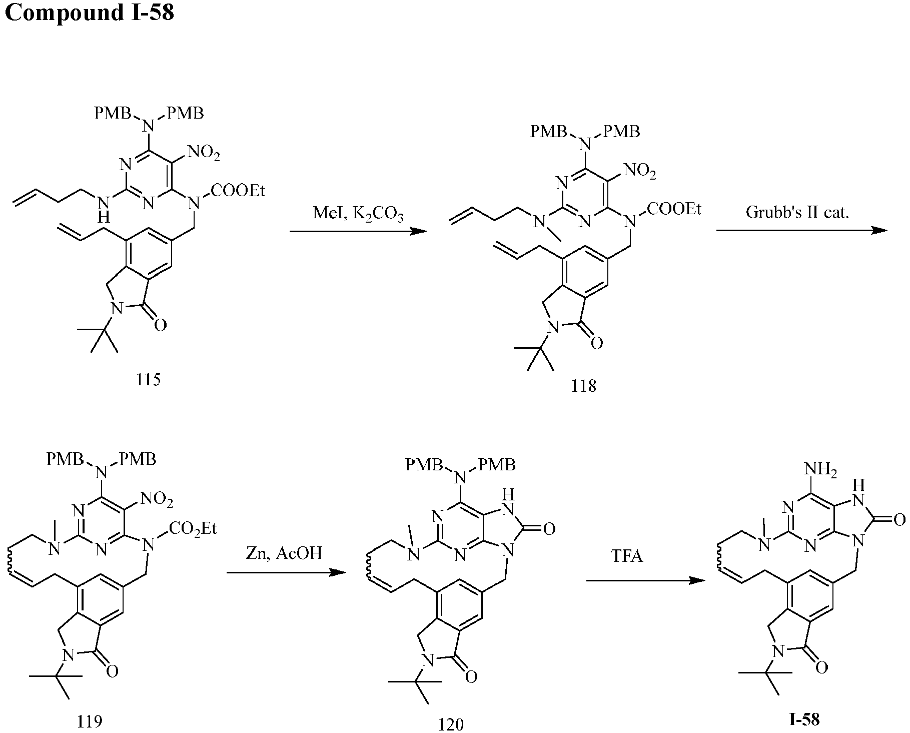

### Step 1: Preparation of compound 118

A mixture solution of **compound 115** (190 mg, 0.24 mmol), CH₃I (62.3 mg, 0.44 mmol) and K₂CO₃ (100 mg, 0.73 mmol) in DMF (5 mL) was stirred at 100 °C for 4 hours. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by silica gel flash chromatography to afford **compound 118** (80 mg, 41%) as a yellow solid. MS: 792.3 (M+H)⁺.

### Step 2: Preparation of compound 119

To a stirred mixture solution of **compound 118** (80 mg, 0.1 mmol) in DCM (200 mL) was added Grubbs'II reagent (21.44 mg, 0.025 mmol) under N₂ protected, then the resulting mixture was stirred at 20 °C for 24 hours. After the reaction was completed, the reaction mixture was concentrated in vacuo to give a crude product, which was purified by silica gel flash chromatography to afford **compound 119** (30 mg, 38%) as a yellow solid. MS: 764.4 (M+H)⁺.

### Step 3: Preparation of compound 120

To a stirred mixture solution of **compound 119** (30 mg, 0.03 mmol) in AcOH (2 mL) was added Zn (12.84 mg, 0.2 mmol) under N₂ protected, then the resulting mixture was stirred at 70 °C for 4 hours. After the reaction was completed, the reaction mixture was filtered and concentrated in vacuo to give a crude product, which was purified by silica gel flash chromatography to afford **compound** 120 (10 mg). MS: 688.1 (M+H)⁺.

### Step 4: Preparation of Compound I-58

A mixture solution of **compound** 120 (10 mg) in TFA (2 mL) was stirred at 70 °C for 1 hour. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by Pre-HPLC to afford **Compound I-58** (white solid, 2.47 mg, mixture of E&Z). MS: 448.1 (M+H)⁺. ¹H NMR (400 MHz, DMSO-*d₆*) δ 9.75 (s, 1H), 8.38 (s, 1H), 7.37 (s, 1H), 6.08 (s, 2H), 5.79-5.70 (m, 1H), 5.52-5.42 (m, 1H), 4.85 (s, 2H), 4.49 (d, J = 17.4 Hz, 2H), 3.32-3.28 (m, 2H), 3.24 (s, 2H), 3.05 (s, 3H), 2.35 (s, 1H), 2.02 (dd, J = 14.7, 7.1 Hz, 1H), 1.48 (s, 9H).

### Example 55

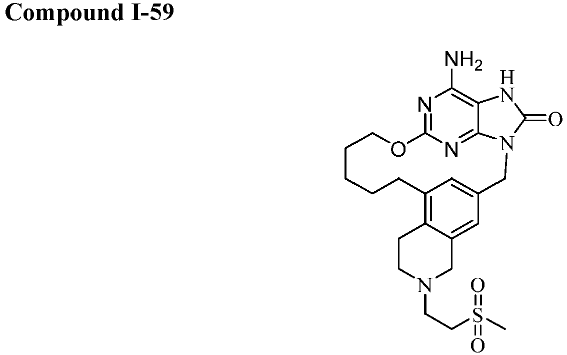

The title **Compound I-59** was prepared in analogy to the preparation of **Compound I*-*35** by using **compound 86** and 2-methylsulfonyl acetaldehyde as a start material. MS: 487.3 (M+H)⁺. ¹H NMR (400 MHz, DMSO-*d₆*) δ 10.02 (s, 1H), 7.61 (s, 1H), 6.82 (s, 1H), 6.41 (s, 2H), 4.81 (br, 2H), 4.64-3.91 (m, 4H), 3.76-3.67 (m, 2H), 3.47-3.25 (m, 4H), 3.11 (s, 3H), 2.97 (br, 2H), 2.66 (br, 2H), 1.69 (br, 2H), 1.34 (br, 4H).

### Example 56

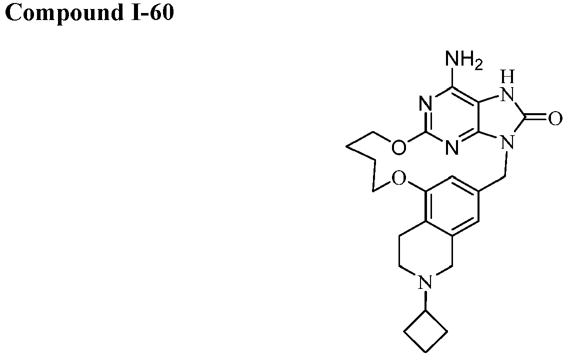

The title **Compound I-60** was prepared in analogy to the preparation of **Compound I-35** by using **Compound I-31** and cyclobutanone as a start material. MS: 437.3 (M+H)⁺. ¹H NMR (400 MHz, DMSO-*d₆*) δ 9.99 (s, 1H), 7.84 (s, 1H), 6.73 (s, 1H), 6.42 (s, 2H), 4.87-4.74 (m, 2H), 4.57 (br, 2H), 4.36 (br, 2H), 4.10-4.00 (m, 2H), 3.65-3.75 (m, 1H), 3.45-3.55 (m, 1H), 3.95-3.15 (m, 1H), 2.87 (d, J= 17.3 Hz, 1H), 2.75 (dd, J= 18.0, 8.4 Hz, 1H), 2.26-2.11 (m, 4H), 1.73 (d, J = 9.3 Hz, 2H), 1.63 (d, J = 7.0 Hz, 2H), 1.53 (br, 2H).

### Example 57

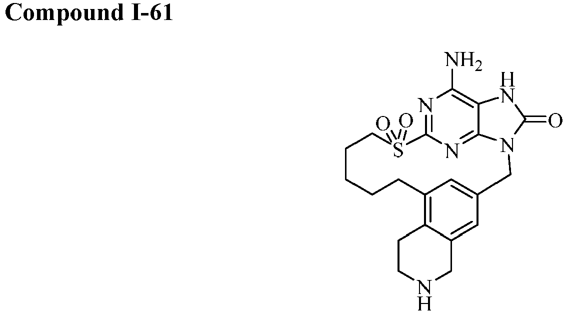

The title **Compound I-61** was prepared in analogy to the preparation of **Compound I-53** by using **compound 109** and **Intermediate 7** as a start material. MS: 429.2 (M+H)⁺. ¹H NMR (400 MHz, DMSO-*d₆*) δ 10.76 (s, 1H), 7.16 (s, 2H), 7.07 (s, 1H), 6.99 (s, 1H), 4.94 (brs, 2H), 4.23 (brs, 2H), 3.41 - 3.33 (m, 4H), 2.85 (s, 2H), 2.59 - 2.54 (m, 2H), 1.52 (m, 4H), 1.20 (m, 2H).

### Example 58

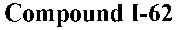

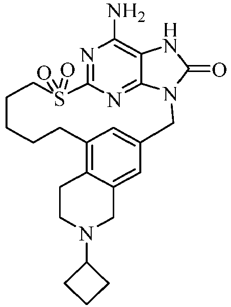

The title **Compound I-62** was prepared in analogy to the preparation of **Compound I-35** by using **Compound I-61** and cyclobutanone as a start material. MS: 483.3 (M+H)⁺. ¹H NMR (400 MHz, DMSO-*d₆*) δ 10.79 (s, 1H), 7.17 (s, 2H), 7.13 (s, 1H), 7.00 (s, 1H), 4.95 (d, J = 4.8 Hz, 2H), 4.41 (d, J = 15.5 Hz, 1H), 4.09 (dd, J = 15.5, 8.0 Hz, 1H), 3.73 (d, J = 8.4 Hz, 1H), 3.56-3.52 (m, 1H), 3.44-3.38 (m, 2H), 3.13-3.10 (m, 1H), 3.03-2.88 (m, 2H), 2.66-2.63 (m, 1H), 2.30-2.15 (m, 4H), 1.79-1.72 (m, 2H), 1.60-1.43 (m, 4H), 1.27-1.18 (m, 2H).

### Example 59

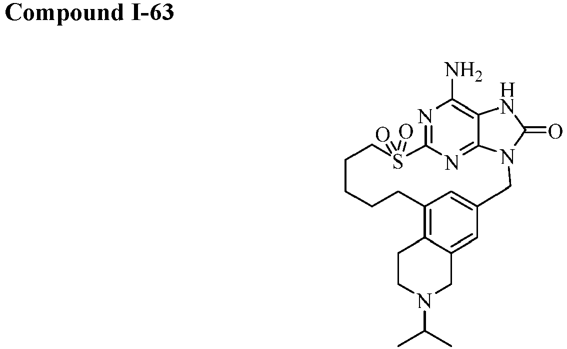

The title **Compound I-63** was prepared in analogy to the preparation of **Compound I-35** by using **Compound I-61** and acetone as a start material. MS: 471.3 (M+H)⁺. ¹H NMR (400 MHz, DMSO-*d₆*) δ 10.79 (s, 1H), 7.17 (s, 2H), 7.13 (s, 1H), 7.00 (s, 1H), 4.96 (s, 2H), 4.35 (d, J = 5.7 Hz, 2H), 3.63-3.52 (m, 3H), 3.30-3.15 (m, 2H), 2.98-2.90 (m, 2H), 2.59-2.50 (m, 2H), 1.61-1.44 (m, 4H), 1.40 (s, 1H), 1.29 (t, 7H).

### Example 60

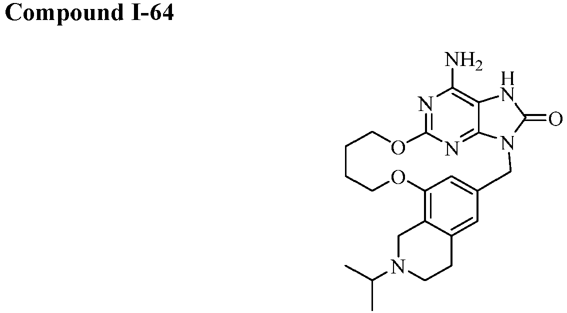

The title **Compound I-64** was prepared in analogy to the preparation of **Compound I-29** by using **compound 6, Intermediate 20** and acetone as a start material. MS: 425.3 (M+H)⁺. ¹H NMR (400 MHz, DMSO-*d₆*) δ 10.00 (s, 1H), 7.84 (s, 1H), 6.74 (s, 1H), 6.43 (s, 2H), 4.81 (br, 2H), 4.57 (br, 2H), 4.36 (d, J = 7.0 Hz, 2H), 4.19 (d, J= 15.6 Hz, 1H), 4.11 - 4.04 (m, 1H), 3.58 (d, J = 11.1 Hz, 2H), 3.19 - 3.10 (m, 1H), 3.03 (d, J = 21.4 Hz, 2H), 1.66 (d, J = 7.6 Hz, 2H), 1.54 (d, J = 7.3 Hz, 2H), 1.29 (dd, J = 6.7, 3.7 Hz, 6H).

### Example 61

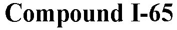

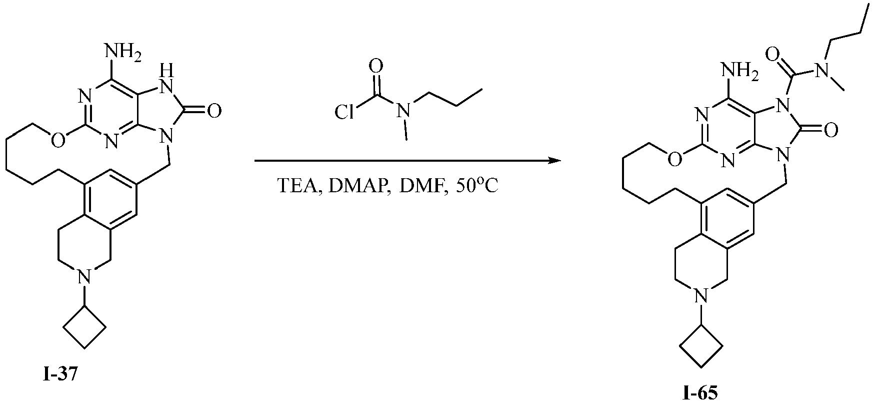

A mixture solution of **Compound I-37** (20 mg, 0.046 mmol), methyl(propyl)carbamic chloride (12.4 mg, 0.092 mmol), DIEA (23.2 mg, 0.23 mmol) and DMAP(5.6 mg, 0.046 mmol) in DMF (1 mL) was stirred at 50 °C for 14 hours. After the reaction was completed, the reaction mixture was concentrated in vacuo and extracted with EA. The combined organic layers were washed with brine, dried over anhydrous Na₂SO₄, filtered and concentrated in vacuo to give a crude product, which was purified by pre-HPLC to afford **Compound I-65** (10.0 mg, 40.8%) as a white solid. MS: 543.3 (M+H)⁺. ¹H NMR (400 MHz, DMSO-*d₆*) δ 7.46 (s, 1H), 6.74 (s, 1H), 6.24 (s, 2H), 4.82 (br, 2H), 4.25 (br, 2H), 3.46-3.38 (m, 2H), 3.26 (s, 3H), 3.25-3.20 (m, 2H), 3.08-3.05 (m, 2H), 3.03-3.00 (m, 1H), 2.70-2.65 (m, 2H), 2.63-2.58 (m, 2H), 2.06-2.02 (m, 2H), 1.88-1.80 (m, 2H), 1.67-1.60 (m, 6H), 1.37-1.30 (m, 4H), 0.91 (t, J = 7.5 Hz, 3H).

### Example 62

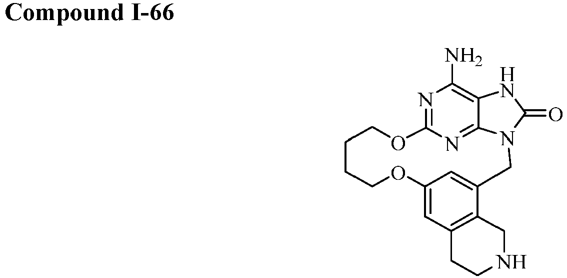

The title **Compound I-66** was prepared in analogy to the preparation of **Compound I-29** by using **compound 6, Intermediate 21** as a start material. MS: 383.3 (M+H)⁺. ¹H NMR (400 MHz, DMSO-*d₆*) δ 10.10 (s, 1H), 7.87 (s, 1H), 6.66 (s, 1H), 6.47 (br, 2H), 4.85 (s, 2H), 4.54-4.48 (m, 2H), 4.39-4.35 (m, 2H), 4.32-4.28 (m, 2H), 3.33-3.28 (m, 2H), 2.92-2.86 (m, 2H), 1.69-1.62 (m, 2H), 1.56-1.50 (m, 2H).

### Example 63

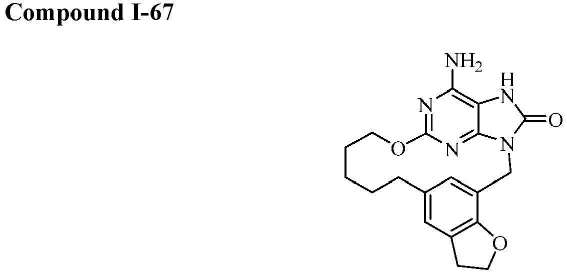

The title **Compound I-67** was prepared in analogy to the preparation of **Example 1** and **Example 2** by using **Intermediate 1** and **Intermediate 22** as a start material. MS: 368.2 (M+H)⁺. ¹H NMR (400 MHz, DMSO-*d₆*) δ 9.83 (s, 1H), 7.19 (s, 1H), 6.90 (s, 1H), 6.37 (s, 2H), 4.77 (s, 2H), 4.39 (t, J = 8.6 Hz, 2H), 4.05 (d, J = 7.2 Hz, 2H), 3.05 (t, J = 8.7 Hz, 2H), 2.55 (t, J = 5.5 Hz, 2H), 1.61 (brs, 2H), 1.27 (br, 4H).

### Example 64

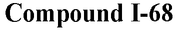

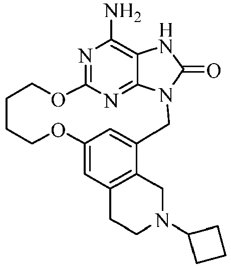

The title **Compound I-68** was prepared in analogy to the preparation of **Compound I-35** by using **Compound I-66** and cyclobutanone as a start material. MS: 437.3 (M+H)⁺. ¹H NMR (400 MHz, DMSO-*d₆*) δ 9.93 (s, 1H), 7.71 (d, J = 2.7 Hz, 1H), 6.52 (d, J = 2.5 Hz, 1H), 6.36 (br, 2H), 4.78 (s, 2H), 4.50-4.43 (m, 2H), 4.28-4.22 (m, 2H), 3.61-3.58 (m, 2H), 2.97-2.89 (m, 1H), 2.73-2.67 (m, 2H), 2.11-2.03 (m, 2H), 1.90-1.81 (m, 2H), 1.68-1.63 (m, 4H), 1.56-1.50 (m, 2H).

### Example 65

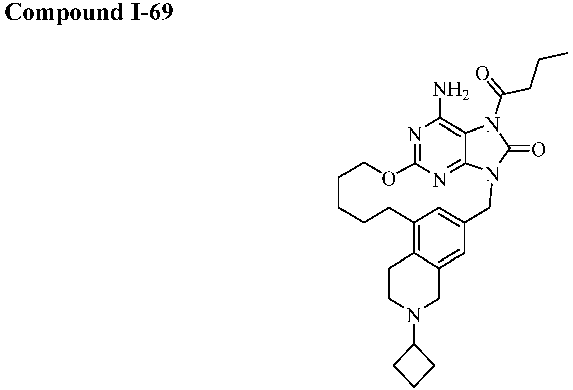

The title **Compound I-69** was prepared in analogy to the preparation of **Compound I-65** by using **Compound I-37** and butanoyl chloride as a start material. MS: 505.4 (M+H)⁺. ¹H NMR (400 MHz, CDCl₃) δ 7.48 (s, 1H), 6.84 (s, 1H), 4.91 (s, 2H), 4.47-4.43 (m, 2H), 3.46 (s, 2H), 3.19-3.15 (m, 2H), 2.89-2.84 (m, 1H), 2.76-2.73 (m, 2H), 2.64-2.58 (m, 4H), 2.15-2.09 (m, 2H), 2.05-1.95 (m, 2H), 1.80-1.75 (m, 5H), 1.58-1.53 (m, 3H), 1.40-1.35 (m, 2H), 1.05 (t, J = 7.4 Hz, 3H).

### Example 66

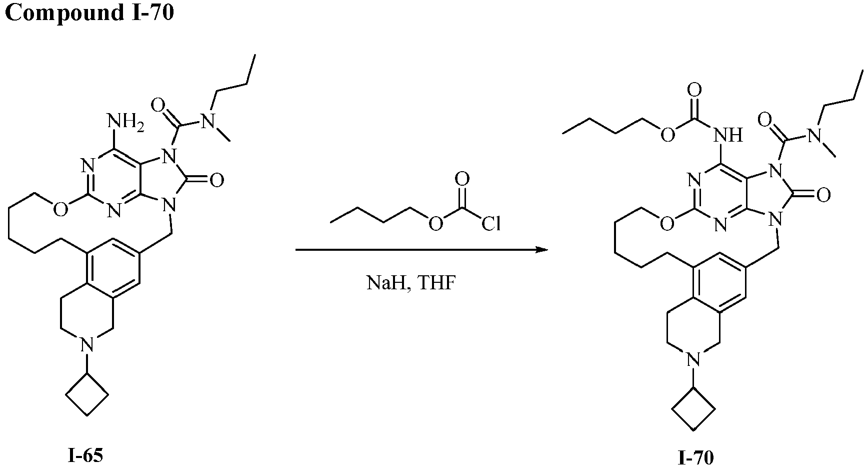

To a stirred mixture solution of **Compound I-65** (30 mg, 0.056 mmol)in THF (2 mL) was added NaH (11.2 mg, 0.28 mmol) at 0 °C and the resulting mixture was stirred at 20 °C for 1 hour. Then butyl carbonochloridate (15.0 mg, 0.11 mmol) was added to the above mixture solution at 0 °C and the resulting mixture was stirred at 20 °C for 16 hours. After the reaction was completed, the reaction mixture was quenched by adding ice-water and extracted with EA. The combined organic layers were washed with a solution of NaHCO₃, brine, dried over anhydrous Na₂SO₄, filtered and concentrated in vacuo to give a crude product, which was purified by pre-HPLC to afford **Compound I-70** (5.0 mg, 14.3%) as a white solid. MS: 634.4 (M+H)⁺.¹H NMR (400 MHz, CDCl₃) δ 7.69 (s, 1H), 7.43 (s, 1H), 6.94 (s, 1H), 5.10-4.85 (m, 2H), 4.10 (s, 2H), 3.80-3.65 (m, 3H), 3.54-3.31 (m, 4H), 3.21 (s, 3H), 3.10-3.02 (m, 2H), 2.83-2.80 (m, 1H), 2.65-2.61 (m, 4H), 2.25-2.22 (m, 2H), 1.95-1.92 (m, 2H), 1.75-1.70 (m, 6H), 1.41-1.37 (m, 5H), 1.02-0.90 (m, 8H).

### Example 1: Efficacy evaluation at the cellular level

### Example 1. Determination of agonistic activity of the compounds of the invention on human TLR7

The TLR7 agonistic activity of the compounds of the invention in HEK-Blue^{™} hTLR7 cell line with stable expression of human TLR7 were assayed using the following method:
1. Weighed the test compounds and prepared stock solutions with DMSO (Sigma) at a concentration of 10 mM. Serially diluted the stocksolutions with DMSO by 3.16 times gradient, which resulted in a total of 10 concentration gradients. The highest concentration was 1 mM.
2. The test compounds and DMSO control were diluted 10-fold in DPBS buffer (Gibco) and then added to a 96-well cell culture plate (Corning) at a volume of 20 µL/well.
3. HEK-Blue^{™} hTLR7 cells (Invivogen) were digested with cell separation solution (Gibco), and counted (TC-20 cell counter, Bio-rad). Cells were diluted to 4.4E5/ml using DMEM cell medium (Gibco). Added the cells to a 96-well cell culture plate at a volume of 180 µL/well. The cells were incubated for 16-22 h at 37°C in a 5% CO₂ incubator.
4. Prepared QUANTI-Blue detection reagent (Invivogen), and added into a new 96-well plate at a volume of 180 µL/well. Tweenty µL of cell culture supernatant was transfered from the cell culture plate and added to the 96-well plate and incubated at 37°C for 1-3 hours.
5. Neo2 multifunctional microplate reader (Bio-Tek) was used to measure the optical absorption at 630nm, and the EC₅₀ was calculated by GraphPad Prism.

The agonistic effect of the compounds of the invention on human TLR7 can be determined by the above experiments, and the measured EC₅₀ value is shown in Table 1.

**Table 1. EC₅₀ value of the agonistic effect of the compounds of the invention on human TLR7**

| Compound | EC50 (nM) | Compound | EC50 (nM) |
|---|---|---|---|
| Reference 1 | 15 | Reference 2 | 31 |
| Reference 3 | 41 | I-1 | 3 |
| I-2 | 2 | I-3 | 10 |
| I-4 | 5 | I-5 | 113 |
| I-6 | 2 | I-7 | 8 |
| I-8 | 15 | I-9 | 9 |
| I-10 | 26 | I-11 | 6 |
| I-12 | 8 | I-13 | 28 |
| I-14 | 38 | I-15 | 228 |
| I-16 | 17 | I-17 | 167 |
| I-18 | 20 | I-19 | 165 |
| I-20A & I-20B | 148 | I-21 | 42 |
| I-22 | 15 | I-23 | 79 |
| I-24 | 84 | I-25 | 95 |
| I-26 | 105 | I-27 | 76 |
| I-28 | 111 | I-29 | 35 |
| I-30 | 75 | I-31 | 39 |
| I-32 | 31 | I-33 | 140 |
| I-34 | 132 | 1-35 | 10 |
| I-36 | 20 | I-37 | 10 |
| I-38 | 47 | 1-39 | 3150 |
| I-40 | 280 | I-41 | 22 |
| I-42 | 7 | I-43 | 3 |
| I-44 | 38 | I-45 | 579 |
| I-46 | 980 | I-47 | 165 |
| I-48 | 11 | I-49 | 73 |
| I-50 | 2 | I-51 | 6 |
| I-52 | 19 | I-53 | 64 |
| I-54 | 1 | 1-55 | 2 |
| I-56 | 293 | I-57 | 1795 |
| I-58 | 1100 | I-59 | 87 |
| I-60 | 14 | I-61 | 13 |
| I-62 | 13 | I-63 | 31 |
| I-64 | 11 | I-66 | 393 |
| I-67 | 316 | I-68 | 25 |

In Table 1, reference compounds 1~3 were prepared in according to patent WO2019209811A1. It can be seen from Table 1 that the compounds of the invention have good TLR7 agonist activity.

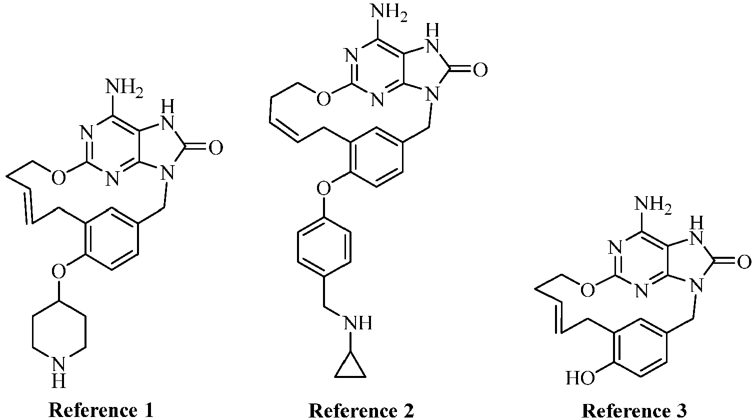

### Example 2: Determination of agonistic activity of the compounds of the invention on human peripheral blood mononuclear cells

1. The frozen human peripheral blood mononuclear cells (Miaotong Biotechnology Co., LTD., PB010C) were dissolved in water bath at 37°C. After counting, the cells were diluted to 0.2M/mL, and 150 uL/well was added to the 96-well cell culture plate, and the cells were incubated for 40 hours.
2. The test compounds were weighed and prepared with DMSO at a concentration of 10 mM. DMSO was used to dilute the test compounds by a 10-fold gradient, with a total of four concentration gradients. The highest concentration was 400 µM.
3. The test compounds, DMSO control and positive controls were diluted 100 times with cell culture medium (RPMI 1640 cell medium, Gibco), and 50 µL was added to 96-well cell culture plates.
4. The cells were incubated at 37°C for about 8 hours, and the supernatant was collected by centrifugation. The level of IFN-α in the supernatant was determined by ELISA (IFN-α ELISA kit, Dakewe).
5. Light absorption at 450nm was measured by microplate reader, and IFN-α level in culture supernatant was quantified according to the standard curve. The results were shown in Table 2.

**Table 2. Levels of IFN-α produced by human peripheral blood monocytes stimulated by the compounds of the invention.**

| Compound | IFN-α concentration post stimulation (pg/mL) | | | |
|---|---|---|---|---|
| | 10nM | 1nM | 0.1nM | DMSO |
| Reference 1 | 159 | 74 | 9 | 8 |
| Reference 2 | 558 | 161 | 8 | 8 |
| Reference 3 | 284 | 9 | 8 | 8 |
| I-35 | 2776 | 2767 | 251 | 12 |
| I-50 | 2173 | 2615 | 1509 | 12 |
| I-26 | 96 | 11 | 12 | 12 |
| I-37 | 2078 | 1973 | 428 | 11 |
| I-51 | 2472 | 2759 | 530 | 12 |
| I-48 | 2784 | 2431 | 13 | 12 |
| I-60 | 3127 | 2412 | 12 | 11 |
| Vesatolimod | 12 | 12 | ND | 12 |

It demonstrated that the compounds of the invention can stimulate human peripheral blood monocytes to produce IFN-α.

## Claims

1. A compound of formula I, a solvate, a prodrug, a metabolite, or a pharmaceutically acceptable salt thereof: 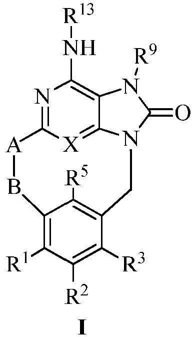
wherein X is N or CH;
A is O, S, S(=O)₂, S(=O)(=NH), NR⁴ or CR⁶R⁷; R⁴, R⁶and R⁷ are independently H or C₁-C₆ alkyl;
B is C₂-C₁₀ alkylene, C₂-C₁₀ unsaturated hydrocarbylene, R¹⁻¹ substituted C₂-C₁₀ alkylene, R¹⁻¹ substituted C₂-C₁₀ unsaturated hydrocarbylene, -Z¹-NH-C(=O)-Z²-, -Z³-NH-C(=O)-Z⁴-L¹-, -Z⁵-L²-, -Z⁶-O-Z⁷-, -Z⁸-O-Z⁹-L³-or -(CH₂)ₙ-L⁵-(CH₂)ᵣ-L⁴-;
L¹, L², L³ and L⁴ are each independently O, S, S(=O)₂, NR⁸, R⁸ is H or C₁-C₆ alkyl;
L⁵ is C₃-C₆ cycloalkylene, halogenated C₃-C₆ cycloalkylene, "3-6 membered heterocycloalkylene having 1-3 hetero atoms selected from one or more of N, O and S" or halogenated "3-6 membered heterocycloalkylene having 1-3 hetero atoms selected from one or more of N, O and S"; n and r are independently 1, 2 or 3;
Z¹, Z², Z³, Z⁴, Z⁶, Z⁷, Z⁸ and Z⁹are independently C₁-C₆ alkylene, C₁-C₆ unsaturated hydrocarbylene, R¹⁻² substituted C₁-C₆ alkylene or R¹⁻² substituted C₁-C₆ unsaturated hydrocarbylene;
Z⁵ is C₂-C₁₀ alkylene, C₂-C₁₀ unsaturated hydrocarbylene, R¹⁻³ is substituted C₂-C₁₀ alkylene or R¹⁻³ substituted C₂-C₁₀ unsaturated hydrocarbylene;
R¹⁻¹, R¹⁻² and R¹⁻³ are independently OH, CN, NH₂, halogen, C₁-C₆ alkyl, C₁-C₆ alkoxy or COOR¹⁻¹⁻¹; and R¹⁻¹⁻¹ is H or C₁-C₃ alkyl;
R⁵ is H, CN, halogen, C₃-C₅ cycloalkyl, C₁-C₆ alkyl or C₁-C₆ alkoxy;
R⁹ is H, CONR¹⁰R¹¹ or C(=O)R¹², R¹⁰and R¹² are independently C₁-C₆alkyl or C₁-C₆alkoxy substituted C₁-C₆alkyl; R¹¹ is C₁-C₆alkyl or R¹⁻³⁰substituted C₁-C₆alkyl; or R¹⁰ and R¹¹ together with the nitrogen atom to which they are attached form a "5-7-membered heterocycloalkyl having 1-2 hetero atoms, and the heteroatom is N";
R¹³ is H, CONR¹⁴R¹⁵, C(=O)R¹⁶ or COOR¹⁷, and R¹⁴, R¹⁵, R¹⁶ and R¹⁷ are independently C₁-C₆alkyl or R¹³⁻¹ substituted C₁-C₆alkyl; R¹³⁻¹ is CN, halogen, C₁-C₆alkoxy or -(CH₂CH₂O)_{q}-R¹³⁻², R¹³⁻² is C₁-C₆alkyl, and q is an integer of 0-460;
R¹⁻³⁰ is C₁-C₆alkoxy, -C(=O)-O-R¹⁻³⁰⁻¹, phenyl, NR¹⁻³⁰⁻²R¹⁻³⁰⁻³, or -O-C(=O)-R¹⁻³⁰⁻⁵;
R¹⁻³⁰⁻¹ and R¹⁻³⁰⁻² are independently C₁-C₆alkyl;
R¹⁻³⁰⁻³ is C₁-C₆alkyl or C(=O)R¹⁻³⁰⁻⁴, and R¹⁻³⁰⁻⁴ is C₁-C₆alkyl or C₁-C₆alkoxy;
R¹⁻³⁰⁻⁵ is C₁-C₆alkoxy or NR¹⁻³⁰⁻⁶R¹⁻³⁰⁻⁷, R¹⁻³⁰⁻⁶ and R¹⁻³⁰⁻⁷ are independently C₁-C₆alkyl, or R¹⁻³⁰⁻⁶ and R¹⁻³⁰⁻⁷ together with the nitrogen atom to which they are attached form a "5-7 membered heterocycloalkyl having 1-2 heteroatoms, and the heteroatom is N";
the definitions of R¹, R² and R³are selected from the following Scheme A or Scheme B:
scheme A: R¹, R² together with the carbon atoms to which they attached form a "4-7 membered heterocycloalkylene having 1-3 heteroatoms selected from one or more of N, O and S", R¹⁻⁴ substituted "4-7 membered heterocycloalkylene having 1-3 heteroatoms selected from one or more of N, O and S", C₄-C₇cycloalkylene or R¹⁻⁵ substituted C₄-C₇cycloalkylene; or, 1 or 2 or more arbitrary methylenes in the "4-7 membered heterocycloalkylene having 1-3 heteroatoms selected from one or more of N, O and S" of the "4-7 membered heterocycloalkylene having 1-3 heteroatoms selected from one or more of N, O and S" or the R¹⁻⁴ substituted "4-7 membered heterocycloalkylene having 1-3 heteroatoms selected from one or more of N, O and S" formed by R¹, R² together with the carbon atoms to which they are attached are independently replaced by carbonyl or S(=O)₂; and R³ is H, halogen, C₁-C₆alkyl or halogenated C₁-C₆alkyl;
scheme B: R¹ is H, halogen, C₁-C₆alkyl or halogenated C₁-C₆alkyl; R², R³ together with the carbon atoms to which they are attached form a "4-7 membered heterocycloalkylene having 1-3 heteroatoms selected from one or more of N, O and S", R¹⁻⁴ substituted "4-7 membered heterocycloalkylene having 1-3 heteroatoms selected from one or more of N, O and S", C₄-C₇cycloalkylene or R¹⁻⁵-substituted C₄-C₇cycloalkylene; or, 1 or 2 or more arbitrary methylenes in the "4-7 membered heterocycloalkylene having 1-3 heteroatoms selected from one or more of N, O and S" of the "4-7 membered heterocycloalkylene having 1-3 heteroatoms selected from one or more of N, O and S" or the R₁₋₄ substituted "4-7 membered heterocycloalkylene having 1-3 heteroatoms selected from one or more of N, O and S" formed by R², R³ together with the carbon atoms to which they are attached are independently replaced by carbonyl or S(=O)₂;
In scheme A or scheme B, R¹⁻⁴ and R¹⁻⁵ are independently OH, halogen, CN, C₁-C₆alkyl, R¹⁻⁶substituted C₁-C₆alkyl, C₁-C₆alkoxy, R¹⁻⁹ substituted C₁-C₆alkoxy, -S(=O)₂R¹⁻⁷, -C(=O)R¹⁻⁸, NR¹⁻¹⁰R¹⁻¹¹, COOR¹⁻¹², SR¹⁻¹³, C₃-C₇cycloalkyl, R¹⁻¹⁹substituted C₃-C₇cycloalkyl, "4-7 membered heterocycloalkyl having 1-3 heteroatoms selected from one or more of N, O and S", R¹⁻²⁰substituted "4-7 membered heterocycloalkyl having 1-3 heteroatoms selected from one or more of N, O and S", "C₁-C₁₀heteroaryl having 1-4 heteroatoms selected from one or more of N, O and S", R¹⁻²¹substituted "C₁-C₁₀ heteroaryl having 1-4 heteroatoms selected from one or more of N, O and S" or -Y(CR¹⁻¹⁴R¹⁻¹⁵)ᵤ-COOR¹⁻¹⁶; Y is O, S, S(=O)₂ or NH; and u is 1, 2 or 3;
R¹⁻⁶ and R¹⁻⁹ are independently halogen, amino, CN, OH, COOR¹⁻¹⁷, S(=O)₂R¹⁻³¹, - C(=O)NH₂, -S(=O)₂NH₂, C₁-C₃alkoxy, C₃-C₇cycloalkyl, COOR¹⁻¹⁸substituted C₃-C₇cycloalkyl, "4-7 membered heterocycloalkyl having 1-3 heteroatoms selected from one or more of N, O and S", R¹⁻²² substituted "4-7 membered heterocycloalkyl having 1-3 heteroatoms selected from one or more of N, O and S", "C₁-C₁₀heteroaryl having 1-4 heteroatoms selected from one or more of N, O and S" or R¹⁻²³ substituted "C₁-C₁₀heteroaryl having 1-4 heteroatoms selected from one or more of N, O and S";
R¹⁻⁷and R¹⁻⁸are independently C₁-C₃alkyl, C₃-C₇cycloalkyl, R¹⁻²⁴substituted C₃-C₇cycloalkyl, "4-7 membered heterocycloalkyl having 1-3 heteroatoms selected from one or more ofN, O and S", R¹⁻²⁵-substituted" 4-7 membered heterocycloalkyl having 1-3 heteroatoms selected from one or more of N, O and S", "C₁-C₁₀heteroaryl having 1-4 heteroatoms selected from one or more of N, O and S" or R¹⁻²⁶ substituted "C₁-C₁₀heteroaryl having 1-4 heteroatoms selected from one or more of N, O and S", C₆-C₁₀aryl, R¹⁻²⁷substituted C₆-C₁₀aryl or NR¹⁻²⁸R¹⁻²⁹;
R¹⁻¹⁰, R¹⁻¹¹, R¹⁻¹², R¹⁻¹⁶, R¹⁻¹⁷, R¹⁻¹⁸, R¹⁻²⁸and R¹⁻²⁹are independently H or C₁-C₃alkyl; R¹⁻³¹is C₁-C₃alkyl;
R¹⁻¹³is H, C₁-C₆alkyl or halogenated C₁-C₆alkyl;
R¹⁻¹⁴ and R¹⁻¹⁵are independently H, C₁-C₆alkyl or halogenated C₁-C₆alkyl;
R¹⁻¹⁹, R¹⁻²⁰, R¹⁻²¹, R¹⁻²², R¹⁻²³and R¹⁻²⁴are independently OH, halogen, amino, CN, or C₁-C₆alkyl.

2. The compound of formula I according to claim 1, a solvate thereof, a prodrug thereof, a metabolite thereof, or a pharmaceutically acceptable salt thereof, wherein when R⁴, R⁶ and R⁷are independently C₁-C₆alkyl, the C₁-C₆alkyl is C₁-C₃alkyl;
and/or, when the R⁸ is C₁-C₆alkyl, the C₁-C₆alkyl is C₁-C₃alkyl;
and/or, when B is C₂-C₁₀ alkylene, the C₂-C₁₀alkylene is C₄-C₆alkylene;
and/or, when B is C₂-C₁₀unsaturated hydrocarbylene, the C₂-C₁₀unsaturated hydrocarbylene is C₄-C₆unsaturated hydrocarbylene;
and/or, when B is R¹⁻¹-substituted C₂-C₁₀alkylene, the C₂-C₁₀alkylene is C₄-C₆alkylene;
and/or, when B is R¹⁻¹-substituted C₂-C₁₀alkylene, the number of R¹⁻¹is one or more, and when the number of R^{1- 1}is multiple, the R¹⁻¹ are the same or different;
and/or, when B is R¹⁻¹-substituted C₂-C₁₀alkylene, the number of the R¹⁻¹is one or more, and when the number of the R¹⁻¹ is multiple, the multiple is 2 or 3;
and/or, when B is R¹⁻¹-substituted C₂-C₁₀unsaturated hydrocarbylene, the C₂-C₁₀unsaturated hydrocarbylene is C₄-C₆unsaturated hydrocarbylene;
and/or, when B is R¹⁻¹-substituted C₂-C₁₀unsaturated hydrocarbylene, the number of R¹⁻¹ is one or more, when the number of R¹⁻¹is multiple, the R¹⁻¹ are the same or different;
and/or, when B is R¹⁻¹-substituted C₂-C₁₀unsaturated hydrocarbylene, the number of the R¹⁻¹is one or more, and when the number of the R¹⁻¹ is multiple, the multiple is 2 or 3;
and/or, when L⁵ is C₃-C₆cycloalkylene, the C₃-C₆cycloalkylene is C₃-C₅cycloalkylene;
and/or, when L⁵ is halogenated C₃-C₆cycloalkylene, the C₃-C₆cycloalkylene is C₃-C₅cycloalkylene;
and/or, when L⁵ is a halogenated C₃-C₆cycloalkylene, the number of the halogens is one or more;
and/or, when L⁵ is "3-6 membered heterocycloalkylene having 1-3 hetero atoms selected from one or more of N, O and S", the heteroatom in the "3-6 membered heterocycloalkylene having 1-3 hetero atoms selected from one or more of N, O and S" is O;
and/or, when L⁵ is "3-6 membered heterocycloalkylene having 1-3 hetero atoms selected from one or more of N, O and S", the number of the heteroatoms in the "3-6 membered heterocycloalkylene having 1-3 hetero atoms selected from one or more of N, O and S" is 1 or 2;
and/or, when L⁵ is halogenated "3-6 membered heterocycloalkylene having 1-3 hetero atoms selected from one or more of N, O and S", the heteroatom in the "3-6 membered heterocycloalkylene having 1-3 hetero atoms selected from one or more of N, O and S" is O;
and/or, when L⁵ is halogenated "the heteroatom in the "3-6 membered heterocycloalkylene having 1-3 hetero atoms selected from one or more of N, O and S", the number of the heteroatoms in the "3-6 membered heterocycloalkylene having 1-3 hetero atoms selected from one or more of N, O and S" is 1 or 2;
and/or, when L⁵ is a halogenated "3-6 membered heterocycloalkylene having 1-3 hetero atoms selected from one or more of N, O and S", the number of the halogen is one or more;
and/or, when Z¹, Z², Z³, Z⁴, Z⁶, Z⁷, Z⁸and Z⁹are independently C₁-C₆alkylene, the C₁-C₆alkylene is C₁-C₄alkylene;
and/or, when Z¹, Z², Z³, Z⁴, Z⁶, Z⁷, Z⁸, and Z⁹are independently C₁-C₆unsaturated hydrocarbylene, the C₁-C₆unsaturated hydrocarbylene is a C₁-C₄unsaturated hydrocarbylene;
and/or, when Z¹, Z², Z³, Z⁴, Z⁶, Z⁷, Z⁸and Z⁹are independently R¹⁻²substituted C₁-C₆alkylene, the C₁-C₆alkylene is C₁-C₄alkylene;
and/or, when Z¹, Z², Z³, Z⁴, Z⁶, Z⁷, Z⁸and Z⁹are independently R¹⁻²substituted C₁-C₆alkylene, the number of R¹⁻²is one or more, and when the number of R¹⁻² is multiple, the R¹⁻² are the same or different;
and/or, when Z¹, Z², Z³, Z⁴, Z⁶, Z⁷, Z⁸and Z⁹are independently R¹⁻²substituted C₁-C₆alkylene, the number of R¹⁻²is one or more, and when the number of R¹⁻²is multiple, the multiple is 2 or 3;
and/or, when Z¹, Z², Z³, Z⁴, Z⁶, Z⁷, Z⁸and Z⁹are independently R¹⁻²substituted C₁-C₆unsaturated hydrocarbylene, the C₁-C₆unsaturated hydrocarbylene is C₁-C₄unsaturated hydrocarbylene;
and/or, when Z¹, Z², Z³, Z⁴, Z⁶, Z⁷, Z⁸and Z⁹are independently R¹⁻²substituted C₁-C₆unsaturated hydrocarbylene, the number of R¹⁻²is one or more, when the number of R¹⁻²is multiple, the R¹⁻² are the same or different;
and/or, when Z¹, Z², Z³, Z⁴, Z⁶, Z⁷, Z⁸and Z⁹are independently R¹⁻²substituted C₁-C₆unsaturated hydrocarbylene, the number of R¹⁻²is one or more, and when the number of R¹⁻²is multiple, the multiple is 2 or 3;
and/or, when Z⁵ is C₂-C₁₀alkylene, the C₂-C₁₀alkylene is C₃-C₅alkylene;
and/or, when Z⁵ is C₂-C₁₀unsaturated hydrocarbylene, the C₂-C₁₀unsaturated hydrocarbylene is C₃-C₅unsaturated hydrocarbylene;
and/or, when Z⁵ is R¹⁻³substituted C₂-C₁₀alkylene, the C₂-C₁₀alkylene is C₃-C₅alkylene;
and/or, when Z⁵ is R¹⁻³ substituted C₂-C₁₀alkylene, the number of R¹⁻³is one or more, and when the number of R¹⁻³is multiple, the R¹⁻³ are the same or different;
and/or, when Z⁵ is R¹⁻³ substituted C₂-C₁₀alkylene, the number of R¹⁻³is one or more, and when the number of R¹⁻³is multiple, the multiple is 2 or 3;
and/or, when Z⁵ is R¹⁻³ substituted C₂-C₁₀unsaturated hydrocarbylene, the C₂-C₁₀unsaturated hydrocarbylene is C₃-C₅unsaturated hydrocarbylene;
and/or, when Z⁵ is R¹⁻³ substituted C₂-C₁₀unsaturated hydrocarbylene, the number of R¹⁻³is one or more, and when the number of R¹⁻³is multiple, the R¹⁻³are the same or different;
and/or, when Z⁵ is R¹⁻³ substituted C₂-C₁₀unsaturated hydrocarbylene, the number of R¹⁻³is one or more, and when the number of R¹⁻³is multiple, the multiple is 2 or 3;
and/or, when R¹⁻¹, R¹⁻², and R¹⁻³are independently halogen, the halogen is fluorine, chlorine, bromine or iodine;
and/or, when R¹⁻¹, R¹⁻², and R¹⁻³are independently C₁-C₆alkyl, the C₁-C₆alkyl is C₁-C₃alkyl;
and/or, when R¹⁻¹, R¹⁻², and R¹⁻³are independently C₁-C₆alkoxy, the C₁-C₆alkoxy is C₁-C₃alkoxy;
and/or, when R¹⁻¹⁻¹ is C₁-C₃alkyl, the C₁-C₃alkyl is methyl, ethyl, n-propyl or isopropyl;
and/or, when R⁵ is halogen, the halogen is fluorine, chlorine, bromine or iodine;
and/or, when the R⁵ is C₁-C₆alkyl, the C₁-C₆alkyl is C₁-C₃alkyl;
and/or, when R¹⁰ and R¹²are independently C₁-C₆alkyl, the C₁-C₆alkyl is C₁-C₃alkyl;
and/or, when R¹⁰and R¹²are independently C₁-C₆alkoxy substituted C₁-C₆alkyl, the C₁-C₆alkyl is C₁-C₃alkyl;
and/or, when R¹⁰and R¹²are independently C₁-C₆alkoxy-substituted C₁-C₆alkyl, the C₁-C₆alkoxy is C₁-C₃alkoxy;
and/or, when the R¹¹ is C₁-C₆alkyl, the C₁-C₆alkyl is C₁-C₃alkyl;
and/or, when R¹¹ is R¹⁻³⁰ substituted C₁-C₆alkyl, the C₁-C₆alkyl is C₁-C₃alkyl;
and/or, when R¹¹ is R¹⁻³⁰substituted C₁-C₆alkyl, the number of R¹⁻³⁰is one or more, and when the number of R¹⁻³⁰is multiple, the R¹⁻³⁰ are the same or different;
and/or, when the R¹¹is R¹⁻³⁰substituted C₁-C₆alkyl, the number of the R¹⁻³⁰is one or more, and when the number of the R¹⁻³⁰is multiple, the multiple is 2 or 3;
and/or, when R¹⁻³⁰ is C₁-C₆alkoxy, the C₁-C₆alkoxy is C₁-C₃alkoxy;
and/or, when R¹⁰ and R¹¹ together with the nitrogen atom to which they are attached form a "5-7 membered heterocycloalkyl having 1-2 heteroatoms, and the heteroatom is N", the "5-7 membered heterocycloalkyl having 1-2 heteroatoms, and the heteroatom is N" is pyrrolidyl or piperidinyl;
and/or, when R¹⁴, R¹⁵, R¹⁶and R¹⁷are independently C₁-C₆alkyl, the C₁-C₆alkyl is C₁-C₄alkyl;
and/or, when R¹⁴, R¹⁵, R¹⁶and R¹⁷are independently R¹³⁻¹ substituted C₁-C₆alkyl, the C₁-C₆alkyl is C₁-C₃alkyl;
and/or, when R¹⁴, R¹⁵, R¹⁶, and R¹⁷are independently R¹³⁻¹substituted C₁-C₆alkyl, the number of R¹³⁻¹is one or more, and when the number of R¹³⁻¹is multiple, the R¹³⁻¹ are the same or different;
and/or, when R¹⁴, R¹⁵, R¹⁶, and R¹⁷are independently R¹³⁻¹substituted C₁-C₆alkyl, the number of the R¹³⁻¹is one or more, and when the number of the R¹³⁻¹is multiple, the multiple is 2 or 3;
and/or, when R¹³⁻¹is C₁-C₆alkoxy, the C₁-C₆alkoxy is C₁-C₃alkoxy;
and/or, the R¹³⁻²is C₁-C₃alkyl;
and/or, when R¹⁻³⁰⁻¹and R¹⁻³⁰⁻²are independently C₁-C₆alkyl, the C₁-C₆alkyl is C₁-C₃alkyl;
and/or, when R¹⁻³⁰⁻³is C₁-C₆alkyl, the C₁-C₆alkyl is C₁-C₃alkyl;
and/or, when R¹⁻³⁰⁻⁴is C₁-C₆alkyl, the C₁-C₆alkyl is C₁-C₃alkyl;
and/or, when R¹⁻³⁰⁻⁴is C₁-C₆alkoxy, the C₁-C₆alkoxy is C₁-C₃alkoxy;
and/or, when R¹⁻³⁰⁻⁵is C₁-C₆alkoxy, the C₁-C₆alkoxy is C₁-C₃alkoxy;
and/or, when R¹⁻³⁰⁻⁶and R¹⁻³⁰⁻⁷are independently C₁-C₆alkyl, the C₁-C₆alkyl is C₁-C₃alkyl;
and/or, when R¹⁻³⁰⁻⁶and R¹⁻³⁰⁻⁷ together with the nitrogen atom to which they are attached form a "5-7 membered heterocycloalkyl having 1-2 heteroatoms, and the heteroatom is N", the "5-7 membered heterocycloalkyl having 1-2 heteroatoms, and the heteroatom is N" is pyrrolyl or piperidinyl;
and/or, when R⁵ is C₁-C₆alkoxy, the C₁-C₆alkoxy is C₁-C₃alkoxy;
and/or, when R¹, R² together with the carbon atoms to which they are attached form a "4-7 membered heterocycloalkylene having 1-3 heteroatoms selected from one or more of N, O and S", the heteroatoms in the "4-7 membered heterocycloalkylene having 1-3 heteroatoms selected from one or more of N, O and S" are N and/or O;
and/or, when R¹, R² together with the carbon atoms to which they are attached form a "4-7 membered heterocycloalkylene having 1-3 heteroatoms selected from one or more of N, O and S", the number of heteroatoms in the "4-7 membered heterocycloalkylene having 1-3 heteroatoms selected from one or more of N, O and S" is 1 or 2;
and/or, when R¹, R² together with the carbon atoms to which they are attached form a "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is 5-6 membered heterocycloalkylene;
and/or, when R¹, R² together with the carbon atoms to which they are attached form a "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene group is replaced by a carbonyl group;
and/or, when R¹, R² together with the carbon atoms to which they are attached form a R¹⁻⁴substituted "4-7 membered heterocycloalkylene having 1-3 heteroatoms selected from one or more of N, O and S", the heteroatoms in the "4-7 membered heterocycloalkylene having 1-3 heteroatoms selected from one or more of N, O and S" are N and/or O;
and/or, when R¹, R² together with the carbon atoms to which they are attached form a R¹⁻⁴substituted "heteroatoms are selected from one or more of N, O and S, and the number of heteroatoms is 1-3 4-7 membered heterocycloalkyls", the "heteroatoms are selected from one or more of N, O and S, 4-7 membered heterocycloalkyl with 1-3 heteroatoms", the number of heteroatoms is 1 or 2;
and/or, whenR¹, R² together with the carbon atoms to which they are attached form a R¹⁻⁴substituted "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is 5-6 membered heterocycloalkylene;
and/or, whenR¹, R² together with the carbon atoms to which they are attached form a R¹⁻⁴substituted "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene is replaced by a carbonyl group;
and/or, whenR¹, R² together with the carbon atoms to which they are attached form a R¹⁻⁴substituted "4-7 membered heterocycloalkylene having 1-3 heteroatoms selected from one or more of N, O and S", the number of R¹⁻⁴is one or more, when the number of R¹⁻⁴is multiple, the R¹⁻⁴ are the same or different;
and/or, whenR¹, R² together with the carbon atoms to which they are attached form a R¹⁻⁴substituted "4-7 membered heterocycloalkylene having 1-3 heteroatoms selected from one or more of N, O and S", the number of R¹⁻⁴ is one or more, and when the number of R¹⁻⁴is multiple, the multiple is 2 or 3;
and/or, when R¹, R² together with the carbon atoms to which they are attached form a C₄-C₇cycloalkylene, the C₄-C₇cycloalkylene is C₅-C₆cycloalkylene;
and/or, when R¹, R² together with the carbon atoms to which they are attached form a R¹⁻⁵-substituted C₄-C₇cycloalkylene, the C₄-C₇cycloalkylene is C₅-C₆cycloalkylene;
and/or, when R¹, R² together with the carbon atoms to which they are attached form a R¹⁻⁵-substituted C₄-C₇cycloalkylene, the number of R¹⁻⁵is one or more, and when the number of R¹⁻⁵is multiple, the R¹⁻⁵ are the same or different;
and/or, when R¹, R² together with the carbon atoms to which they are attached form a R¹⁻⁵-substituted C₄-C₇cycloalkylene, the number of R¹⁻⁵ is one or more, and when the number of R¹⁻⁵is multiple, the multiple is 2 or 3;
and/or, in scheme A, when R³is halogen, the halogen is fluorine, chlorine, bromine or iodine;
and/or, in scheme A, when R³is C₁-C₆alkyl, the C₁-C₆alkyl is C₁-C₃alkyl;
and/or, in scheme A, when R³is a halogenated C₁-C₆alkyl, the C₁-C₆alkyl is C₁-C₃alkyl;
and/or, in scheme A, when R³is a halogenated C₁-C₆alkyl, the halogen is fluorine, chlorine, bromine or iodine;
and/or, in scheme A, when R³is a halogenated C₁-C₆alkyl, the number of the halogens is one or more;
and/or, when R², R³ together with the carbon atoms to which they are attached form a "4-7 membered heterocycloalkylene having 1-3 heteroatoms selected from one or more of N, O and S", the heteroatom in the "4-7 membered heterocycloalkylene having 1-3 heteroatoms selected from one or more of N, O and S" is N and/or O;
and/or, when R², R³ together with the carbon atoms to which they are attached form a "4-7 membered heterocycloalkylene having 1-3 heteroatoms selected from one or more of N, O and S", the number of heteroatoms "4-7 membered heterocycloalkylene having 1-3 heteroatoms selected from one or more of N, O and S" is 1 or 2;
and/or, when R², R³ together with the carbon atoms to which they are attached form a "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is a 5-to 6-membered heterocycloalkylene;
and/or, when R², R³ together with the carbon atoms to which they are attached form a "4-7 membered heterocycloalkylene having 1-3 heteroatoms selected from one or more of N, O and S", the methylene "4-7 membered heterocycloalkylene having 1-3 heteroatoms selected from one or more of N, O and S" is not replaced, or one of the methylene is replaced by carbonyl group;
and/or, when R², R³ together with the carbon atoms to which they are attached form a R¹⁻⁴ substituted "4-7 membered heterocycloalkylene having 1-3 heteroatoms selected from one or more of N, O and S", the heteroatom in the "4-7 membered heterocycloalkylene having 1-3 heteroatoms selected from one or more of N, O and S" is N and/or O;
and/or, when R², R³ together with the carbon atoms to which they are attached form a R¹⁻⁴ substituted "4-7 membered heterocycloalkylene having 1-3 heteroatoms selected from one or more of N, O and S", the number of heteroatoms in the "4-7 membered heterocycloalkylene having 1-3 heteroatoms selected from one or more of N, O and S" is 1 or 2;
and/or, when R², R³ together with the carbon atoms to which they are attached form a R¹⁻⁴ substituted "4-7 membered heterocycloalkylene having 1-3 heteroatoms selected from one or more of N, O and S", the 4-7 membered heterocycloalkylene in the "4-7 membered heterocycloalkylene having 1-3 heteroatoms selected from one or more of N, O and S" is a 5-to 6-membered heterocycloalkylene;
and/or, when R², the R³ together with the carbon atoms to which they are attached form a R¹⁻⁴ substituted "4-7 membered heterocycloalkylene having 1-3 heteroatoms selected from one or more of N, O and S", the methylene in the "4-7 membered heterocycloalkylene having 1-3 heteroatoms selected from one or more of N, O and S" is not replaced, or one methylene is replaced by a carbonyl group;
and/or, when R², R³ together with the carbon atoms to which they are attached form a R¹⁻⁴ substituted "4-7 membered heterocycloalkylene having 1-3 heteroatoms selected from one or more of N, O and S", the number of the R¹⁻⁴is one or more, when the number of R¹⁻⁴is multiple, the R¹⁻⁴ are the same or different;
and/or, when R², R³ together with the carbon atoms to which they are attached form a R¹⁻⁴ substituted "4-7 membered heterocycloalkylene having 1-3 heteroatoms selected from one or more of N, O and S", the number of the R¹⁻⁴is one or more, when the number of R¹⁻⁴is multiple, the multiple is 2 or 3;
and/or, when R², R³ together with the carbon atoms to which they are attached form a C₄-C₇cycloalkylene, the C₄-C₇cycloalkylene is C₅-C₆cycloalkylene;
and/or, when R², R³ together with the carbon atoms to which they are attached form a R¹⁻⁵-substituted C₄-C₇cycloalkylene, the number of the R¹⁻⁵is one or more, and when the number of the R¹⁻⁵is multiple, the R¹⁻⁵are the same or different;
and/or, when R², R³ and the carbon atoms to which they are attached together form the R¹⁻⁵-substituted C₄-C₇cycloalkylene, the number of the R¹⁻⁵is one or more, and when the number of the R¹⁻⁵is multiple, the number is 2 or 3;
and/or, in scheme B, when R¹ is halogen, the halogen is fluorine, chlorine, bromine or iodine;
and/or, in scheme B, when R¹ is C₁-C₆alkyl, the C₁-C₆alkyl is C₁-C₃alkyl;
and/or, in scheme B, when R¹ is halogenated C₁-C₆alkyl, the C₁-C₆alkyl is C₁-C₃alkyl;
and/or, in scheme B, when R¹ is a halogenated C₁-C₆alkyl, the halogen is fluorine, chlorine, bromine or iodine;
and/or, in scheme B, when R¹ is a halogenated C₁-C₆alkyl, the number of the halogen is one or more;
and/or, when R¹⁻⁴ and R¹⁻⁵are independently halogen, the halogen is fluorine, chlorine, bromine or iodine;
and/or, when R¹⁻⁴ and R¹⁻⁵ are independently C₁-C₆alkyl, the C₁-C₆alkyl is C₁-C₄alkyl;
and/or, when R¹⁻⁴ and R¹⁻⁵are independently a R¹⁻⁶ substituted C₁-C₆alkyl, the C₁-C₆alkyl is a C₁-C₄alkyl;
and/or, when R¹⁻⁴ and R¹⁻⁵are independently a R¹⁻⁶ substituted C₁-C₆alkyl, the number of the R¹⁻⁶is one or more, and when the number of the R¹⁻⁶is multiple, the R¹⁻⁶are the same or different;
and/or, when R¹⁻⁴ and R¹⁻⁵are independently a R¹⁻⁶ substituted C₁-C₆alkyl, the number of R¹⁻⁶is one or more, and when the number of R¹⁻⁶is multiple, the number of R¹⁻⁶ is 2 or 3;
and/or, when R¹⁻⁴ and R¹⁻⁵are independently C₁-C₆alkoxy, the C₁-C₆alkoxy is C₁-C₃alkoxy;
and/or, when R¹⁻⁴ and R¹⁻⁵are independently a R¹⁻⁹ substituted C₁-C₆alkoxy, the C₁-C₆alkoxy is a C₁-C₃alkoxy;
and/or, when R¹⁻⁴ and R¹⁻⁵are independently a R¹⁻⁹ substituted C₁-C₆alkoxy, the number of the R¹⁻⁹is one or more, and when the number of the R¹⁻⁹is multiple, the R¹⁻⁹are the same or different;
and/or, when R¹⁻⁴ and R¹⁻⁵ are independently a R¹⁻⁹ substituted C₁-C₆alkoxy, the number of R¹⁻⁹ is one or more, and when the number of R¹⁻⁹is multiple, the number of R¹⁻⁹ is 2 or 3;
and/or, when R¹⁻⁴ and R¹⁻⁵ are independently C₃-C₇cycloalkyl, the C₃-C₇cycloalkyl is C₃-C₅cycloalkyl;
and/or, when R¹⁻⁴ and R¹⁻⁵ are independently a R¹⁻¹⁹ substituted C₃-C₇cycloalkyl, the C₃-C₇cycloalkyl is C₃-C₅cycloalkyl;
and/or, when R¹⁻⁴and R¹⁻⁵ are independently a R¹⁻¹⁹ substituted C₃-C₇cycloalkyl, the number of the R¹⁻¹⁹is one or more, and when the number of the R¹⁻¹⁹is multiple, the R¹⁻¹⁹ are the same or different;
and/or, when R¹⁻⁴and R¹⁻⁵ are independently a R¹⁻¹⁹ substituted C₃₋C₇cycloalkyl, the number of the R¹⁻¹⁹ is one or more, and when the number of the R¹⁻¹⁹ is multiple, the multiple is 2 or 3;
and/or, when R¹⁻⁴ and R¹⁻⁵ are independently "4-7 membered heterocycloalkyl having 1-3 heteroatoms selected from one or more of N, O and S", the heteroatom in the "4-7 membered heterocycloalkyl having 1-3 heteroatoms selected from one or more of N, O and S" is N or O;
and/or, when R¹⁻⁴ and R¹⁻⁵ are independently "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1;
and/or, when R¹⁻⁴ and R¹⁻⁵ are independently "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl;
and/or, when R¹⁻⁴ and R¹⁻⁵ are independently R¹⁻²⁰ substituted "4-7 membered heterocycloalkyl having 1-3 heteroatoms selected from one or more of N, O and S", the heteroatoms in the "4-7 membered heterocycloalkyl having 1-3 heteroatoms selected from one or more of N, O and S" is N or O;
and/or, when R¹⁻⁴ and R¹⁻⁵are independently R¹⁻²⁰ substituted "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1;
and/or, when R¹⁻⁴and R¹⁻⁵are independently R¹⁻²⁰ substituted "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl;
and/or, when R¹⁻⁴and R¹⁻⁵are independently R¹⁻²⁰ substituted "4-7 membered heterocycloalkyl having 1-3 heteroatoms selected from one or more of N, O and S", the number of R¹⁻²⁰ is one or more, when the number of R¹⁻²⁰ is multiple, the R¹⁻²⁰ are the same or different;
and/or, when R¹⁻⁴and R¹⁻⁵are independently R¹⁻²⁰ substituted "4-7 membered heterocycloalkyl having 1-3 heteroatoms selected from one or more of N, O and S", the number of R¹⁻²⁰is one or more, when the number of R¹⁻²⁰is multiple, the multiple is 2 or 3;
and/or, when R¹⁻⁴and R¹⁻⁵are independently "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" is N and/or O;
and/or, when R¹⁻⁴and R¹⁻⁵are independently "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1 or 2;
and/or, when R¹⁻⁴and R¹⁻⁵are independently "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl;
and/or, when R¹⁻⁴and R¹⁻⁵are independently R¹⁻²¹ substituted "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" is N and/or O;
and/or, when R¹⁻⁴and R¹⁻⁵are independently R¹⁻²¹ substituted "C₁-C₁₀heteroaryl having 1-4 heteroatoms selected from one or more of N, O and S", the number of heteroatoms in the "Ci-C₁₀heteroaryl having 1-4 heteroatoms selected from one or more of N, O and S" is 1 or 2;
and/or, when R¹⁻⁴and R¹⁻⁵are independently R¹⁻²¹ substituted "C₁-C₁₀heteroaryl having 1-4 heteroatoms selected from one or more of N, O and S", the C₁-C₁₀heteroaryl of the "Ci-C₁₀heteroaryl having 1-4 heteroatoms selected from one or more of N, O and S" is C₃-C₅heteroaryl;
and/or, when R¹⁻⁴and R¹⁻⁵are independently R¹⁻²¹ substituted "C₁-C₁₀heteroaryl having 1-4 heteroatoms selected from one or more of N, O and S", the number of R¹⁻²¹ is one or more, when the number of R¹⁻²¹ is multiple, the R¹⁻²¹ are the same or different;
and/or, when R¹⁻⁴and R¹⁻⁵are independently R¹⁻²¹ substituted "C₁-C₁₀heteroaryl having 1-4 heteroatoms selected from one or more of N, O and S", the number of R¹⁻²¹is one or more, when the number of R¹⁻²¹is multiple, the multiple is 2 or 3;
and/or, when R¹⁻⁶ and R¹⁻⁹are independently halogen, the halogen is fluorine, chlorine, bromine or iodine;
and/or, when R¹⁻⁶and R¹⁻⁹are independently C₁-C₃alkoxy, the C₁-C₃alkoxy is methoxy, ethoxy, n-propoxy or isopropoxy;
and/or, when R¹⁻⁶and R¹⁻⁹are independently C₃-C₇cycloalkyl, the C₃-C₇cycloalkyl is C₃-C₅cycloalkyl;
and/or, when R¹⁻⁶and R¹⁻⁹are independently COOR¹⁻¹⁸substituted C₃-C₇cycloalkyl, the C₃-C₇cycloalkyl is C₃-C₅cycloalkyl;
and/or, when R¹⁻⁶and R¹⁻⁹are independently a COOR¹⁻¹⁸ substituted C₃₋C₇cycloalkyl, the number of the COOR¹⁻¹⁸ is one or more, and when the number of the COOR¹⁻¹⁸ is multiple, the COOR¹⁻¹⁸ are the same or different;
and/or, when R¹⁻⁶and R¹⁻⁹are independently a COOR¹⁻¹⁸ substituted C₃-C₇cycloalkyl, the number of the COOR¹⁻¹⁸ is one or more, and when the number of the COOR¹⁻¹⁸ is multiple, the multiple is 2 or 3;
and/or, when R¹⁻⁶and R¹⁻⁹are independently "4-7 membered heterocycloalkylene having 1-3 heteroatoms selected from one or more of N, O and S", the heteroatom is N;
and/or, when R¹⁻⁶ and R¹⁻⁹ are independently "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1;
and/or, when R¹⁻⁶ and R¹⁻⁹ are independently "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl;
and/or, when R¹⁻⁶ and R¹⁻⁹are independently R¹⁻²² substituted "4-7 membered heterocycloalkyl having 1-3 heteroatoms selected from one or more of N, O and S", the heteroatoms in the "4-7 membered heterocycloalkyl having 1-3 heteroatoms selected from one or more of N, O and S" are N;
and/or, when R¹⁻⁶ and R¹⁻⁹are independently R¹⁻²² substituted "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1;
and/or, when R¹⁻⁶and R¹⁻⁹are independently R¹⁻²² substituted "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl;
and/or, when R¹⁻⁶and R¹⁻⁹are independently R¹⁻²² substituted "4-7 membered heterocycloalkyl having 1-3 heteroatoms selected from one or more of N, O and S", the number of R¹⁻²² is one or more, when the number of R¹⁻²² is multiple, the R¹⁻²² are the same or different;
and/or, when R¹⁻⁶and R¹⁻⁹are independently R¹⁻²² substituted "4-7 membered heterocycloalkyl having 1-3 heteroatoms selected from one or more of N, O and S", the number of R¹⁻²²is one or more, when the number of R¹⁻²²is multiple, the multiple is 2 or 3;
and/or, when R¹⁻⁶and R¹⁻⁹are independently "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" are N;
and/or, when R¹⁻⁶and R¹⁻⁹are independently "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1;
and/or, when R¹⁻⁶and R¹⁻⁹are independently "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl;
and/or, when R¹⁻⁶ and R¹⁻⁹are independently R¹⁻²³ substituted "C₁-C₁₀heteroaryl having 1-3 heteroatoms selected from one or more of N, O and S", the heteroatoms in the "Ci-C₁₀heteroaryl having 1-4 heteroatoms selected from one or more of N, O and S" are N;
and/or, when R¹⁻⁶ and R¹⁻⁹ are independently R¹⁻²³ substituted "C₁-C₁₀heteroaryl having 1-4 heteroatoms selected from one or more of N, O and S", the number of heteroatom in the "Ci-C₁₀heteroaryl having 1-3 heteroatoms selected from one or more of N, O and S" is 1;
and/or, when R¹⁻⁶and R¹⁻⁹are independently R1-23 substituted "C₁-C₁₀heteroaryl having 1-4 heteroatoms selected from one or more of N, O and S", the C₁-C₁₀heteroaryl of the "Ci-C₁₀heteroaryl having 1-4 heteroatoms selected from one or more of N, O and S" is C₃-C₅heteroaryl;
and/or, when R¹⁻⁶and R¹⁻⁹are independently R¹⁻²³ substituted "C₁-C₁₀heteroaryl having 1-4 heteroatoms selected from one or more of N, O and S", the number of R¹⁻²³ is one or more, when the number of R¹⁻²³ is multiple, the R¹⁻²³ are the same or different;
and/or, when R¹⁻⁶and R¹⁻⁹are independently R¹⁻²³ substituted "C₁-C₁₀heteroaryl having 1-4 heteroatoms selected from one or more of N, O and S", the number of R¹⁻²³is one or more, when the number of R¹⁻²³is multiple, the multiple is 2 or 3;
and/or, when R¹⁻⁷and R¹⁻⁸are independently C₁-C₃alkyl, the Ci-C alkyl₃is methyl, ethyl, n-propyl or isopropyl;
and/or, when R¹⁻⁷ and R¹⁻⁸ are independently C₃-C₇cycloalkyl, the C₃-C₇cycloalkyl is C₃-C₅cycloalkyl;
and/or, when R¹⁻⁷ and R¹⁻⁸ are independently a R¹⁻²⁴ substituted C₃-C₇cycloalkyl, the C₃-C₇cycloalkyl is a C₃-C₅cycloalkyl;
and/or, when R¹⁻⁷and R¹⁻⁸are independently a R¹⁻²⁴ substituted C₃₋C₇cycloalkyl, the number of the R¹⁻²⁴ is one or more, and when the number of the R¹⁻²⁴ is multiple, the R¹⁻²⁴are the same or different;
and/or, when R¹⁻⁷and R¹⁻⁸ are independently a R¹⁻²⁴ substituted C₃-C₇cycloalkyl, the number of the R¹⁻²⁴ is one or more, and when the number of the R¹⁻²⁴ is multiple, the multiple is 2 or 3;
and/or, when R¹⁻⁷ and R¹⁻⁸are independently "4-7 membered heterocycloalkyl having 1-3 heteroatoms selected from one or more of N, O and S", the heteroatom in the "4-7 membered heterocycloalkyl having 1-3 heteroatoms selected from one or more of N, O and S" is N and/or O;
and/or, when R¹⁻⁷ and R¹⁻⁸are independently "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1 or 2;
and/or, when R¹⁻⁷ and R¹⁻⁸ are independently "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl;
and/or, when R¹⁻⁷ and R¹⁻⁸are independently R¹⁻²⁵ substituted "4-7 membered heterocycloalkyl having 1-3 heteroatoms selected from one or more of N, O and S", the heteroatoms in the "4-7 membered heterocycloalkyl having 1-3 heteroatoms selected from one or more of N, O and S" are N and/or O;
and/or, when R¹⁻⁷ and R¹⁻⁸are independently R¹⁻²⁵ substituted "4-7 membered heterocycloalkyl having 1-3 heteroatoms selected from one or more of N, O and S", the number of the heteroatoms in the "4-7 membered heterocycloalkyl having 1-3 heteroatoms selected from one or more of N, O and S" is 1 or 2;
and/or, when R¹⁻⁷and R¹⁻⁸are independently R¹⁻²⁵ substituted "4-7 membered heterocycloalkyl having 1-3 heteroatoms selected from one or more of N, O and S", 4-7 membered heterocycloalkyl of the "4-7 membered heterocycloalkyl having 1-3 heteroatoms selected from one or more of N, O and S" is a 5-6 membered heterocycloalkyl;
and/or, when R¹⁻⁷and R¹⁻⁸are independently R¹⁻²⁵ substituted "4-7 membered heterocycloalkyl having 1-3 heteroatoms selected from one or more of N, O and S", the number of R¹⁻²⁵ is one or more, when the number of R¹⁻²⁵ is multiple, the R¹⁻²⁵ are the same or different;
and/or, when R¹⁻⁷and R¹⁻⁸are independently R¹⁻²⁵ substituted "4-7 membered heterocycloalkyl having 1-3 heteroatoms selected from one or more of N, O and S", the number of R¹⁻²⁵is one or more, when the number of R¹⁻²⁵is multiple, the multiple is 2 or 3;
and/or, when R¹⁻⁷and R¹⁻⁸are independently "C₁-C₁₀heteroaryl having 1-4 heteroatoms selected from one or more of N, O and S", the heteroatoms in the "C₁-C₁₀heteroaryl having 1-4 heteroatoms selected from one or more of N, O and S" is N and/or O;
and/or, when R¹⁻⁷and R¹⁻⁸are independently "C₁-C₁₀heteroaryl having 1-4 heteroatoms selected from one or more of N, O and S", the number of heteroatoms in the "C₁-C₁₀heteroaryl having 1-4 heteroatoms selected from one or more of N, O and S" is 1 or 2;
and/or, when R¹⁻⁷and R¹⁻⁸are independently "C₁-C₁₀heteroaryl having 1-4 heteroatoms selected from one or more of N, O and S", the C₁-C₁₀heteroaryl of the "C₁-C₁₀heteroaryl having 1-4 heteroatoms selected from one or more of N, O and S" is C₃-C₅heteroaryl;
and/or, when R¹⁻⁷and R¹⁻⁸are independently R¹⁻²⁶ substituted "C₁-C₁₀heteroaryl having 1-4 heteroatoms selected from one or more of N, O and S", the heteroatoms in the "Ci-C₁₀heteroaryl having 1-4 heteroatoms selected from one or more of N, O and S" is N and/or O;
and/or, when R¹⁻⁷and R¹⁻⁸are independently R¹⁻²⁶ substituted "C₁-C₁₀heteroaryl having 1-4 heteroatoms selected from one or more of N, O and S", the number of heteroatoms in the "Ci-C₁₀heteroaryl having 1-4 heteroatoms selected from one or more of N, O and S" is 1 or 2;
and/or, when R¹⁻⁷and R¹⁻⁸are independently R¹⁻²⁶ substituted "C₁-C₁₀heteroaryl having 1-4 heteroatoms selected from one or more of N, O and S", the C₁-C₁₀heteroaryl of the "Ci-C₁₀heteroaryl having 1-4 heteroatoms selected from one or more of N, O and S" is C₃-C₅heteroaryl;
and/or, when R¹⁻⁷and R¹⁻⁸are independently C₆-C₁₀aryl, the C₆-C₁₀aryl is phenyl;
and/or, when R¹⁻⁷and R¹⁻⁸are independently R¹⁻²⁷ substituted C₆-C₁₀aryl , the C₆-C₁₀aryl is phenyl;
and/or, when R¹⁻⁷and R¹⁻⁸are independently a R¹⁻²⁷ substituted C₆-C₁₀aryl, the number of the R¹⁻²⁷is one or more, and when the number of the R¹⁻²⁷ is multiple, the R¹⁻²⁷are the same or different;
and/or, when R¹⁻⁷and R¹⁻⁸are independently a R¹⁻²⁷ substituted C₆-C₁₀aryl, the number of R¹⁻²⁷is one or more, and when the number of R¹⁻²⁷ is multiple, the multiple is 2 or 3;
and/or, when R¹⁻¹⁰, R¹⁻¹¹, R¹⁻¹², R¹⁻¹⁶, R¹⁻¹⁷, R¹⁻¹⁸, R¹⁻²⁸and R¹⁻²⁹are independently C₁-C₃alkyl, the C₁-C₃alkyl is methyl, ethyl, n-propyl or isopropyl;
and/or, when R¹⁻¹³is C₁-C₆alkyl, the C₁-C₆alkyl is C₁-C₃alkyl;
and/or, when R¹⁻¹³is a halogenated C₁-C₆alkyl, the C₁-C₆alkyl is C₁-C₃alkyl;
and/or, when R¹⁻¹³is a halogenated C₁-C₆alkyl, the halogen is fluorine, chlorine, bromine or iodine;
and/or, when R¹⁻¹³is a halogenated C₁-C₆alkyl, the number of the halogen is one or more;
and/or, when R¹⁻¹⁴ and R¹⁻¹⁵ are independently C₁-C₆alkyl, the C₁-C₆alkyl is C₁-C₃alkyl;
and/or, when R¹⁻¹⁴and R¹⁻¹⁵are independently halogenated C₁-C₆alkyl, the C₁-C₆alkyl is C₁-C₃alkyl;
and/or, when R¹⁻¹⁴and R¹⁻¹⁵are independently halogenated C₁-C₆alkyl, the halogen is fluorine, chlorine, bromine or iodine;
and/or, when R¹⁻¹⁴and R¹⁻¹⁵are independently halogenated C₁-C₆alkyl, the number of the halogen is one or more;
and/or, when R¹⁻¹⁹, R¹⁻²⁰, R¹⁻²¹, R¹⁻²² , R¹⁻²³, and R¹⁻²⁴are independently halogen, the halogen is fluorine, chlorine, bromine or iodine;
and/or, when R¹⁻¹⁹, R¹⁻²⁰, R¹⁻²¹, R¹⁻²², R¹⁻²³, and R¹⁻²⁴are independently C₁-C₆alkyl, the C₁-C₆alkyl is C₁-C₃alkyl.

3. The compound of formula I according to claim 1, a solvate thereof, a prodrug thereof, a metabolite thereof, or a pharmaceutically acceptable salt thereof, wherein when R⁴, R⁶ and R⁷are independently C₁-C₆alkyl, the C₁-C₆alkyl is methyl;
and/or, when R⁸ is C₁-C₆alkyl, the C₁-C₆alkyl is methyl;
and/or, when B is C₂-C₁₀alkylene, the C₂-C₁₀alkylene is(CH₂)ᵥ-, and v is 4, 5 or 6;
and/or, when B is C₂-C₁₀ unsaturated alkylene, the C₂-C₁₀unsaturated alkylene is 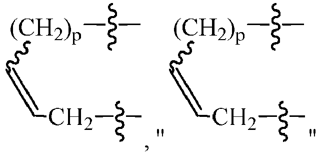 is Z configuration olefin 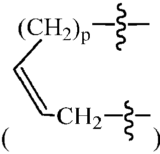 and/or E configuration olefin 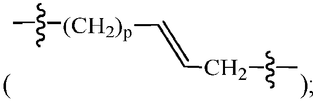 and p is 1, 2 or 3;
and/or, when B is R¹⁻¹ substituted C₂-C₁₀alkylene, the C₂-C₁₀alkylene is(CH₂)ᵥ-, v is 4, 5 or 6;
and/or, when B is R¹⁻¹ substituted C₂-C₁₀unsaturated hydrocarbylene, the C₂-C₁₀unsaturated hydrocarbylene is 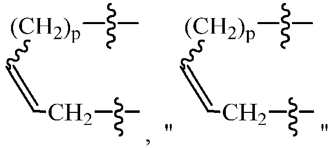 is Z configuration olefin 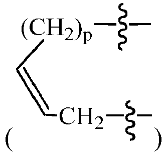 and/or E configuration olefin 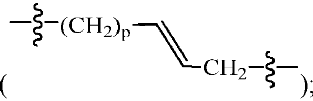 and p is 1, 2 or 3;
and/or, when L⁵ is C₃-C₆cycloalkylene, the C₃-C₆cycloalkylene is cyclopropylene, cyclobutylene, or cyclopentylene;
and/or, when L⁵ is a halogenated C₃-C₆cycloalkylene, the C₃-C₆cycloalkylene is cyclopropylidene, cyclobutylene or cyclopentylidene;
and/or, when L⁵ is a halogenated C₃-C₆cycloalkylene, the number of the halogens is one or multiple, and the multiple is 2 or 3;
and/or, when L⁵ is a halogenated "3-6 membered heterocycloalkylene having 1-3 heteroatoms selected from one or more of N, O and S", the number of the halogen is one or multiple, and the multiple is 2 or 3;
and/or, when Z⁵ is C₂-C₁₀alkylene, the C₂-C₁₀alkylene is-(CH₂)_{w}-; w is 3, 4 or 5;
and/or, when Z⁵ is C₂-C₁₀unsaturated hydrocarbylene, the C₂-C₁₀unsaturated hydrocarbylene is 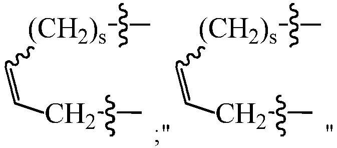 is Z configuration olefin 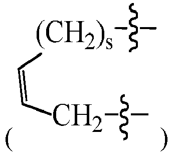 and/or E configuration olefin 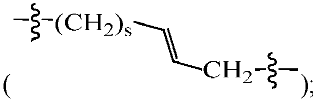 ; and s is 1 or 2;
and/or when Z⁵ is R¹⁻³substituted C₂-C₁₀alkylene, C₂-C₁₀alkylene is-(CH₂)_{w}-;w is 3, 4 or 5;
and/or, when Z⁵ is R¹⁻³substituted C₂-C₁₀unsaturated hydrocarbylene, the C₂-C₁₀unsaturated hydrocarbylene is 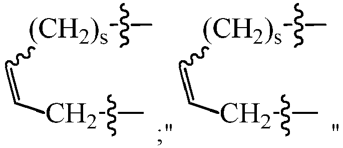 is Z configuration olefin 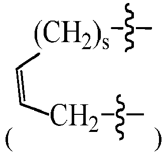 and/or E configuration olefin 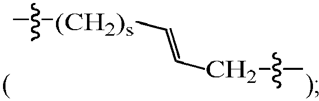 and s is 1 or 2;
and/or, when R¹⁻¹, R¹⁻², and R¹⁻³are independently C₁-C₆alkyl, the C₁-C₆alkyl is methyl, ethyl, n-propyl or isopropyl;
and/or, when R¹⁻¹, R¹⁻², and R¹⁻³are independently C₁-C₆alkoxy, the C₁-C₆alkoxy is methoxy, ethoxy, n-propoxy or isopropoxy;
and/or, when R⁵ is C₁-C₆alkyl, the C₁-C₆alkyl is methyl, ethyl, n-propyl or isopropyl;
and/or, when R¹⁰ and R¹²are independently C₁-C₆alkyl, the C₁-C₆alkyl is methyl, ethyl, n-propyl or isopropyl;
and/or, when R¹⁰ and R¹² are independently C₁-C₆alkoxy substituted C₁-C₆alkyl, the C₁-C₆alkyl is methyl, ethyl, n-propyl or isopropyl;
and/or, when R¹⁰ and R¹² are independently C₁-C₆alkoxy substituted C₁-C₆alkyl, the C₁-C₆alkoxy is methoxy, ethoxy, n-propoxy or isopropoxy;
and/or, when R¹¹ is C₁-C₆alkyl, the C₁-C₆alkyl is methyl, ethyl, n-propyl or isopropyl;
and/or, when R¹¹ is R¹⁻³⁰substituted C₁-C₆alkyl, wherein the C₁-C₆alkyl is methyl, ethyl, n-propyl or isopropyl;
and/or, when R¹⁻³⁰ is C₁-C₆alkoxy, the C₁-C₆alkoxy is methoxy, ethoxy, n-propoxy or isopropoxy;
and/or, when R¹⁴, R¹⁵, R¹⁶ and R¹⁷ are independently C₁-C₆alkyl, the C₁-C₆alkyl is methyl, ethyl, n-propyl, isopropyl, n-butyl, isobutyl, sec-butyl or t-butyl;
and/or, when R¹⁴, R¹⁵, R¹⁶, and R¹⁷are independently R¹³⁻¹-substituted C₁-C₆alkyl, wherein the C₁-C₆ alkyl is methyl, ethyl, n-propyl or isopropyl;
and/or, when R¹³⁻¹ is C₁-C₆alkoxy, wherein the C₁-C₆alkoxy is methoxy, ethoxy, n-propoxy or isopropoxy;
and/or, the R¹³⁻² is methyl, ethyl, n-propyl or isopropyl;
and/or, when R¹⁻³⁰⁻¹ and R¹⁻³⁰⁻² independently C₁-C₆alkyl, the C₁-C₆alkyl is methyl, ethyl, n-propyl or isopropyl;
and/or, when R¹⁻³⁰⁻³ is C₁-C₆alkyl, the C₁-C₆alkyl is methyl, ethyl, n-propyl or isopropyl;
and/or, when R¹⁻³⁰⁻⁴ is C₁-C₆alkyl, the C₁-C₆alkyl is methyl, ethyl, n-propyl or isopropyl;
and/or, when R¹⁻³⁰⁻⁴ is C₁-C₆alkoxy, the C₁-C₆alkoxy is methoxy, ethoxy, n-propoxy or isopropoxy;
and/or, when R¹⁻³⁰⁻⁵ is C₁-C₆alkoxy, the C₁-C₆alkoxy is methoxy, ethoxy, n-propoxy or isopropoxy;
and/or, when R¹⁻³⁰⁻⁶ and R¹⁻³⁰⁻⁷ independently C₁-C₆alkyl, the C₁-C₆alkyl is methyl, ethyl, n-propyl or isopropyl;
and/or, when R⁵ is C₁-C₆alkoxy, the C₁-C₆alkoxy is methoxy, ethoxy, n-propoxy or isopropoxy;
and/or, when the R¹, R² together with the carbon atoms to which they are attached form a "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² is 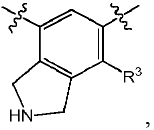 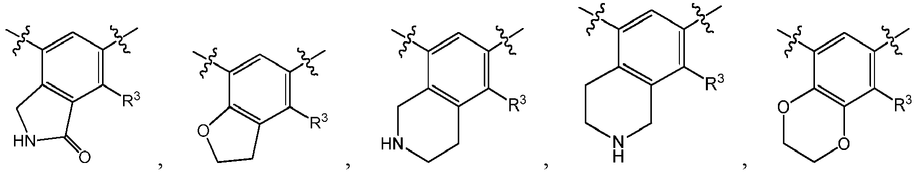 or 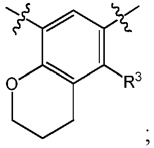
and/or, when R¹, R² together with the carbon atoms to which they are attached form a R¹⁻⁴substituted "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² is 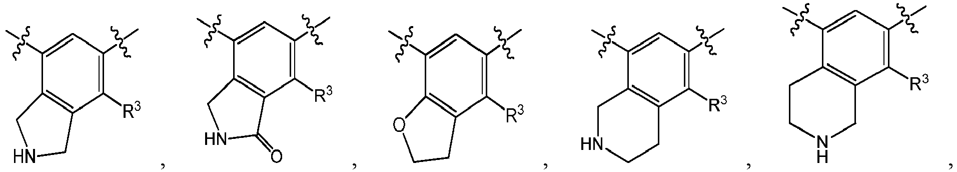 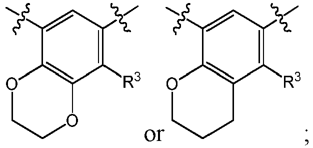
and/or, when R¹, R² together with the carbon atoms to which they are attached form a C₄-C₇cycloalkylene, the C₄-C₇cycloalkylene is cyclopentylene or cyclohexylene;
and/or, when R¹, R² together with the carbon atoms to which they are attached form a R¹⁻⁵substituted C₄-C₇cycloalkylene, the C₄-C₇cycloalkylene is cyclopentenylene or cyclohexylene;
and/or, in Scheme A, when R³ is C₁-C₆alkyl, the C₁-C₆alkyl is methyl, ethyl, n-propyl or isopropyl;
and/or, in Scheme A, when R³ is a halogenated C₁-C₆alkyl, the C₁-C₆alkyl is methyl, ethyl, n-propyl or isopropyl group;
and/or, in Scheme A, when R³ is a halogenated C₁-C₆alkyl, the number of the halogens is one or multiple, and the multiple is 2 or 3;
and/or, when the R², R³ together with the carbon atoms to which they are attached form a "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R² and R³ from 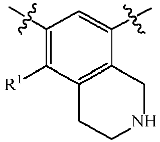 or 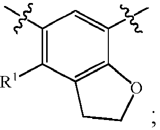
and/or, when R², R³ together with the carbon atoms to which they are attached form a R¹⁻⁴substituted "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² from 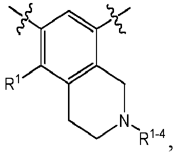 and the number of R¹⁻⁴ is one or more, when the number of R¹⁻⁴is multiple, the R¹⁻⁴ are the same or different;
and/or, when R², R³ together with the carbon atoms to which they are attached form a C₄-C₇cycloalkylene, the C₄-C₇cycloalkylene is cyclopentylene or cyclohexylene;
and/or, in Scheme B, when R¹ is C₁-C₆alkyl, the C₁-C₆alkyl is methyl, ethyl, n-propyl or isopropyl;
and/or, in Scheme B, when R¹ is a halogenated C₁-C₆alkyl, wherein the C₁-C₆alkyl is methyl, ethyl, n-propyl or isopropyl;
and/or, in Scheme B, when the R¹ is a halogenated C₁-C₆alkyl, the number of the halogens is one or more, and the number is 2 or 3;
and/or, when R¹⁻⁴ and R¹⁻⁵ are independently C₁-C₆alkyl, the C₁-C alkyl₆ is methyl, ethyl, isopropyl or tert-butyl;
and/or, when R¹⁻⁴ and R¹⁻⁵ are independently R¹⁻⁶ substituted C₁-C₆alkyl, the C₁-C₆ alkyl is methyl, ethyl, isopropyl or isobutyl;
and/or, when R¹⁻⁴and R¹⁻⁵are independently C₁-C₆alkoxy, the C₁-C₆alkoxy is methoxy, ethoxy, n-propoxy or isopropoxy;
and/or, when R¹⁻⁴ and R¹⁻⁵ are independently R¹⁻⁹substituted C₁-C₆alkoxy, the C₁-C₆alkoxy is methoxy, ethoxy, n-propoxy or isopropoxy;
and/or, when R¹⁻⁴ and R¹⁻⁵ are independently C₃-C₇cycloalkyl, the C₃-C₇cycloalkyl is cyclopropyl or cyclobutyl;
and/or, when R¹⁻⁴ and R¹⁻⁵ are independently R¹⁻¹⁹ substituted C₃-C₇cycloalkyl, the C₃-C₇cycloalkyl is cyclopropyl or cyclobutyl;
and/or, when R¹⁻⁴ and R¹⁻⁵are independently "4-7 membered heterocycloalkyl having 1-3 heteroatoms selected from one or more of N, O and S", the "4-7 membered heterocycloalkyl having 1-3 heteroatoms selected from one or more of N, O and S" is piperidinyl or tetrahydropyranyl;
and/or, when R¹⁻⁴ and R¹⁻⁵ are independently R¹⁻²⁰ substituted "4-7 membered heterocycloalkyl having 1-3 heteroatoms selected from one or more of N, O and S", the "4-7 membered heterocycloalkyl having 1-3 heteroatoms selected from one or more of N, O and S" is piperidinyl or tetrahydropyranyl;
and/or, when R¹⁻⁴ and R¹⁻⁵ are independently "C₁-C₁₀heteroaryl having 1-4 heteroatoms selected from one or more of N, O and S", the "C₁-C₁₀heteroaryl having 1-4 heteroatoms selected from one or more of N, O and S" is pyrimidyl, pyridyl or oxazolyl;
and/or, when R¹⁻⁴and R¹⁻⁵are independently R¹⁻²¹ substituted "C₁-C₁₀heteroaryl having 1-4 heteroatoms selected from one or more of N, O and S", wherein the "C₁-C₁₀heteroaryl having 1-4 heteroatoms selected from one or more of N, O and S" is pyrimidyl, pyridyl or oxazolyl;
and/or, when R¹⁻⁶ and R¹⁻⁹ are independently C₁-C₃alkoxy, the C₁-C₃alkoxys are methoxy;
and/or, when R¹⁻⁶and R¹⁻⁹are independently C₃-C₇cycloalkyl, the C₃-C₇cycloalkyl is cyclopropyl or cyclobutyl;
and/or, when R¹⁻⁶ and R¹⁻⁹ are independently COOR¹⁻¹⁸ substituted C₃-C₇cycloalkyl, the C₃-C₇cycloalkyl is cyclopropyl or cyclobutyl;
and/or, when R¹⁻⁶ and R¹⁻⁹are independently "4-7 membered heterocycloalkyl having 1-3 heteroatoms selected from one or more of N, O and S", the "4-7 membered heterocycloalkyl having 1-3 heteroatoms selected from one or more of N, O and S" is tetrahydropyrrolyl;
and/or, when R¹⁻⁶ and R¹⁻⁹ are independently R¹⁻²² substituted "4-7 membered heterocycloalkyl having 1-3 heteroatoms selected from one or more of N, O and S", wherein the "4-7 membered heterocycloalkyl having 1-3 heteroatoms selected from one or more of N, O and S" is tetrahydropyrrolyl;
and/or, when R¹⁻⁶ and R¹⁻⁹ are independently "C₁-C₁₀heteroaryl having 1-4 heteroatoms selected from one or more of N, O and S", the "C₁-C₁₀heteroaryl having 1-4 heteroatoms selected from one or more of N, O and S" is pyridinyl;
and/or, when R¹⁻⁶ and R¹⁻⁹ are independently R¹⁻²³substituted "C₁-C₁₀heteroaryl having 1-4 heteroatoms selected from one or more of N, O and S", wherein the "C₁-C₁₀heteroaryl having 1-4 heteroatoms selected from one or more of N, O and S" is pyridinyl;
and/or, when R¹⁻⁷ and R¹⁻⁸ are independently C₁-C₃alkyl, the C₁-C₃alkyl is methyl;
and/or, when R¹⁻⁷ and R¹⁻⁸ are independently C₃-C₇cycloalkyl, the C₃-C₇cycloalkyl is cyclopropyl or cyclobutyl;
and/or, when R¹⁻⁷ and R¹⁻⁸ are independently R¹⁻²⁴ substituted C₃-C₇cycloalkyl, the C₃-C₇cycloalkyl is cyclopropyl or cyclobutyl;
and/or, when R¹⁻¹³ is C₁-C₆alkyl, the C₁-C₆alkyl is methyl, ethyl, n-propyl or isopropyl;
and/or, when R¹⁻¹³ is a halogenated C₁-C₆alkyl, wherein the C₁-C₆alkyl is methyl, ethyl, n-propyl or isopropyl;
and/or, when R¹⁻¹³ is a halogenated C₁-C₆alkyl, wherein the number of the halogen is one or multiple, and the multiple is 2 or 3;
and/or, when R¹⁻¹⁴and R¹⁻¹⁵are independently C₁-C₆alkyl, the C₁-C₆ alkyl is methyl, ethyl, n-propyl or isopropyl;
and/or, when R¹⁻¹⁴and R¹⁻¹⁵are independently halogenated C₁-C₆alkyl, the C₁-C₆alkyl is methyl, ethyl, n-propyl or isopropyl;
and/or, when R¹⁻¹⁴ and R¹⁻¹⁵ are independently halogenated C₁-C₆alkyl, wherein the number of the halogen is one or multiple, and the multiple is 2 or 3;
and/or, when R¹⁻¹⁹, R¹⁻²⁰, R¹⁻²¹, R¹⁻²², R¹⁻²³and R¹⁻²⁴ are independently C₁-C₆alkyl, the C₁-C₆alkyl are methyl, ethyl, n-propyl or isopropyl.

4. The compound of formula I according to claim 1, a solvate thereof, a prodrug thereof, a metabolite thereof, or a pharmaceutically acceptable salt thereof , wherein when L⁵ is C₃-C₆cycloalkylene, the C₃-C₆cycloalkylene is cyclopropylene, 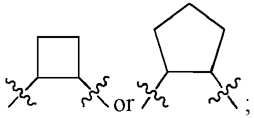
and/or, when L⁵ is halogenated C₃-C₆cycloalkylene, the C₃-C₆cycloalkylene is cyclopropylene, 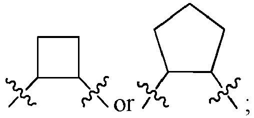
and/or, when R¹, R² together with the carbon atoms to which they are attached form a C₄-C₇cycloalkylene, the C₄-C₇cycloalkylene is 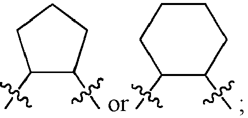
and/or, when R¹, R² together with the carbon atoms to which they are attached form a R¹⁻⁵substituted C₄-C₇cycloalkylene, wherein the C₄-C₇cycloalkylene is 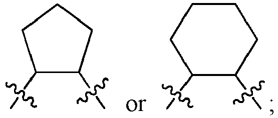
and/or, when R², R³ together with the carbon atoms to which they are attached form a C₄-C₇cycloalkylene, the C₄-C₇cycloalkylene is 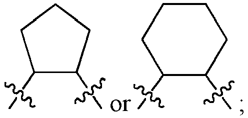
and/or, when R¹, R² together with the carbon atoms to which they are attached form a R¹⁻⁴ substituted "4-7 membered heterocycloalkylene having 1-3 heteroatoms selected from one or more of N, O and S", the "4-7 membered heterocycloalkylene having 1-3 heteroatoms selected from one or more of N, O and S" together with the benzene ring bound to R¹ and R² form 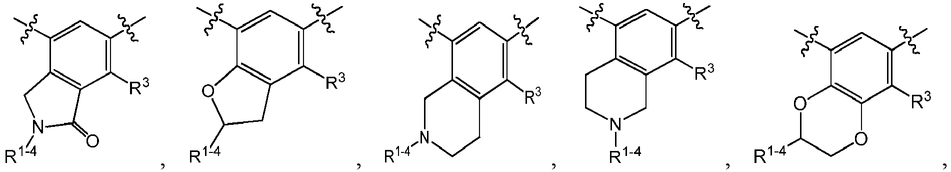 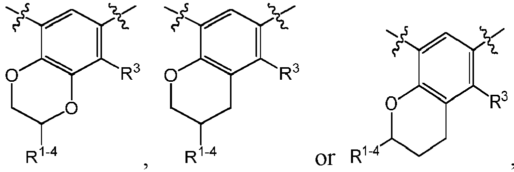 , and the number of R¹⁻⁴ is one or more, when the number of R¹⁻⁴ is multiple, each R¹⁻⁴ are the same or different;
and/or, when R¹, R²and their attached carbon atoms together form R¹⁻⁵substituted C₄-C₇cycloalkylene, the R¹⁻⁵substituted C₄-C₇cycloalkylene together with the benzene ring bonded to R¹ and R² form 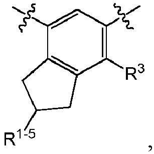 and the number of R¹⁻⁵ is 1 or 2, when the number of R¹⁻⁵ is 2, the R¹⁻⁵ are the same or different;
and/or, when R¹⁻⁴ and R¹⁻⁵are independently "4-7 membered heterocycloalkyl having 1-3 heteroatoms selected from one or more of N, O and S", the "4-7 membered heterocycloalkyl having 1-3 heteroatoms selected from one or more of N, O and S" is 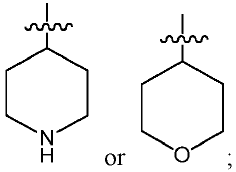
and/or, when R¹⁻⁴ and R¹⁻⁵ are independently R¹⁻²⁰ substituted "4-7 membered heterocycloalkyl having 1-3 heteroatoms selected from one or more of N, O and S", the "4-7 membered heterocycloalkyl having 1-3 heteroatoms selected from one or more of N, O and S" is 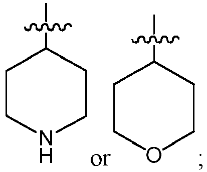
and/or, when R¹⁻⁴ and R¹⁻⁵ are independently "C₁-C₁₀heteroaryl having 1-4 heteroatoms selected from one or more of N, O and S", the "C₁-C₁₀heteroaryl having 1-4 heteroatoms selected from one or more of N, O and S" is 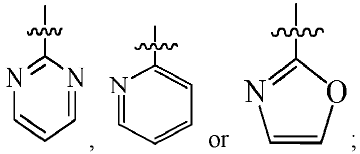
and/or, when R¹⁻⁴and R¹⁻⁵are independently R¹⁻²¹ substituted "C₁-C₁₀heteroaryl having 1-4 heteroatoms selected from one or more of N, O and S", wherein the "C₁-C₁₀heteroaryl having 1-4 heteroatoms selected from one or more of N, O and S" is 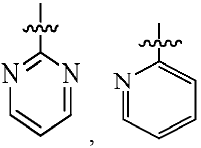 or 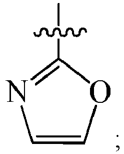
and/or, when R¹⁻⁶ and R¹⁻⁹are independently "4-7 membered heterocycloalkyl having 1-3 heteroatoms selected from one or more of N, O and S", the "4-7 membered heterocycloalkyl having 1-3 heteroatoms selected from one or more of N, O and S" is 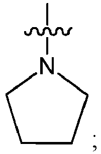
and/or, when R¹⁻⁶ and R¹⁻⁹ are independently R¹⁻²² substituted "4-7 membered heterocycloalkyl having 1-3 heteroatoms selected from one or more of N, O and S", the "4-7 membered heterocycloalkyl having 1-3 heteroatoms selected from one or more of N, O and S" is 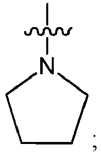
and/or, when R¹⁻⁶ and R¹⁻⁹ are independently "C₁-C₁₀heteroaryl having 1-4 heteroatoms selected from one or more of N, O and S", the "C₁-C₁₀heteroaryl having 1-4 heteroatoms selected from one or more of N, O and S" is 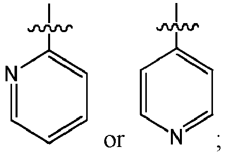
and/or, when R¹⁻⁶and R¹⁻⁹are independently R¹⁻²³substituted "C₁-C₁₀heteroaryl having 1-4 heteroatoms selected from one or more of N, O and S", wherein the "C₁-C₁₀heteroaryl having 1-4 heteroatoms selected from one or more of N, O and S" is

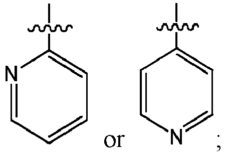

5. The compound of formula I according to claim 1, a solvate thereof, a prodrug thereof, a metabolite thereof, or a pharmaceutically acceptable salt thereof, wherein X is N;
and/or, A is O, S, S(=O)₂, NH or NCH₃;
and/or, n and r are 1;
and/or, L⁵ is C₃-C₆cycloalkylene;
and/or, L² and L⁴ are independently O;
and/or, Z⁵ is C₂-C₁₀alkylene or C₂-C₁₀unsaturated hydrocarbylene;
and/or, B is C₂-C₁₀alkylene, C₂-C₁₀unsaturated hydrocarbylene, -Z⁵-L²- or -(CH₂)ₙ-L⁵-(CH₂)ᵣ-L⁴-;
and/or, R¹⁻⁴is C₁-C₆alkyl, R¹⁻⁶substituted C₁-C₆alkyl, -S(=O)₂R¹⁻⁷, -C(=O)R¹⁻⁸, COOR¹⁻¹², C₃-C₇cycloalkyl, "4-7 membered heterocycloalkyl having 1-3 heteroatoms selected from one or more of N, O and S", "C₁-C₁₀ heteroaryl having 1-4 heteroatoms selected from one or more of N, O and S" or R¹⁻²¹substituted "C₁-C₁₀ heteroaryl having 1-4 heteroatoms selected from one or more of N, O and S";
and/or, R¹⁻⁶ is halogen, OH, COOR¹⁻¹⁷, S(=O)₂R¹⁻³¹, C₁-C₃alkoxy, "4-7 membered heterocycloalkyl having 1-3 heteroatoms selected from one or more of N, O and S" (e. g. 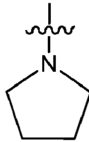 ) or "C₁-C₁₀ heteroaryl having 1-4 heteroatoms selected from one or more of N, O and S" (e. g. 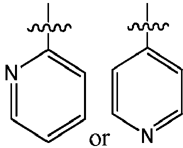 );
and/or, R¹⁻⁷and R¹⁻⁸ are independently C₁-C₃alkyl (e. g. methyl) or C₆-C₁₀aryl (e. g. phenyl);
and/or, R¹⁻²¹ is C₁-C₃alkyl;
and/or, R¹⁻⁵ is C₁-C₄alkyl or COOH;
and/or, in scheme A, the R³is H;
and/or, in scheme A, R¹, R² together with the carbon atoms to which they are attached form a "5-6 membered heterocycloalkylene having 1-2 heteroatoms selected from one or more of N and/or O", R¹⁻⁴substituted "5-6 membered heterocycloalkylene having 1-2 heteroatoms selected from one or more of N and/or O", C₅-C₆cycloalkylene or R¹⁻⁵substituted C₅-C₆cycloalkylene; wherein the methylene in the "5-6 membered heterocycloalkylene having 1-2 heteroatoms selected from one or more of N and/or O" and R¹⁻⁴substituted "5-6 membered heterocycloalkylene having 1-2 heteroatoms selected from one or more of N and/or O" is not replaced, or one methylene isreplaced by a carbonyl group;
and/or, in scheme B, R¹is H;
and/or, in scheme B, R², R³ together with the carbon atoms to which they are attached form a "5-6 membered heterocycloalkylene having 1-2 heteroatoms selected from one or more of N and/or O", R¹⁻⁴substituted "5-6 membered heterocycloalkylene having 1-2 heteroatoms selected from one or more of N and/or O"; wherein the methylene in the "5-6 membered heterocycloalkylene having 1-2 heteroatoms selected from one or more of N and/or O" and R¹⁻⁴substituted "5-6 membered heterocycloalkylene having 1-2 heteroatoms selected from one or more of N and/or O" is not replaced;
and/or, R⁵ is H;
and/or, R¹⁰ is C₁-C₆alkyl, and R¹¹is C₁-C₆alkyl;
and/or, R¹² is C₁-C₆alkyl;
and/or, R⁹ is H;
and/or, R¹³ is H or COOR¹⁷;
and/or, R¹⁷ is C₁-C₆alkyl.

6. The compound of formula I according to claim 5, a solvate thereof, a prodrug thereof, a metabolite thereof, or a pharmaceutically acceptable salt thereof, wherein A is O, S or S(=O)₂;
and/or, B is C₄-C₆alkylene, C₄-C₆unsaturated hydrocarbylene, -Z⁵-L²- or -(CH₂)ₙ-L⁵-(CH₂)ᵣ-L⁴-, and Z⁵ is C₃-C₅alkylene or C₃-C₅unsaturated hydrocarbylene;
and/or, R¹⁻⁷ and R¹⁻⁸are independently C₁-C₃alkyl (e. g. methyl);
and/or, R¹³ is H.

7. The compound of formula I according to claim 5, a solvate thereof, a prodrug thereof, a metabolite thereof, or a pharmaceutically acceptable salt thereof, wherein, A is O or S(=O)₂;
and/or, B is 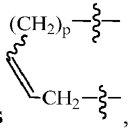 -(CH₂)ᵥ-, 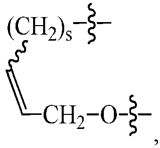 -(CH₂)_{w}-O- or -CH₂-L⁵-CH₂-O-; p is 1, 2 or 3; v is 4, 5 or 6; s is 1 or 2; w is 3, 4 or 5; and L⁵ is C₃-C₅cycloalkylene;
and/or, where R¹⁻⁴ is COOH, C₁-C₄alkyl, R¹⁻⁶substituted C₁-C₄alkyl, -C(=O)R¹⁻⁸,-S(=O)₂R¹⁻⁷, 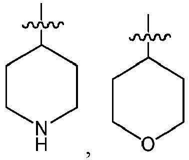 or cyclobutyl; R¹⁻⁶ is COOR¹⁻¹⁷, C₁-C₃alkoxy, 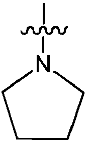 or 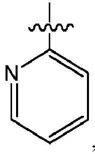 , R¹⁻⁷is methyl; and R¹⁻⁸is methyl;
and/or, 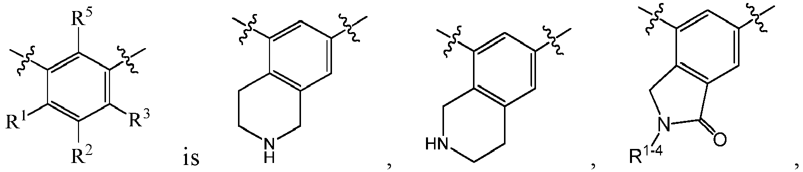 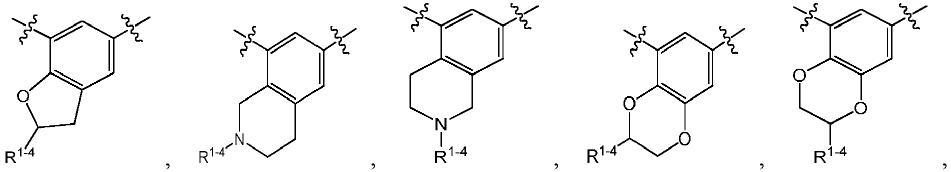 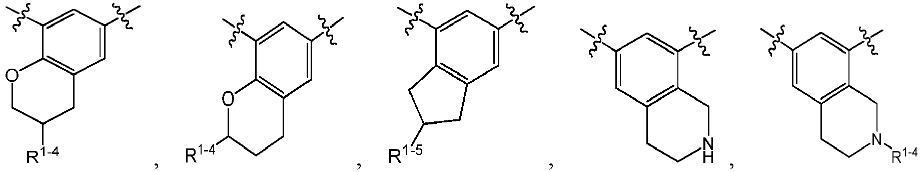 or 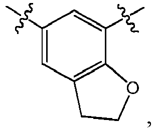 the number of R¹⁻⁴ is 1 or more, when number of of R¹⁻⁴ is multiple, the R¹⁻⁴ are the same or different; the number of R¹⁻⁵ is 1 or 2, and when the number of R¹⁻⁵is 2, the R¹⁻⁵ are the same or different.

8. The compound of formula I according to claim 5, a solvate thereof, a prodrug thereof, a metabolite thereof, or a pharmaceutically acceptable salt thereof, wherein, -A-B- is 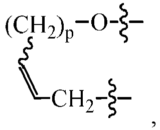 -O-(CH₂)ᵥ-, 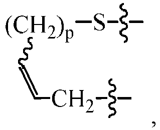 -S-(CH₂)ᵥ-, 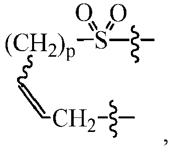 -S(=O)₂-(CH₂)ᵥ-, 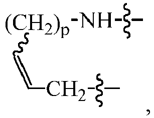 -NH-(CH₂)ᵥ-, 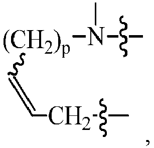 -N(CH₃)-(CH₂)ᵥ-, 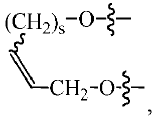 -O-(CH₂)_{w}-O-or -O-CH₂-L⁵-CH₂-O-(e. g. 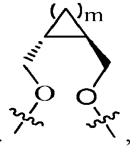 m is 1, 2 or 3);p is 1, 2 or 3; v is 4, 5 or 6; s is 1 or 2; w is 3, 4 or 5; and L⁵ is C₃-C₅cycloalkylene.

9. The compound of formula I according to claim 5, a solvate thereof, a prodrug thereof, a metabolite thereof, or a pharmaceutically acceptable salt thereof, wherein, -A-B- is 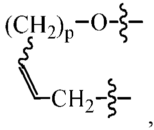 -O-(CH₂)ᵥ-, 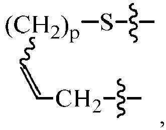 -S-(CH₂)ᵥ-, 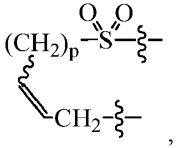 -S(=O)₂-(CH₂)ᵥ-, 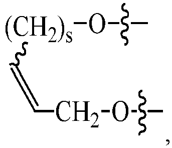 -O-(CH₂)_{w}-O-, 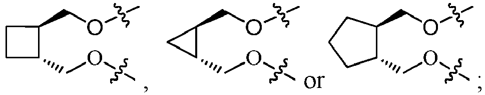 p is 1, 2 or 3; v is 4, 5 or 6; s is 1 or 2; and w is 3 or 4.

10. The compound of formula I according to claim 1, a solvate thereof, a prodrug thereof, a metabolite thereof, or a pharmaceutically acceptable salt thereof, wherein, the compound of Formula I is any of the following compounds:

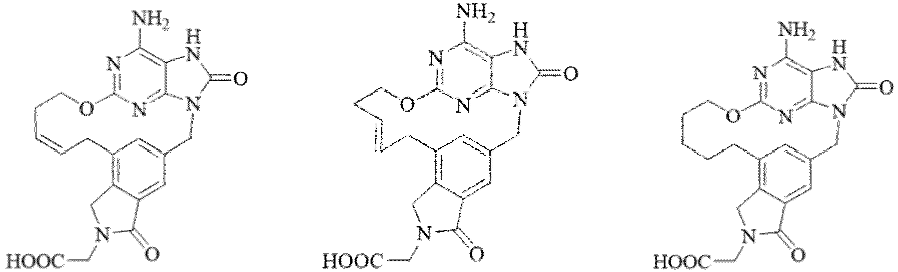

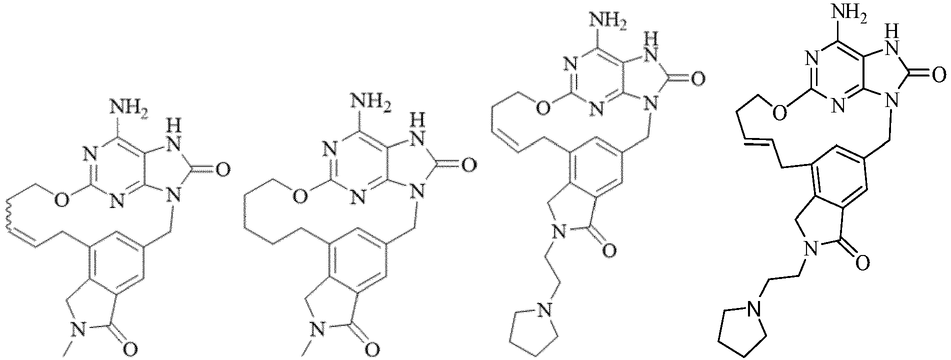

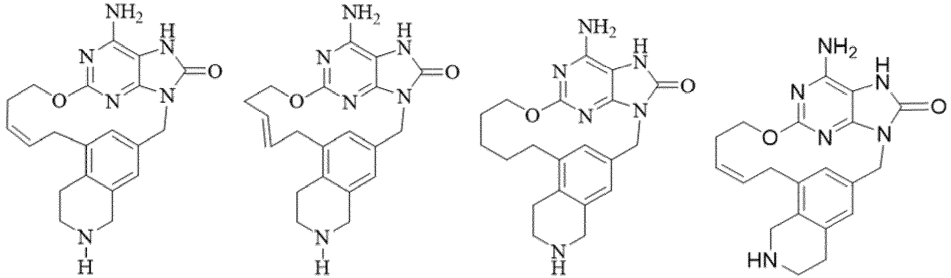

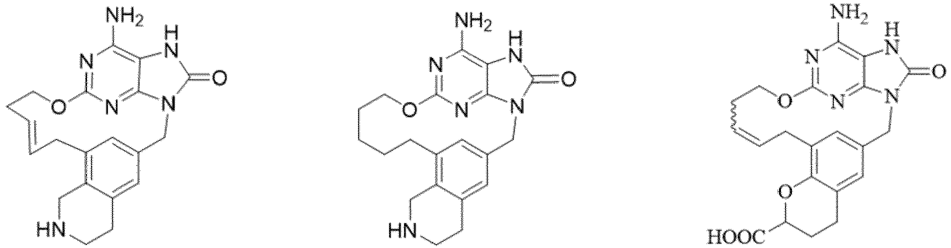

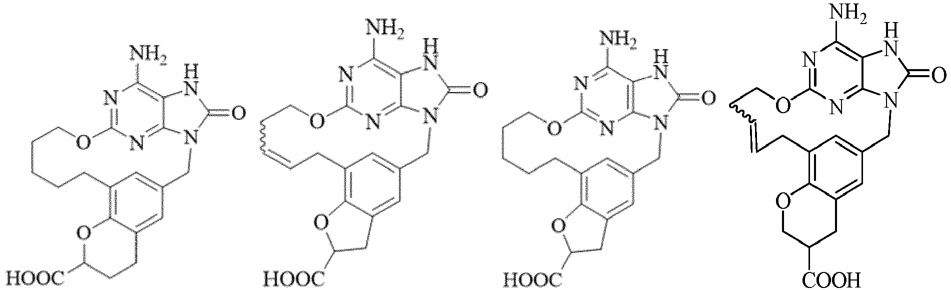

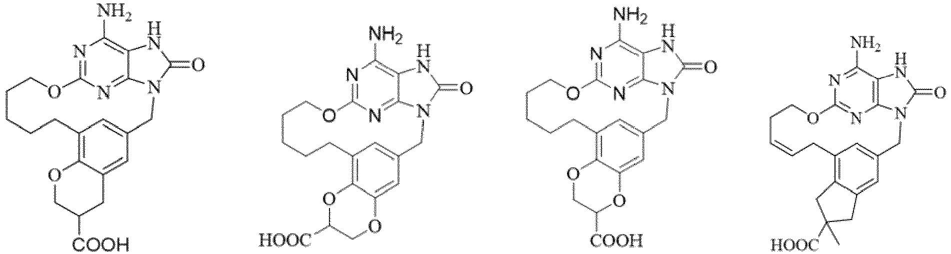

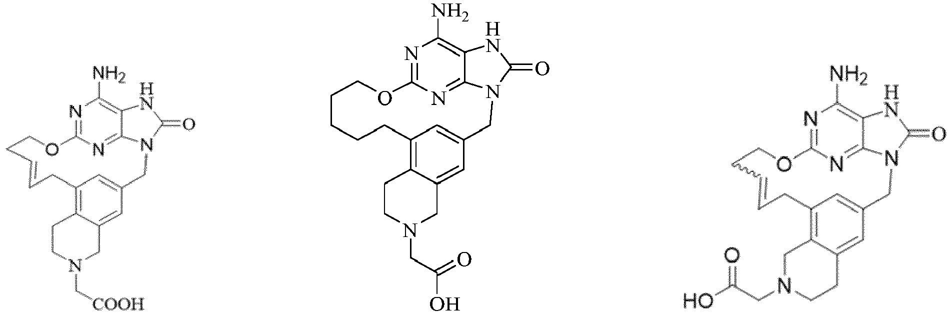

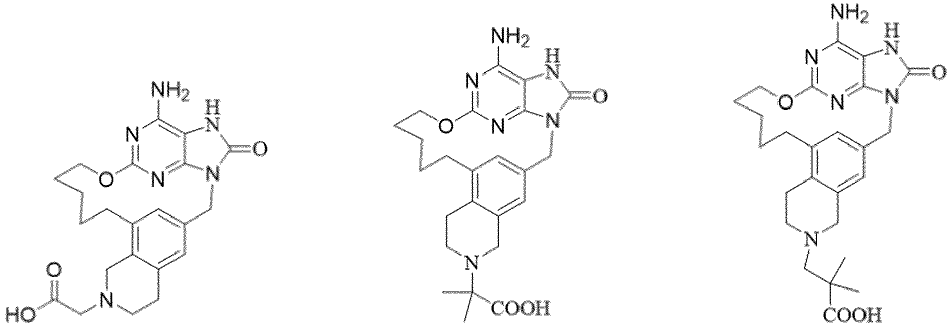

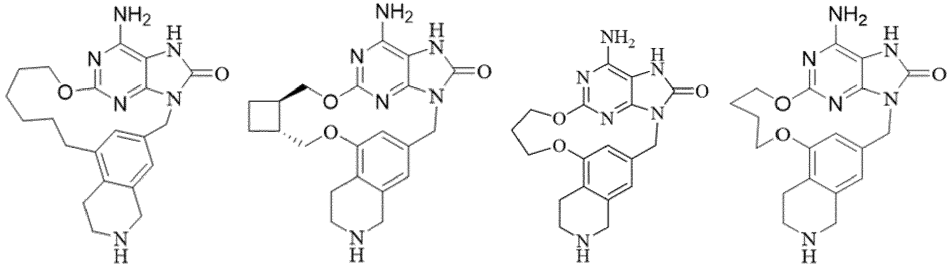

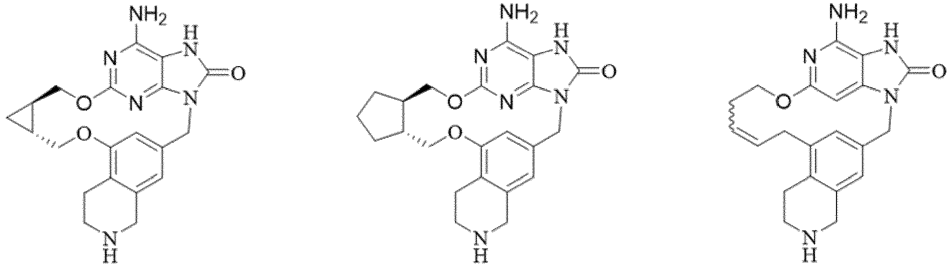

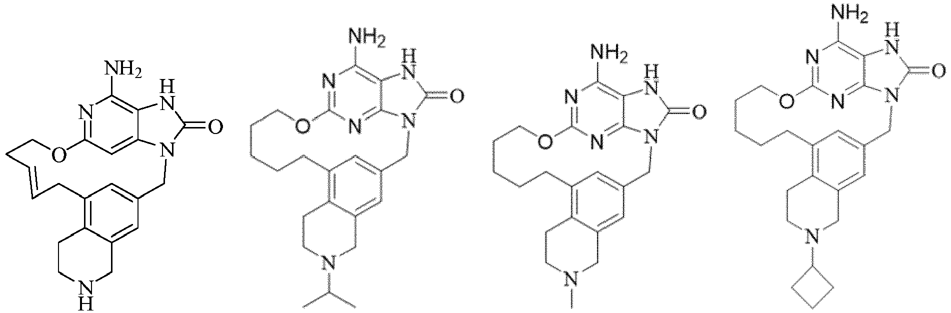

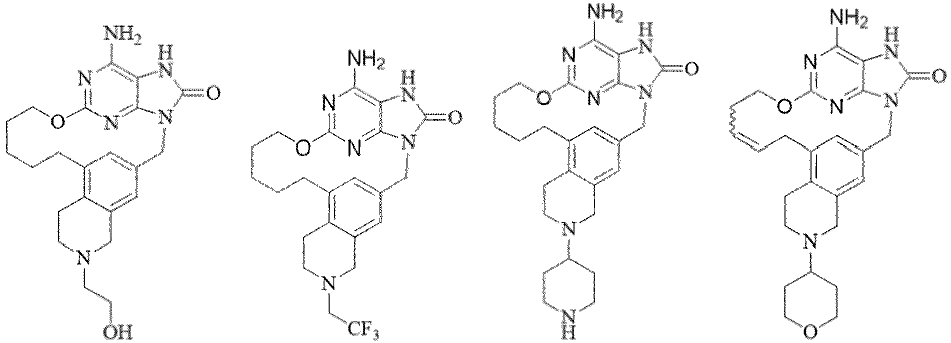

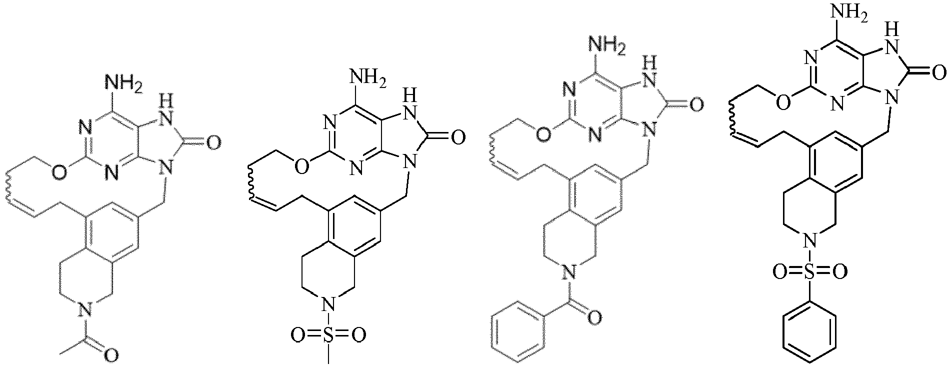

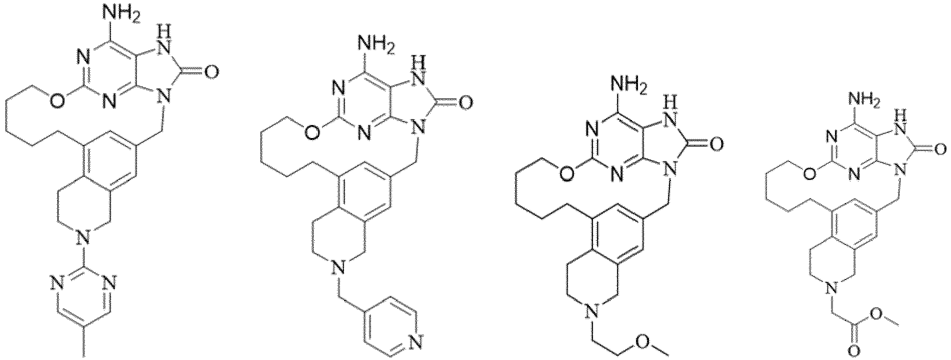

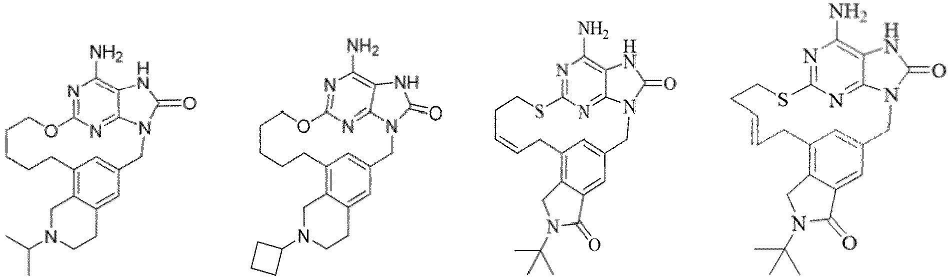

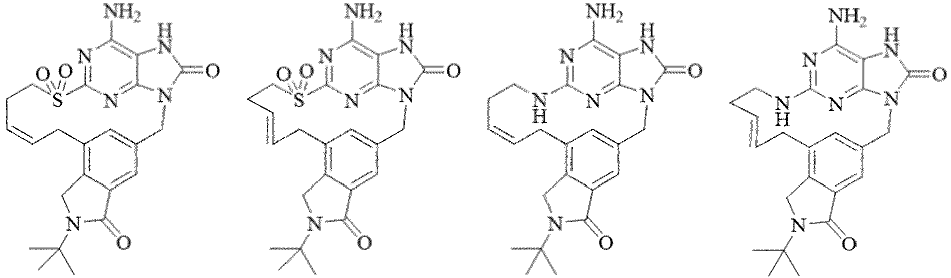

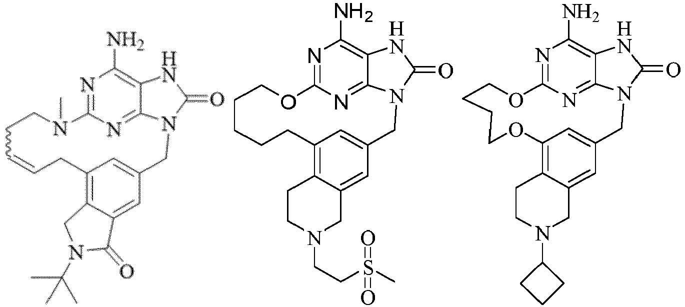

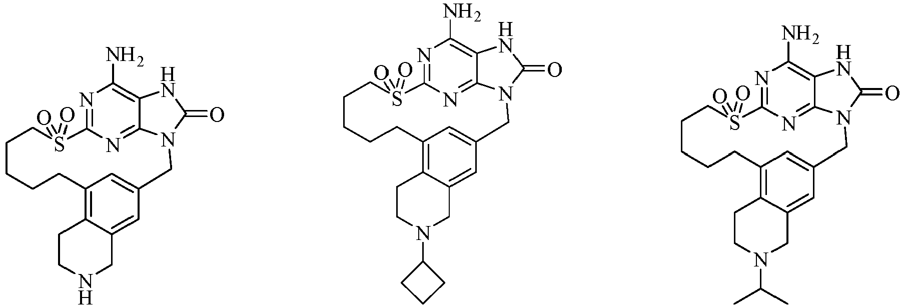

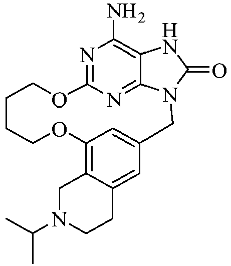

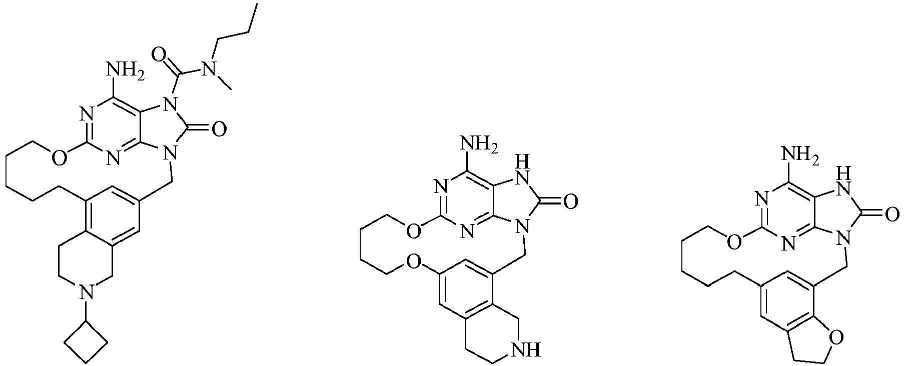

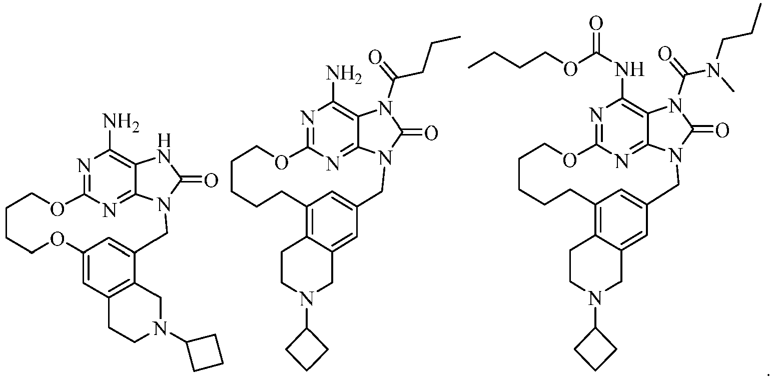

11. A compound of formula II: 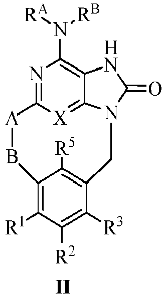 wherein, R^{A} and R^{B} are independently H or an amino protecting group, and R^{A} and R^{B} are not simultaneously H; X, A, B, R¹, R², R³ and R⁵ are as defined in any one of claims 1 to 10; and the amino protecting group is preferably p-methoxybenzyl.

12. The compound of formula II according to claim 11, wherein the compound of formula II is any one of the following compounds:

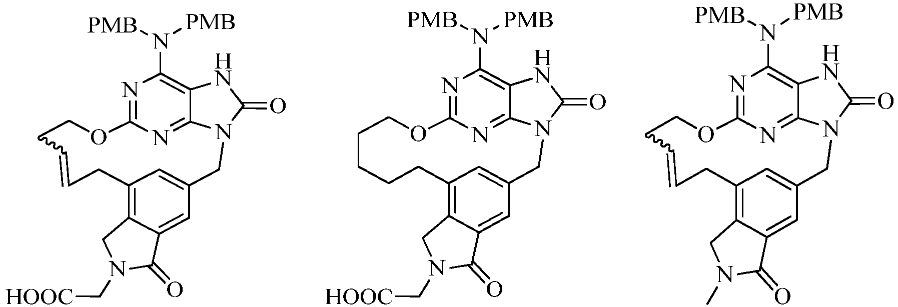

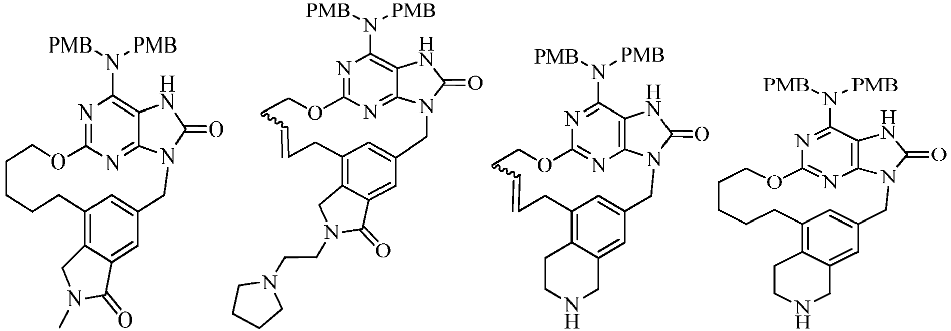

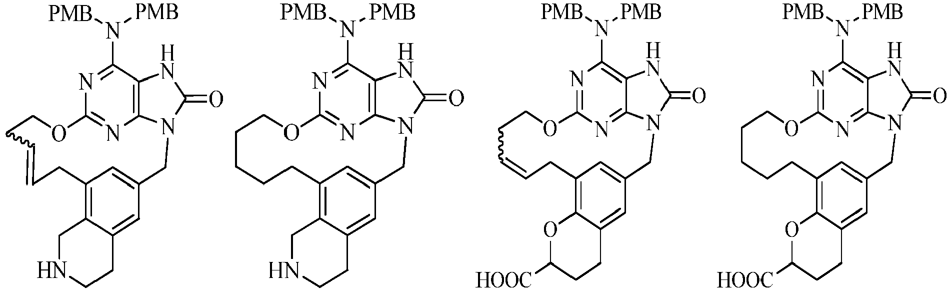

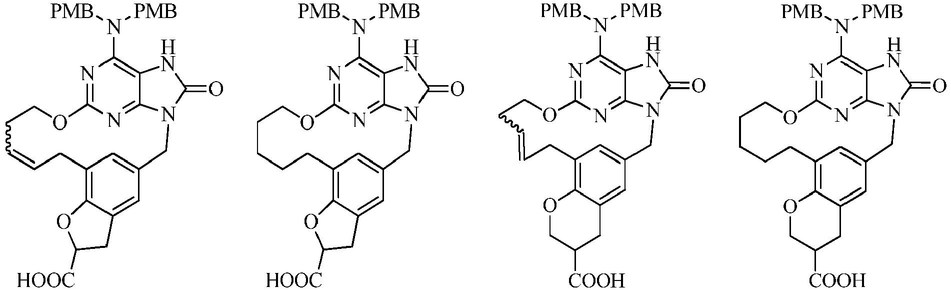

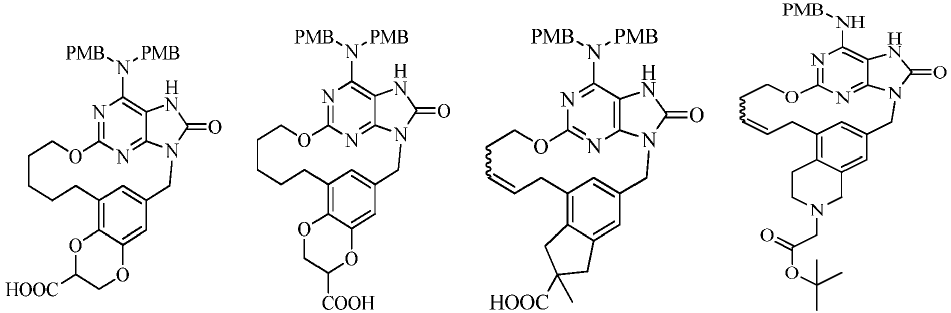

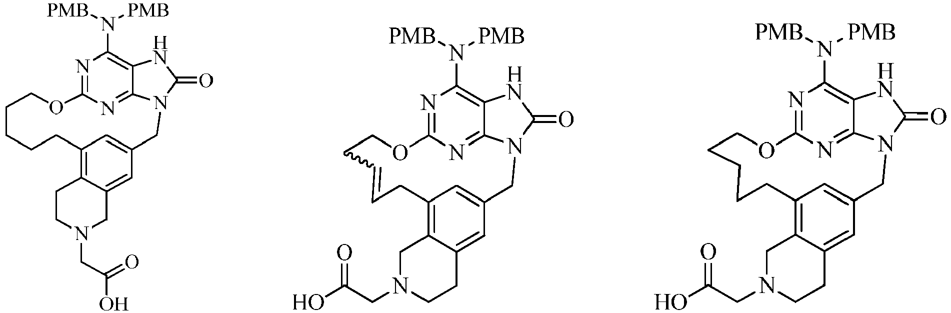

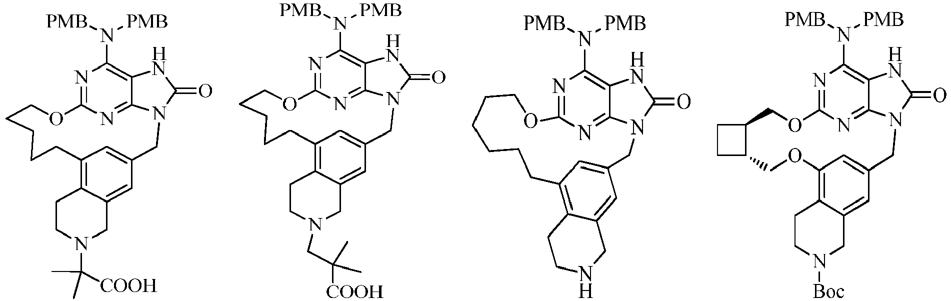

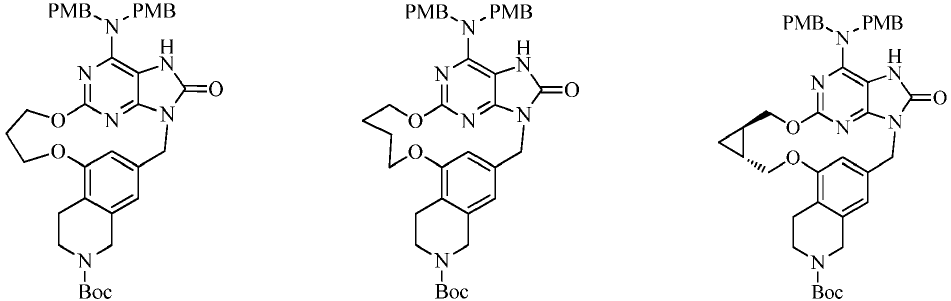

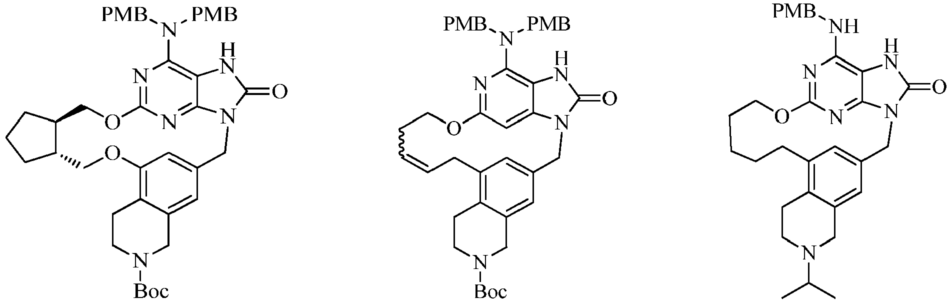

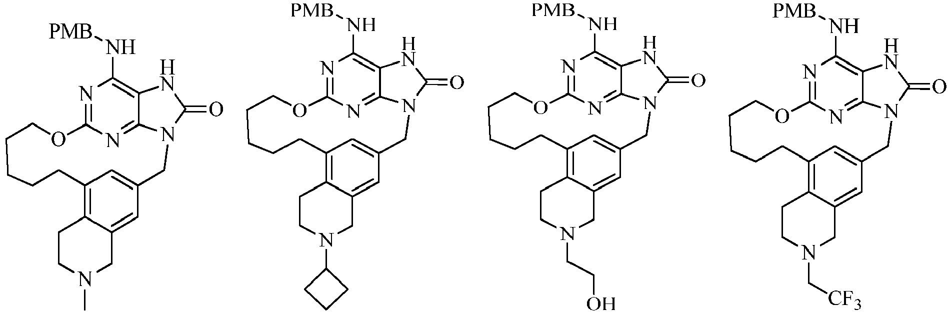

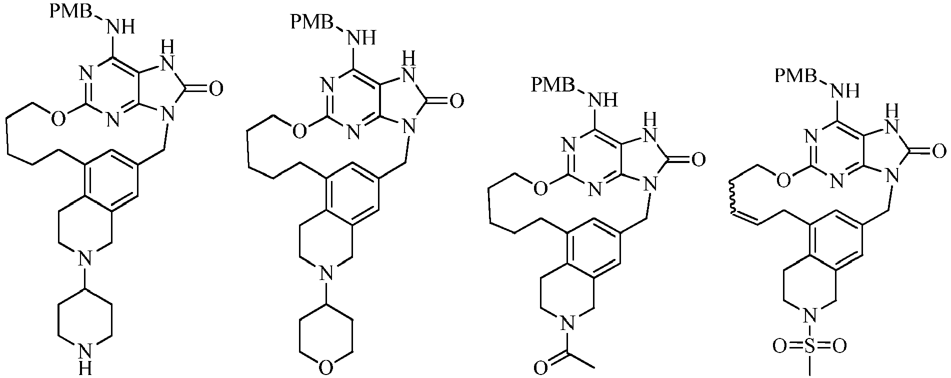

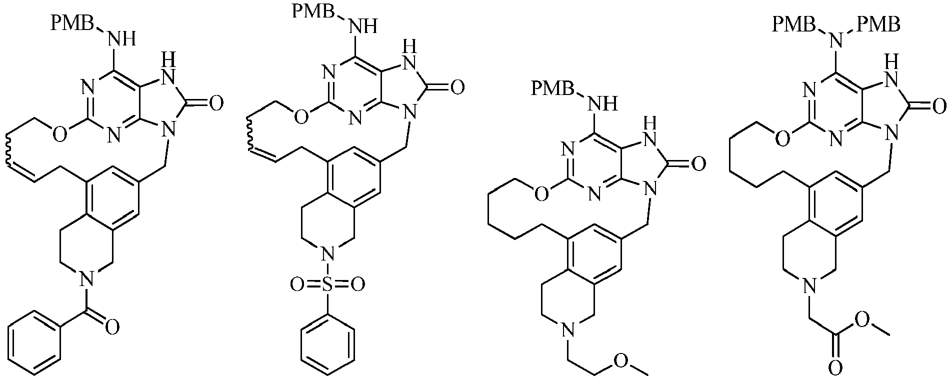

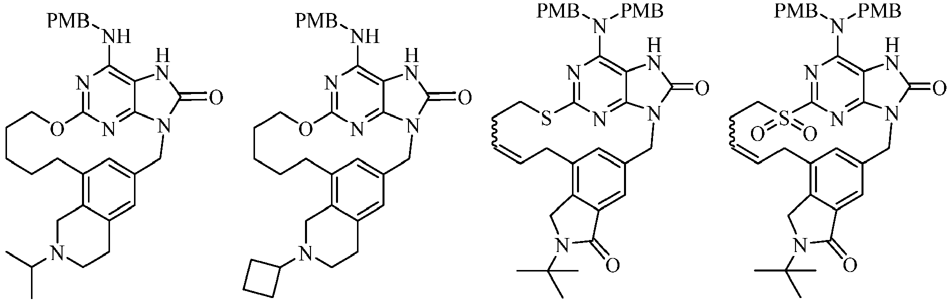

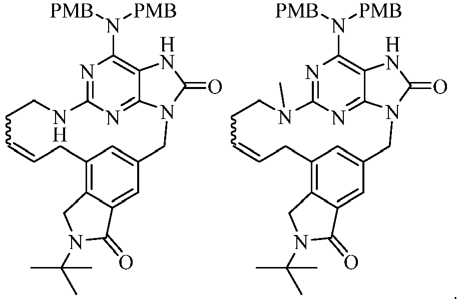

13. A method for preparing the compound of formula I according to any one of claims 1 to 10, comprising the following method I, method II and method III:
the method I comprises the following steps: performing the following deprotection reaction on the compound as shown in formula **II** to obtain the compound as shown in formula **I**; 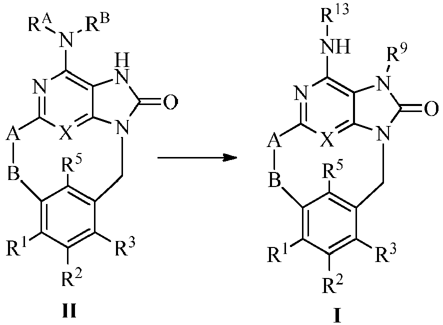
in the method I, R^{A} and R^{B} are defined as described in claim 11 or 12, R⁹ and R¹³ are H, and A, B, R¹, R², R³ and R⁵ are defined as described in any one of claims 1 to10;
the method II comprises the following steps: performing an acylation reaction of the compound of formula **I-A** and the compound of formula **III-A** to obtain the compound of formula **I**; 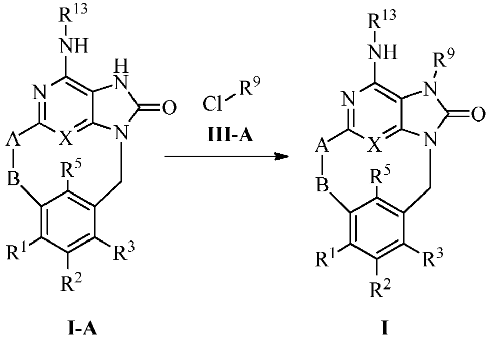
in the method II, R⁹ is CONR¹⁰R¹¹or C(=O)R¹², and R¹⁰, R¹¹, R¹², A, B, R¹, R², R³, R⁵ and R¹³ are defined as described in any one of claims 1 to 10;
the method III comprises the following steps: performing an acylation reaction of the compound of formula **I-B** and the compound of formula **III-B** to obtain the compound of formula **I**; 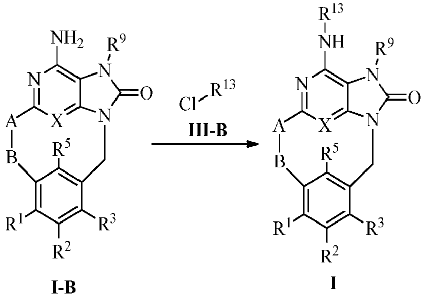
in the method III, R¹³ is CONR¹⁴R¹⁵, C(=O)R¹⁶ or COOR¹⁷, and R¹⁴, R¹⁵, R¹⁶, R¹⁷, A, B, R¹, R², R³, R⁵ and R⁹ are defined as described in any one of claims 1 to 10.

14. A pharmaceutical composition, comprising a compound according to any one of claims 1 to 10, a solvate thereof, a prodrug thereof, a metabolite thereof, or a pharmaceutically acceptable salt thereof, and a pharmaceutical excipient.

15. A use of a compound of formula I according to any one of claims 1 to 10, a solvate thereof, a prodrug thereof, a metabolite thereof, or a pharmaceutically acceptable salt thereof, or the pharmaceutical composition according to claim 14 in the preparation of a medicament.

16. The use of claim 15, wherein the medicament is for treating or preventing of tumors or infections caused by viruses, preferably the viruse is one or more of HBV, HCV, HIV and influenza viruses.

17. A use of the compound of formula I, a solvate thereof, a prodrug thereof, a metabolite thereof, or a pharmaceutically acceptable salt thereof according to any one of claims 1 to 10, or a pharmaceutical composition according to claim 14 in the preparation of a TLR7 agonist.
